# EUROPEAN PATENT APPLICATION

(11) **EP 1 707 205 A2**
(43) Date of publication of application: **04.10.2006**
(21) Application number: 06116683.1
(22) Date of filing: 26.06.2003
(51) Int. Cl.: A61K 31/46, A61K 31/4178, A61P 11/00, A61K 31/517, A61K 31/416, A61K 31/5377, A61K 31/435

(54) **Pharmaceutical compositions of anticholinergics and P38 kinase inhibitors in the treatment of respiratory diseases**

(30) Priority: 09.07.2002 EP 02015231
(62) Divisional of application: 03738089.6
(71) Applicant: Boehringer Ingelheim Pharma GmbH & Co. KG, 55216 Ingelheim am Rhein (DE)
(72) Inventor: Maede, Christopher John Montague, Am Strudel 15, 88437, Maselheim (DE); Pairet, Michel, Obere Str. 17 88400 Biberach (DE); Pieper, Michael P., Geschwister-Scholl.Str.45 88400 Biberach (DE)

(57) **Abstract**

The present invention relates to novel pharmaceutical compositions based on novel anticholinergics and p38 kinase inhibitors, processes for preparing them and their use in the treatment of respiratory diseases.

## Description

The present invention relates to novel pharmaceutical compositions based on novel anticholinergics and p38 kinase inhibitors, processes for preparing them and their use in the treatment of respiratory diseases.

### Description of the invention

The present invention relates to novel pharmaceutical compositions based on novel anticholinergics and p38 kinase inhibitors, processes for preparing them and their use in the treatment of respiratory diseases.

Surprisingly, it has been found that an unexpectedly beneficial therapeutic effect, particularly a synergistic effect can be observed in the treatment of diseases of the upper or lower respiratory tract, particularly in the treatment of allergic or non-allergic rhinitis, if one or more, preferably one anticholinergic of general formula **A** is or are used together with one or more, preferably one, p38 kinase inhibitor **B**. Thanks to this synergistic effect the pharmaceutical combinations according to the invention can be used in lower doses than is the case when the individual compounds are used in monotherapy in the usual way.

The effects mentioned above are observed both when the two active substances are administered simultaneously in a single active substance formulation and when they are administered successively in separate formulations. According to the invention, it is preferable if the two active substance ingredients are administered simultaneously in a single formulation.

Within the scope of the present invention the term anticholinergics **A** denotes compounds of formula 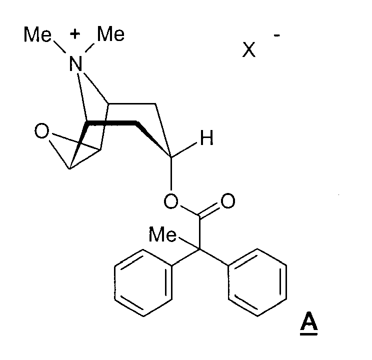 wherein
- X⁻: denotes an anion (counter-ion), preferably an anion selected from the group consisting of chloride, bromide, iodide, sulphate, phosphate, methansulphonate, nitrate, maleate, acetate, citrate, fumarate, tartrate, oxalate, succinate, benzoate and p-toluenesulphonate.

Preferably, those salts **A** are applied, wherein
- X⁻: denotes an anion (counter-ion), preferably an anion selected from the group consisting of chloride, bromide, methansulphonate and p-toluenesulphonate, preferably bromide.

More preferably, those salts **A** are applied, wherein
- X⁻: denotes an anion (counter-ion), preferably an anion selected from the group consisting of chloride, bromide and methansulphonate, preferably bromide.

Of particular importance is the anticholinergic of formula **A** wherein X - denotes bromide.

The salts of formula **A** are known from the international patent application WO02/32899.

Within the scope of the present patent application, any reference to the cation of formula 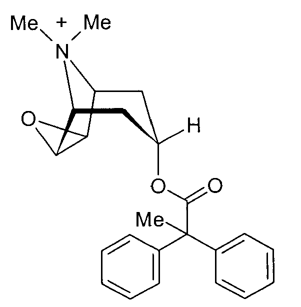 is indicated by use of the number **A'**. Any reference to compounds **A**naturally also includes a reference to the cation **A'**.

Any reference to compounds **A** within the scope of the present patent application naturally also includes a reference to the salts and/or solvates thereof.
p38 kinase inhibitors applicable within the scope of the invention are known in the art. Within the scope of the present invention the term p38 kinase inhibitors (hereinafter **B**) denotes compounds selected from the compounds that are disclosed for instance in US Patents 5,716,972, US 5,686,455, US 5,656,644, US 5,593,992, US 5,593,991, US 5,663,334, US 5,670,527, US 5,559,137, 5,658,903, US 5,739,143, US 5,756,499, US 6,277,989, US 6,340,685, and US 5,716,955 and PCT applications WO 92/12154, WO 94/19350, WO 95/09853, WO 95/09851, WO 95/09847, WO 95/09852, WO 97/25048, WO 97/25047, WO 97/33883, WO 97/35856, WO 97/35855, WO 97/36587, WO 97/47618, WO 97/16442, WO 97/16441, WO 97/12876, WO 98/25619, WO 98/06715, WO 98/07425, WO 98/28292, WO 98/56377, WO 98/07966, WO 98/56377, WO 98/22109, WO 98/24782, WO 98/24780, WO 98/22457, WO 98/52558, WO 98/52559, WO 98/52941, WO 98/52937, WO 98/52940, WO 98/56788, WO 98/27098, WO 98/47892, WO 98/47899, WO 98/50356, WO 98/32733, WO 99/58523, WO 99/01452, WO 99/01131, WO 99/01130, WO 99/01136, WO 99/17776, WO 99/32121, WO 99/58502, WO 99/58523, WO 99/57101, WO 99/61426, WO 99/59960, WO 99/59959, WO 99/00357, WO 99/03837, WO 99/01441, WO 99/01449, WO 99/03484, WO 99/15164, WO 99/32110, WO 99/32111, WO 99/32463, WO 99/64400, WO 99/43680, WO 99/17204, WO 99/25717, WO 99/50238, WO 99/61437, WO 99/61440, WO 00/26209, WO 00/18738, WO 00/17175, WO 00/20402, WO 00/01688, WO 00/07980, WO 00/07991, WO 00/06563, WO 00/12074, WO 00/12497, WO 00/31072, WO 00/31063, WO 00/23072, WO 00/31065, WO 00/35911, WO 00/39116, WO 00/43384, WO 00/41698, WO 00/69848, WO 00/26209, WO 00/63204, WO 00/07985, WO 00/59904, WO 00/71535, WO 00/10563, WO 00/25791, WO 00/55152, WO 00/55139, WO 00/17204, WO 00/36096, WO 00/55120, WO 00/55153, WO 00/56738, WO 01/21591, WO 01/29041, WO 01/29042, WO 01/62731, WO 01/05744, WO 01/05745, WO 01/05746, WO 01/05749, WO 01/05751, WO 01/27315, WO 01/42189, WO 01/00208, WO 01/42241, WO 01/34605, WO 01/47897, WO 01/64676, WO 01/37837, WO 01/38312, WO 01/38313, WO 01/36403, WO 01/38314, WO 01/47921, WO 01/27089, DE 19842833, and JP 2000 86657 whose disclosures are all incorporated herein by reference in their entirety.

Of particular interest for the pharmaceutical compositions according to the invention are those p38 inhibitors **B** disclosed in US 6,277,989, US 6,340,685, WO 00/12074, WO 00/12497, WO 00/59904, WO 00/71535, WO 01/64676, WO 99/61426, WO 00/10563, WO 00/25791, WO 01/37837, WO 01/38312, WO 01/38313, WO 01/38314, WO 01/47921, WO 99/61437, WO 99/61440, WO 00/17175, WO 00/17204, WO 00/36096, WO 98/27098, WO 99/00357, WO 99/58502, WO 4 99/64400, WO 99/01131, WO 00/43384, WO 00/55152, WO 00/55139, and WO 01/36403.

In a preferred embodiment the invention relates to pharmaceutical compositions containing **A** and **B,** characterized in that the p38 kinase inhibitor **B** is selected from the compounds of formula **1** as disclosed in WO 99/01131 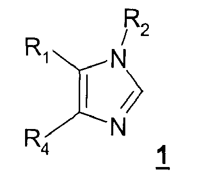 wherein
- R₁: is 4-pyridyl, pyrimidinyl, 4-pyridazinyl, 1,2,4-triazin-5-yl, quinolyl, isoquinolinyl, or quinazolin-4-yl ring, which ring is substituted with Y-Rₐ and optionally with an additional independent substituent selected from C₁₋₄ alkyl, halogen, hydroxyl, C₁₋₄ alkoxy, C₁₋₄ akylthio, C₁₋₄ aklylsulfinyl, CH₂OR₁₂, amino, mono and di- C₁₋₆ alkyl substituted amino, an N-heterocyclyl ring which ring has from 5 to 7 members and optionally contains an additional heteroatom selected from oxygen, sulfur or NR₁₅, N(R₁₀)C(O)R_{b} or NHRₐ;
- Y: is oxygen or sulfur;
- R₄: is phenyl, naphth-1-yl or naphth―yl, or a heteroaryl, which is optionally substituted by one or two substituents, each of which is independently selected, and which, for a 4-phenyl, 4naphth-1-yl, 5-naphth-2-yl or 6-naphth-2-yl substituent, is halogen, cyano, nitro, C(Z)NR₇R₁₇, C(Z)OR₁₆, (CR₁₀R₂₀)ᵥCOR₁₂, SR₅, SOR₅, OR₁₂, halo-substituted-C₁₋₄ alkyl, C₁₋₄ alkyl, ZC(Z)R₁₂, NR₁₀C(Z)R₁₆, or (CR₁₀R₂₀)ᵥNR₁₀R₂₀ and which, for other positions of substitution, is halogen, cyano, C(Z)NR₁₃R₁₄, C(Z)OR₃, (CR₁₀R₂₀)_{m"}COR₃, S(O)ₘR₃, OR₃, halo-substituted-C₁₋₄ alkyl, C₁₋₄ alkyl, (CR₁₀R₂₀)_{m"}R₁₀C(Z)R₃, NR₁₀S(O)_{m'}R₈, NR₁₀S(O)_{m'}NR₇R₁₇, ZC(Z)R₃ or (CR₁₀R₂₀)_{m"}NR₁₃R₁₄;
- Z: is oxygen or sulfur;
- n: is an integer having a value of 1 to 10;
- m: is 0, or integer 1 or 2;
- m': is an integer having a value of 1 or 2;
- m": is 0, or an integer having a value of 1 to 5;
- v: is 0, or an integer having a value of 1 to 2;
- R₂: is -C(H) (A) (R₂₂);
- A: is optionally substituted aryl, heterocyclyl, or heteroaryl ring, or A is substituted C₁₋₁₀ alkyl;
- R₂₂: is an optionally substituted C₁₋₁₀ alkyl;
- Rₐ: is aryl, arylC₁₋₆ alkyl, heterocyclic, heterocyclylC₁₋₆ alkyl, heteroaryl, heteroarylC₁₋₆alkyl, wherein each of these moieties may be optionally substituted;
- R_{b}: is hydrogen, C₁₋₆ alkyl, C₃₋₇ cycloalkyl, aryl, aryl C₁₋₄ alkyl, heteroaryl, heteroarylC₁₋₄ alkyl, heterocyclyl, or heterocyclylC₁₋₄ alkyl, wherein each of these moieties may be optionally substituted;
- R₃: is heterocyclyl, heterocyclyl C₁₋₁₀ alkyl or R₈;
- R₅: is hydrogen, C₁₋₄ alkyl, C₂₋₄ alkenyl, C₂₋₄ alkynyl or NR₇R₁₇, excluding the moieties SR₅ being SNR₇R₁₇and SOR₅ being SOH;
- R₆: is hydrogen, a pharmaceutically acceptable cation, C₁₋₁₀ alkyl, C₃₋₇ cycloalkyl, aryl, aryl C₁₋₄ alkyl, heteroaryl, heteroaryl C₁₋₄ alkyl, heterocyclyl, aryl, or C₁₋₁₀ alkanoyl;
- R₇ and R₁₇: is each independently selected from hydrogen or C₁₋₄ alkyl or R₇ and R₁₇ together with the nitrogen to which they are attached form a heterocyclic ring of 5 to 7 members which ring optionally contains an additional heteroatom selected from oxygen, sulfur or NR₁₅;
- R₈: is C₁₋₁₀ alkyl, halo-substituted C₁₋₁₀ alkyl, C₂₋₁₀ alkenyl, C₂₋₁₀ alkynyl, C₃₋₇ cycloalkyl, C₅₋₇ cycloalkenyl, aryl, aryl C₁₋₁₀ alkyl, heteroaryl, heteroaryl C₁₋₁₀ alkyl, (CR₁₀R₂₀)ₙOR₁₁, (CR₁₀R₂₀)ₙS(O)ₘR₁₈, (CR₁₀R₂₀)ₙNHS(O)₂R₁₈, (CR₁₀R₂₀)ₙNR₁₃R₁₄; wherein the aryl, arylalkyl, heteroaryl, heteroaryl alkyl may be optionally substituted;
- R₉: is hydrogen, C(Z) R₁₁ or optionally substituted C₁₋₁₀ alkyl, S(O)₂R₁₈, optionally substituted aryl or optionally substituted aryl C₁₋₄ alkyl;
- R₁₀ and R₂₀: is each independently selected from hydrogen or C₁₋₄ alkyl;
- R₁₁: is hydrogen, C₁₋₁₀ alkyl, C₃₋₇ cycloalkyl, heterocyclyl, heterocyclyl C₁₋₁₀ alkyl, aryl, arylC₁₋₁₀ alkyl, heteroaryl or heteroaryl C₁₋₁₀ alkyl, wherein these moieties may be optionally substituted;
- R₁₂: is hydrogen or R_{16;}
- R₁₃ an R₁₄: is each independently selected from hydrogen or optionally substituted C₁₋₄ alkyl, optionally substituted aryl or optionally substituted arylC₁₋₄ alkyl, or together with the nitrogen which they are attached form a heterocyclic ring of 5 to 7 members which ring optionally contains an additional heteroatom selected from oxygen, sulfur or NR₉;
- R₁₅: is R₁₀ or C(Z)-C₁₋₄ alkyl;
- R₁₆: is C₁₋₄ alkyl, halo-substituted-C₁₋₄ alkyl, or C₃₋₇ cycloalkyl;
- R₁₈: is C₁₋₁₀ alkyl, C₃₋₇ cycloalkyl, heterocyclyl, aryl, aryl₁₋₁₀ alkyl, heterocyclyl, heterocyclyl- C₁₋₁₀alkyl, heteroaryl or heteroaryl₁₋₁₀ alkyl;
or a pharmaceutically acceptable salt thereof.

In the aforementioned compounds of formula **1** R₂ is a substituted alkyl derivative. It is recognised that the first methylene carbon in this chain is a tertiary carbon, and it will contain one hydrogen moiety. This ethylene group has two additional substituents, an R₂₂ moiety and an A moiety, -C(H)(A)(R₂₂). Both A and R₂₂ may not be unsubstituted C₁₋₁₀ alkyl moiety.

In a preferred embodiment, R₂ is a ―C(AA₁)(A) moiety, wherein AA₁ is the R₂₂ moiety, but is specifically the side chain residue (R) of an amino acid, as is further described herein.

Suitably, A is an optionally substituted C₁₃₋₇ cycloalkyl, aryl, heteroaryl, or heterocyclic ring, or A is a substituted C₁₋₁₀ alkyl moiety.

When A is an aryl, heteroaryl and heterocyclic ring, the ring may be substituted independently one or more times, preferably, 1 to 3 times by C₁₋₁₀ alkyl; halogen; halo substituted C₁₋₁₀ alkyl such as CF₃; (CR₁₀R₂₀)ₜOR₁₁; (CR₁₀R₂₀)ₜNR₁₂R₁₄, especially amino or mono-or di-C₁₋₄ alkylamino; (CR₁₀R₂₀)ₜS(O)m R₁₈, wherein m is 0, 1 or 2; SH; NR₁₀C(Z)R₃ (such NHCO(C₁₋₁₀ alkyl)); or NR₁₀S(O)m R₈ (such as NHSO₂(C₁₋₁₀ alkyl)).

Suitably, t is 0, or an integer of 1 to 4.

When A is an optionally substituted cycloalkyl it is as defined below with the R₂₂ substitution.

When A is an optionally substituted heterocyclil ring, the ring is preferably a morpholino, pyrrolidinyl, piperazinyl or a piperidinyl ring.

When A is an optionally substituted aryl moiety, it is preferably a phenyl ring.

When A is an optionally substituted heteroaryl ring, it is as defined below in the definition section.

When A is a substituted C₁₋₁₀ alkyl moiety, the alkyl chain may be straight or branched. The chain is substituted independently 1 or more times, preferably 1 to 3 times by halogen, such as fluorine, chlorine, bromine or iodine; halosubstituted C₁₋₁₀ alkyl, such as CF₃; C₃₋₇ cycloaklyl, C₁₋₁₀ alkloxy, such as methoxy or ethoxy; hydroxy substituted C₁₋₁₀ alkoxy; halosubstituted C₁₋₁₀ alkoxy, such as OCF₂CF₂H; OR₁₁; S(O)ₘR₁₈ (wherein m is 0, 1 or 2); NR₁₃R₁₄; C(Z)NR₁₃R₁₄; S(O)_{m'}NR₁₃R₁₄; NR₂₃C(Z)R₁₁; NHS(O)₂R₁₈; C(Z)R₁₁; OC(Z)R₁₁; C(Z)OR₁₁; C(Z)NR₁₁OR₉; N(OR₆)C(Z)NR₁₃R₁₄; N(OR₆)C(Z)R₁₁; C(=NOR₆)R₁₁; NR₂₃C(=NR₁₉)NR₁₃R₁₄; OC(Z)NR₁₃R₁₄; NR₂₃C(Z)NR₁₃R₁₄; or NR₂₃C(Z)OR₁₀.

Preferably A is a C₃₋₇ cycloalkyl, or a C₁₋₆ alkyl, more preferably a C₁₋₂ alkyl, i.e. a methylene or ethylene moiety, more preferably a methylene moiety which is substituted by one of the above noted groups.

Preferably, when A is a C₁₋₁₀alkyl, it is substituted by OR₁₁ where R₁₁ is preferably hydrogen, aryl or arylalkyl; NR₁₃R₁₄; OC(Z)R₁₁; C(Z)OR₁₁.

More preferably, A is substituted by OR₁₁ where R₁₁ is hydrogen.

Suitably, R₂₂ is a C₁₋₁₀ alkyl chain, which chain may be straight or branched and which may be optionally substituted independently, one or more times, preferably 1 to 3 times, by halogen, such as fluorine, chlorine or iodine; halo substituted C₁₋₁₀ alkyl; C₁₋₁₀ alkoxy, such as methoxy or ethoxy; hydroxy substituted C₁₋₁₀ alkoxy; halosubstituted C₁₋₁₀ alkoxy, such as OCF₂CF₂H; OR₁₁; S(O)ₘR₁₈; NR₁₃R₁₄; C(Z)NR₁₃R₁₄; S(O)ₘ,NR₁₃R₁₄; NR₂₃C(Z)R₁₁; NHS(O)₂R₁₈; C(Z)R₁₁; OC(Z)OR₁₁; C(Z)OR₁₁; C(Z)NR₁₁OR₉; N(OR₆)C(Z)NR₁₃R₁₄; N(OR₆)C(Z)R₁₁; C(=NOR₆)R₁₁; NR₂₃C(=NR₁₉)NR₁₃R₁₄; OC(Z)NR₁₃R₁₄; NR₂₃C(Z)NR₁₃R₁₄; NR₂₃C(Z)OR₁₀; optionally substituted C₃₋₇ cycloalkyl; optionally substituted aryl, such as phenyl; optionally substituted heteroaryl; or an optionally substituted heterocyclic. The optional substituents on these cycloalkyl, aryl, heteroaryl, and heterocyclic moieties are as defined herein below.

It is noted that those R₂₂ substituent groups which contain carbon as the first connecting group, i.e. C(Z)OR₁₁; C(Z)NR₁₁OR₉, C(Z)R₁₁, C(Z)NR₁₃R₁₄, and C(=NOR₆)R₁₁, may be the sole carbon in alkyl chain. Therefore, the R₂₂ group may, for instance, be a carboxy, an aldehyde, or an amide, as well as being a substituent of a methylene unit, such as carbamoylmethyl, or acetamidomethyl.

Preferably R₂₂ is a C₁₋₆ unsubstituted or substituted alkyl group, such as a C₁₋₃ alkylene such as methyl, ethyl or isopropyl, or a methylene or ethylene moiety substituted by one of the above noted moieties, or as noted above those substituent groups which contain a carbon may substituent for the first methylene unit of the alkyl chain, such as carboxy, C(O)OR₁₁; C(O)NR₁₃R₁₄ or R₂₂ is an optionally substituted aryl group, such as a benzyl or phenethyl. In other words, R₂₂ can be an optionally substituted alkyl group, or R₂₂ can be C(Z)OR₁₁; C(Z)NR₁₁OR₉, C(Z)R₁₁, C(Z)NR₁₃R₁₄, or C(=NOR₆)R₁₁.

Preferably R₂₂ is C₁₋₆ unsubstituted or substituted alkyl group, more preferably a C₁₋₂ alkylene chain, such as a methylene or ethylene moiety, more preferably methylene.

Preferably the alkyl chain is substituted by OR₁₁, where R₁₁ is preferably hydrogen, aryl or arylalkyl; S(O)ₘR₁₈, where m is 0 and R₁₈ is a C₁₋₆ alkyl; or an optionally substituted aryl, i.e. a benzyl or phenethyl moiety.

More preferably, R₂₂ is phenyl, benzyl, CH₂OH, or CH₂-O-aryl.

Preferably, one or both of A and R₂₂ contain hydroxy moieties, such as in C₁₋₆ alkyl OR₁₁, wherein R₁₁ is hydrogen, i.e. CH₂CH₂OH.

Suitably, when AA₁ is the (R) side chain residue of an amino acid, it is a C₁₋₆ alkyl group, which may be straight or branched. This means the R group of the core amino acid of the structure R-C(H)(COOH)(NH₂). The R residue term is for example, CH₃ for alanine, (CH₃)₂CH- for valine, (CH₃)₂CH-CH₂-for leucine, phenyl-CH₂- for phenylalanine, CH₃-S-CH₂-CH₂- for methionine, etc. All generally recognised primary amino acids are included in this groups, such as but not limited to, alanine, arginine, asparagine, aspartic acid, cysteine, glutamine, glutamic acid, glycine, histidine, isoleucine, leucine, lysine, methionine, phenylalanine, serine, threonine, tryptophan, tyrosine, valine, hydroxylysine, methylhistidine, and other naturally accurring amino acids not found in proteins, such as β-alanine, γ-aminobutyric acid, homocysteine, homoserine, citrulline, ornithine, canavanine, djenkolic acid, and β-cyanoalanine, or other naturally occurring non-mammalian amino acids.

Preferably AA₁ is the residue of phenylalanine, or alanine.
Preferably A is a hydroxy substituted C₁₋₁₀ alkyl and R₂₂ is a C₁₋₁₀ alkyl or a hydroxy substituted C₁₋₁₀ alkyl.

In a further preferred embodiment the invention relates to pharmaceutical compositions containing **A** and **B,** characterized in that the p38 kinase inhibitor **B** is selected from the following compounds disclosed in WO 99/01131:
1-(1,3-Dihydroxyprop-2-yl)-(4-fluorophenyl)-5-(2-phenoxypyrimidin-4-yl)imidazole; *trans*-1-(4-Hydroxycyclohexyl)-4-(4-fluorophenyl)5-[(2-methoxy)pyrimidin-4-yl]imidazole;
1-(4-Piperidinyl)-4-(4-fluorophenyl)-5-(2-methoxy-4-pyrimidinyl)imidazole;
(4-Fluorophenyl)-2-(4-methylsulfinylphenyl)-5-(4-pyridyl)-imidazole;

In yet another preferred embodiment the invention relates to pharmaceutical compositions containing **A** and **B**, characterized in that the p38 kinase inhibitor **B** is selected from the compounds of formula **2** as disclosed in US 6,277,989 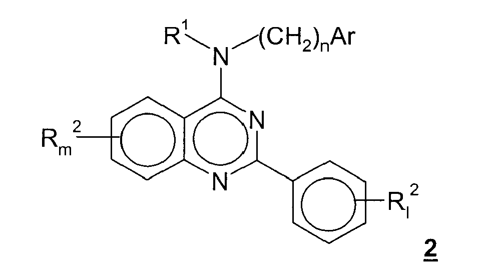 and the pharmaceutically acceptable salts thereof,
wherein
- R¹: is H, alkyl(1-6C) or arylalkyl optionally substituted on the aryl group with 1-3 substituents independently selected from alkyl (1-6C), halo, OR, NR₂, SR, -OOCR, -NROCR, RCO, -COOR, -CONR₂, -SO₂NR₂, CN, CF₃, and NO₂, wherein each R is independently H or lower alkyl (1-4C);
each R² is independently alkyl (1-6C), halo, OR, SR, OOCR, NROCR, COOR, RCO, CONR₂, SO₂NR₂, CN, CF₃ or NO₂, wherein each R is independently H or lower alkyl (1-4C);
each of l, m, and n is independently 0, 1 or 2; and
- Ar: is phenyl, 2-, 3- or 4-pyridyl, indolyl, 2- or 4-pyrimidyl, or benzimidazolyl, each optionally substituted with optionally substituted alkyl, alkenyl, alkynyl, aryl, N-aryl, NH-aroyl, halo, OR, NR₂, SR, -OOCR, -NROCR, RCO, -COOR,-CONR₂, SO₂NR₂, CN, CF₃, or NO₂, wherein each R is independently H or alkyl(1-4C);

Preferably the invention relates to pharmaceutical compositions containing **A** and **B**, characterized in that the p38 kinase inhibitor **B** is selected from the compounds of formula **2** as disclosed in US 6,277,989 , wherein
- R¹: is H;
- R²: is halo, m is 0, 1, or 2, and I is 1 or 2;
- Ar: is 4-pyridyl.

In a particularily preferred embodiment the invention relates to pharmaceutical compositions containing **A** and **B,** characterized in that the p38 kinase inhibitor **B** is selected from the follwoing compounds disclosed US 6,277,989:
2-phenyl-4-(4-pyridylamino)-quinazoline;
2-(2-bromophenyl)-4-(4-pyridylamino)-quinazoline;
2-(2-chlorophenyl)-4-(4-pyridylamino)-quinazoline;
2-(2-fluorophenyl)-4-(4-pyridylamino)-quinazoline;
2-(2-methylphenyl)-4-(4-pyridylamino)-quinazoline;
2-(4-fluorophenyl)-4-(4-pyridylamino)-quinazoline;
2-(3-methoxyanilyl)-4-(4-pyridylamino)-quinazoline;
2-(2,6-dichlorophenyl)-4-(4-pyridylamino)-quinazoline;
2-(2,6-dibromophenyl)-4-(4-pyridylamino)-quinazoline;
2-(2,6-difluorophenyl)-4-(4-pyridylamino)-quinazoline;
2-(2-fluorophenyl)-4-(4-pyridylamino)-6,7-dimethoxyquinazoline;
2-(4-fluorophenyl)-4-(4-pyridylamino)-6,7-dimethoxyquinazoline;
2-(2-fluorophenyl)-4-(4-pyridylamino)-6-nitroquinazoline;
2-(2-fluorophenyl)-4-(4-pyridylamino -6-aminoquinazoline;
2-(2-fluorophenyl)-4-(4-pyridylamino)-7-aminoquinazoline;
2-(2-fluorophenyl)-4-(4-pyridylamino)-6-(3-methoxybenzylamino)-quinazoline;
2-(2-fluorophenyl)-4-(4-pyridylamino)-6-(4-methoxybenzylamino)-quinazoline;
2-(2-fluorophenyl)-4-(4-pyridylamino)-6-(2-isobutylamino)-quinazoline; and
2-(2-fluorophenyl)-4-(4-pyridylamino)-6-(4-methylmercaptobenzylamino)-quina zoline; and the pharmaceutically acceptable salts thereof.

In yet another preferred embodiment the invention relates to pharmaceutical compositions containing **A** and **B,** characterized in that the p38 kinase inhibitor **B** is selected from the compounds of formula **3a, 3b, 3c,** or **3d** as disclosed in US 6,340,685 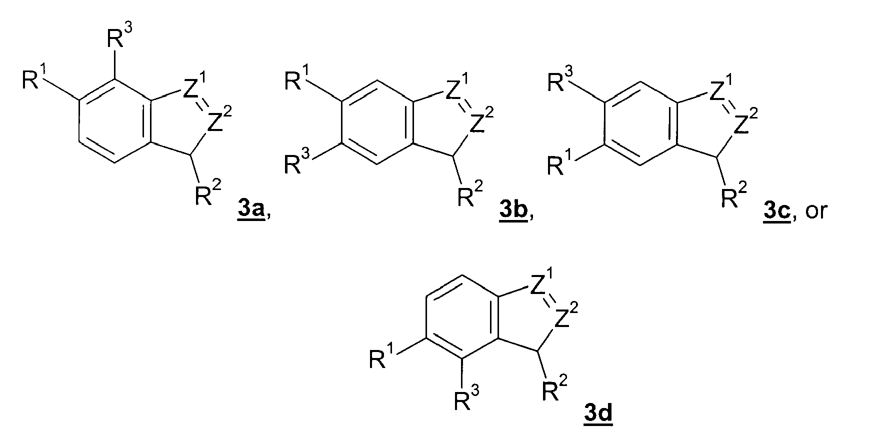 and the pharmaceutically acceptable salts thereof,
wherein each of Z¹ and Z² is independently CR⁴ or N;
where each R⁴ is independently selected from H and alkyl(1-6C);
wherein said alkyl optionally includes one or more heteroatoms selected from O, S and N, and wherein said alkyl is optionally substituted by one or more substituents selected from halo, OR, SR, NR₂, RCO, COOR, CONR₂, OOCR, NROCR, CN, =O, a 5 or 6 membered saturated carbocyclic ring or heterocyclic ring containing 1-2 N, and a 6-membered aromatic ring optionally containing 1-2 N heteroatoms, wherein R in the foregoing optional substituents is H or alkyl (1-6C);
- R¹: is 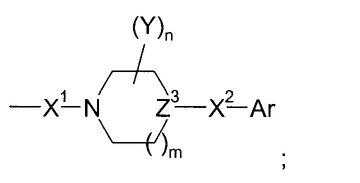
wherein
- X¹: is CO, SO, CHOH or SO₂ ;
- m: is 1;
- Y: is optionally substituted alkyl, optionally substituted aryl, or optionally substituted arylalkyl;
- n: is 0, 1 or 2;
- Z³: is N;
- X²: is CH or CH₂ ; and
- Ar: consists of one or two phenyl moieties directly coupled to X², said one or two phenyl moieties being optionally substituted by a substituent selected from halo, nitro, alkyl (1-6C), alkenyl (1-6C), CN, CF₃, RCO, COOR, CONR₂, NR₂, OR, SR, OOCR, NROCR, (wherein R in the foregoing is H or 1-6C alkyl), and phenyl, itself optionally substituted by the foregoing substituents;
- R²: is selected from H, and alkyl (1-6C);
wherein said alkyl optionally includes one or more heteroatoms which are selected from O, S and N, and wherein said alkyl is optionally substituted by one or more substituents selected from halo, OR, SR, NR₂, RCO, COOR, CONR₂, OOCR, NROCR, (where R in the foregoing is H or 1-6C alkyl) CN, =O, a 5 or 6 membered saturated carbocyclic ring or heterocyclic ring containing 1-2 N, and a 6-membered aromatic ring optionally containing 1-2 N heteroatoms;
- R³: is H, halo, NO₂, alkyl (1-6C), alkenyl (1-6C), CN, OR, SR, NR₂, RCO, COOR, CONR₂, OOCR, or NROCR where R is H or alkyl (1-6C).

In a particularily preferred embodiment the invention relates to pharmaceutical compositions containing **A** and **B**, characterized in that the p38 kinase inhibitor **B** is selected from the follwoing compounds disclosed US 6,340,685:
4-(2,6-difluorobenzyl)-piperazinyl-benzimidazole-5-carboxamide;
4-(2,3-difluorobenzyl)-piperazinyl-benzimidazole-5-carboxamide;
4-(3,5-difluorobenzyl)-piperazinyl-benzimidazole-5-carboxamide;
4-(3-chlorobenzyl)-piperazinyl-benzimidazole-5-carboxamide;
4-(4-carboxymethyl benzyl)-piperazinyl-benzimidazole-5-carboxamide;
4-(4-methoxybenzyl)-piperazinyl-benzimidazole-5-carboxamide;
4-(4-trifluoromethoxybenzyl)-piperazinyl-benzimidazole-5-carboxamide;
4-(4-methylbenzyl)-piperazinyl-benzimidazole-5arboxamide;
4-(2,4-dichlorobenzoyl)-piperazinyl-benzimidazole-5-carboxamide;
4-(3,4-dichlorobenzoylm)piperazinyl-benzinidazole-5-carboxamnide;
4-[trans-3-(trifluoromethyl)-cinnamoyl]-piperazinyl-benzimidazole-5-carboxm ide;
4-(4-chlorobenzoyl)-piperazinyl-benzimidazole-5-carboxamide;
4-benzomethylbenzoylpiperazyl-benzimidazole-5-carboxamide;
4-(2-trifluoromethylbenzoyl)-piperazinyl-benzimidazole-5-carboxamide;
4-(4-methxybenzoyl)-piperazinyl-benzimidazole-5-carboxamide;
4-(3,4-dichlorophenyl)-piperazinyl-benzimnidazole-5-carboxamide;
4-(4-chlorobenzhydryl)-piperazinyl-benzimidazole-5-carboxamide;
4-trans-1-cinnamyl piperazinyl-benzimidazole-5-carboxamide;
4-(4-chlorophenyl)-piperazinyl-benzimidazole-5-carboxamide;
4-[bis(4-fluorophenyl)-methyl]-piperazinyl-benzimidazole-5-carboxamide;
4-(4-chlorobenzyl)-piperazinyl-benzimidazole-5-carboxamide;
4-(2-chlorobenzyl)-piperazinyl-benzimidazole-5-carboxamide;
4-benzylpiperazinyl-benzinudazole-5-carboxamnide;
4-(4-methylthiobenzyl)-piperazinyl-benzimidazole-5-carboxamide;
4-(3,4,5-trimethoxybenzyl)-piperazinyl-benzimidazole-5-carboxamide;
4-(2-naphthylmethyl)-piperazinyl-benzimidazole-5-carboxamide;
4-(4-diethylaminobenzyl)-piperazinyl-benzimidazole-5-carboxamide;
4-(biphenylmethyl)-piperazinyl-benzimidazole-5-carboxamide;
4-(4-phenoxybenzyl)-piperazinyl-benzimidazole-5-carboxamide;
4-(4-quinolinylmethyl)-piperazinyl-benzimidazole-5-carboxamide;
4-(4-chlorobenzyl)-piperazinyl-1-(2-propyl)-indole-5-carboxamide;
4-(3-chlorobenzyl)-piperazinyl-benzimidazole-5-carboxamide;
4-(3-chlorobenzyl)-piperazinyl-N-(2-propyl)-benzimidazole-5-carboxamide;
4-(3-chlorobenzyl)-piperazinyl-N-(2-propyl)-benzimidazole-6-carboxamide;
4-(3-chlorobenzyl)-piperazinyl-N-methyl-benzimidazole-5-carboxamide;
4-(3-chlorobenzyl)-piperazinyl-N-methyl-benzimidazole-6-carboxamide;
4-(3-chlorobenzyl)-piperazinyl-N-ethyl-benzimidazole-5-carboxamide; and
4-(3-chlorobenzyl)-piperazinyl-N-ethyl-benzimidazole-6-carboxamide.

In another preferred embodiment the invention relates to pharmaceutical compositions containing **A** and **B**, characterized in that the p38 kinase inhibitor **B** is selected from the compounds of formula **4** as disclosed in WO 00/43384 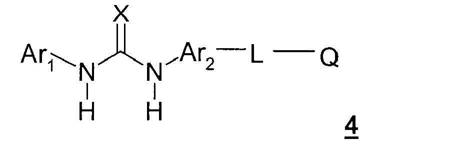 wherein
- Ar₁: is a heterocyclic group selected from the group consisting of pyrrole, pyrrolidine, pyrazole, imidazole, oxazole, thiazole, furan and thiophene; and wherein Ar₁ may be substituted by one or more R₁,R₂ or R₃;
- Ar₂: is phenyl, naphthyl, quinoline, isoquinoline, tetrahydronaphthyl, tetrahydroquinoline, tetrahydroisoquinoline, benzimidazole, benzofuran, indanyl, indenyl or indole each being optionally substituted with one to three R₂ groups;
- L,: a linking group, is a
C₁₋₁₀ saturated or unsaturated branched or unbranched carbon chain;
wherein one or more methylene groups are optionally independently replaced by O,N or S; and
wherein said linking group is optionally substituted with 0-2 oxo groups and one or more C₁₋₄ branched or unbranched alkyl which may be substituted by one or more halogen atoms;
- Q: is selected from the group consisting of:
a) phenyl, naphthyl, pyridine, pyrimidine, pyridazine, imidazole, benzimidazole, furan, thiophene, pyran, naphthyridine, oxazo[4,5-*b*]pyridine and imidazo[4,5-*b*]pyridine, which are optionally substituted with one to three groups selected from the group consisting of halogen, C₁₋₆ alkyl, C₁₋₆ alkoxy, hydroxy, mono- or di-(C₁₋₃ alkyl)amino, C₁₋₆ alkyl-S(O)ₘ and phenylamino wherein the phenyl ring is optionally substituted with one to two groups consisting of halogen, C₁₋₆ alkyl and C₁₋₆ alkoxy;
b) tetrahydropyran, tetrahydrofuran, 1,3-dioxolanone, 1,3-dioxanone, 1,4-dioxane, morpholine, thiomorpholine, thiomorpholine sulfoxide, thiomorpholine sulfone, piperidine, piperidinone, tetrahydropyrimidone, cyclohexanone, cyclohexanol, pentamethylene sulfide, pentamethylene sulfoxide, pentamethylene sulfone, tetramethylene sulfide, tetramethylene sulfoxide and tetramethylene sulfone which are optionally substituted with one to three groups selected from the group consisting of C₁₋₆ alkyl, C₁₋₆ alkoxy, hydroxy, mono- or di-(C₁₋₃ alkyl)amino-C₁₋₃ alkyl, phenylamino-C₁₋₃ alkyl and C₁₋₃ alkoxy-C₁₋₃ alkyl;
c) C₁₋₆ alkoxy, secondary or tertiary amine wherein the amino nitrogen is covalently bonded to groups selected from the group consisting of C₁₋₃ alkyl and C₁₋₅ alkoxyalkyl and phenyl wherein the phenyl ring is optionally substituted with one to two groups consisting of halogen, C₁₋₆ alkoxy, hydroxy or mono- or di-(C₁₋₃ alkyl)amino, C₁₋₆ alkyl-S(O)ᵣ, phenyl-S(O)ₜ, wherein the phenyl ring is optionally substituted with one to two groups consisting of halogen, C₁₋₆ alkoxy, hydroxy or mono- or di-(C₁₋₃ alkyl)amino;
- R₁: is selected from the group consisting of:
(a) C₃₋₁₀ branched or unbranched alkyl, which may optionally be partially or fully halogenated, and optionally substituted with one to three phenyl, naphthyl or heterocyclic groups selected from the group consisting of pyridinyl, pyrimidinyl, pyrazinyl, pyridazinyl, pyrrolyl, imidazolyl, pyrazolyl, thienyl, furyl, isoxazolyl and isothiazolyl; each such phenyl, naphthyl or heterocycle selected from the group hereinabove described, being substituted with 0 to 5 groups selected from the group consisting of halogen, C₁₋₆ branched or unbranched alkyl which is optionally partially or fully halogenated, C₃₋₈ cycloalkyl, C₅₋₈ cycloalkenyl, hydroxy, cyano, C₁₋₃ alkyloxy which is optionally partially or fully halogenated, NH₂C(O) and di(C₁₋₃)alkylaminocarbonyl;
(b) C₃₋₇ cycloalkyl selected from the group consisting of cyclopropyl, cyclobutyl, cyclopentanyl, cyclohexanyl, cycloheptanyl, bicyclopentanyl, bicyclohexanyl and bicycloheptanyl, which may optionally be partially or fully halogenated and which may optionally be substituted with one to three C₁₋₃ alkyl groups, or an analog of such cycloalkyl group wherein one to three ring methylene groups are replaced by groups independently selected from O, S, CHOH, >C=O, >C=S and NH;
(c) C₃₋₁₀ branched alkenyl which may optionally be partially or fully halogenated, and which is optionally substituted with one to three C₁₋₅ branched or unbranched alkyl, phenyl, naphthyl or heterocyclic groups, with each such heterocyclic group being independently selected from the group consisting of pyridinyl, pyrimidinyl, pyrazinyl, pyridazinyl, pyrrolyl, imidazolyl, pyrazolyl, thienyl, furyl, isoxazolyl and isothiazolyl, and each such phenyl, naphthyl or heterocyclic group being substituted with 0 to 5 groups selected from halogen, C₁₋₆ branched or unbranched alkyl which is optionally partially or fully halogenated, cyclopropyl, cyclobutyl, cyclopentanyl, cyclohexanyl, cycloheptanyl, bicyclopentanyl, bicyclohexanyl and bicycloheptanyl, hydroxy, cyano, C₁₋₃ alkyloxy which is optionally partially or fully halogenated, NH₂C(O), mono- or di(C₁₋₃)alkylaminocarbonyl;
(d) C₅₋₇ cycloalkenyl selected from the group consisting of cyclopentenyl, cyclohexenyl, cyclohexadienyl, cycloheptenyl, cycloheptadienyl, bicyclohexenyl and bicycloheptenyl, wherein such cycloalkenyl group may optionally be substituted with one to three C₁₋₃ alkyl groups;
(e) cyano; and,
(f) methoxycarbonyl, ethoxycarbonyl and propoxycarbonyl;
- R₂: is selected from the group consisting of:
a C₁₋₆ branched or unbranched alkyl which may optionally be partially or fully halogenated, acetyl, aroyl, C₁₋₄ branched or unbranched alkoxy, which may optionally be partially or fully halogenated, halogen, methoxycarbonyl and phenylsulfonyl;
- R₃: is selected from the group consisting of:
a) a phenyl, naphthyl or heterocyclic group selected from the group consisting of pyridinyl, pyrimidinyl, pyrazinyl, pyridazinyl, pyrrolyl, imidazolyl, pyrazolyl, thienyl, furyl, tetrahydrofuryl, isoxazolyl, isothiazolyl, quinolinyl, isoquinolinyl, indolyl, benzimidazolyl, benzofuranyl, benzoxazolyl, benzisoxazolyl, benzpyrazolyl, benzothiofuranyl, cinnolinyl, pterindinyl, phthalazinyl, naphthypyridinyl, quinoxalinyl, quinazolinyl, purinyl and indazolyl; wherein such phenyl, naphthyl or heterocyclic group is optionally substituted with one to five groups selected from the group consisting of a C₁₋₆ branched or unbranched alkyl, phenyl, naphthyl, heterocycle selected from the group hereinabove described, C₁₋₆ branched or unbranched alkyl which is optionally partially or fully halogenated, cyclopropyl, cyclobutyl, cyclopentanyl, cyclohexanyl, cycloheptanyl, bicyclopentanyl, bicyclohexanyl, bicycloheptanyl, phenyl C₁₋₅ alkyl, naphthyl C₁₋₅ alkyl, halo, hydroxy, cyano, C₁₋₃ alkyloxy which may optionally be partially or fully halogenated, phenyloxy, naphthyloxy, heteraryloxy wherein the heterocyclic moiety is selected from the group hereinabove described, nitro, amino, mono- or di-(C₁₋₃)alkylamino, phenylamino, naphthylamino, heterocyclylamino wherein the heterocyclyl moiety is selected from the group hereinabove described, NH₂C(O), a mono- or di-(C₁₋₃)alkyl aminocarbonyl, C₁₋₅ alkyl-C(O)-C₁₋₄ alkyl, amino-C₁₋₅ alkyl, mono- or di-(C₁₋₃)alkylamino-C₁₋₅ alkyl, amino-S(O)₂, di-(C₁₋₃)alkylamino-S(O)₂, R₄-C₁₋₅alkyl, R₅-C₁₋₅alkoxy, R₆-C(O)-C₁₋₅ alkyl and R₇ -C₁₋₅ alkyl(R₈)N;
b) a fused aryl selected from the group consisting of benzocyclobutanyl, indanyl, indenyl, dihydronaphthyl, tetrahydronaphthyl, benzocycloheptanyl and benzocycloheptenyl, or a fused heterocyclyl selected from the group consisting of cyclopentenopyridine, cyclohexanopyridine, cyclopentanopyrimidine, cyclohexanopyrimidine, cyclopentanopyrazine, cyclohexanopyrazine, cyclopentanopyridazine, cyclohexanopyridazine, cyclopentanoquinoline, cyclohexanoquinoline, cyclopentanoisoquinoline, cyclohexanoisoquinoline, cyclopentanoindole, cyclohexanoindole, cyclopentanobenzimidazole, cyclohexanobenzimidazole, cyclopentanobenzoxazole, cyclohexanobenzoxazole, cyclopentanoimidazole, cyclohexanoimidazole, cyclopentanothiophene and cyclohexanothiophene; wherein the fused aryl or fused heterocyclyl ring is substituted with 0 to 3 groups independently selected from phenyl, naphthyl and heterocyclyl selected from the group consisting of pyridinyl, pyrimidinyl, pyrazinyl, pyridazinyl, pyrrolyl, imidazolyl, pyrazolyl, thienyl, furyl, isoxazolyl, and isothiazolyl, C₁₋₆ branched or unbranched alkyl which is optionally partially or fully halogenated, halo, cyano, C₁₋₃ alkyloxy which is optionally partially or fully halogenated, phenyloxy, naphthyloxy, heterocyclyloxy wherein the heterocyclyl moiety is selected from the group hereinabove described, nitro, amino, mono- or di-(C₁₋₃)alkylamino, phenylamino, naphthylamino, heterocyclylamino wherein the heterocyclyl moiety is selected from the group hereinabove described, NH₂C(O), a mono- or di-(C₁₋₃)alkyl aminocarbonyl, C₁₋₄ alkyl-OC(O), C₁₋₅ alkyl-C(O)-C₁₋₄ branched or unbranched alkyl, an amino-C₁₋₅ alkyl, mono- or di-(C₁₋₃)alkylamino-C₁₋₅ alkyl, R₉-C₁₋₅ alkyl, R₁₀-C₁₋₅ alkoxy, R₁₁―C(O)-C₁₋₅ alkyl, and R₁₂-C₁₋₅ alkyl(R₁₃)N;
c) cycloalkyl selected from the group consisting of cyclopentanyl, cyclohexanyl, cycloheptanyl, bicyclopentanyl, bicyclohexanyl and bicycloheptanyl, which the cycloalkyl may optionally be partially or fully halogenated and which may optionally be substituted with one to three C₁₋₃ alkyl groups;
d) C₅₋₇ cycloalkenyl, selected from the group consisting of cyclopentenyl, cyclohexenyl, cyclohexadienyl, cycloheptenyl, cycloheptadienyl, bicyclohexenyl and bicycloheptenyl, wherein such cycloalkenyl group may optionally be substituted with one to three C₁₋₃ alkyl groups; and
e) acetyl, aroyl, alkoxycarbonylalkyl or phenylsulfonyl;
f) C₁₋₆ branched or unbranched alkyl which may optionally be partially or fully halogenated;
or R₁ and R₂ taken together may optionally form a fused phenyl or pyridinyl ring,
and wherein each R₈, R₁₃ is independently selected from the group consisting of:
hydrogen and C₁₋₄ branched or unbranched alkyl which may optionally be partially or fully halogenated;
each R₄, R₅, R₆, R₇, R₉, Rio, R₁₁ and R₁₂ is independently selected from the group consisting of:
   morpholine, piperidine, piperazine, imidazole and tetrazole;
      m = 0, 1,2;
      r = 0, 1, 2;
      t = 0, 1, 2;
      X = O or S and physiologically acceptable acids or salts thereof.

In a preferred embodiment the invention relates to pharmaceutical compositions containing **A** and **B**, characterized in that the p38 kinase inhibitor **B** is selected from the compounds of formula **4** as disclosed in WO 00/43384 wherein Ar₂ is naphthyl, tetrahydronaphthyl, indanyl or indenyl.

A more preferred subgeneric aspect of the invention relates to pharmaceutical compositions containing **A** and **B**, characterized in that the p38 kinase inhibitor **B** is a compound of the formula **4** wherein Ar₂ is naphthyl.

A yet more preferred subgeneric aspect of the invention relates to pharmaceutical compositions containing **A** and **B**, characterized in that the p38 kinase inhibitor **B** is selected from compounds of the formula **4**, as described in the immediate previous paragraph, wherein:
- Ar₁: is thiophene or pyrazole;
- Ar₂: is 1-naphthyl;
- L: is C₁₋₆ saturated or unsaturated branched or unbranched carbon chain wherein one or more methylene groups are optionally independently replaced by O,N or S; and
wherein said linking group is optionally substituted with 0-2 oxo groups and one or more C₁₋₄ branched or unbranched alkyl which may be substituted by one or more halogen atoms;
- R₁: is selected from the group consisting of C₁₋₄alkyl branched or unbranched, cyclopropyl and cyclohexyl which may optionally be partially or fully halogenated and which may optionally be substituted with one to three C₁₋₃ alkyl groups;
- R₃: is selected from the group consisting of C₁₋₄alkyl branched or unbranched, cyclopropyl, phenyl, pyridinyl each being optionally substituted as described above, alkoxycarbonylalkyl; C₁₋₆alkyl branched or unbranched; cyclopropyl or cyclopentyl optionally substituted as described above.

A yet further preferred subgeneric aspect of the invention relates to pharmaceutical compositions containing **A** and **B**, characterized in that the p38 kinase inhibitor **B** is selected from compounds of the formula **4**, as described in the immediate previous paragraph, wherein Ar₁ is pyrazole.

A still yet further preferred subgeneric aspect of previous the invention relates to pharmaceutical compositions containing **A** and **B**, characterized in that the p38 kinase inhibitor **B** is selected from compounds of the formula **4**, as described in the immediate paragraph, wherein L is C₁₋₅ saturated carbon chain wherein one or more methylene groups are optionally independently replaced by O,N or S; and
wherein said linking group is optionally substituted with 0-2 oxo groups and one or more C₁₋₄ branched or unbranched alkyl which may be substituted by one or more halogen atoms;

Particularly preferred embodiments of L are propoxy, ethoxy, methoxy, methyl, propyl, C₃₋₅ acetylene or methylamino each being optionally substituted are described herein.

A more particularly preferred embodiment of L is ethoxy optionally substituted.

The following compounds are representative of the compounds of formula **4** and are of particular interest as component **B** in the compositions according to the invention:
1-[5-*tert*-Butyl-2-*p*-tolyl-2H-pyrazol-3-yl]-3-[4-(2-morpholin-4-yl-ethoxy)naphthalen-1-yl]-urea;
1-[5-*tert*-Butyl-2-*p*-tolyl-2H-pyrazol-3-yl]-3-[4-(2-(*cis*-2,6-dimethylmorpholin-4-yl)ethoxy)naphthalen-1-yl]-urea;
1-[5-*tert*-Butyl-2-p-tolyl-2H-pyrazol-3-yl]-3-[4-(2-(*trans*-2,6-dimethylmorpholin-4-yl)ethoxy)naphthalen-1-yl]-urea;
1-[5-*tert*-Butyl-2-*p*-tolyl-2H-pyrazol-3-yl]-3-[4-(2-(2-(methoxymethyl)morpholin-4-yl)ethoxy)naphthalen-1-yl]-urea;
1-[5-*tert*-Butyl-2-*p*-tolyl-2H-pyrazol-3-yl]-3-[4-(2-(morpholin-4-yl)-2-oxoethoxy)naphthalen-1-yl]-urea;
1-[5-*tert*-Butyl-2-*p*-tolyl-2H-pyrazol-3-yl]-3-[4-(2-(morpholin-4-yl)-2-methylethoxy)naphthalen-1-yl]-u rea;
1-[5-*tert*-Butyl-2-*p*-tolyl-2H-pyrazol-3-yl]-3-[4-(2-(morpholin-4-yl)-1-methylethoxy)naphthalen-1-yl]-urea;
1-[5-*tert*-Butyl-2-*p*-tolyl-2H-pyrazol-3-yl]-3-[4-(2-thiomorpholin-4-yl-ethoxy)naphthalen-1-yl]-u rea;
1-[5-*tert*-Butyl-2-*p*-tolyl-2H-pyrazol-3-yl]-3-[4-(2-(1-oxothiomorpholin-4-yl)ethoxy)naphthalen-1-yl]-urea;
1-[5-*tert*-Butyl-2-*p*-tolyl-2H-pyrazol-3-yl]-3-[4-(2-morpholin-4-yl-ethoxy)-3-methylnaphthalen-1-yl]-urea;
1-[5-*tert*-Butyl-2-*p*-tolyl-2H-pyrazol-3-yl]-3-[4-(2-piperidin-4-yl-ethoxy)naphthalen-1-yl]-urea;
1-[5-*tert*-Butyl-2-*p*-tolyl-2H-pyrazol-3-yl]-3-[4-(2-(1-acetylpiperidin-4-yl)ethoxy)naphthalen-1-yl]-urea;
1-[5-*tert*-Butyl-2-*p*-tolyl-2H-pyrazol-3-yl]-3-[4-(2-thiazolidin-3-yl-ethoxy)naphthalen-1-yl]-urea;
1-[5-tert-Butyl-2-p-tolyl-2H-pyrazol-3-yl]-3-[4-(2-(morpholin-4-yl-carbonyloxo)ethoxy)naphthalen-1-yl]-urea;
1-[5-tert-Butyl-2-p-tolyl-2H-pyrazol-3-yl]-3-[4-(2-(tetrahydropyran-4-yl)ethoxy)naphthalen-1-yl]-urea;
1-[5-*tert*-Butyl-2-*p*-tolyl-2H-pyrazol-3-yl]-3-[4-(2-(N-methyl-2-methoxyethylamino)ethoxy)naphthalen-1-yl]-urea;
1-[5-*tert*-Butyl-2-*p*-tolyl-2H-pyrazol-3-yl]-3-[4-(2-(1-oxo-tetrahydrothiophen-3-yl)ethoxy)naphthalen-1-yl]-urea;
1-[5-*tert*-Butyl-2-*p*-tolyl-2H-pyrazol-3-yl]-3-[4-(3-morpholin-4-yl-propyl)naphthalen-1-yl]-urea;
1-[5-*tert*-Butyl-2-*p*-tolyl-2H-pyrazol-3-yl]-3-[4-(morpholin-4-yl-methyl)naphthalen-1-yl]-urea;
1-[5-*tert*-Butyl-2-*p*-tolyl-2H-pyrazol-3-yl]-3-[4-(3-thiazolidin-3-yl-propyl)naphthalen-1-yl]-urea;
1-[5-tert-Butyl-2-p-tolyl-2H-pyrazol-3-yl]-3-[4-(3-(tetrahydopyran-2-yl-oxy)propyl)naphthalen-1-yl]-urea;
1-[5-*tert*-Butyl-2-*p*-tolyl-2H-pyrazol-3-yl]-3-[4-(2-pyridin-4-yl-ethyl)naphthalen-1-yl]-urea;
1-[5-*tert*-Butyl-2-*p*-tolyl-2H-pyrazol-3-yl]-3-[4-(2-pyridin-4-yl-ethenyl)naphthalen-1-yl]-urea;
1-[5-*tert*-Butyl-2-*p*-tolyl-2H-pyrazol-3-yl]-3-[4-(3-(morpholin-4-yl)propyn-1-yl)naphthalen-1-yl]-urea;
1-[5-*tert*-Butyl-2-p-tolyl-2 H-pyrazol-3-yl]-3-[4-(3-(tetrahyd ropyran-2-yl-oxy)propyn-1-yl)naphthalen-1-yl]-urea;
1-[5-*tert*-Butyl-2-*p*-tolyl-2H-pyrazol-3-yl]-3-[4-(3-(methoxymethyloxy)propyn-1-yl)naphthalen-1-yl]-urea;
1-[5-*tert*-Butyl-2-*p*-tolyl-2H-pyrazol-3-yl]-3-[4-(3-(morpholin-4-yl)-3-methylpropyn-1-yl)naphthalen-1-yl]-urea;
1-[5-*tert*-Butyl-2-p-tolyl-2H-pyrazol-3-yl]-3-[4-(3-(morpholin-4-yl)-3,3-dimethylpropyn-1-yl)naphthalen-1-yl]-urea;
1-[5-*tert*-Butyl-2-*p*-tolyl-2H-pyrazol-3-yl]-3-[4-(3-(tetrahydropyran-2-yl-oxy)butyn-1-yl)naphthalen-1-yl]-urea;
1-[5-*tert*-Butyl-2-*p*-tolyl-2H-pyrazol-3-yl]-3-[4-(3-(furan-2-ylcarbonyloxy)propyn-1-yl)naphthalen-1-yl]-urea;
1-[5-*tert*-Butyl-2-*p*-tolyl-2H-pyrazol-3-yl]-3-[4-(3-(piperdin-1-yl)propyn-1-yl)naphthalen-1-yl]-urea;
1-[5-*tert*-Butyl-2-*p*-tolyl-2H-pyrazol-3-yl]-3-[4-(3-(2-methoxymethylmorpholin-4-yl)propyn-1-yl)naphthalen-1-yl]-u rea;
1-[5-*tert*-Butyl-2-*p*-tolyl-2H-pyrazol-3-yl]-3-[4-(pyridin-4-yl-methoxy)naphthalen-1-yl]-urea;
1-[5-*tert*-Butyl-2-*p*-tolyl-2H-pyrazol-3-yl]-3-[4-(2-pyridin-4-yl-ethoxy)naphthalen-1-yl]-urea;
1-[5-*tert*-Butyl-2-*p*-tolyl-2H-pyrazol-3-yl]-3-[4-(3-pyridin-4-yl-propoxy)naphthalen-1-yl]-urea;
1-[5-*tert*-Butyl-2-*p*-tolyl-2H-pyrazol-3-yl]-3-[4-(2-imidazol-1-yl-ethoxy)naphthalen-1-yl]-urea;
1-[5-*tert*-Butyl-2-*p*-tolyl-2H-pyrazol-3-yl]-3-[4-(2-benzimidazol-1-yl-ethoxy)naphthalen-1-yl]-urea;
1-[5-*tert*-Butyl-2-*p*-tolyl-2H-pyrazol-3-yl]-3-[4-(2-(3,4-dimethoxyphenyl)-ethoxy)naphthalen-1-yl]-urea;
1-[5-*tert*-Butyl-2-*p*-tolyl-2H-pyrazol-3-yl]-3-[4-(pyridin-4-yl-methylamino)naphthalen-1-yl]-urea;
1-[5-*tert*-Butyl-2-*p*-tolyl-2H-pyrazol-3-yl]-3-[4-(pyridin-4-yl-carbonylamino)naphthalen-1-yl]-urea;
1-[5-*tert*-Butyl-2-*p*-tolyl-2H-pyrazol-3-yl]-3-[4-(morpholin-4-yl-acetamido)naphthalen-1-yl]-urea;
1-[5-*tert*-Butyl-2-*p*-tolyl-2H-pyrazol-3-yl]-3-[4-(pyridin-3-yl-methylamino)naphthalen-1-yl]-urea;
1-[5-*tert*-Butyl-2-*p*-tolyl-2H-pyrazol-3-yl]-3-[4-(pyridin-3-yl-carbonylamino)naphthalen-1-yl]-urea;
1-[5-*iso*-Propyl-2-phenyl-2H-pyrazol-3-yl]-3-[4-(2-morpholin-4-yl-ethoxy)naphthalen-1-yl]-urea;
1-[5-(Tetrahydropyran-3-yl)-2-phenyl-2H-pyrazol-3-yl]-3-[4-(2-morpholin-4-yl-ethoxy)naphthalen-1-yl]-urea;
1-[5-cyclohexyl-2-phenyl-2H-pyrazol-3-yl]-3-[4-(2-morpholin-4-yl-ethoxy)naphthalen-1-yl]-urea;
1-[5-(2,2,2-trifluoroethyl)-2-phenyl-2H-pyrazol-3-yl]-3-[4-(2-morpholin-4-yl-ethoxy)naphthalen-1-yl]-urea;
1-[5-(1-methylcycloprop-1-yl)-2-phenyl-2H-pyrazol-3-yl]-3-[4-(2-morpholin-4-yl-ethoxy)naphthalen-1-yl]-urea;
1-[5-ethoxycarbonyl-2-phenyl-2H-pyrazol-3-yl]-3-[4-(2-morpholin-4-yl-ethoxy)naphthalen-1-yl]-urea;
1-[5-(1-methylcyclohex-1-yl)-2-phenyl-2H-pyrazol-3-yl]-3-[4-(2-morpholin-4-yl-ethoxy)naphthalen-1-yl]-urea;
1-[5-*tert*-butyl-2-methyl-2H-pyrazol-3-yl]-3-[4-(2-morpholin-4-yl-ethoxy)naphthalen-1-yl]-urea;
1-[5-*tert*-butyl-2-benzyl-2H-pyrazol-3-yl]-3-[4-(2-morpholin-4-yl-ethoxy)naphthalen-1-yl]-urea;
1-[5-*tert*-butyl-2-(4-chlorophenyl)-2H-pyrazol-3-yl]-3-[4-(2-morpholin-4-yl-ethoxy)naphthalen-1-yl]-urea;
1-[5-*tert*-butyl-2-butyl-2H-pyrazol-3-yl]-3-[4-(2-morpholin-4-yl-ethoxy)naphthalen-1-yl]-urea;
1-[5-*tert*-butyl-2-(ethoxycarbonylmethyl)-2H-pyrazol-3-yl]-3-[4-(2-morpholin-4-yl-ethoxy)naphthalen-1-yl]-urea;
1-[5-*tert*-butyl-2-(4-methyl-3-carbamylphenyl)-2H-pyrazol-3-yl]-3-[4-(2-morpholin-4-yl-ethoxy)naphthalen-1-yl]-urea;
1-[5-*tert*-butyl-2-(4-methyl-3-(2-ethoxycarbonylvinyl)phenyl)-2H-pyrazol-3-yl]-3-[4-(2-morpholin-4-yl-ethoxy)naphthalen-1-yl]-urea;
1-[5-*tert*-butyl-2-(4-methyl-3-(morpholin-4-yl)methylphenyl)-2H-pyrazol-3-yl]-3-[4-(2-morpholin-4-yl-ethoxy)naphthalen-1-yl]-urea;
1-[5-*tert*-butyl-2-(4-methyl-3-dimethylaminomethylphenyl)-2H-pyrazol-3-yl]-3-[4-(2-morpholin-4-yl-ethoxy)naphthalen-1-yl]-urea;
1-[5-*tert*-butyl-2-(3-(2-morpholin-4-yl-ethyl)phenyl)-2H-pyrazol-3-yl]-3-[4-(2-morpholin-4-yl-ethoxy)naphthalen-1-yl]-u rea;
1-[5-tert-butyl-2-(3-(tetrahydropyran-4-ylamino)phenyl)-2H-pyrazol-3-yl]-3-[4-(2-morpholin-4-yl-ethoxy)naphthalen-1-yl]-urea;
1-[5-*tert*-butyl-2-(3-dimethylaminomethylphenyl)-2H-pyrazol-3-yl]-3-[4-(2-morpholin-4-yl-ethoxy)naphthalen-1-yl]-urea;
1-[5-*tert*-butyl-2-(4-(tetrahydropyran-4-ylamino)phenyl)-2H-pyrazol-3-yl]-3-[4-(2-morpholin-4-yl-ethoxy)naphthalen-1-yl]-urea;
1-[5-*tert*-butyl-2-(4-(3-benzylureido)phenyl)-2H-pyrazol-3-yl]-3-[4-(2-morpholin-4-yl-ethoxy)naphthalen-1-yl]-urea;
1-[5-*tert*-butyl-2-(2-chloropyridin-5-yl)-2H-pyrazol-3-yl]-3-[4-(2-morpholin-4-yl-ethoxy)naphthalen-1-yl]-urea;
1-[5-*tert*-butyl-2-(2-methylpyridin-5-yl)-2H-pyrazol-3-yl]-3-[4-(2-morpholin-4-yl-ethoxy)naphthalen-1-yl]-urea;
1-[5-*tert*-butyl-2-(2-methoxypyridin-5-yl)-2H-pyrazol-3-yl]-3-[4-(2-morpholin-4-yl-ethoxy)naphthalen-1-yl]-urea;
1-[5-*tert*-butyl-2-(pyridin-3-yl)-2H-pyrazol-3-yl]-3-[4-(2-morpholin-4-yl-ethoxy)naphthalen-1-yl]-urea;
1-[5-*tert*-butyl-2-(2-methylpyridin-5-yl)-2H-pyrazol-3-yl]-3-[4-(2-pyridin-4-yl-ethoxy)naphthalen-1-yl]-u rea;
1-[5-*tert*-butyl-2-(2-methylpyridin-5-yl)-2H-pyrazol-3-yl]-3-[4-(2-(*trans*-2,6-dimethylmorpholin-4-yl)ethoxy)naphthalen-1-yl]-urea;
1-[5-*tert*-butyl-2-(2-methylpyridin-5-yl)-2H-pyrazol-3-yl]-3-[4-(3-morpholin-4-yl-propyn-1-yl)naphthalen-1-yl]-urea;
1-[5-*tert*-Butyl-2-*p*-tolyl-2H-pyrazol-3-yl]-3-[4-(2-(2-dimethylaminomethylmorpholin-4-yl)ethoxy)naphthalen-1-yl]-urea;
1-[5-*tert*-butyl-2-*iso*-propyl-2H-pyrazol-3-yl]-3-[4-(2-morpholin-4-yl-ethoxy)naphthalen-1-yl]-u rea;
1-[5-*tert*-butyl-2-cyclopropyl-2H-pyrazol-3-yl]-3-[4-(2-morpholin-4-yl-ethoxy)naphthalen-1-yl]-urea;
1-[5-*tert*-butyl-2-(thiophen-3-yl)-2H-pyrazol-3-yl]-3-[4-(2-morpholin-4-yl-ethoxy)naphthalen-1-yl]-urea;
1-[5-*tert*-butyl-2-cyclopentyl-2H-pyrazol-3-yl]-3-[4-(2-morpholin-4-yl-ethoxy)naphthalen-1-yl]-urea;
1-[5-tert-butyl-2-iso-propyl-2H-pyrazol-3-yl]-3-[4-(tetrahyropyran-4-yl-ethoxy)naphthalen-1-yl]-urea;
1-[5-*tert*-butyl-2-cyclopropyl-2H-pyrazol-3-yl]-3-[4-(1-oxo-tetrahydrothiophen-3-yl-ethoxy)naphthalen-1-yl]-urea;
1-[5-tert-butyl-2-(thiophen-3-yl)-2H-pyrazol-3-yl]-3-[4-(2-pyridinyl-4-yl-ethoxy)naphthalen-1-yl]-urea;
1-[5-*tert*-butyl-2-cyclopentyl-2H-pyrazol-3-yl]-3-[4-(pyridin-4-yl-methoxy)naphthalen-1-yl]-urea;
1-[5-*tert*-Butyl-2-*p*-tolyl-2H-pyrazol-3-yl]-3-[4-(3-(pyridin-4-yl)propyn-1-yl)naphthalen-1-yl]-urea;
1-[5-*tert*-Butyl-2-*p*-tolyl-2H-pyrazol-3-yl]-3-[4-(3-(2-methylaminopyridin-4-yl)propyn-1-yl)naphthalen-1-yl]-urea;
1-[5-*tert*-Butyl-2-*p*-tolyl-2H-pyrazol-3-yl]-3-[4-(3-(1-oxo-tetrahydothiophen-3-yl)propyn-1-yl)naphthalen-1-yl]-urea;
1-[5-*tert*-Butyl-2-*p*-tolyl-2H-pyrazol-3-yl]-3-[4-(3-(thiazolidin-3-yl)propyn-1-yl)naphthalen-1-yl]-urea;
1-[5-*tert*-Butyl-2-*p*-tolyl-2H-pyrazol-3-yl]-3-[4-(3-(tetrahydropyran-4-yl)propyn-1-yl)naphthalen-1-yl]-urea;
1-[5-tert-Butyl-2-p-tolyl-2H-pyrazol-3-yl]-3-[4-(2-methylaminopyrimidin-4-yl-methoxy)naphthalen-1-yl]-urea;
1-[5-*tert*-Butyl-2-*p*-tolyl-2H-pyrazol-3-yl]-3-[4-(2-(2-methylaminopyrimidin-4-yl)ethoxy)naphthalen-1-yl]-urea;
1-[5-*tert*-Butyl-2-*p*-tolyl-2H-pyrazol-3-yl]-3-[4-(2-(4-methoxybenzimidazol-1-yl)ethoxy)naphthalen-1-yl]-urea;
1-[5-*tert*-Butyl-2-*p*-tolyl-2H-pyrazol-3-yl]-3-[4-(2-(4-methylaminobenzimidazol-1-yl)ethoxy)naphthalen-1-yl]-urea;
1-[5-*tert*-Butyl-2-*p*-tolyl-2H-pyrazol-3-yl]-3-[4-(2-(2-imidazo[4,5-b]pyridin-1-yl)ethoxy)naphthalen-1-yl]-urea;
1-[5-*tert*-Butyl-2-*p*-tolyl-2H-pyrazol-3-yl]-3-[4-(2-[1,8]naphthyridin-4-yl)ethoxy)naphthalen-1-yl]-urea;
1-[5-*tert*-Butyl-2-*p*-tolyl-2H-pyrazol-3-yl]-3-[4-(2-(3,4-dihydro-2H-pyrano[2,3-b]pyridin-5-yl)ethoxy)naphthalen-1-yl]-urea;
1-[5-*tert*-Butyl-2-pyridin-3-yl-2H-pyrazol-3-yl]-3-[4-(2-methylaminopyrimidin-4-yl-methoxy)naphthalen-1-yl]-urea;
1-[5-*tert*-Butyl-2-(2-methylpyridin-5-yl) -2H-pyrazol-3-yl]-3-[4-(2-(2-methylaminopyrimidin-4-yl)ethoxy)naphthalen-1-yl]-urea;
1-[5-*tert*-Butyl-2-(2-methylpyridin-5-yl)-2H-pyrazol-3-yl]-3-[4-(2-(4-methoxybenzimidazol-1-yl)ethoxy)naphthalen-1-yl]-urea;
1-[5-tert-Butyl-2-(2-methylpyridin-5-yl)-2H-pyrazol-3-yl]-3-[4-(2-(4-methylaminobenzimidazol-1-yl)ethoxy)naphthalen-1-yl]-urea;
1-(5-*tert*-Butyl-2-(2-methylpyridin-5-yl)-2H-pyrazol-3-yl]-3-[4-(2-(2-imidazo[4,5-b]pyridin-1-yl)ethoxy)naphthalen-1-yl]-urea;
1-[5-*tert*-Butyl-2-(2-methylpyridin-5-yl)-2H-pyrazol-3-yl]-3-[4-(2-[1,8]naphthyridin-4-yl)ethoxy)naphthalen-1-yl]-urea;
1-[5-*tert*-Butyl-2-(2-methylpyridin-5-yl)-2H-pyrazol-3-yl]-3-[4-(2-(3,4-dihydro-2H-pyrano[2,3-b]pyridin-5-yl)ethoxy)naphthalen-1-yl]-urea;
1-[5-*tert*-Butyl-2-cyclopropyl -2H-pyrazol-3-yl]-3-[4-(2-methylaminopyrimidin-4-yl-methoxy)naphthalen-1-yl]-urea;
1-[5-*tert*-Butyl-2-cyclopropyl-2H-pyrazol-3-yl]-3-[4-(2-(2-methylaminopyrimidin-4-yl)ethoxy)naphthalen-1-yl]-urea;
1-[5-*tert*-Butyl-2-cyclopropyl-2H-pyrazol-3-yl]-3-[4-(2-(4-methoxybenzimidazol-1-yl)ethoxy)naphthalen-1-yl]-urea;
1-[5-*tert*-Butyl-2-cyclopropyl-2H-pyrazol-3-yl]-3-[4-(2-(4-methylaminobenzimidazol-1-yl)ethoxy)naphthalen-1-yl]-urea;
1-[5-*tert*-Butyl-2-methyl-2H-pyrazol-3-yl]-3-[4-(2-(2-imidazo[4,5-b]pyridin-1-yl)ethoxy)naphthalen-1-yl]-urea;
1-[5-tert-Butyl-2-methyl-2H-pyrazol-3-yl]-3-[4-(2-[1,8]naphthyridin-4-yl)ethoxy)naphthalen-1-yl]-u rea;
1-[5-*tert*-Butyl-2-methyl-2H-pyrazol-3-yl]-3-[4-(2-(3,4-dihydro-2H-pyrano[2,3-b]pyridin-5-yl)ethoxy)naphthalen-1-yl]-urea
and their physiologically acceptable acids or salts thereof.

In a particularily preferred embodiment the invention relates to pharmaceutical compositions containing A and B, characterized in that the p38 kinase inhibitor B is selected from the following compounds of formula 4 as disclosed in WO 00/43384:
1-[5-*tert*-Butyl-2-*p*-tolyl-2H-pyrazol-3-yl]-3-[4-(2-morpholin-4-yl-ethoxy)naphthalen-1-yl]-urea;
1-[5-*tert*-Butyl-2-*p*-tolyl-2H-pyrazol-3-yl]-3-[4-(2-(*cis*-2,6-dimethylmorpholin-4-yl)ethoxy)naphthalen-1-yl]-urea;
1-(5-*tert*-Butyl-2-*p*-tolyl-2H-pyrazol-3-yl]-3-[4-(2-(*trans*-2,6-dimethylmorpholin-4-yl)ethoxy)naphthalen-1-yl]-urea;
1-[5-*tert*-Butyl-2-*p*-tolyl-2H-pyrazol-3-yl]-3-[4-(2-(2-(methoxymethyl)morpholin-4-yl)ethoxy)naphthalen-1-yl]-urea;
1-[5-*tert*-Butyl-2-*p*-tolyl-2H-pyrazol-3-yl]-3-[4-(2-(morpholin-4-yl)-2-oxoethoxy)naphthalen-1-yl]-urea;
1-[5-*tert*-Butyl-2-*p*-tolyl-2H-pyrazol-3-yl]-3-[4-(2-(morpholin-4-yl)-2-methylethoxy)naphthalen-1-yl]-u rea;
1-[5-*tert*-Butyl-2-*p*-tolyl-2H-pyrazol-3-yl]-3-[4-(2-(morpholin-4-yl)-1-methylethoxy)naphthalen-1-yl]-urea;
1-[5-*tert*-Butyl-2-*p*-tolyl-2H-pyrazol-3-yl]-3-[4-(2-thiomorpholin-4-yl-ethoxy)naphthalen-1-yl]-urea;
1-[5-*tert*-Butyl-2-*p*-tolyl-2H-pyrazol-3-yl]-3-[4-(2-(1-oxothiomorpholin-4-yl)ethoxy)naphthalen-1-yl]-urea;
1-[5-*tert*-Butyl-2-*p*-tolyl-2H-pyrazol-3-yl]-3-[4-(2-morpholin-4-yl-ethoxy)-3-methylnaphthalen-1-yl]-urea;
1-[5-*tert*-Butyl-2-*p*-tolyl-2H-pyrazol-3-yl]-3-[4-(2-(morpholin-4-yl-carbonyloxo)ethoxy)naphthalen-1-yl]-urea;
1-[5-*tert*-Butyl-2-*p*-tolyl-2H-pyrazol-3-yl]-3-[4-(2-(tetrahydropyran-4-yl)ethoxy)naphthalen-1-yl]-urea;
1-[5-*tert*-Butyl-2-*p*-tolyl-2H-pyrazol-3-yl]-3-[4-(2-(1-oxo-tetrahydrothiophen-3-yl)ethoxy)naphthalen-1-yl]-urea;
1-[5-*tert*-Butyl-2-*p*-tolyl-2H-pyrazol-3-yl]-3-[4-(3-morpholin-4-yl-propyl)naphthalen-1-yl]-urea;
1-[5-*tert*-Butyl-2-*p*-tolyl-2H-pyrazol-3-yl]-3-[4-(morpholin-4-yl-methyl)naphthalen-1-yl]-urea;
1-[5-*tert*-Butyl-2-*p*-tolyl-2H-pyrazol-3-yl]-3-[4-(2-pyridin-4-yl-ethyl)naphthalen-1-yl]-urea;
1-[5-*tert*-Butyl-2-p-tolyl-2H-pyrazol-3-yl]-3-[4-(3-(morpholin-4-yl)propyn-1-yl)naphthalen-1-yl]-urea;
1-[5-*tert*-Butyl-2-*p*-tolyl-2H-pyrazol-3-yl]-3-[4-(3-(tetrahydropyran-2-yl-oxy)propyn-1-yl)naphthalen-1-yl]-urea;
1-[5-*tert*-Butyl-2-*p*-tolyl-2H-pyrazol-3-yl]-3-[4-(3-(tetrahydropyran-2-yl-oxy)butyn-1-yl)naphthalen-1-yl]-urea;
1-[5-*tert*-Butyl-2-*p*-tolyl-2H-pyrazol-3-yl]-3-[4-(3-(piperdin-1-yl)propyn-1-yl)naphthalen-1-yl]-urea;
1-[5-*tert*-Butyl-2-*p*-tolyl-2H-pyrazol-3-yl]-3-[4-(3-(2-methoxymethylmorpholin-4-yl)propyn-1-yl)naphthalen-1-yl]-urea;
1-[5-*tert*-Butyl-2-*p*-tolyl-2H-pyrazol-3-yl]-3-[4-(pyridin-4-yl-methoxy)naphthalen-1-yl]-urea;
1-[5-*tert*-Butyl-2-*p*-tolyl-2H-pyrazol-3-yl]-3-[4-(2-pyridin-4-yl-ethoxy)naphthalen-1-yl]-urea;
1-[5-*tert*-Butyl-2-*p*-tolyl-2H-pyrazol-3-yl]-3-[4-(3-pyridin-4-yl-propoxy)naphthalen-1-yl]-urea;
1-[5-*tert*-Butyl-2-*p*-tolyl-2H-pyrazol-3-yl]-3-[4-(2-imidazol-1-yl-ethoxy)naphthalen-1-yl]-urea;
1-[5-*tert*-Butyl-2-*p*-tolyl-2H-pyrazol-3-yl]-3-[4-(2-(3,4-dimethoxyphenyl)-ethoxy)naphthalen-1-yl]-urea;
1-[5-*tert*-Butyl-2-*p*-tolyl-2H-pyrazol-3-yl]-3-[4-(pyridin-4-yl-methylamino)naphthalen-1-yl]-urea;
1-[5-*iso*-Propyl-2-phenyl-2H-pyrazol-3-yl]-3-[4-(2-morpholin-4-yl-ethoxy)naphthalen-1-yl]-urea;
1-[5-cyclohexyl-2-phenyl-2H-pyrazol-3-yl]-3-[4-(2-morpholin-4-yl-ethoxy)naphthalen-1-yl]-urea;
1-[5-(2,2,2-trifluoroethyl)-2-phenyl-2H-pyrazol-3-yl]-3-[4-(2-morpholin-4-yl-ethoxy)naphthalen-1-yl]-urea;
1-[5-(1-methylcycloprop-1-yl)-2-phenyl-2H-pyrazol-3-yl]-3-[4-(2-morpholin-4-yl-ethoxy)naphthalen-1-yl]-urea;
1-[5-(1-methylcyclohex-1-yl)-2-phenyl-2H-pyrazol-3-yl]-3-[4-(2-morpholin-4-yl-ethoxy)naphthalen-1-yl]-urea;
1-[5-*tert*-butyl-2-methyl-2H-pyrazol-3-yl]-3-[4-(2-morpholin-4-yl-ethoxy)naphthalen-1-yl]-urea;
1-[5-tert-butyl-2-(4-chlorophenyl)-2H-pyrazol-3-yl]-3-[4-(2-morpholin-4-yl-ethoxy)naphthalen-1-yl]-urea;
1-[5-*tert*-butyl-2-butyl-2H-pyrazol-3-yl]-3-[4-(2-morpholin-4-yl-ethoxy)naphthalen-1-yl]-urea;
1-[5-*tert*-butyl-2-(4-methyl-3-carbamylphenyl)-2H-pyrazol-3-yl]-3-[4-(2-morpholin-4-yl-ethoxy)naphthalen-1-yl]-urea;
1-[5-tert-butyl-2-(4-methyl-3-(morpholin-4-yl)methylphenyl)-2H-pyrazol-3-yl]-3-[4-(2-morpholin-4-yl-ethoxy)naphthalen-1-yl]-urea;
1-[5-tert-butyl-2-(4-methyl-3-dimethylaminomethylphenyl)-2H-pyrazol-3-yl]-3-[4-(2-morpholin-4-yl-ethoxy)naphthalen-1-yl]-urea;
1-[5-*tert*-butyl-2-(3-dimethylaminomethylphenyl)-2H-pyrazol-3-yl]-3-[4-(2-morpholin-4-yl-ethoxy)naphthalen-1-yl]-urea;
1-[5-*tert*-butyl-2-(2-chloropyridin-5-yl)-2H-pyrazol-3-yl]-3-[4-(2-morpholin-4-yl-ethoxy)naphthalen-1-yl]-urea;
1-[5-tert-butyl-2-(2-methylpyridin-5-yl)-2H-pyrazol-3-yl]-3-[4-(2-morpholin-4-yl-ethoxy)naphthalen-1-yl]-urea;
1-[5-*tert*-butyl-2-(2-methoxypyridin-5-yl)-2H-pyrazol-3-yl]-3-[4-(2-morpholin-4-yl-ethoxy)naphthalen-1-yl]-urea;
1-[5-*tert*-butyl-2-(pyridin-3-yl)-2H-pyrazol-3-yl]-3-[4-(2-morpholin-4-yl-ethoxy)naphthalen-1-yl]-urea;
1-[5-*tert*-butyl-2-(2-methylpyridin-5-yl)-2H-pyrazol-3-yl]-3-[4-(2-pyridin-4-yl-ethoxy)naphthalen-1-yl]-urea;
1-[5-*tert*-butyl-2-(2-methylpyridin-5-yl)-2H-pyrazol-3-yl]-3-[4-(2-(*trans*-2,6-dimethylmorpholin-4-yl)ethoxy)naphthalen-1-yl]-urea;
1-[5-*tert*-butyl-2-(2-methylpyridin-5-yl)-2H-pyrazol-3-yl]-3-[4-(3-morpholin-4-yl-propyn-1-yl)naphthalen-1-yl]-urea.

Particularly preferred p38 kinase inhibitors B within the scope of the present invention are the following compounds of the formula **4** :
1-[5-*tert*-Butyl-2-*p*-tolyl-2H-pyrazol-3-yl]-3-[4-(2-morpholin-4-yl-ethoxy)naphthalen-1-yl]-urea;
1-[5-*tert*-Butyl-2-*p*-tolyl-2H-pyrazol-3-yl]-3-[4-(2-(1-oxothiomorpholin-4-yl)ethoxy)naphthalen-1-yl]-urea;
1-[5-*tert*-butyl-2-(2-methylpyridin-5-yl)-2H-pyrazol-3-yl]-3-[4-(2-pyridin-4-yl-ethoxy)naphthalen-1-yl]-urea;
1-[5-*tert*-butyl-2-(2-methoxypyridin-5-yl)-2H-pyrazol-3-yl]-3-[4-(2-morpholin-4-yl-ethoxy)naphthalen-1-yl]-urea or
1-[5-*tert*-butyl-2-methyl-2H-pyrazol-3-yl]-3-[4-(2-morpholin-4-yl-ethoxy)naphthalen-1-yl]-urea.

In another preferred embodiment the invention relates to pharmaceutical compositions containing **A** and **B**, characterized in that the p38 kinase inhibitor **B** is selected from the compounds of formula **5** as disclosed in WO 00/55139 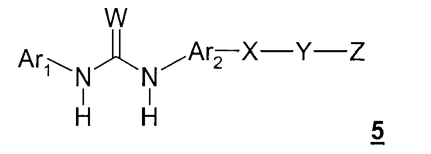 wherein:
- Ar₁: is selected from the group consisting of:
pyrrole, pyrrolidine, pyrazole, imidazole, oxazole, thiazole, furan and thiophene;
wherein Ar₁ may be substituted by one or more R₁, R₂ or R₃;
- Ar₂: is:
phenyl, naphthyl, quinoline, isoquinoline, tetrahydronaphthyl, tetrahydroquinoline, tetrahydroisoquinoline, benzimidazole, benzofuran, indanyl, indenyl or indole each being optionally substituted with zero to three R₂ groups;
- X: is:
a) a C₅₋₈ cycloalkyl or cycloalkenyl optionally substituted with 0-2 oxo group or 0-3 C₁₋₄ branched or unbranched alkyl, C₁₋₄ alkoxy or C₁₋₄ alkylamino chains;
b) phenyl, furan, thiophene, pyrrole, imidazolyl, pyridine, pyrimidine, pyridinone, dihydropyridinone, maleimide, dihydromaleimide, piperdine, piperazine or pyrazine each being optionally independently substituted with 0-3 C₁₋₄ branched or unbranched alkyl, C₁₋₄alkoxy, hydroxy, nitrile, mono- or di-(C₁₋₃ alkyl)amino, C₁₋₆ alkyl-S(O)ₘ, or halogen;
- Y: is:
a bond or a C₁₋₄ saturated or unsaturated branched or unbranched carbon chain optionally partially or fully halogenated, wherein one or more methylene groups are optionally replaced by O, NH, S(O), S(O)₂ or S and wherein Y is optionally independently substituted with 0-2 oxo groups and one or more C₁₋₄ branched or unbranched alkyl which may be substituted by one or more halogen atoms;
- Z: is:
a) phenyl, pyridine, pyrimidine, pyridazine, imidazole, furan, thiophene, pyran, which are optionally substituted with one to three groups consisting of halogen, C₁₋₆ alkyl, C₁₋₆ alkoxy, hydroxy, mono- or di-(C₁₋₃ alkyl)amino, C₁₋₆ alkyl-S(O)ₘ , COOH and phenylamino wherein the phenyl ring is optionally substituted with one to two groups consisting of halogen, C₁₋₆ alkyl and C₁₋₆ alkoxy;
b) tetrahydropyran, tetrahydrofuran, 1,3-dioxolanone, 1,3-dioxanone, 1,4-dioxane, morpholine, thiomorpholine, thiomorpholine sulfoxide, piperidine, piperidinone, piperazine, tetrahydropyrimidone, cyclohexanone, cyclohexanol, pentamethylene sulfide, pentamethylene sulfoxide, pentamethylene sulfone, tetramethylene sulfide, tetramethylene sulfoxide or tetramethylene sulfone which are optionally substituted with one to three groups consisting of nitrile, C₁₋₆ alkyl, C₁₋₆ alkoxy, hydroxy, mono- or di-(C₁₋₃ alkyl)amino-C₁₋₃ alkyl, phenylamino-C₁₋₃ alkyl and C₁₋₃ alkoxy-C₁₋₃ alkyl;
c) C₁₋₆ alkoxy, secondary or tertiary amine wherein the amino nitrogen is covalently bonded to groups selected from the group consisting of C₁₋₃ alkyl, C₁₋₅ alkoxyalkyl, pyridinyl-C₁₋₃ alkyl, imidazolyl-C₁₋₃ alkyl, tetrahydrofuranyl-C₁₋₃ alkyl, phenylamino, wherein the phenyl ring is optionally substituted with one to two halogen, C₁₋₆ alkoxy, hydroxy or mono- or di-(C₁₋₃ alkyl)amino, C₁₋₆ alkyl-S(O)ₘ, and phenyl-S(O)ₘ, wherein the phenyl ring is optionally substituted with one to two halogen, C₁₋₆ alkoxy, hydroxy or mono- or di-(C₁₋₃ alkyl)amino;
- R₁: is :
a) C₃₋₁₀ branched or unbranched alkyl optionally partially or fully halogenated and optionally substituted with one to three phenyl, naphthyl or heterocyclic groups selected from the group consisting of pyridinyl, pyrimidinyl, pyrazinyl, pyridazinyl, pyrrolyl, imidazolyl, pyrazolyl, thienyl, furyl, isoxazolyl and isothiazolyl; each such phenyl, naphthyl or heterocycle selected from the group hereinabove described in this paragraph, and being substituted with 0 to 5 groups selected from the group consisting of halogen, C₁₋₆ branched or unbranched alkyl which is optionally partially or fully halogenated, C₃₋₈ cycloalkyl, C₅₋₈ cycloalkenyl, hydroxy, nitrile, C₁₋₃ alkyloxy which is optionally partially or fully halogenated, NH₂C(O) and di(C₁₋₃)alkylaminocarbonyl;
b) C₃₋₇ cycloalkyl selected from the group consisting of cyclopropyl, cyclobutyl, cyclopentanyl, cyclohexanyl, cycloheptanyl, bicyclopentanyl, bicyclohexanyl and bicycloheptanyl each being optionally be partially or fully halogenated and optionally substituted with one to three C₁₋₃ alkyl groups, or an analog of such cycloalkyl group wherein one to three ring methylene groups are replaced by groups independently selected from the group consisting of O, S, CHOH, >C=O, >C=S and NH;
c) C₃₋₁₀ branched alkenyl optionally partially or fully halogenated and optionally substituted with one to three C₁₋₅ branched or unbranched alkyl, phenyl, naphthyl or heterocyclic groups, with each such heterocyclic group being independently selected from the group consisting of pyridinyl, pyrimidinyl, pyrazinyl, pyridazinyl, pyrrolyl, imidazolyl, pyrazolyl, thienyl, furyl, isoxazolyl and isothiazolyl, and each such phenyl, naphthyl or heterocyclic group being substituted with 0 to 5 groups selected from the group consisting of halogen, C₁₋₆ branched or unbranched alkyl which is optionally partially or fully halogenated, cyclopropyl, cyclobutyl, cyclopentanyl, cyclohexanyl, cycloheptanyl, bicyclopentanyl, bicyclohexanyl, bicycloheptanyl, hydroxy, nitrile, C₁₋₃ alkoxy which is optionally partially or fully halogenated, NH₂C(O) and mono- or di(C₁₋₃)alkylaminocarbonyl;
d) a C₅₋₇ cycloalkenyl selected from the group consisting of cyclopentenyl, cyclohexenyl, cyclohexadienyl, cycloheptenyl, cycloheptadienyl, bicyclohexenyl and bicycloheptenyl, wherein such cycloalkenyl group is optionally substituted with one to three C₁₋₃ alkyl groups;
e) nitrile; or
f) C₁₋₆ branched or unbranched alkoxycarbonyl, C₁₋₆ branched or unbranched alkylaminocarbonyl, C₁₋₆ branched or unbranched alkylcarbonylamino-C₁₋₃-alkyl;
- R₂: is:
a C₁₋₆ branched or unbranched alkyl optionally partially or fully halogenated, acetyl, aroyl, C₁₋₄ branched or unbranched alkoxy optionally partially or fully halogenated, halogen, methoxycarbonyl or phenylsulfonyl;
- R₃: is:
a) phenyl, naphthyl or heterocyclic group selected from the group consisting of pyridinyl, pyrimidinyl, pyrazinyl, pyridazinyl, pyrrolyl, imidazolyl, pyrazolyl, thienyl, furyl, tetrahydrofuryl, isoxazolyl, isothiazolyl, quinolinyl, isoquinolinyl, indolyl, benzimidazolyl, benzofuranyl, benzoxazolyl, benzisoxazolyl, benzpyrazolyl, benzothiofuranyl, cinnolinyl, pterindinyl, phthalazinyl, naphthypyridinyl, quinoxalinyl, quinazolinyl, purinyl and indazolyl, wherein such phenyl, naphthyl or heterocyclic group is optionally substituted with one to five groups selected from the group consisting of phenyl, naphthyl, heterocycle selected from the group hereinabove described in this paragraph, C₁₋₆ branched or unbranched alkyl which is optionally partially or fully halogenated, cyclopropyl, cyclobutyl, cyclopentyl, cyclohexyl, cycloheptyl, bicyclopentyl, bicyclohexyl, bicycloheptyl, phenyl C₁₋₅ alkyl, naphthyl C₁₋₅ alkyl, halogen, hydroxy, nitrile, C₁₋₃ alkyloxy which may optionally be partially or fully halogenated, phenyloxy, naphthyloxy, heteraryloxy wherein the heterocyclic moiety is selected from the group hereinabove described in this paragraph, nitro, amino, mono- or di-(C₁₋₃)alkylamino, phenylamino, naphthylamino, heterocyclylamino wherein the heterocyclyl moiety is selected from the group hereinabove described in this paragraph, NH₂C(O), a mono- or di-(C₁₋₃)alkyl aminocarbonyl, C₁₋₅ alkyl-C(O)-C₁₋₄ alkyl, amino-C₁₋₅ alkyl, mono- or di-(C₁₋₃)alkylamino-C₁₋₅ alkyl, amino-S(O)₂, di-(C₁₋₃)alkylamino-S(O)₂, R₄-C₁₋₅alkyl, R₅-C₁₋₅alkoxy, R₆-C(O)-C₁₋₅ alkyl and R₇-C₁₋₅ alkyl(R₈)N, carboxy-mono- or di-(C₁₋₅)-alkyl-amino;
b) a fused aryl selected from the group consisting of benzocyclobutanyl, indanyl, indenyl, dihydronaphthyl, tetrahydronaphthyl, benzocycloheptanyl and benzocycloheptenyl, or a fused heterocyclyl selected from the group consisting of cyclopentenopyridine, cyclohexanopyridine, cyclopentanopyrimidine, cyclohexanopyrimidine, cyclopentanopyrazine, cyclohexanopyrazine, cyclopentanopyridazine, cyclohexanopyridazine, cyclopentanoquinoline, cyclohexanoquinoline, cyclopentanoisoquinoline, cyclohexanoisoquinoline, cyclopentanoindole, cyclohexanoindole, cyclopentanobenzimidazole, cyclohexanobenzimidazole, cyclopentanobenzoxazole, cyclohexanobenzoxazole, cyclopentanoimidazole, cyclohexanoimidazole, cyclopentanothiophene and cyclohexanothiophene; wherein the fused aryl or fused heterocyclyl ring is substituted with 0 to 3 groups independently selected from the group consisting of phenyl, naphthyl and heterocyclyl selected from the group consisting of pyridinyl, pyrimidinyl, pyrazinyl, pyridazinyl, pyrrolyl, imidazolyl, pyrazolyl, thienyl, furyl, isoxazolyl, and isothiazolyl, C₁₋₆ branched or unbranched alkyl which is optionally partially or fully halogenated, halogen, nitrile, C₁₋₃ alkoxy which is optionally partially or fully halogenated, phenyloxy, naphthyloxy, heterocyclyloxy wherein the heterocyclyl moiety is selected from the group hereinabove described in this paragraph, nitro, amino, mono- or di-(C₁₋₃)alkylamino, phenylamino, naphthylamino, heterocyclylamino wherein the heterocyclyl moiety is selected from the group hereinabove described in this paragraph, NH₂C(O), a mono- or di-(C₁₋₃)alkyl aminocarbonyl, C₁₋₄ alkyl-OC(O), C₁₋₅ alkyl-C(O)-C₁₋₄ branched or unbranched alkyl, an amino-C₁₋₅ alkyl, mono-or di-(C₁₋₃)alkylamino-C₁₋₅ alkyl, R₉-C₁₋₅ alkyl, R₁₀ -C₁₋₅ alkoxy, R₁₁ -C(O)-C₁₋₅ alkyl, and R₁₂-C₁₋₅ alkyl(R₁₃)N;
c) cycloalkyl selected from the group consisting of cyclopentyl, cyclohexyl, cycloheptyl, bicyclopentyl, bicyclohexyl and bicycloheptyl, wherein the cycloalkyl is optionally partially or fully halogenated and optionally substituted with one to three C₁₋₃ alkyl groups;
d) C₅₋₇ cycloalkenyl selected from the group consisting of cyclopentenyl, cyclohexenyl, cyclohexadienyl, cycloheptenyl, cycloheptadienyl, bicyclohexenyl and bicycloheptenyl, wherein such cycloalkenyl group is optionally substituted with one to three C₁₋₃ alkyl groups;
e) acetyl, aroyl, alkoxycarbonylalkyl or phenylsulfonyl; or
f) C₁₋₆ branched or unbranched alkyl optionally partially or fully halogenated;
or R₁ and R₂ taken together may optionally form a fused phenyl or pyridinyl ring;
each R₈ and R₁₃ is independently selected from the group consisting of:
hydrogen and C₁₋₄ branched or unbranched alkyl optionally be partially or fully halogenated;
each R₄, R₅, R₆, R₇, R₉, R₁₀, R₁₁ and R₁₂ is independently selected from the group consisting of morpholine, piperidine, piperazine, imidazole and tetrazole;
m is 0, 1 or 2;
W is O or S and pharmaceutically acceptable derivatives thereof.

In another preferred embodiment the invention relates to pharmaceutical compositions containing **A** and **B,** characterized in that the p38 kinase inhibitor **B** is selected from the compounds of formula **5** as disclosed in WO 00/55139
wherein:
- Ar₂: is naphthyl, tetrahydronaphthyl, indanyl or indenyl and
W is O.

In another preferred embodiment the invention relates to pharmaceutical compositions containing **A** and **B**, characterized in that the p38 kinase inhibitor **B** is selected from the compounds of formula **5** as disclosed in WO 00/55139
wherein:
- Ar₁: is selected from thiophene and pyrazole;
- X: is C₅₋₇ cycloalkyl or C₅₋₇cycloalkenyl optionally substituted with 0-2 oxo groups or 0-3 C₁₋₄ branched or unbranched alkyl, C₁₋₄ alkoxy or C₁₋₄ alkylamino; or
X is phenyl, pyridine, tetrahydropyridine, pyrimidine, furan or thiophene each being optionally independently substituted with 0-3 C₁₋₄ branched or unbranched alkyl, C₁₋₄alkoxy, hydroxy, nitrile, mono- or di-(C₁₋₃ alkyl)amino, C₁₋₆ alkyl-S(O)ₘ or halogen;
- R₁: is C₁₋₄alkyl branched or unbranched, cyclopropyl or cyclohexyl optionally partially or fully halogenated and optionally substituted with one to three C₁₋₃ alkyl groups;
- R₃: is C₁₋₄alkyl branched or unbranched, phenyl, pyrimidinyl, pyrazolyl or pyridinyl each being optionally substituted as described hereinabove in the broadest generic aspect, alkoxycarbonylalkyl or cyclopropyl or cyclopentyl optionally substituted as described hereinabove in the broadest generic aspect.

In yet another preferred embodiment the invention relates to pharmaceutical compositions containing **A** and **B**, characterized in that the p38 kinase inhibitor **B** is selected from the compounds of formula **5** as disclosed in WO 00/55139
wherein:
- Ar₁: is pyrazole;
- X: is cyclopentenyl, cyclohexenyl or cycloheptenyl, optionally substituted with an oxo group or 0-3 C₁₋₄ branched or unbranched alkyl, C₁₋₄alkoxy or C₁₋₄alkylamino; or X is phenyl, pyridine, furan or thiophene each being optionally independently substituted with 0-3 C₁₋₄ branched or unbranched alkyl, C₁₋₄alkoxy, hydroxy, nitrile, mono- or di-(C₁₋₃ alkyl)amino, C₁₋₆ alkyl-S(O)ₘ or halogen.

In yet still another preferred embodiment the invention relates to pharmaceutical compositions containing **A** and **B**, characterized in that the p38 kinase inhibitor B is selected from the compounds of formula **5** as disclosed in WO 00/55139
wherein:
- Y: is -CH2-, -CH2CH2-, -CH2NH-, -CH2CH2NH- or a bond;
and
- Z: is phenyl, imidazole, furan, piperazine, tetrahydropyran, morpholine, thiomorpholine, thiomorpholine sulfoxide, piperidine, pyridine, secondary or tertiary amine wherein the amino nitrogen is covalently bonded to groups selected from the group consisting of C₁₋₃ alkyl and C₁₋₅ alkoxyalkyl, phenylamino wherein the phenyl ring is optionally substituted with one to two halogen, C₁₋₆ alkoxy, hydroxy or mono- or di-(C₁₋₃ alkyl)amino, C₁₋₆ alkyl-S(O)ₘ and phenyl-S(O)ₘ wherein the phenyl ring is optionally substituted with one to two halogen, C₁₋₆ alkoxy, hydroxy or mono- or di-(C₁₋₃ alkyl)amino.

In a further embodiment the invention relates to pharmaceutical compositions containing **A** and **B**, characterized in that the p38 kinase inhibitor **B** is selected from the compounds of formula **5** as disclosed in WO 00/55139 wherein:
- Ar₁: is 5-*tert*-butyl-pyrazol-3-yl; wherein the pyrazole ring may be substituted by R₃;
- R₃: is C₁₋₄alkyl branched or unbranched, phenyl, pyrimidinyl, pyrazolyl, pyridinyl each being optionally substituted as described hereinabove in the broadest generic aspect, alkoxycarbonylalkyl or cyclopropyl or cyclopentyl optionally substituted as described hereinabove in the broadest generic aspect.

In another preferred embodiment the invention relates to pharmaceutical compositions containing **A** and **B**, characterized in that the p38 kinase inhibitor **B** is selected from the compounds of formula **5** as disclosed in WO 00/55139
wherein X is pyridinyl.

In another preferred embodiment the invention relates to pharmaceutical compositions containing **A** and **B**, characterized in that the p38 kinase inhibitor **B** is selected from the compounds of formula **5** as disclosed in WO 00/55139
wherein the pyridinyl is attached to Ar₁ via the 3-pyridinyl position.

In another preferred embodiment the invention relates to pharmaceutical compositions containing **A** and **B**, characterized in that the p38 kinase inhibitor **B** is selected from the compounds of formula **5** as disclosed in WO 00/55139 that are mentioned below:
1-[5-*tert*-butyl-2-*p*-tolyl-2H-pyrazol-3-yl]-3-[4-(4-(morpholin-4-yl)phenyl)naphthalen-1-yl]urea;
1-[5-*tert*-butyl-2-*p*-tolyl-2H-pyrazol-3-yl]-3-[4-(4-(morpholin-4-yl-methyl)phenyl)naphthalen-1-yl]urea;
1-[5-*tert*-butyl-2-*p*-tolyl-2H-pyrazol-3-yl]-3-[4-(4-(2-(morpholin-4-yl)ethyl)phenyl)naphthalen-1-yl]urea;
1-[5-*tert*-butyl-2-*p*-tolyl-2H-pyrazol-3-yl]-3-[4-(4-dimethylaminophenyl)naphthalen-1-yl]urea;
1-[5-*tert*-butyl-2-*p*-tolyl-2H-pyrazol-3-yl]-3-[4-(3-(morpholin-4-yl)phenyl)naphthalen-1-yl]urea;
1-[5-*tert*-butyl-2-*p*-tolyl-2H-pyrazol-3-yl]-3-[4-(3-(morpholin-4-yl-methyl)phenyl)naphthalen-1-yl]urea;
1-[5-tert-butyl-2-p-tolyl-2H-pyrazol-3-yl]-3-[4-(6-morpholin-4-ylmethyl-pyridin-3-yl)naphthalen-1-yl]urea;
1-[5-*tert*-butyl-2-*p*-tolyl-2H-pyrazol-3-yl]-3-[4-(5-morpholin-4-ylmethyl-pyridin-2-yl)naphthalen-1-yl]urea;
1-[5-*tert*-butyl-2-*p*-tolyl-2H-pyrazol-3-yl]-3-[4-(5-morpholin-4-ylmethyl-fur-2-yl)naphthalen-1-yl]urea;
1-[5-*tert*-butyl-2-(6-methyl-pyridin-3-yl)-2H-pyrazol-3-yl]-3-[4-(6-morpholin-4-ylmethyl-pyridin-3-yl)naphthalen-1-yl]urea;
1-[5-*tert*-butyl-2-methyl-2H-pyrazol-3-yl]-3-[4-(6-morpholin-4-ylmethyl-pyridin-3-yl)naphthalen-1-yl]urea;
1-[5-*tert*-butyl-2-phenyl-2H-pyrazol-3-yl]-3-[4-(4-piperdin-1-ylmethyl-phenyl)naphthalen-1-yl]urea;
1-[5-*tert*-butyl-2-phenyl-2H-pyrazol-3-yl]-3-[4-(4-(4-methylpiperazin-1-yl)methylphenyl)naphthalen-1-yl]urea;
1-[5-*tert*-butyl-2-*p*-tolyl-2H-pyrazol-3-yl]-3-[4-(3,4-di(morpholin-4-yl-methyl)phenyl)naphthalen-1-yl]urea;
1-[5-*tert*-butyl-2-(6-methyl-pyridin-3-yl)-2H-pyrazol-3-yl]-3-[4-(6-pyridin-4-ylmethyl-pyridin-3-yl)naphthalen-1-yl]urea;
1-[5-*tert*-butyl-2-(6-methyl-pyridin-3-yl)-2H-pyrazol-3-yl]-3-[4-(6-(1-oxo-thiomorpholin-4-ylmethyl)pyridin-3-yl)naphthalen-1-yl]urea;
1-[5-tert-butyl-2-p-tolyl-2H-pyrazol-3-yl]-3-[4-(6-(1-oxo-thiomorpholin-4-ylmethyl)pyridin-3-yl)naphthalen-1-yl]urea;
1-[5-*tert*-butyl-2-(6-methyl-pyridin-3-yl)-2H-pyrazol-3-yl]-3-[4-(6-tetrahydropyran-4-ylmethyl-pyridin-3-yl)naphthalen-1-yl]urea;
1-[5-*tert*-butyl-2-(6-methyl-pyridin-3-yl)-2H-pyrazol-3-yl]-3-[4-(6-(1-oxo-tetrahydrothiophen-3-ylmethyl)pyridin-3-yl)naphthalen-1-yl]urea;
1-[5-*tert*-butyl-2-(6-methyl-pyridin-3-yl)-2H-pyrazol-3-yl]-3-[4-(6-(imidazol-1-ylmethyl)pyridin-3-yl)naphthalen-1-yl]urea;
1-[2-(3-d imethylaminomethylphenyl)-5-(1-methyl-cyclohexyl)-2H-pyrazol-3-yl]-3-[4-(6-morpholin-4-ylmethyl-pyridin-3-yl)naphthalen-1-yl]urea;
1-[2-(5-(1-methyl-cyclohexyl)-2-(6-methyl-pyridin-3-yl)-2H-pyrazol-3-yl]-3-[4-(6-morpholin-4-ylmethyl-pyridin-3-yl)naphthalen-1-yl]urea;
1-[5-*tert*-butyl-2-*p*-tolyl-2H-pyrazol-3-yl]-3-[4-(2-morpholin-4-ylmethyl-pyrimidin-5-yl)naphthalen-1-yl]urea;
1-[5-*tert*-butyl-2-(6-methyl-pyridin-3-yl)-2H-pyrazol-3-yl]-3-[4-(3-methoxy-5-(2-morpholin-4-yl-ethoxy)phenyl)naphthalen-1-yl]urea;
1-[5-*tert*-butyl-2-(6-methyl-pyridin-3-yl)-2H-pyrazol-3-yl]-3-[4-(3-(2-morpholin-4-yl-ethoxy)phenyl)naphthalen-1-yl]urea;
1-[5-tert-butyl-2-(6-methyl-pyridin-3-yl)-2H-pyrazol-3-yl]-3-[4-3-(dimethylamino)phenyl)naphthalen-1-yl]urea;
1-[5-*tert*-butyl-2-(6-methyl-pyridin-3-yl)-2H-pyrazol-3-yl]-3-[4-3-(methylsulfonyl)phenyl)naphthalen-1-yl]urea;
5-*tert*-butyl-3-{3-[4-(6-morpholin-4-ylmethyl-pyridin-3-yl)naphthalen-1-yl]ureido}thiophene-2-carboxylic acid methyl ester;
5-*tert*-butyl-3-{3-[4-(6-morpholin-4-ylmethyl-pyridin-3-yl)naphthalen-1-yl]ureido}thiophene-2-carboxylic acid methylamide;
5-*tert*-butyl-1-methyl-3-{3-[4-(6-morpholin-4-ylmethyl-pyridin-3-yl)naphthalen-1-yl]ureido}-1H-pyrrole-2-carboxylic acid methyl ester;
5-*tert*-butyl-1-methyl-3-{3-[4-(6-morpholin-4-ylmethyl-pyridin-3-yl)naphthalen-1-yl]ureido}-1H-pyrrole-2-carboxylic acid methylamide;
2-acetylamino N-(5-*tert*-butyl-3-{3-[4-(6-morpholin-4-ylmethyl-pyridin-3-yl)naphthalen-1-yl]ureido}thiophen-2-ylmethyl)acetamide;
1-[5-*tert*-butyl-2-*p*-tolyl-2H-pyrazol-3-yl]-3-[4-(3-morpholin-4-yl-cyclohex-1-enyl)naphthalen-1-yl]urea;
1-[5-*tert*-butyl-2-*p*-tolyl-2H-pyrazol-3-yl]-3-[4-(3-morpholin-4-yl-cylohept-1-enyl)naphthalen-1-yl]urea;
1-[5-*tert*-butyl-2-*p*-tolyl-2H-pyrazol-3-yl]-3-[4-(3-(2-morpholin-4-yl-ethylamino)cyclohex-1-enyl)naphthalen-1-yl]u rea;
1-[5-*tert*-butyl-2-*p*-tolyl-2H-pyrazol-3-yl]-3-[4-(3-morpholin-4-yl-cyclohept-1-enyl)naphthalen-1-yl]urea;
1-[5-*tert*-butyl-2-(6-methyl-pyridin-3-yl)-2H-pyrazol-3-yl]-3-[4-(3-(pyridin-4-yl-methylamino)cyclohex-1-enyl)naphthalen-1-yl]urea;
1-[5-*tert*-butyl-2-(6-methyl-pyridin-3-yl)-2H-pyrazol-3-yl]-3-[4-(3-(dimethylaminoethylamino)cyclohex-1-enyl)naphthalen-1-yl]urea;
1-[5-*tert*-butyl-2-(6-methyl-pyridin-3-yl)-2H-pyrazol-3-yl]-3-[4-(3-(pyridin-3-yl-methylamino)cyclohex-1-enyl)naphthalen-1-yl]urea;
1-[5-*tert*-butyl-2-(6-methyl-pyridin-3-yl)-2H-pyrazol-3-yl]-3-[4-(3-(phenyl-methylamino)cyclohex-1-enyl)naphthalen-1-yl]urea;
1-[5-*tert*-butyl-2-(6-methyl-pyridin-3-yl)-2H-pyrazol-3-yl]-3-[4-(3-(2-phenylethylamino)cyclohex-1-enyl)naphthalen-1-yl]urea;
1-[5-*tert*-butyl-2-(6-methyl-pyridin-3-yl)-2H-pyrazol-3-yl]-3-[4-(3-(furan-2-yl-methylamino)cyclohex-1-enyl)naphthalen-1-yl]urea;
1-[5-*tert*-butyl-2-(6-methyl-pyridin-3-yl)-2H-pyrazol-3-yl]-3-[4-(3-(2-pyridin-2-yl-ethylamino)cyclohex-1-enyl)naphthalen-1-yl]u rea;
1-[5-*tert*-butyl-2-(6-methyl-pyridin-3-yl)-2H-pyrazol-3-yl]-3-[4-(3-(2-piperdin-1-yl-ethylamino)cyclohex-1-enyl)naphthalen-1-yl]urea;
1-[5-*tert*-butyl-2-(6-methyl-pyridin-3-yl)-2H-pyrazol-3-yl]-3-[4-(3-(2-imidazol-4-yl-ethylamino)cyclohex-1-enyl)naphthalen-1-yl]urea;
1-[5-*tert*-butyl-2-(6-methyl-pyridin-3-yl)-2H-pyrazol-3-yl]-3-[4-(3-(pyridin-2-yl-methylamino)cyclohex-1-enyl)naphthalen-1-yl]urea;
1-[5-*tert*-butyl-2-(6-methyl-pyridin-3-yl)-2H-pyrazol-3-yl]-3-[4-(3-(2-(4-methoxyphenyl)ethylamino)cyclohex-1-enyl)naphthalen-1-yl]urea;
1-[5-*tert*-butyl-2-*p*-tolyl-2H-pyrazol-3-yl]-3-[4-(4-morpholin-4-ylmethyl-3-oxo-cyclohex-1-enyl)naphthalen-1-yl]urea;
1-[5-*tert*-butyl-2-*p*-tolyl-2H-pyrazol-3-yl]-3-[4-(4-( 1-oxo-tetrahydrothiophen-3-ylmethyl)-3-oxo-cyclohex-1-enyl)naphthalen-1-yl]urea;
1-[5-*tert*-butyl-2-*p*-tolyl-2H-pyrazol-3-yl]-3-[4-(4-(1-oxo-thiomorpholin-4-ylmethyl)-3-oxo-cyclohex-1-enyl)naphthalen-1-yl]urea;
1-[5-*tert*-butyl-2-*p*-tolyl-2H-pyrazol-3-yl]-3-[4-(4-methylpiperazin-1-ylmethyl)-3-oxo-cyclohex-1-enyl)naphthalen-1-yl]urea;
1-[5-*tert*-butyl-2-(6-methyl-pyridin-3-yl)-2H-pyrazol-3-yl]-3-[4-{6-oxo-1-(tetrahydropyran-4-ylmethyl)-1,2,3,6-tetrahydro-pyridin-4-yl}naphthalen-1-yl]urea;
1-[5-*tert*-butyl-2-(6-methyl-pyridin-3-yl)-2H-pyrazol-3-yl]-3-[4-(2-oxo-1-pyrid in-4-ylmethyl-piperdin-4-yl)naphthalen-1-yl]urea;
1-[5-*tert*-butyl-2-p-tolyl-2H-pyrazol-3-yl]-3-[4-(6-oxo-1-pyrid in-4-yl-1,2,3,6-tetrahyd ro-pyridin-4-yl)naphthalen-1-yl]urea;
1-[5-*tert*-butyl-2-(6-methyl-pyridin-3-yl)-2H-pyrazol-3-yl]-3-[4-(6-oxo-1-pyridin-4-yl-1,2,3,6-tetrahydro-pyridin-4-yl)naphthalen-1-yl]urea;
5-*tert*-butyl-3-{3-[4-(6-oxo-1-pyridin-4-yl-1,2,3,6-tetrahydro-pyridin-4-yl)naphthalen-1-yl]ureido}thiophene-2-carboxylic acid methyl ester;
5-*tert*-butyl)-1-methyl-3-{3-[4-(6-oxo-1-pyridin-4-yl-1,2,3,6-tetrahydro-pyridin-4-yl)naphthalen-1-yl]ureido}pyrrole-2-carboxylic acid methyl ester;
5-*tert*-butyl-1-methyl-3-{3-[4-(6-oxo-1-pyridin-4-yl-1,2,3,6-tetrahydro-pyridin-4-yl)naphthalen-1-yl]ureido}pyrrole-2-carboxylic acid methyl amide;
5-*tert*-butyl-3-{3-[4-(3-morpholin-4-yl-cyclohex-1-enyl)naphthalen-1-yl]ureido}thiophene-2-carboxylic acid methyl ester;
5-*tert*-butyl-1-methyl-3-{3-[4-(3-morpholin-4-yl-cyclohex-1-enyl)naphthalen-1-yl]ureido}pyrrole-2-carboxylic acid methyl ester; and
5-*tert*-butyl-1-methyl-3-{3-[4-(3-morpholin-4-yl-cyclohex-1-enyl)naphthalen-1-yl]ureido}pyrrole-2-carboxylic acid methyl amide and
the pharmaceutically acceptable derivatives thereof.

Preferably the invention relates to pharmaceutical compositions containing **A** and **B**, characterized in that the p38 kinase inhibitor **B** is selected from the following compounds of formula **5** :
1-[5-tert-butyl-2-p-tolyl-2H-pyrazol-3-yl]-3-[4-(4-(morpholin-4-yl-methyl)phenyl)naphthalen-1-yl]urea;
1-[5-tert-butyl-2-p-tolyl-2H-pyrazol-3-yl]-3-[4-(4-(2-(morpholin-4-yl)ethyl)phenyl)naphthalen-1-yl]urea;
1-[5-tert-butyl-2-p-tolyl-2H-pyrazol-3-yl]-3-[4-(3-(morpholin-4-yl-methyl)phenyl)naphthalen-1-yl]urea;
1-[5-tert-butyl-2-p-tolyl-2H-pyrazol-3-yl]-3-[4-(6-morpholin-4-ylmethyl-pyridin-3-yl)naphthalen-1-yl]urea;
1-[5-tert-butyl-2-p-tolyl-2H-pyrazol-3-yl]-3-[4-(5-morpholin-4-ylmethyl-pyridin-2-yl)naphthalen-1-yl]urea;
1-[5-tert-butyl-2-p-tolyl-2H-pyrazol-3-yl]-3-[4-(5-morpholin-4-ylmethyl-fur-2-yl)naphthalen-1-yl]urea;
1-[5-*tert*-butyl-2-(6-methyl-pyridin-3-yl)-2H-pyrazol-3-yl]-3-[4-(6-morpholin-4-ylmethyl-pyridin-3-yl)naphthalen-1-yl]urea;
1-[5-*tert*-butyl-2-methyl-2H-pyrazol-3-yl]-3-[4-(6-morpholin-4-ylmethyl-pyridin-3-yl)naphthalen-1-yl]urea and
the pharmaceutically acceptable derivatives thereof.

In another embodiment the invention relates to pharmaceutical compositions containing **A** and **B**, characterized in that the p38 kinase inhibitor **B** is selected from the compounds of formula **5a** as disclosed in WO 00/55139 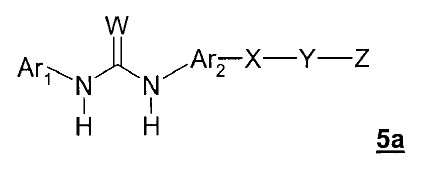 wherein:
- Ar₁: is:
pyrrole, pyrrolidine, pyrazole, imidazole, oxazole, thiazole, furan and thiophene;
wherein Ar₁ is optionally substituted by one or more R₁, R₂ or R₃;
- Ar₂: is:
phenyl, naphthyl, quinoline, isoquinoline, tetrahydronaphthyl, tetrahydroquinoline, tetrahydroisoquinoline, benzimidazole, benzofuran, indanyl, indenyl and indole each being optionally substituted with zero to three R₂ groups;
- X: is:
a C₅₋₈ cycloalkyl or cycloalkenyl optionally substituted with one to two oxo groups or one to three C₁₋₄ alkyl, C₁₋₄ alkoxy or C₁₋₄ alkylamino chains each being branched or unbranched;
phenyl, furanyl, thienyl, pyrrolyl, pyrazolyl, imidazolyl, pyridinyl, tetrahydropyridinyl, pyrimidinyl, pyridinonyl, dihydropyridinonyl, maleimidyl, dihydromaleimidyl, piperdinyl, benzimidazole, 3H-imidazo[4,5-b]pyridine, piperazinyl, pyridazinyl or pyrazinyl; each being optionally independently substituted with one to three C₁₋₄alkyl, C₁₋₄alkoxy, hydroxy, nitrile, amino, mono- or di-(C₁₋₃ alkyl)amino, mono- or di-(C₁₋₃ alkylamino)carbonyl, NH₂C(O), C₁₋₆ alkyl-S(O)ₘ or halogen;
- Y: is:
a bond or a C₁₋₄ saturated or unsaturated branched or unbranched carbon chain optionally partially or fully halogenated, wherein one or more C atoms are optionally replaced by O, N, or S(O)ₘ and wherein Y is optionally independently substituted with one to two oxo groups, nitrile, phenyl, hydroxy or one or more C₁₋₄ alkyl optionally substituted by one or more halogen atoms;
- Z: is:
aryl, indanyl, heteroaryl selected from benzimidazolyl, pyridinyl, pyrimidinyl, pyridazinyl, pyrazinyl, imidazolyl, pyrazolyl, triazolyl, tetrazolyl, furanyl, thienyl and pyranyl, heterocycle selected from piperazinyl, tetrahydropyrimidonyl, cyclohexanonyl, cyclohexanolyl, 2-oxa- or 2-thia-5-aza-bicyclo[2.2.1]heptanyl, pentamethylene sulfidyl, pentamethylene sulfoxidyl, pentamethylene sulfonyl, tetramethylene sulfidyl, tetramethylene sulfoxidyl or tetramethylene sulfonyl, tetrahydropyranyl, tetrahydrofuranyl, 1,3-dioxolanonyl, 1,3-dioxanonyl, 1,4-dioxanyl, morpholino, thiomorpholino, thiomorpholino sulfoxidyl, thiomorpholino sulfonyl, piperidinyl, piperidinonyl, pyrrolidinyl and dioxolanyl, each of the aforementioned Z are optionally substituted with one to three halogen, C₁₋₆ alkyl, C₁₋₆ alkoxy, C₁₋₃ alkoxy-C₁₋₃ alkyl, C₁₋₆ alkoxycarbonyl, aroyl, heteroaroyl, heterocycleC₁₋₃acyl wherein the heteroaryl and heterocycle are as defined hereinabove in this paragraph, C₁₋₃acyl, oxo, hydroxy, pyridinyl-C₁₋₃ alkyl, imidazolyl-C₁₋₃ alkyl, tetrahydrofuranyl-C₁₋₃ alkyl, nitrile-C₁₋₃ alkyl, nitrile, carboxy, phenyl wherein the phenyl ring is optionally substituted with one to two halogen, C₁₋₆ alkoxy, hydroxy or mono- or di-(C₁₋₃ alkyl)amino, amino-S(O)ₘ, C₁₋₆ alkyl-S(O)ₘ or phenyl-S(O)ₘ wherein the phenyl ring is optionally substituted with one to two halogen, C₁₋₆ alkoxy, hydroxy, halogen or mono- or di-(C₁₋₃ alkyl)amino;
or Z is optionally substituted with one to three amino, aminocarbonyl or amino-C₁₋₃ alkyl wherein the N atom is optionally independently mono- or di-substituted by aminoC₁₋₆alkyl, C₁₋₃alkyl, arylC₀₋₃alkyl, C₁₋₅ alkoxyC₁₋₃ alkyl, C₁₋₅ alkoxy, aroyl, C₁₋₃acyl, C₁₋₃alkyl-S(O)ₘ- or arylC₀₋₃alkyl-S(O)ₘ- each of the aforementioned alkyl and aryl attached to the amino group is optionally substituted with one to two halogen, C₁₋₆ alkyl, C₁₋₆ alkoxy, hydroxy or mono-or di-(C₁₋₃ alkyl)amino;
or Z is optionally substituted with one to three aryl, heterocycle or heteroaryl as hereinabove described in this paragraph each in turn is optionally substituted by halogen, C₁₋₆ alkyl or C₁₋₆ alkoxy;
or Z is hydroxy, hydroxyC₁₋₃alkyl, halogen, nitrile, amino wherein the N atom is optionally independently mono- or di-substituted by C₁₋₆alkyl, aminoC₁₋₆alkyl, arylC₀₋₃alkyl, C₁₋₅ alkoxyC₁₋₃ alkyl, C₁₋₅ alkoxy, aroyl, C₁₋₃acyl, C₁₋₃alkyl-S(O)ₘ-, arylC₀₋₃alkyl-S(O)ₘ-, nitrileC₁₋₄alkyl or C₁₋₃alkoxyC₁₋₃alkyl, each of the aforementioned alkyl and aryl attached to the amino group is optionally substituted with one to two halogen, C₁₋₆ alkyl, C₁₋₆ alkoxy, hydroxy or mono-or di-(C₁₋₃ alkyl)amino, C₁₋₆ alkoxyheteroarylC₀₋₃alkyl, heteroarylC₀₋₃alkyl or heterocycyleC₀₋₃alkyl wherein the heteroaryl and heterocycle is hereinabove described in this paragraph,
or Z is C₁₋₆alkyl branched or unbranched, C₁₋₆alkoxy, C₁₋₃acylamino,
nitrileC₁₋₄alkyl, C₁₋₆ alkyl-S(O)ₘ, and phenyl-S(O)ₘ, wherein the phenyl ring is optionally substituted with one to two halogen, C₁₋₆ alkoxy, hydroxy or mono-or di-(C₁₋₃ alkyl)amino;
- R₁: is:
a) C₁₋₁₀ branched or unbranched alkyl optionally partially or fully halogenated, and optionally substituted with one to three phenyl, naphthyl or heterocyclic groups selected from the group consisting of pyridinyl, pyrimidinyl, pyrazinyl, pyridazinyl, pyrrolyl, imidazolyl, pyrazolyl, thienyl, furyl, isoxazolyl and isothiazolyl; each such phenyl, naphthyl or heterocycle, selected from the group hereinabove described, being substituted with 0 to 5 groups selected from the group consisting of halogen, C₁₋₆ branched or unbranched alkyl which is optionally partially or fully halogenated, C₃₋₈ cycloalkyl, C₅₋₈ cycloalkenyl, hydroxy, nitrile, C₁₋₃ alkyloxy which is optionally partially or fully halogenated, NH₂C(O) and di(C₁₋₃)alkylaminocarbonyl;
b) C₃₋₇ cycloalkyl selected from the group consisting of cyclopropyl, cyclobutyl, cyclopentyl, cyclohexyl, cycloheptyl, bicyclopentyl, bicyclohexyl and bicycloheptyl, each optionally partially or fully halogenated and optionally substituted with one to three C₁₋₃ alkyl groups, or an analog of such cycloalkyl group wherein one to three ring methylene groups are replaced by groups independently selected from the group consisting of O, S, CHOH, >C=O, >C=S and NH;
c) C₃₋₁₀ branched alkenyl optionally partially or fully halogenated and optionally substituted with one to three C₁₋₅ branched or unbranched alkyl, phenyl, naphthyl or heterocyclic groups, with each such heterocyclic group being independently selected from the group consisting of pyridinyl, pyrimidinyl, pyrazinyl, pyridazinyl, pyrrolyl, imidazolyl, pyrazolyl, thienyl, furyl, isoxazolyl and isothiazolyl, and each such phenyl, naphthyl or heterocyclic group being substituted with 0 to 5 groups selected from the group consisting of halogen, C₁₋₆ branched or unbranched alkyl which is optionally partially or fully halogenated, cyclopropyl, cyclobutyl, cyclopentanyl, cyclohexanyl, cycloheptanyl, bicyclopentanyl, bicyclohexanyl, bicycloheptanyl, hydroxy, nitrile, C₁₋₃ alkoxy which is optionally partially or fully halogenated, NH₂C(O) and mono- or di(C₁₋₃)alkylaminocarbonyl;
d) a C₅₋₇ cycloalkenyl selected from the group consisting of cyclopentenyl, cyclohexenyl, cyclohexadienyl, cycloheptenyl, cycloheptadienyl, bicyclohexenyl and bicycloheptenyl, wherein such cycloalkenyl group is optionally substituted with one to three C₁₋₃ alkyl groups;
e) nitrile; or
f) C₁₋₆ branched or unbranched alkoxycarbonyl, C₁₋₆ branched or unbranched alkylaminocarbonyl, C₁₋₆ branched or unbranched alkylcarbonylamino-C₁₋₃-alkyl;
- R₂: is:
a C₁₋₆ branched or unbranched alkyl optionally partially or fully halogenated and optionally substituted with nitrile,
or R₂ is acetyl, aroyl, C₁₋₄ branched or unbranched alkoxy optionally partially or fully halogenated, halogen, methoxycarbonyl or phenylsulfonyl;
- R₃: is:
a) phenyl, naphthyl or heterocyclic group selected from the group consisting of pyridinyl, pyrimidinyl, pyrazinyl, pyridazinyl, pyrrolyl, imidazolyl, pyrazolyl, thienyl, furyl, tetrahydrofuryl, isoxazolyl, isothiazolyl, quinolinyl, isoquinolinyl, indolyl, benzimidazolyl, benzofuranyl, benzoxazolyl, benzisoxazolyl, benzpyrazolyl, benzothiofuranyl, cinnolinyl, pterindinyl, phthalazinyl, naphthypyridinyl, quinoxalinyl, quinazolinyl, purinyl and indazolyl, wherein such phenyl, naphthyl or heterocyclic group is optionally substituted with one to five groups selected from the group consisting of a phenyl, naphthyl, heterocycle selected from the group hereinabove described in this paragraph, C₁₋₆ branched or unbranched alkyl which is optionally partially or fully halogenated, cyclopropyl, cyclobutyl, cyclopentyl, cyclohexyl, cycloheptyl, bicyclopentyl, bicyclohexyl, bicycloheptyl, phenyl C₁₋₅ alkyl, naphthyl C₁₋₅ alkyl, halogen, hydroxy, oxo, nitrile, C₁₋₃ alkoxy optionally partially or fully halogenated, C₁₋₃ alkoxyC₁₋₅alkyl, C₁₋₃thioalkyl, C₁₋₃thioalkylC₁₋₅alkyl, phenyloxy, naphthyloxy, heteraryloxy wherein the heterocyclic moiety is selected from the group hereinabove described in this paragraph, nitro, amino, mono- or di-(C₁₋₃)alkylamino, phenylamino, naphthylamino, heterocyclylamino wherein the heterocyclyl moiety is selected from the group hereinabove described in this paragraph, NH₂C(O), a mono- or di-(C₁₋₃)alkyl aminocarbonyl, C₁₋₅ alkyl-C(O)-C₁₋₄ alkyl, amino-C₁₋₅ alkyl, mono- or di-(C₁₋₃)alkylamino-C₁₋₅ alkyl, amino-S(O)₂, di-(C₁₋₃)alkylamino-S(O)₂, R₄-C₁₋₅ alkyl, R₅-C₁₋₅ alkoxy, R₆―C(O)-C₁₋₅ alkyl and R₇-C₁₋₅ alkyl(R₈)N, carboxy-mono- or di-(C₁₋₅ )-alkyl-amino;
b) a fused aryl selected from the group consisting of benzocyclobutanyl, indanyl, indenyl, dihydronaphthyl, tetrahydronaphthyl, benzocycloheptanyl and benzocycloheptenyl, or a fused heterocyclyl selected from the group consisting of cyclopentenopyridine, cyclohexanopyridine, cyclopentanopyrimidine, cyclohexanopyrimidine, cyclopentanopyrazine, cyclohexanopyrazine, cyclopentanopyridazine, cyclohexanopyridazine, cyclopentanoquinoline, cyclohexanoquinoline, cyclopentanoisoquinoline, cyclohexanoisoquinoline, cyclopentanoindole, cyclohexanoindole, cyclopentanobenzimidazole, cyclohexanobenzimidazole, cyclopentanobenzoxazole, cyclohexanobenzoxazole, cyclopentanoimidazole, cyclohexanoimidazole, cyclopentanothiophene and cyclohexanothiophene; wherein the fused aryl or fused heterocyclyl ring is substituted with 0 to 3 groups independently selected from the group consisting of phenyl, naphthyl and heterocyclyl selected from the group consisting of pyridinyl, pyrimidinyl, pyrazinyl, pyridazinyl, pyrrolyl, imidazolyl, pyrazolyl, thienyl, furyl, isoxazolyl, and isothiazolyl, C₁₋₆ branched or unbranched alkyl which is optionally partially or fully halogenated, halogen, nitrile, C₁₋₃ alkoxy which is optionally partially or fully halogenated, phenyloxy, naphthyloxy, heterocyclyloxy wherein the heterocyclyl moiety is selected from the group hereinabove described, nitro, amino, mono- or di-(C₁₋₃)alkylamino, phenylamino, naphthylamino, heterocyclylamino wherein the heterocyclyl moiety is selected from the group hereinabove described, NH₂C(O), a mono- or di-(C₁₋₃)alkyl aminocarbonyl, C₁₋₄ alkyl-OC(O), C₁₋₅ alkyl-C(O)-C₁₋₄ branched or unbranched alkyl, an amino-C₁₋₅ alkyl, mono- or di-(C₁₋₃)alkylamino-C₁₋₅ alkyl, R₉-C₁₋₅ alkyl, R₁₀-C₁₋₅ alkoxy, R₁₁―C(O)-C₁₋₅ alkyl and R₁₂ -C₁₋₅ alkyl(R₁₃)N;
c) cycloalkyl selected from the group consisting of cyclopropyl, cyclobutyl, cyclopentyl, cyclohexyl, cycloheptyl, bicyclopentyl, bicyclohexyl and bicycloheptyl, wherein the cycloalkyl is optionally partially or fully halogenated and optionally substituted with one to three C₁₋₃ alkyl groups;
d) C₅₋₇ cycloalkenyl selected from the group consisting of cyclopentenyl, cyclohexenyl, cyclohexadienyl, cycloheptenyl, cycloheptadienyl, bicyclohexenyl and bicycloheptenyl, wherein such cycloalkenyl group is optionally substituted with one to three C₁₋₃ alkyl groups;
e) acetyl, aroyl, C₁₋₆alkoxycarbonylC₁₋₆alkyl or phenylsulfonyl; or
f) C₁₋₆ branched or unbranched alkyl optionally partially or fully halogenated;
or R₁ and R₂ taken together optionally form a fused phenyl or pyridinyl ring;
each R₈ and R₁₃ is independently selected from the group consisting of:
hydrogen and C₁₋₄ branched or unbranched alkyl optionally partially or fully halogenated;
each R₄, R₅, R₆, R₇, R₉, R₁₀, R₁₁ and R₁₂ is independently selected from the group consisting of morpholine, piperidine, piperazine, imidazole and tetrazole;
m is 0, 1 or 2;
W is O or S;
wherein X is directly attached to one or two -Y-Z, and
pharmaceutically acceptable derivatives thereof.

In another embodiment the invention relates to pharmaceutical compositions containing **A** and **B**, characterized in that the p38 kinase inhibitor **B** is selected from the compounds of formula **5a** wherein:
- Ar₂: is naphthyl, tetrahydronaphthyl, indanyl or indenyl and
- W: is O.

In another embodiment the invention relates to pharmaceutical compositions containing **A** and **B**, characterized in that the p38 kinase inhibitor **B** is selected from the compounds of formula **5a** wherein:
- Ar₁: is thiophene or pyrazole each substituted independently by one to three R₁, R₂ or R₃;
- X: is:
a C₅₋₇ cycloalkyl or cycloalkenyl optionally substituted with one to two oxo groups or one to three C₁₋₄ alkyl, C₁₋₄ alkoxy or C₁₋₄ alkylamino chains each being branched or unbranched;
phenyl, indanyl, furanyl, thienyl, imidazolyl, pyridinyl, pyrazinyl, tetrahydrapyridinyl, pyrimidinyl, pyridinonyl, piperdinyl, benzimidazole or piperazinyl; each being optionally independently substituted with one to three C₁₋₄ alkyl, C₁₋₄alkoxy, hydroxy, nitrile, amino, mono- or di-(C₁₋₃ alkyl)amino, mono- or di-(C₁₋₃ alkylamino)carbonyl, NH₂C(O), C₁₋₆ alkyl-S(O)ₘ or halogen;
- Y: is:
a bond or a C₁₋₄ saturated or unsaturated branched or unbranched carbon chain optionally partially or fully halogenated, wherein one or more C atoms are optionally replaced by O or N, and wherein Y is optionally independently substituted with one to two oxo groups, nitrile, phenyl, hydroxy or one or more C₁₋₄ alkyl optionally substituted by one or more halogen atoms;
- Z is:: phenyl, heteroaryl selected from pyridinyl, imidazolyl, furanyl and thienyl, heterocycle selected from piperazinyl, 2-oxa-5-aza-bicyclo[2.2.1]heptanyl, pentamethylene sulfidyl, pentamethylene sulfoxidyl, pentamethylene sulfonyl, tetrahydrofuranyl, morpholino, thiomorpholino and piperidinyl,
each of the aforementioned Z are optionally substituted with one to three halogen, C₁₋₆ alkyl, C₁₋₆ alkoxy, C₁₋₃ alkoxy-C₁₋₃ alkyl, C₁₋₆ alkoxycarbonyl, aroyl, morpholinocarbonyl, C₁₋₃acyl, oxo, hydroxy, pyridinyl-C₁₋₃ alkyl, imidazolyl-C₁₋₃ alkyl, tetrahydrofuranyl-C₁₋₃ alkyl, nitrile-C₁₋₃ alkyl, nitrile, carboxy, phenyl wherein the phenyl ring is optionally substituted with one to two halogen, C₁₋₆ alkoxy, hydroxy or mono- or di-(C₁₋₃ alkyl)amino, amino-S(O)ₘ, C₁₋₆ alkyl-S(O)ₘ or phenyl-S(O)ₘ wherein the phenyl ring is optionally substituted with one to two halogen, C₁₋₆ alkoxy, hydroxy, halogen or mono- or di-(C₁₋₃ alkyl)amino;
or Z is optionally substituted with one to three amino, aminocarbonyl or amino-C₁₋₃ alkyl wherein the N atom is optionally independently mono- or di-substituted by aminoC₁₋₆alkyl, C₁₋₃alkyl, arylC₀₋₃alkyl, C₁₋₅ alkoxyC₁₋₃ alkyl, C₁₋₅ alkoxy, aroyl, C₁₋₃acyl, C₁₋₃alkyl-S(O)ₘ- or arylC₀₋₃alkyl-S(O)ₘ- each of the aforementioned alkyl and aryl attached to the amino group are optionally substituted with one to two halogen, C₁₋₆ alkyl or C₁₋₆ alkoxy;
or Z is optionally substituted with one to three aryl, heterocycle or heteroaryl as hereinabove described in this paragraph each in turn is optionally substituted by halogen, C₁₋₆ alkyl or C₁₋₆ alkoxy;
or Z is hydroxy, hydroxyC₁₋₃alkyl, halogen, nitrile, amino wherein the N atom is optionally independently mono- or di-substituted by aroyl, C₁₋₃acyl, C₁₋₆alkyl, C₁₋₅ alkoxyC₁₋₃ alkyl, pyridinylC₁₋₃alkyl, tetrahydrafuranylC₁₋₃alkyl, nitrileC₁₋₄alkyl or phenyl wherein the phenyl ring is optionally substituted with one to two halogen, C₁₋₆ alkoxy, hydroxy or mono- or di-(C₁₋₃ alkyl)amino,
or Z is C₁₋₆alkyl branched or unbranched, C₁₋₆alkoxy or nitrileC₁₋₄alkyl;
- R₁: is:
C₁₋₄ branched or unbranched alkyl optionally partially or fully halogenated;
cyclopropyl, cyclobutyl, cyclopentyl, cyclohexyl and cycloheptyl optionally partially or fully halogenated and optionally substituted with one to three C₁₋₃ alkyl groups, or an analog of such cycloalkyl group wherein one to three ring methylene groups are replaced by groups independently selected from the group consisting of O, S and NH;
C₃₋₁₀ branched alkenyl optionally partially or fully halogenated and optionally substituted with one to three C₁₋₅ branched or unbranched alkyl;
cyclopentenyl and cyclohexenyl optionally substituted with one to three C₁₋₃ alkyl groups;
- R₂: is:
a C₁₋₆ branched or unbranched alkyl optionally partially or fully halogenated and optionally substituted with nitrile;
- R₃: is:
phenyl or heterocyclic group selected from the group consisting of pyridinyl, pyrimidinyl, pyrazinyl, pyridazinyl and pyrazolyl, wherein such phenyl or heterocyclic group is optionally substituted with one to five groups selected from the group consisting of a phenyl, heterocycle selected from the group hereinabove described in this paragraph, C₁₋₆ branched or unbranched alkyl which is optionally partially or fully halogenated, cyclopropyl, cyclobutyl, cyclopentyl, cyclohexyl, cycloheptyl, bicyclopentyl, bicyclohexyl, bicycloheptyl, phenyl C₁₋₅ alkyl, naphthyl C₁₋₅ alkyl, halogen, hydroxy, oxo, nitrile, C₁₋₃ alkoxy optionally be partially or fully halogenated, C₁₋₃ alkoxyC₁₋₅alkyl, C₁₋₃thioalkyl, C₁₋₃thioalkylC₁₋₅alkyl, phenyloxy, naphthyloxy, heteraryloxy wherein the heterocyclic moiety is selected from the group hereinabove described in this paragraph, nitro, amino, mono- or di-(C₁₋₃)alkylamino, phenylamino, naphthylamino, heterocyclylamino wherein the heterocyclyl moiety is selected from the group hereinabove described in this paragraph, NH₂C(O), a mono- or di-(C₁₋₃)alkyl aminocarbonyl, C₁₋₅ alkyl-C(O)-C₁₋₄ alkyl, amino-C₁₋₅ alkyl, mono-or di-(C₁₋₃)alkylamino-C₁₋₅ alkyl, amino-S(O)₂, di-(C₁₋₃)alkylamino-S(O)₂, R₄-C₁₋₅alkyl, R₅-C₁₋₅ alkoxy, R₆-C(O)-C₁₋₅ alkyl and R₇-C₁₋₅ alkyl(R₈)N, carboxy-mono- or di-(C₁₋₅)-alkyl-amino;
a fused aryl selected from the group consisting of benzocyclobutanyl, indanyl, indenyl; wherein the fused aryl is substituted with 0 to 3 groups independently selected from the group consisting of phenyl, naphthyl and heterocyclyl selected from the group consisting of pyridinyl, pyrimidinyl, pyrazinyl, pyridazinyl, pyrrolyl, imidazolyl, pyrazolyl, thienyl, furyl, isoxazolyl, and isothiazolyl, C₁₋₆ branched or unbranched alkyl which is optionally partially or fully halogenated, halogen, nitrile, C₁₋₃ alkoxy which is optionally partially or fully halogenated, phenyloxy, naphthyloxy, heterocyclyloxy wherein the heterocyclyl moiety is selected from the group hereinabove described in this paragraph, nitro, amino, mono- or di-(C₁₋₃)alkylamino, phenylamino, naphthylamino, heterocyclylamino wherein the heterocyclyl moiety is selected from the group hereinabove described in this paragraph, NH₂C(O), a mono- or di-(C₁₋₃)alkyl aminocarbonyl, C₁₋₄ alkyl-OC(O), C₁₋₅ alkyl-C(O)-C₁₋₄ branched or unbranched alkyl, an amino-C₁₋₅ alkyl, mono- or di-(C₁₋₃)alkylamino-C₁₋₅ alkyl, R₉-C₁₋₅ alkyl, R₁₀ -C₁₋₅ alkoxy, R₁₁―C(O)-C₁₋₅ alkyl and R₁₂ -C₁₋₅ alkyl(R₁₃)N;
cycloalkyl selected from the group consisting of cyclopropyl, cyclobutyl, cyclopentyl, cyclohexyl, cycloheptyl, wherein the cycloalkyl is optionally partially or fully halogenated and optionally substituted with one to three C₁₋₃ alkyl groups;
C₁₋₆alkoxycarbonylC₁₋₆alkyl;
or R₁ and R₂ taken together optionally form a fused phenyl or pyridinyl ring;
each R₈ and R₁₃ is independently selected from the group consisting of:
hydrogen and C₁₋₄ branched or unbranched alkyl optionally partially or fully halogenated;
and
each R₄, R₅, R₆, R₇, R₉, R₁₀, R₁₁ and R₁₂ is independently selected from the group consisting of morpholine, piperidine, piperazine, imidazole and tetrazole;
wherein X is directly attached to one -Y-Z.

In another embodiment the invention relates to pharmaceutical compositions containing **A** and **B**, characterized in that the p38 kinase inhibitor **B** is selected from the compounds of formula **5a** wherein:
- Ar₁: is pyrazole;
- X: is:
cyclopentenyl, cyclohexenyl, cycloheptenyl, optionally substituted with an oxo group or one to three C₁₋₄ alkyl, C₁₋₄ alkoxy or C₁₋₄ alkylamino chains each being branched or unbranched;
phenyl, furanyl, thienyl, pyridinyl, pyrazinyl piperidinyl or pyrimidinyl each being optionally independently substituted with one to three C₁₋₂ alkyl, C₁₋₂alkoxy, hydroxy or halogen;
- Z: is:
phenyl, heteroaryl selected from pyridinyl, imidazolyl and furanyl, heterocycle selected from 2-oxa-5-aza-bicyclo[2.2.1]heptanyl, pentamethylene sulfidyl, pentamethylene sulfoxidyl, pentamethylene sulfonyl, tetrahydrofuranyl, tetrahydropyranyl, piperazinyl, morpholino, thiomorpholino, thiomorpholino sulfoxide and piperidinyl,
each of the aforementioned Z are optionally substituted with one to three halogen, C₁₋₆ alkyl, C₁₋₆ alkoxy, C₁₋₃ alkoxy-C₁₋₃ alkyl, C₁₋₆ alkoxycarbonyl, aroyl, morpholinocarbonyl, C₁₋₃acyl, oxo, hydroxy, pyridinyl-C₁₋₃ alkyl, imidazolyl-C₁₋₃ alkyl, tetrahydrofuranyl-C₁₋₃ alkyl, nitrile-C₁₋₃ alkyl, nitrile, carboxy, phenyl wherein the phenyl ring is optionally substituted with one to two halogen, C₁₋₆ alkoxy, hydroxy or mono- or di-(C₁₋₃ alkyl)amino, amino-S(O)ₘ, C₁₋₆ alkyl-S(O)ₘ, or phenyl-S(O)ₘ wherein the phenyl ring is optionally substituted with one to two halogen, C₁₋₆ alkoxy, hydroxy, halogen or mono- or di-(C₁₋₃ alkyl)amino;
or Z is optionally substituted with one to three amino, aminocarbonyl or amino-C₁₋₃ alkyl wherein the N atom is optionally independently mono- or di-substituted by aminoC₁₋₆alkyl, C₁₋₃alkyl, arylC₀₋₃alkyl, C₁₋₅ alkoxyC₁₋₃ alkyl, C₁₋₅ alkoxy, aroyl, C₁₋₃acyl, C₁₋₃alkyl-S(O)ₘ-, pyridinylC₀₋₃alkyl, tetrahydrafuranylC₀₋₃alkyl, or arylC₀₋₃alkyl-S(O)ₘ- each of the aforementioned alkyl and aryl attached to the amino group is optionally substituted with one to two halogen, C₁₋₆ alkyl or C₁₋₆ alkoxy;
or Z is hydroxy, hydroxyC₁₋₃alkyl, halogen, nitrile, amino wherein the N atom is optionally independently mono- or di-substituted by C₁₋₆alkyl, pyridinylC₀₋₃alkyl, tetrahydrafuranylC₀₋₃alkyl, C₁₋₅ alkoxyC₁₋₃ alkyl, C₁₋₃acyl, nitrileC₁₋₄alkyl or phenyl wherein the phenyl ring is optionally substituted with one to two halogen, C₁₋₆ alkoxy, hydroxy or mono- or di-(C₁₋₃ alkyl)amino,
or Z is C₁₋₆alkyl branched or unbranched, C₁₋₆alkoxy or nitrileC₁₋₄alkyl;
- R₁: is:
C₁₋₄ branched or unbranched alkyl optionally partially or fully halogenated;
cyclopropyl, cyclobutyl, cyclopentanyl, cyclohexanyl and cycloheptanyl optionally partially or fully halogenated and optionally substituted with one to three C₁₋₃ alkyl groups, or an analog of such cycloalkyl group wherein one to three ring methylene groups are replaced by groups independently selected from the group consisting of O, S and NH;
C₃₋₁₀ branched alkenyl optionally partially or fully halogenated and optionally substituted with one to three C₁₋₃ branched or unbranched alkyl;
cyclopentenyl and cyclohexenyl optionally substituted with one to three C₁₋₃ alkyl groups;
- R₂: is:
a C₁₋₆ branched or unbranched alkyl optionally partially or fully halogenated and optionally substituted with nitrile;
- R₃: is:
phenyl or heterocyclic group selected from the group consisting of pyridinyl, pyrimidinyl, pyridazinyl and pyrazolyl, wherein such phenyl or heterocyclic group is optionally substituted with one to five groups selected from the group consisting of a phenyl, heterocycle selected from the group hereinabove described in this paragraph, C₁₋₆ branched or unbranched alkyl which is optionally partially or fully halogenated, phenyl C₁₋₅ alkyl, halogen, hydroxy, oxo, nitrile, C₁₋₃ alkoxy optionally partially or fully halogenated, C₁₋₃thioalkyl, C₁₋₃thioalkylC₁₋₅alkyl, amino, mono- or di-(C₁₋₃)alkylamino, NH₂C(O) or a mono- or di-(C₁₋₃)alkyl aminocarbonyl,
C₁₋₆alkoxycarbonylC₁₋₆alkyl;
or R₃ is cyclopropyl or cyclopentyl each optionally partially or fully halogenated and optionally substituted with one to three C₁₋₃ alkyl groups
or R₁ and R₂ taken together optionally form a fused phenyl or pyridinyl ring.

In another embodiment the invention relates to pharmaceutical compositions containing **A** and **B**, characterized in that the p38 kinase inhibitor **B** is selected from the compounds of formula **5a** wherein:
- Y: is -CH₂-, -O-(CH₂)₀₋₃-, -CH₂CH₂-, -CH₂NH-, -CH₂CH₂-NH-, NH-CH₂CH₂-, -CH₂-NH-CH₂-, -NH-, -NH-C(O)-, -C(O)-, -CH(OH)-, -CH₂(CH₂CH₃)- or a bond;
- X: is:
cyclohexenyl optionally substituted with an oxo group or one to three C₁₋₄ alkyl, C₁₋₄ alkoxy or C₁₋₄ alkylamino chains each being branched or unbranched;
phenyl, pyridinyl, pyrazinyl, piperidinyl or pyrimidinyl each being optionally independently substituted with one to three C₁₋₂ alkyl, C₁₋₂alkoxy, hydroxy or halogen;
- Z: is:
phenyl, heteroaryl selected from pyridinyl, imidazolyl and furanyl, heterocycle selected from 2-oxa-5-aza-bicyclo[2.2.1]heptanyl, pentamethylene sulfidyl, pentamethylene sulfoxidyl, pentamethylene sulfonyl, tetrahydrofuranyl, tetrahydropyranyl, piperazinyl, morpholino, thiomorpholino, thiomorpholino sulfoxide and piperidinyl,
each of the aforementioned Z are optionally substituted with one to three halogen, C₁₋₆ alkyl, C₁₋₆ alkoxy, C₁₋₃ alkoxy-C₁₋₃ alkyl, C₁₋₆ alkoxycarbonyl, aroyl, morpholinocarbonyl, C₁₋₃acyl, oxo, hydroxy, pyridinyl-C₁₋₃ alkyl, imidazolyl-C₁₋₃ alkyl, tetrahydrofuranyl-C₁₋₃ alkyl, nitrile-C₁₋₃ alkyl, nitrile, carboxy, phenyl wherein the phenyl ring is optionally substituted with one to two halogen, C₁₋₆ alkoxy, hydroxy or mono- or di-(C₁₋₃ alkyl)amino, amino-S(O)ₘ, C₁₋₆ alkyl-S(O)ₘ, or phenyl-S(O)ₘ wherein the phenyl ring is optionally substituted with one to two halogen, C₁₋₆ alkoxy, hydroxy, halogen or mono- or di-(C₁₋₃ alkyl)amino;
or Z is optionally substituted with one to three amino or aminocarbonyl wherein the N atom is optionally independently mono- or di-substituted by aminoC₁₋₆alkyl, C₁₋₃alkyl, arylC₀₋₃alkyl, C₁₋₅ alkoxyC₁₋₃ alkyl, C₁₋₅ alkoxy, aroyl, C₁₋₃acyl, C₁₋₃alkyl-S(O)ₘ- or arylC₀₋₃alkyl-S(O)ₘ- each of the aforementioned alkyl and aryl attached to the amino group is optionally substituted with one to two halogen, C₁₋₆ alkyl or C₁₋₆ alkoxy;
or Z is hydroxy, hydroxyC₁₋₃alkyl, halogen, nitrile, amino wherein the N atom is optionally independently mono- or di-substituted by C₁₋₃alkyl, pyridinylC₁₋₂alkyl, tetrahydrafuranylC₁₋₂alkyl, C₁₋₃ alkoxyC₁₋₃ alkyl, C₁₋₃acyl, nitrileC₁₋₄alkyl, phenyl wherein the phenyl ring is optionally substituted with one to two halogen, C₁₋₆ alkoxy, hydroxy or mono- or di-(C₁₋₃ alkyl)amino,
or Z is C₁₋₆alkyl branched or unbranched, C₁₋₆alkoxy or nitrileC₁₋₄alkyl;
- R₁: is:
C₁₋₄ branched or unbranched alkyl optionally partially or fully halogenated;
- R₂: is:
a C₁₋₃ branched or unbranched alkyl optionally partially or fully halogenated and optionally substituted with nitrile;
- R₃: is:
phenyl or heterocyclic group selected from the group consisting of pyridinyl, pyrimidinyl, and pyrazolyl, wherein such phenyl or heterocyclic group is optionally substituted with one to five groups selected from the group consisting of C₁₋₃ branched or unbranched alkyl which is optionally partially or fully halogenated, C₁₋₃ alkoxy which optionally partially or fully halogenated, C₁₋₃thioalkyl, C₁₋₃thioalkylC₁₋₅alkyl, amino or NH₂C(O);
C₁₋₃alkoxycarbonyl;
or R₃ is cyclopropyl or cyclopentyl each optionally partially or fully halogenated and optionally substituted with one to three C₁₋₃ alkyl groups.

In a further embodiment the invention relates to pharmaceutical compositions containing **A** and **B**, characterized in that the p38 kinase inhibitor **B** is selected from the compounds of formula **5a** wherein:
- Ar₁: is 5-tert-butyl-pyrazol-3-yl; wherein the pyrazole ring is substituted independently by one to two R₂ or R₃;
- X: is:
cyclohexenyl;
phenyl, pyridinyl, pyrazinyl, piperidinyl or pyrimidinyl each being optionally independently substituted with C₁₋₂alkoxy or hydroxy;
- Z: is:
phenyl, heteroaryl selected from pyridinyl and furanyl, heterocycle selected from 2-oxa-5-aza-bicyclo[2.2.1]heptanyl, pentamethylene sulfidyl,
pentamethylene sulfoxidyl, tetrahydrofuranyl, piperazinyl, morpholino, thiomorpholino and piperidinyl,
each of the aforementioned Z are optionally substituted with one to three C₁₋₃ alkyl, C₁₋₃ alkoxy, oxo , hydroxy or NH₂C(O)-;
or Z is hydroxyC₁₋₃alkyl, amino wherein the N atom is optionally independently mono- or di-substituted by pyridinylmethyl, tetrahydrafuranylmethyl, C₁₋₃ alkoxyC₁₋₃ alkyl, C₁₋₃acyl or nitrileC₁₋₄alkyl,
or Z is nitrileC₁₋₄alkyl;
- R₃: is:
phenyl or heterocyclic group selected from the group consisting of pyridinyl, pyrimidinyl, and pyrazolyl, wherein such phenyl or heterocyclic group is optionally substituted with one to two groups selected from the group consisting of C₁₋₂ alkyl which is optionally partially or fully halogenated, C₁₋₂ alkoxy which optionally partially or fully halogenated, C₁₋₂thioalkyl, C₁₋₂thioalkylC₁₋₃alkyl, amino or NH₂C(O);
C₁₋₃alkoxycarbonyl;
or R₃ is cyclopropyl or cyclopentyl each optionally partially or fully halogenated and optionally substituted with one to three C₁₋₃ alkyl groups.

In a still further embodiment the invention relates to pharmaceutical compositions containing **A** and **B**, characterized in that the p38 kinase inhibitor **B** is selected from the compounds of formula **5a** wherein X is pyridinyl.

In a yet still further embodiment the invention relates to pharmaceutical compositions containing **A** and **B**, characterized in that the p38 kinase inhibitor **B** is selected from the compounds of formula **5a** wherein the pyridinyl is attached to Ar₁ via the 3-pyridinyl position.

Preferably the invention relates to pharmaceutical compositions containing **A** and **B**, characterized in that the p38 kinase inhibitor **B** is selected from the following compounds of formula **5a:**
1-[5-tert-butyl-2-p-tolyl-2H-pyrazol-3-yl]-3-[4-(4-morpholin-4-yl-methylphenyl)-naphthalen-1-yl]-urea;
1-[5-tert-butyl-2-p-tolyl-2H-pyrazol-3-yl]-3-[3-(4-morpholin-4-yl-methylphenyl)-naphthalen-1-yl]-urea;
1-[5-tert-butyl-2-p-tolyl-2H-pyrazol-3-yl]-3-[4-(5-morpholin-4-yl-methylfuran-2-yl)-naphthalen-1-yl]-urea;
1-[5-tert-butyl-2-p-tolyl-2H-pyrazol-3-yl]-3-[4-(3-(morpholin-4-yl-methyl)cyclohexenyl)-naphthalen-1-yl]-urea;
1-[5-tert-butyl-2-p-tolyl-2H-pyrazol-3-yl]-3-[4-(2-(4-morpholin-4-yl)ethylphenyl)-naphthalen-1-yl]-urea;
1-[5-tert-butyl-2-p-tolyl-2H-pyrazol-3-yl]-3-[4-(4-dimethylaminomethylphenyl)-naphthalen-1-yl]-u rea;
1-[5-tert-butyl-2-p-tolyl-2H-pyrazol-3-yl]-3-[4-(5-(morpholin-4-yl-methyl)pyridin-2-yl)-naphthalen-1-yl]-urea;
1-[5-tert-butyl-2-p-tolyl-2H-pyrazol-3-yl]-3-[4-(6-(morpholin-4-yl-methyl)pyridin-3-yl)-naphthalen-1-yl]-urea;
1-[5-tert-butyl-2-(6-methyl-pyridin-3-yl)-2H-pyrazol-3-yl]-3-[4-(6-(morpholin-4-yl-methyl)pyridin-3-yl)-naphthalen-1-yl]-urea;
1-[5-tert-butyl-2-methyl-2H-pyrazol-3-yl]-3-[4-(6-(morpholin-4-yl-methyl)pyridin-3-yl)-naphthalen-1-yl]-urea;
1-[5-tert-butyl-2-(6-methyl-pyridin-3-yl)-2H-pyrazol-3-yl]-3-[4-(3-(2-(morpholin-4-yl)ethylamino)cyclohexenyl)-naphthalen-1-yl]-urea;
1-[5-tert-butyl-2-p-tolyl-2H-pyrazol-3-yl]-3-[4-(3,4-(morpholin-4-yl-methyl)phenyl)-naphthalen-1-yl]-urea;
1-[5-tert-butyl-2-p-tolyl-2H-pyrazol-3-yl]-3-[4-(4-methylpiperzin-1-yl-methyl)phenyl)-naphthalen-1-yl]-u rea;
1-[5-tert-butyl-2-p-tolyl-2H-pyrazol-3-yl]-3-[4-(piperdin-1-yl-methyl)phenyl)-naphthalen-1-yl]-urea;
1-[5-tert-butyl-2-(6-methyl-pyridin-3-yl)-2H-pyrazol-3-yl]-3-[4-(3-(2-(pyridin-2-yl)ethylamino)cyclohexenyl)-naphthalen-1-yl]-urea;
1-[5-tert-butyl-2-p-tolyl-2H-pyrazol-3-yl]-3-[4-(4-(2-(pyridin-4-yl)ethylaminomethyl)phenyl)naphthalen-1-yl]-urea;
1-[5-tert-butyl-2-p-tolyl-2H-pyrazol-3-yl]-3-[4-(4-(pyridin-3-yl-methylaminomethyl)phenyl)naphthalen-1-yl]-urea;
1-[5-tert-butyl-2-(6-methyl-pyridin-3-yl)-2H-pyrazol-3-yl]-3-[4-(4-(3,4-dimethoxyphenylmethyl)-3-hydroxyphenyl)naphthalen-1-yl]-urea;
1-[5-tert-butyl-2-p-tolyl-2H-pyrazol-3-yl]-3-[4-(6-oxo-1,6-dihydro-pyridin-3-yl)naphthalen-1-yl]-urea;
1-[5-tert-butyl-2-(6-methyl-pyridin-3-yl)-2H-pyrazol-3-yl]-3-[4-(4-(morpholin-4-yl-methyl)phenyl)naphthalen-1-yl]-urea;
1-[5-tert-butyl-2-(6-methyl-pyridin-3-yl)-2H-pyrazol-3-yl]-3-[4-(4-(morpholin-4-yl-methyl)imidazol-1-yl)naphthalen-1-yl]-urea;
1-[5-tert-butyl-2-p-tolyl-2H-pyrazol-3-yl]-3-[4-(4-(morpholin-4-yl-methyl)imidazol-1-yl)naphthalen-1-yl]-urea;
1-[5-tert-butyl-2-(6-methyl-pyridin-3-yl)-2H-pyrazol-3-yl]-3-[4-(4-(furan-3-yl-methyl)-3-hydroxyphenyl)naphthalen-1-yl]-urea;
1-[5-te rt-butyl-2-(6-methyl-pyrid i n-3-yl )-2 H-pyrazol-3-yl]-3-[4-(6-(4-hydroxybutylamino)pyridin-3-yl)-naphthalen-1-yl]-urea;
1-[5-tert-butyl-2-(6-methyl-pyridin-3-yl)-2H-pyrazol-3-yl]-3-[4-(4-(pyridin-3-yl-methyl)-3-hydroxyphenyl)naphthalen-1-yl]-urea;
1-[5-tert-butyl-2-(4-methyl-3-carbamylphenyl)-2H-pyrazol-3-yl]-3-[4-(6-(morpholin-4-yl-methyl)pyridin-3-yl)naphthalen-1-yl]-urea;
1-[5-tert-butyl-2-(6-methyl-pyridin-3-yl)-2H-pyrazol-3-yl]-3-[4-(4-(imidazol-2-yl-methyl)-3-hydroxyphenyl)naphthalen-1-yl]-urea;
1-[5-tert-butyl-2-(6-methyl-pyridin-3-yl)-2H-pyrazol-3-yl]-3-[4-(4-(3-hydroxymorpholin-4-yl-methyl)phenyl)naphthalen-1-yl]-urea;
1-[5-tert-butyl-2-(6-methyl-pyridin-3-yl)-2H-pyrazol-3-yl]-3-[4-(4-(N-2-methoxyethy-N-methylaminomethyl)phenyl)naphthalen-1-yl]-urea;
1-[5-tert-butyl-2-(6-methyl-pyridin-3-yl)-2H-pyrazol-3-yl]-3-[4-(4-(4-hydroxymorpholin-4-yl-methyl)phenyl)naphthalen-1-yl]-urea;
1-[5-tert-butyl-2-(6-methyl-pyridin-3-yl)-2H-pyrazol-3-yl]-3-[4-(3-(morpholin-4-yl-methyl)cyclohexenyl)-naphthalen-1-yl]-urea;
1-[5-tert-butyl-2-(6-methyl-pyridin-3-yl)-2H-pyrazol-3-yl]-3-[4-(4-(tetrahydrofuran-3-yl-methyl)-3-hydroxyphenyl)naphthalen-1-yl]-urea;
1-[5-tert-butyl-2-(6-methyl-pyridin-3-yl)-2H-pyrazol-3-yl]-3-[4-(4-(N,N-di-(2-methoxyethyl)aminomethyl)phenyl)naphthalen-1-yl]-urea;
1-[5-tert-butyl-2-(6-methyl-pyridin-3-yl)-2H-pyrazol-3-yl]-3-[4-(6-(3-cyanopropoxy)pyridin-3-yl)naphthalen-1-yl]-urea;
1-[5-tert-butyl-2-(6-methyl-pyridin-3-yl)-2H-pyrazol-3-yl]-3-[4-(4-morpholin-4-yl-methyl-piperdinyl)naphthalen-1-yl]-urea;
1-[5-tert-butyl-2-(6-methyl-pyridin-3-yl)-2H-pyrazol-3-yl]-3-[4-(4-(N,N-di-(2-cyanoethyl)aminomethyl)phenyl)naphthalen-1-yl]-urea;
1-[5-tert-butyl-2-p-tolyl-2H-pyrazol-3-yl]-3-[4-(1-morpholin-4-yl-indan-5-yl)-naphthalen-1-yl]-urea;
1-[5-tert-butyl-2-(6-methyl-pyridin-3-yl)-2H-pyrazol-3-yl]-3-[4-(4-(furan-2-yl-methyl)-3-hydroxyphenyl)naphthalen-1-yl]-urea;
1-[5-tert-butyl-2-(6-methyl-pyridin-3-yl)-2H-pyrazol-3-yl]-3-[4-(4-(thiomorpholin-4-yl-methyl)phenyl)naphthalen-1-yl]-urea;
1-[5-tert-butyl-2-(6-methyl-pyridin-3-yl)-2H-pyrazol-3-yl]-3-[4-(4-(3-carboxamidomorpholin-4-yl-methyl)phenyl)naphthalen-1-yl]-urea;
1-[5-tert-butyl-2-(6-methyl-pyridin-3-yl)-2H-pyrazol-3-yl]-3-[4-(4-(2-methyl-3-oxo-piperzin-1-yl-methyl)phenyl)naphthalen-1-yl]-urea;
1-[5-tert-butyl-2-(2-methylpyrimidin-5-yl)-2H-pyrazol-3-yl]-3-[4-(6-(morpholin-4-yl-methyl)pyridin-3-yl)naphthalen-1-yl]-urea;
1-[5-tert-butyl-2-(6-methyl-pyridin-3-yl)-2H-pyrazol-3-yl]-3-[4-(6-(4-hydroxybutyloxy)pyridin-3-yl)-naphthalen-1-yl]-urea;
1-[3-tert-butyl-1'H-[1,4']bipyrazol-5-yl]-3-[4-(6-(morpholin-4-yl-methyl)pyridin-3-yl)naphthalen-1-yl]-urea;
1-[5-tert-butyl-2-(6-methyl-pyridin-3-yl)-2H-pyrazol-3-yl]-3-[4-(4-(furan-2-yl-methyl)-3-methoxyphenyl)naphthalen-1-yl]-urea;
1-[5-tert-butyl-2-(6-methyl-pyridin-3-yl)-2H-pyrazol-3-yl]-3-[4-(5-(morpholin-4carbonyl)pyrazin-2-yl)naphthalen-1-yl]-urea;
1-[5-tert-butyl-2-(6-methyl-pyridin-3-yl)-2H-pyrazol-3-yl]-3-[4-(6-(tetrahydrothiopyran-4-yl-amino)pyridin-3-yl)-naphthalen-1-yl]-urea;
1-[5-tert-butyl-2-(2-cyanoethyl)-2H-pyrazol-3-yl]-3-[4-(6-(morpholin-4-yl-methyl)pyridin-3-yl)-naphthalen-1-yl]-urea;
1-[5-tert-butyl-2-(6-methyl-pyridin-3-yl)-2H-pyrazol-3-yl]-3-[4-(6-(2,6-dimethylmorpholin-4-yl-methyl)pyridin-3-yl)-naphthalen-1-yl]-urea;
1-[5-tert-butyl-2-(2-methoxypyridin-5-yl)-2H-pyrazol-3-yl]-3-[4-(6-(morpholin-4-yl-methyl)pyridin-3-yl)-naphthalen-1-yl]-urea;
1-[5-tert-butyl-2-(2-aminoypyridin-5-yl)-2H-pyrazol-3-yl]-3-[4-(6-(morpholin-4-yl-methyl)pyridin-3-yl)-naphthalen-1-yl]-urea;
1-[5-tert-butyl-2-(6-oxo-1,6-dihydropyridin-3-yl)-2H-pyrazol-3-yl]-3-[4-(6-(morpholin-4-yl-methyl)pyridin-3-yl)-naphthalen-1-yl]-urea;
1-[5-tert-butyl-2-(6-methyl-pyridin-3-yl)-2H-pyrazol-3-yl]-3-[4-(6-(morpholin-4-yl-4-carbonyl)pyridin-3-yl)-naphthalen-1-yl]-urea;
1-[5-tert-butyl-2-(6-methyl-pyridin-3-yl)-2H-pyrazol-3-yl]-3-[4-(6-(2-oxa-5-aza-bicyclo[2.2.1]hept-5-yl-methyl)pyridin-3-yl)-naphthalen-1-yl]-urea;
1-[5-tert-butyl-2-p-tolyl-2H-pyrazol-3-yl]-3-[4-(4-(3-carbamylphenyl)naphthalen-1-yl]-urea;
1-[5-tert-butyl-2-(6-methyl-pyridin-3-yl)-2H-pyrazol-3-yl]-3-[4-(4-(N-(2-cyanoethyl)-N-(pyridin-3-yl-methyl)aminomethyl)phenyl)-naphthalen-1-yl]-urea;
1-[5-tert-butyl-2-(6-methyl-pyridin-3-yl)-2H-pyrazol-3-yl]-3-[4-(4-(N-(2-cyanoethyl)-N-(pyridin-2-yl-methyl)aminomethyl)phenyl)-naphthalen-1-yl]-urea;
1-(5-tert-butyl-2-(6-methyl-pyridin-3-yl)-2H-pyrazol-3-yl]-3-[4-(4-(N-(2-cyanoethyl)-N-(tetrahydrofuran-2-yl-methyl)aminomethyl)phenyl)-naphthalen-1-yl]-urea;
1-[5-tert-butyl-2-(6-methyl-pyridin-3-yl)-2H-pyrazol-3-yl]-3-[4-(6-(morpholin-4-yl-methyl)-4-methoxypyridin-3-yl)-naphthalen-1-yl]-urea;
1-[5-tert-butyl-2-(6-methyl-pyridin-3-yl)-2H-pyrazol-3-yl]-3-[4-(6-(1-morpholin-4-yl-propyl)pyridin-3-yl)-naphthalen-1-yl]-urea;
1-[5-tert-butyl-2-(6-methyl-pyridin-3-yl)-2H-pyrazol-3-yl]-3-[4-(6-(N-(3-methoxypropyl)amino)pyridin-3-yl)-naphthalen-1-yl]-urea;
1-[5-tert-butyl-2-(6-methyl-pyridin-3-yl)-2H-pyrazol-3-yl]-3-[4-(6-(N-(3-methoxypropyl)-N-methylamino)pyridin-3-yl)-naphthalen-1-yl]-urea;
1-[3-tert-butyl-1'-methyl-1'H-[1,4']bipyrazol-5-yl]-3-[4-(6-(morpholin-4-yl-methyl)pyridin-3-yl)naphthalen-1-yl]-urea;
1-[5-tert-butyl-2-benzyl-2H-pyrazol-3-yl]-3-[4-(6-(morpholin-4-yl-methyl)pyridin-3-yl)-naphthalen-1-yl]-urea;
1-[5-tert-butyl-2-(6-methyl-pyridin-3-yl)-2H-pyrazol-3-yl]-3-[4-(4-(N-N-di-(2-cyanoethyl)aminomethyl)phenyl)-naphthalen-1-yl]-urea;
1-[5-tert-butyl-2-p-tolyl-2H-pyrazol-3-yl]-3-[4-(4-(4-carbamylphenyl)naphthalen-1-yl]-urea;
1-[5-tert-butyl-2-(6-methyl-pyridin-3-yl)-2H-pyrazol-3-yl]-3-[4-(6-(1-oxo-tetrahydrothiopyran-4yl-amino)pyridin-3-yl)-naphthalen-1-yl]-urea;
1-[5-tert-butyl-2-(6-methyl-pyridin-3-yl)-2H-pyrazol-3-yl]-3-[4-(6-(tetrahydropyran-4yl-amino)pyridin-3-yl)-naphthalen-1-yl]-urea;
1-[3-tert-butyl-1'-(3-cyanopropyl)-1'H-[1,4']bipyrazol-5-yl]-3-[4-(6-(morpholin-4-yl-methyl)pyridin-3-yl)naphthalen-1-yl]-urea;
1-[5-tert-butyl-2-p-tolyl-2H-pyrazol-3-yl]-3-[4-(3-methanesulfinylphenyl)naphthalen-1-yl]-urea;
1-[5-tert-butyl-2-p-tolyl-2H-pyrazol-3-yl]-3-[4-(3-methanesulfonylphenyl)naphthalen-1-yl]-urea;
1-[5-tert-butyl-2-p-tolyl-2H-pyrazol-3-yl]-3-[4-(3-sulfonamidophenyl)naphthalen-1-yl]-urea;
1-[5-tert-butyl-2-p-tolyl-2H-pyrazol-3-yl]-3-[4-(3-(morpholin-4-yl)carbonylphenyl)naphthalen-1-yl]-urea;
1-[5-tert-butyl-2-(6-methyl-pyridin-3-yl)-2H-pyrazol-3-yl]-3-[4-(5-(tetrahydrothiopyran-4yl-amino)pyrazin-2-yl)-naphthalen-1-yl]-urea;
1-[5-tert-butyl-2-(6-methyl-pyridin-3-yl)-2H-pyrazol-3-yl]-3-[4-(6-(methylcarbonylamino)pyridin-3-yl)-naphthalen-1-yl]-urea;
1-[5-tert-butyl-2-p-tolyl-2H-pyrazol-3-yl]-3-[4-(6-(morpholin-4-yl-4-carbonyl)phenyl)-naphthalen-1-yl]-urea;
1-[3-tert-butyl-1'-(3-methylsulfanylpropyl)-1'H-[1,4']bipyrazol-5-yl]-3-[4-(6-(morpholin-4-yl-methyl)pyridin-3-yl)naphthalen-1-yl]-urea;
1-[5-tert-butyl-2-p-tolyl-2H-pyrazol-3-yl]-3-[4-(5-(morpholin-4-yl-carbonyl)pyridin-3-yl)-naphthalen-1-yl]-urea;
1-[5-tert-butyl-2-(6-methyl-pyrid in-3-yl)-2H-pyrazol-3-yl]-3-[4-(5-(morpholin-4-yl-methyl)pyrazin-2-yl)-naphthalen-1-yl]-urea;
1-[5-tert-butyl-2-(6-methyl-pyridin-3-yl)-2H-pyrazol-3-yl]-3-[4-(6-aminopyridin-3-yl)naphthalen-1-yl]-urea;
1-[5-tert-butyl-2-(6-methyl-pyridin-3-yl)-2H-pyrazol-3-yl]-3-[4-(6-(1-methylpiperdin-4-yl-amino)pyridin-3-yl)naphthalen-1-yl]-urea;
1-[5-tert-butyl-2-(2-methylpyrimidin-5-yl)-2H-pyrazol-3-yl]-3-[4-(6-(2-methyl-3-oxo-piperzin-1-yl-methyl)pyridin-3-yl)naphthalen-1-yl]-urea;
1-[5-tert-butyl-2-(2-methylpyrimidin-5-yl)-2H-pyrazol-3-yl]-3-[4-(6-(morpholin-4-yl-carbonyl)pyridin-3-yl)naphthalen-1-yl]-urea;
1-[5-tert-butyl-2-(2-methylpyrimidin-5-yl)-2H-pyrazol-3-yl]-3-[4-(6-(N,N-di-(2-methoxyethyl)aminomethyl)pyridin-3-yl)naphthalen-1-yl]-urea;
1-[5-tert-butyl-2-(2-methylpyrimidin-5-yl)-2H-pyrazol-3-yl]-3-[4-(6-(1-oxo-thiomorpholin-4-yl-methyl)pyridin-3-yl)naphthalen-1-yl]-urea;
1-[5-tert-butyl-2-(2-methylpyrimidin-5-yl)-2H-pyrazol-3-yl]-3-[4-(6-(tetrahydropyran-4-yl-amino)pyridin-3-yl)naphthalen-1-yl]-urea;
1-[5-tert-butyl-2-(2-methylpyrimidin-5-yl)-2H-pyrazol-3-yl]-3-[4-(5-(morpholin-4-yl-methyl)pyrazin-2-yl)naphthalen-1-yl]-urea;
1-[5-tert-butyl-2-(2-methylthiopyrimidin-5-yl)-2H-pyrazol-3-yl]-3-[4-(6-(morpholin-4-yl-methyl)pyridin-3-yl)naphthalen-1-yl]-urea;
1-[5-tert-butyl-2-p-tolyl-2H-pyrazol-3-yl]-3-[4-(6-(2-methyl-3-oxo-piperzin-1-yl-methyl)pyridin-3-yl)naphthalen-1-yl]-urea;
1-[5-tert-butyl-2-p-tolyl-2H-pyrazol-3-yl]-3-[4-(6-(pyridin-3-yl-oxy)pyridin-3-yl)naphthalen-1-yl]-urea
1-[5-tert-butyl-2-p-tolyl-2H-pyrazol-3-yl]-3-[4-(6-(pyridin-3-yl-amino)pyridin-3-yl)naphthalen-1-yl]-urea;
1-[5-tert-butyl-2-(2-methoxypyrimidin-5-yl)-2H-pyrazol-3-yl]-3-[4-(6-(morpholin-4-yl-methyl)pyridin-3-yl)naphthalen-1-yl]-urea;
1-[5-tert-butyl-2-p-tolyl-2H-pyrazol-3-yl]-3-[4-(5-carbamylpyridin-3-yl)naphthalen-1-yl]-urea;
1-[5-tert-butyl-2-(2-aminopyrimidin-5-yl)-2H-pyrazol-3-yl]-3-[4-(6-(morpholin-4-yl-methyl)pyridin-3-yl)naphthalen-1-yl]-urea;
1-[5-tert-butyl-2-(2-methylpyrimidin-5-yl)-2H-pyrazol-3-yl]-3-[4-(4-(morpholin-4-yl-methyl)phenyl)naphthalen-1-yl]-urea;
1-[3-tert-butyl-1'-methyl-1'H-[1,4']bipyrazol-5-yl]-3-[4-(6-(morpholin-4-yl-methyl)phenyl)naphthalen-1-yl]-urea;
1-[5-tert-butyl-2-(2-cyclopropylpyrimidin-5-yl)-2H-pyrazol-3-yl]-3-[4-(6-(morpholin-4-yl-methyl)pyridin-3-yl)naphthalen-1-yl]-urea;
1-[5-tert-butyl-2-p-tolyl-2H-pyrazol-3-yl]-3-[4-(2-(pyridin-3-yl-amino)pyrimidin-5-yl)naphthalen-1-yl]-urea;
1-[5-tert-butyl-2-p-tolyl-2 H-pyrazol-3-yl]-3-[4-(6-( 1-oxo-tetrahydrothiopyran-4-yl-amino)pyridin-3-yl)naphthalen-1-yl]-urea;
1-[5-tert-butyl-2-p-tolyl-2H-pyrazol-3-yl]-3-[4-(6-(thiomorpholin-4-yl-methyl)pyridin-3-yl)naphthalen-1-yl]-urea;
1-[5-tert-butyl-2-p-tolyl-2H-pyrazol-3-yl]-3-[4-(3-benzyl-3H-imidazo[4,5-b]pyridin-6-yl)naphthalen-1-yl]-urea;
1-[5-tert-butyl-2-(6-methyl-pyrid in-3-yl)-2H-pyrazol-3-yl]-3-[4-(6-(pyrid in-3-yl-methyl)pyridin-3-yl)naphthalen-1-yl]-urea;
1-[5-tert-butyl-2-p-tolyl-2H-pyrazol-3-yl]-3-[4-(2-(morpholin-4-yl-carbonyl)pyrimidin-5-yl)naphthalen-1-yl]-urea;
1-[5-tert-butyl-2-p-tolyl-2H-pyrazol-3-yl]-3-[4-(2-(morpholin-4-yl-methyl)pyrimidin-5-yl)naphthalen-1-yl]-urea;
1-[5-tert-butyl-2-p-tolyl-2H-pyrazol-3-yl]-3-[4-(3-amino-4-carbamylphenyl)naphthalen-1-yl]-urea;
1-[5-tert-butyl-2-p-tolyl-2H-pyrazol-3-yl]-3-[4-(6-(1-oxo-thiomorpholin-4-yl-methyl)pyridin-3-yl)naphthalen-1-yl]-urea;
1-[5-tert-butyl-2-p-tolyl-2H-pyrazol-3-yl]-3-[4-(6-(pyridin-3-yl-methyl)pyrid in-3-yl)naphthalen-1-yl]-urea;
1-[5-tert-butyl-2-p-tolyl-2H-pyrazol-3-yl]-3-[4-(6-(hydroxy-pyridin-3-yl-methyl)pyridin-3-yl)naphthalen-1-yl]-urea;
1-[5-tert-butyl-2-(2-methylpyrimidin-5-yl)-2H-pyrazol-3-yl]-3-[4-(2-(morpholin-4-yl-methyl)pyrimidin-5-yl)naphthalen-1-yl]-urea;
and the pharmaceutically acceptable derivatives thereof.

In another embodiment the invention relates to pharmaceutical compositions containing A and B, characterized in that the p38 kinase inhibitor B is selected from the following compounds of formula **5a**:
1-[5-tert-butyl-2-p-tolyl-2H-pyrazol-3-yl]-3-[4-(5-(morpholin-4-yl-methyl)pyridin-2-yl)-naphthalen-1-yl]-urea;
1-[5-tert-butyl-2-p-tolyl-2H-pyrazol-3-yl]-3-[4-(6-(morpholin-4-yl-methyl)pyridin-3-yl)-naphthalen-1-yl]-u rea;
1-[5-tert-butyl-2-(6-methyl-pyridin-3-yl)-2H-pyrazol-3-yl]-3-[4-(3-(2-(pyridin-2-yl)ethylamino)cyclohexenyl)-naphthalen-1-yl]-urea;
1-[5-tert-butyl-2-p-tolyl-2H-pyrazol-3-yl]-3-[4-(4-(pyridin-3-yl-methylaminomethyl)phenyl)naphthalen-1-yl]-urea;
1-[5-tert-butyl-2-(6-methyl-pyridin-3-yl)-2H-pyrazol-3-yl]-3-[4-(4-(morpholin-4-yl-methyl)phenyl)naphthalen-1-yl]-urea;
1-[5-tert-butyl-2-(6-methyl-pyridin-3-yl)-2H-pyrazol-3-yl]-3-[4-(6-(4-hydroxybutylamino)pyridin-3-yl)-naphthalen-1-yl]-urea;
1-[5-tert-butyl-2-(4-methyl-3-carbamylphenyl)-2H-pyrazol-3-yl]-3-[4-(6-(morpholin-4-yl-methyl)pyridin-3-yl)naphthalen-1-yl]-urea;
1-[5-tert-butyl-2-(6-methyl-pyridin-3-yl)-2H-pyrazol-3-yl]-3-[4-(4-(3-hydroxypiperidin-1-yl-methyl)phenyl)naphthalen-1-yl]-urea;
1-[5-tert-butyl-2-(6-methyl-pyridin-3-yl)-2H-pyrazol-3-yl]-3-[4-(4-(4-hydroxymorpholin-4-yl-methyl)phenyl)naphthalen-1-yl]-urea;
1-[5-tert-butyl-2-(6-methyl-pyridin-3-yl)-2H-pyrazol-3-yl]-3-[4-(3-(morpholin-4-yl-methyl)cyclohexenyl)-naphthalen-1-yl]-urea;
1-[5-tert-butyl-2-(6-methyl-pyridin-3-yl)-2H-pyrazol-3-yl]-3-[4-(4-(tetrahydrofuran-3-yl-methyl)-3-hydroxyphenyl)naphthalen-1-yl]-urea;
1-[5-tert-butyl-2-(6-methyl-pyridin-3-yl)-2H-pyrazol-3-yl]-3-[4-(4-(N,N-di-(2-methoxyethyl)aminomethyl)phenyl)naphthalen-1-yl]-urea;
1-[5-tert-butyl-2-(6-methyl-pyridin-3-yl)-2H-pyrazol-3-yl]-3-[4-(6-(3-cyanopropoxy)pyridin-3-yl)naphthalen-1-yl]-urea;
1-[5-tert-butyl-2-(6-methyl-pyridin-3-yl)-2H-pyrazol-3-yl]-3-[4-(4-morpholin-4-yl-methyl-piperdinyl)naphthalen-1-yl]-urea;
1-[5-tert-butyl-2-(6-methyl-pyridin-3-yl)-2H-pyrazol-3-yl]-3-[4-(4-(N,N-di-(2-cyanoethyl)aminomethyl)phenyl)naphthalen-1-yl]-urea;
1-[5-tert-butyl-2-(6-methyl-pyridin-3-yl)-2H-pyrazol-3-yl]-3-[4-(4-(furan-2-yl-methyl)-3-hydroxyphenyl)naphthalen-1-yl]-urea;
1-[5-tert-butyl-2-(6-methyl-pyridin-3-yl)-2H-pyrazol-3-yl]-3-[4-(4-(thiomorpholin-4-yl-methyl)phenyl)naphthalen-1-yl]-urea;
1-[5-tert-butyl-2-(6-methyl-pyridin-3-yl)-2H-pyrazol-3-yl]-3-[4-(4-(3-carboxamidopiperidin-1-yl-methyl)phenyl)naphthalen-1-yl]-urea;
1-[5-tert-butyl-2-(6-methyl-pyridin-3-yl)-2H-pyrazol-3-yl]-3-[4-(4-(2-methyl-3-oxo-piperzin-1-yl-methyl)phenyl)naphthalen-1-yl]-urea;
1-[5-tert-butyl-2-(2-methylpyrimidin-5-yl)-2H-pyrazol-3-yl]-3-[4-(6-(morpholin-4-yl-methyl)pyridin-3-yl)naphthalen-1-yl]-urea;
1-[5-tert-butyl-2-(6-methyl-pyridin-3-yl)-2H-pyrazol-3-yl]-3-[4-(6-(4-hydroxybutyloxy)pyridin-3-yl)-naphthalen-1-yl]-u rea;
1-[3-tert-butyl-1'H-[1,4']bipyrazol-5-yl]-3-[4-(6-(morpholin-4-yl-methyl)pyridin-3-yl)naphthalen-1-yl]-urea;
1-[5-tert-butyl-2-(6-methyl-pyridin-3-yl)-2H-pyrazol-3-yl]-3-[4-(6-(tetrahydrothiopyran-4-yl-amino)pyridin-3-yl)-naphthalen-1-yl]-urea;
1-[5-tert-butyl-2-(2-cyanoethyl)-2H-pyrazol-3-yl]-3-[4-(6-(morpholin-4-yl-methyl)pyridin-3-yl)-naphthalen-1-yl]-urea;
1-[5-tert-butyl-2-(6-methyl-pyridin-3-yl)-2H-pyrazol-3-yl]-3-[4-(6-(2,6-dimethylmorpholin-4-yl-methyl)pyridin-3-yl)-naphthalen-1-yl]-urea;
1-[5-tert-butyl-2-(2-methoxypyridin-5-yl)-2H-pyrazol-3-yl]-3-[4-(6-(morpholin-4-yl-methyl)pyridin-3-yl)-naphthalen-1-yl]-urea;
1-[5-tert-butyl-2-(2-aminoypyridin-5-yl)-2H-pyrazol-3-yl]-3-[4-(6-(morpholin-4-yl-methyl)pyridin-3-yl)-naphthalen-1-yl]-urea;
1-[5-tert-butyl-2-(6-methyl-pyridin-3-yl)-2H-pyrazol-3-yl]-3-[4-(6-(morpholin-4-yl-4-carbonyl)pyridin-3-yl)-naphthalen-1-yl]-urea;
1-[5-tert-butyl-2-(6-methyl-pyrid i n-3-yl)-2H-pyrazol-3-yl]-3-[4-(6-(2-oxa-5-aza-bicyclo[2.2.1 ]hept-5-yl-methyl)pyridin-3-yl)-naphthalen-1-yl]-urea;
1-[5-tert-butyl-2-(6-methyl-pyridin-3-yl)-2H-pyrazol-3-yl]-3-[4-(4-(N-(2-cyanoethyl)-N-(pyridin-3-yl-methyl)aminomethyl)phenyl)-naphthalen-1-yl]-urea;
1-[5-tert-butyl-2-(6-methyl-pyridin-3-yl)-2H-pyrazol-3-yl]-3-[4-(4-(N-(2-cyanoethyl)-N-(tetrahydrofuran-2-yl-methyl)aminomethyl)phenyl)-naphthalen-1-yl]-urea;
1-[5-tert-butyl-2-(6-methyl-pyridin-3-yl)-2H-pyrazol-3-yl]-3-[4-(6-(morpholin-4-yl-methyl)-4-methoxypyridin-3-yl)-naphthalen-1-yl]-urea;
1-[5-tert-butyl-2-(6-methyl-pyridin-3-yl)-2H-pyrazol-3-yl]-3-[4-(6-(1-morpholin-4-yl-propyl)pyridin-3-yl)-naphthalen-1-yl]-urea;
1-[3-tert-butyl-1'-methyl-1'H-[1,4']bipyrazol-5-yl]-3-[4-(6-(morpholin-4-yl-methyl)pyridin-3-yl)naphthalen-1-yl]-urea;
1-[5-tert-butyl-2-(6-methyl-pyridin-3-yl)-2H-pyrazol-3-yl]-3-[4-(6-(1-oxo-tetrahydrothiopyran-4yl-amino)pyridin-3-yl)-naphthalen-1-yl]-urea;
1-[5-tert-butyl-2-(6-methyl-pyridin-3-yl)-2H-pyrazol-3-yl]-3-[4-(6-(tetrahydropyran-4yl-amino)pyridin-3-yl)-naphthalen-1-yl]-urea;
1-[5-tert-butyl-2-(6-methyl-pyridin-3-yl)-2H-pyrazol-3-yl]-3-[4-(5-(tetrahydrothiopyran-4yl-amino)pyrazin-2-yl)-naphthalen-1-yl]-urea;
1-[5-tert-butyl-2-(6-methyl-pyridin-3-yl)-2H-pyrazol-3-yl]-3-[4-(6-(methylcarbonylamino)pyridin-3-yl)-naphthalen-1-yl]-urea;
1-[3-tert-butyl-1'-(3-methylsulfanylpropyl)-1'H-[1,4']bipyrazol-5-yl]-3-[4-(6-(morpholin-4-yl-methyl)pyridin-3-yl)naphthalen-1-yl]-urea;
1-[5-tert-butyl-2-(2-methylpyrimidin-5-yl)-2H-pyrazol-3-yl]-3-[4-(6-(1-oxo-thiomorpholin-4-yl-methyl)pyridin-3-yl)naphthalen-1-yl]-urea;
1-[5-tert-butyl-2-(2-methylpyrimidin-5-yl)-2H-pyrazol-3-yl]-3-[4-(6-(tetrahydropyran-4-yl-amino)pyridin-3-yl)naphthalen-1-yl]-u rea;
1-[5-tert-butyl-2-(2-methylthiopyrimidin-5-yl)-2H-pyrazol-3-yl]-3-[4-(6-(morpholin-4-yl-methyl)pyridin-3-yl)naphthalen-1-yl]-urea;
1-[5-tert-butyl-2-(2-aminopyrimidin-5-yl)-2H-pyrazol-3-yl]-3-[4-(6-(morpholin-4-yl-methyl)pyridin-3-yl)naphthalen-1-yl]-urea;
1-[3-tert-butyl-1'-methyl-1'H-[1,4']bipyrazol-5-yl]-3-[4-(6-(morpholin-4-yl-methyl)phenyl)naphthalen-1-yl]-urea;
1-[5-tert-butyl-2-p-tolyl-2H-pyrazol-3-yl]-3-[4-(6-(1-oxo-tetrahydrothiopyran-4-yl-amino)pyridin-3-yl)naphthalen-1-yl]-urea;
1-[5-tert-butyl-2-p-tolyl-2H-pyrazol-3-yl]-3-[4-(6-(thiomorpholin-4-yl-methyl)pyridin-3-yl)naphthalen-1-yl]-urea;
1-[5-tert-butyl-2-p-tolyl-2H-pyrazol-3-yl]-3-[4-(2-(morpholin-4-yl-carbonyl)pyrimidin-5-yl)naphthalen-1-yl]-urea;
1-[5-tert-butyl-2-p-tolyl-2H-pyrazol-3-yl]-3-[4-(2-(morpholin-4-yl-methyl)pyrimidin-5-yl)naphthalen-1-yl]-urea;
1-[5-tert-butyl-2-p-tolyl-2H-pyrazol-3-yl]-3-[4-(6-(1-oxo-thiomorpholin-4-yl-methyl)pyridin-3-yl)naphthalen-1-yl]-urea;
1-[5-tert-butyl-2-(2-methylpyrimidin-5-yl)-2H-pyrazol-3-yl]-3-[4-(2-(morpholin-4-yl-methyl)pyrimidin-5-yl)naphthalen-1-yl]-urea and
the pharmaceutically acceptable derivatives thereof.

In another preferred embodiment the invention relates to pharmaceutical compositions containing **A** and **B**, characterized in that the p38 kinase inhibitor **B** is selected from the compounds of formula **6** as disclosed in WO 00/55139 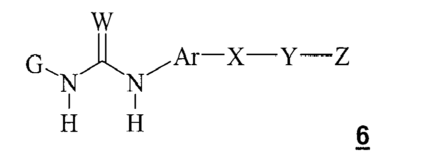 wherein:
- G: is:
an aromatic C₆₋₁₀ carbocycle or a nonaromatic C₃₋₁₀ carbocycle saturated or unsaturated;
a 6-10 membered heteroaryl containing 1 or more heteroatoms chosen from O, N and S;
a 5-8 membered monocyclic heterocycle containing one or more heteroatoms chosen from O, N and S;
or
an 8-11 membered bicyclic heterocycle, containing one or more heteroatoms chosen from O, N and S;
wherein G is substituted by one or more R₁, R₂ or R₃;
- Ar: is:
phenyl, naphthyl, quinolinyl, isoquinolinyl, tetrahydronaphthyl, tetrahydroquinolinyl, tetrahydroisoquinolinyl, benzimidazolyl, benzofuranyl, dihydrobenzofuranyl, indolinyl, benzothienyl, dihydrobenzothienyl, indanyl, indenyl or indolyl each being optionally substituted by one or more R₄ or R₅;
- X: is:
a C₅₋₈ cycloalkyl or cycloalkenyl optionally substituted with one to two oxo groups or one to three C₁₋₄ alkyl, C₁₋₄ alkoxy or C₁₋₄ alkylamino chains;
phenyl, furanyl, thienyl, pyrrolyl, pyrazolyl, imidazolyl, pyridinyl, pyrimidinyl, pyridinonyl, dihydropyridinonyl, maleimidyl, dihydromaleimidyl, piperdinyl, benzimidazole, 3H-imidazo[4,5-b]pyridine, piperazinyl, pyridazinyl or pyrazinyl;
- Y: is:
a bond or a C₁₋₄ saturated or unsaturated branched or unbranched carbon chain optionally partially or fully halogenated, wherein one or more methylene groups are optionally replaced by O, N, or S(O)ₘ and wherein Y is optionally independently substituted with one to two oxo groups, phenyl or one or more C₁₋₄ alkyl optionally substituted by one or more halogen atoms;
- Z: is:
phenyl, pyridinyl, pyrimidinyl, pyridazinyl, pyrazinyl, imidazolyl, pyrazolyl, triazolyl, tetrazolyl, furanyl, thienyl, pyranyl each being optionally substituted with one to three halogen, C₁₋₆ alkyl, C₁₋₆ alkoxy, hydroxy, amino, mono- or di-(C₁₋₃ alkyl)amino, C₁₋₆ alkyl-S(O)ₘ, CN, CONH₂, COOH or phenylamino wherein the phenyl ring is optionally substituted with one to two halogen, C₁₋₆ alkyl or C₁₋₆ alkoxy;
tetrahydropyranyl, tetrahydrofuranyl, 1,3-dioxolanonyl, 1,3-dioxanonyl, 1,4-dioxanyl, morpholinyl, thiomorpholinyl, thiomorpholino sulfoxidyl, thiomorpholino sulfonyl, piperidinyl, piperidinonyl, piperazinyl, tetrahydropyrimidonyl, cyclohexanonyl, cyclohexanolyl, pentamethylene sulfidyl, pentamethylene sulfoxidyl, pentamethylene sulfonyl, tetramethylene sulfide, tetramethylene sulfoxidyl or tetramethylene sulfonyl each being optionally substituted with one to three nitrile, C₁₋₆ alkyl, C₁₋₆ alkoxy, hydroxy, amino, mono- or di-(C₁₋₃ alkyl)amino-C₁₋₃ alkyl, CONH₂, phenylamino-C₁₋₃ alkyl or C₁₋₃ alkoxy-C₁₋₃ alkyl;
halogen, C₁₋₄ alkyl, nitrile, amino, hydroxy, C₁₋₆ alkoxy, NH₂C(O), mono- or di(C₁₋₃alkyl) aminocarbonyl, mono- or di(C₁₋₆alkyl)amino, secondary or tertiary amine wherein the amino nitrogen is covalently bonded to C₁₋₃ alkyl or C₁₋₅ alkoxyalkyl, pyridinyl-C₁₋₃ alkyl, imidazolyl-C₁₋₃ alkyl, tetrahydrofuranyl-C₁₋₃ alkyl, nitrile-C₁₋₃ alkyl, carboxamide-C₁₋₃ alkyl, phenyl, wherein the phenyl ring is optionally substituted with one to two halogen, C₁₋₆ alkoxy, hydroxy or mono- or di-(C₁₋₃ alkyl)amino, C₁₋₆ alkyl-S(O)ₘ, or phenyl-S(O)ₘ, wherein the phenyl ring is optionally substituted with one to two halogen, C₁₋₆ alkoxy, hydroxy, halogen or mono- or di-(C₁₋₃ alkyl)amino;
C₁₋₆ alkyl-S(O)ₘ, and phenyl-S(O)ₘ, wherein the phenyl ring is optionally substituted with one to two halogen, C₁₋₆ alkoxy, hydroxy or mono- or di-(C₁₋₃ alkyl)amino;
each R₁ is independently:
C₁₋₁₀ alkyl optionally be partially or fully halogenated, and optionally substituted with one to three C₃₋₁₀ cycloalkanyl, hydroxy, phenyl, naphthyl, pyridinyl, pyrimidinyl, pyrazinyl, pyridazinyl, pyrrolyl, imidazolyl, pyrazolyl, thienyl, furyl, isoxazolyl or isothiazolyl; each of the aforementioned being optionally substituted with one to five groups selected from halogen, C₁₋₆ alkyl which is optionally partially or fully halogenated, C₃₋₈ cycloalkanyl, C₅₋₈ cycloalkenyl, hydroxy, nitrile, C₁₋₃ alkoxy which is optionally partially or fully halogenated or NH₂C(O), mono- or di(C₁₋₃alkyl)amino, and mono- or di(C₁₋₃alkyl)aminocarbonyl;
cyclopropyloxy, cyclobutyloxy, cyclopentyloxy, cyclohexyloxy, or cycloheptyloxy each being optionally partially or fully halogenated and optionally substituted with one to three C₁₋₃ alkyl groups optionally partially or fully halogenated, CN, hydroxyC₁₋₃alkyl or aryl; or an analog of such cycloalkyl group wherein one to three ring methylene groups are independently replaced by O, S(O)ₘ, CHOH, >C=O, >C=S or NH;
phenyloxy or benzyloxy each being optionally partially or fully halogenated and optionally substituted with one to three C₁₋₃ alkyl groups optionally partially or fully halogenated, CN, hydroxyC₁₋₃alkyl or aryl; or an analog of such cycloaryl group wherein one to two ring methyne groups are independently replaced by N;
cyclopropanyl, cyclobutanyl, cyclopentanyl, cyclohexanyl, cycloheptanyl, bicyclopentanyl, bicyclohexanyl or bicycloheptanyl, each being optionally partially or fully halogenated and optionally substituted with one to three C₁₋₃ alkyl groups optionally partially or fully halogenated, CN, hydroxyC₁₋₃alkyl or aryl; or an analog of such cycloalkyl group wherein one to three ring methylene groups are independently replaced by O, S(O)ₘ, CHOH, >C=O, >C=S or NH;
C₃₋₁₀ branched or unbranced alkenyl each being optionally partially or fully halogenated, and optionally be substituted with one to three C₁₋₅ branched or unbranched alkyl, phenyl, naphthyl, pyridinyl, pyrimidinyl, pyrazinyl, pyridazinyl, pyrrolyl, imidazolyl, pyrazolyl, thienyl, furyl, isoxazolyl or isothiazolyl, each of the aforementioned being substituted with zero to five halogen, C₁₋₆ alkyl which is optionally partially or fully halogenated, cyclopropanyl, cyclobutanyl, cyclopentanyl, cyclohexanyl, cycloheptanyl, bicyclopentanyl, bicyclohexanyl and bicycloheptanyl, hydroxy, nitrile, C₁₋₃ alkyloxy which is optionally partially or fully halogenated, NH₂C(O), mono- or di(C₁₋₃alkyl)aminocarbonyl; the C₃₋₁₀ branched or unbranced alkenyl being optionally interrupted by one or more heteroatoms chosen from O, N and S(O)ₘ;
cyclopentenyl, cyclohexenyl, cyclohexadienyl, cycloheptenyl, cycloheptadienyl, bicyclohexenyl or bicycloheptenyl, wherein such cycloalkenyl group is optionally substituted with one to three C₁₋₃ alkyl groups;
nitrile, halogen;
methoxycarbonyl, ethoxycarbonyl and propoxycarbonyl;
silyl containing three C₁₋₄ alkyl groups optionally partially or fully halogenated;
C₃₋₆ alkynyl branched or unbranched carbon chain optionally partially or fully halogenated, wherein one or more methylene groups are optionally replaced by O, NH or S(O)ₘ and wherein said alkynyl group is optionally independently substituted with one to two oxo groups, pyrrolidinyl, pyrrolyl, one or more C₁₋₄ alkyl optionally substituted by one or more halogen atoms, nitrile, morpholino, piperidinyl, piperazinyl, imidazolyl, phenyl, pyridinyl, tetrazolyl, or mono- or di(C₁₋₃alkyl)amino optionally substituted by one or more halogen atoms;
each R₂, R₄, and R₅ is
a C₁₋₆ branched or unbranched alkyl optionally partially or fully halogenated, acetyl, aroyl, C₁₋₄ branched or unbranched alkoxy, each being optionally partially or fully halogenated, halogen, nitrile, methoxycarbonyl, C₁₋₃ alkylS(O)ₘ optionally partially or fully halogenated, or phenylsulfonyl;
C₁₋₆ alkoxy, hydroxy, amino, or mono- or di-(C₁₋₄ alkyl)amino, nitrile, halogen;
OR₆;
nitro; or
mono- or di-(C₁₋₄ alkyl)amino-S(O)₂ optionally partially or fully halogenated, or H₂NSO₂;
each R₃ is independently:
phenyl, naphthyl, morpholinyl, pyridinyl, pyrimidinyl, pyrazinyl, pyridazinyl, pyrrolyl, pyrrolidinyl, imidazolyl, pyrazolyl, thiazolyl, oxazoyl, triazolyl, tetrazolyl, thienyl, furyl, tetrahydrofuryl, isoxazolyl, isothiazolyl, quinolinyl, isoquinolinyl, indolyl, benzimidazolyl, benzofuranyl, benzoxazolyl, benzisoxazolyl, benzpyrazolyl, benzothiofuranyl, cinnolinyl, pterindinyl, phthalazinyl, naphthypyridinyl, quinoxalinyl, quinazolinyl, purinyl or indazolyl, each of the aforementioned is optionally substituted with one to three phenyl, naphthyl, heterocycle or heteroaryl as hereinabove described in this paragraph, C₁₋₆ branched or unbranched alkyl which is optionally partially or fully halogenated, cyclopropanyl, cyclobutanyl, cyclopentanyl, cyclohexanyl, cycloheptanyl, bicyclopentanyl, bicyclohexanyl, bicycloheptanyl, phenyl C₁₋₅ alkyl, naphthyl C₁₋₅ alkyl, halogen, hydroxy, oxo, nitrile, C₁₋₃ alkyloxy optionally partially or fully halogenated, phenyloxy, naphthyloxy, heteroaryloxy or heterocyclicoxy wherein the heterocyclic or heteroaryl moiety is as hereinabove described in this paragraph, nitro, amino, mono- or di-(C₁₋₃alkyl)amino, phenylamino, naphthylamino, heteroaryl or heterocyclic amino wherein the heteroaryl heterocyclic moiety is as hereinabove described in this paragraph, NH₂C(O), a mono- or di-(C₁₋₃alkyl) aminocarbonyl, C₁₋₅ alkyl-C(O)-C₁₋₄ alkyl, amino-C₁₋₅ alkyl, mono- or di-(C₁₋₃alkyl)amino-C₁₋₅ alkyl, amino-S(O)₂, di-(C₁₋₃alkyl)amino-S(O)₂, R₇-C₁₋₅ alkyl, R₈-C₁₋₅ alkoxy, R₉-C(O)-C₁₋₅ alkyl, R₁₀-C₁₋₅ alkyl(R₁₁)N, carboxy-mono- or di-(C₁₋₅alkyl)-amino;
a fused aryl selected from benzocyclobutanyl, indanyl, indenyl, dihydronaphthyl, tetrahydronaphthyl, benzocycloheptanyl and benzocycloheptenyl, or a fused heteroaryl selected from cyclopentenopyridinyl, cyclohexanopyridinyl, cyclopentanopyrimidinyl, cyclohexanopyrimidinyl, cyclopentanopyrazinyl, cyclohexanopyrazinyl, cyclopentanopyridazinyl, cyclohexanopyridazinyl, cyclopentanoquinolinyl, cyclohexanoquinolinyl, cyclopentanoisoquinolinyl, cyclohexanoisoquinolinyl, cyclopentanoindolyl, cyclohexanoindolyl, cyclopentanobenzimidazolyl, cyclohexanobenzimidazolyl, cyclopentanobenzoxazolyl, cyclohexanobenzoxazolyl, cyclopentanoimidazolyl, cyclohexanoimidazolyl, cyclopentanothienyl and cyclohexanothienyl; wherein the fused aryl or fused heteroaryl ring is independently substituted with zero to three phenyl, naphthyl, pyridinyl, pyrimidinyl, pyrazinyl, pyridazinyl, pyrrolyl, imidazolyl, pyrazolyl, thienyl, furyl, isoxazolyl, isothiazolyl, C₁₋₆ alkyl which is optionally partially or fully halogenated, halogen, nitrile, C₁₋₃ alkyloxy which is optionally partially or fully halogenated, phenyloxy, naphthyloxy, heteroaryloxy or heterocyclicoxy wherein the heteroaryl or heterocyclic moiety is as hereinabove described in this paragraph, nitro, amino, mono- or di-(C₁₋₃alkyl)amino, phenylamino, naphthylamino, heteroaryl or heterocyclic amino wherein the heteroaryl or heterocyclic moiety is as hereinabove described in this paragraph, NH₂C(O), mono- or di-(C₁₋₃alkyl)aminocarbonyl, C₁₋₄ alkyl-OC(O), C₁₋₅ alkyl-C(O)-C₁₋₄ alkyl, amino-C₁₋₅ alkyl, mono- or di-(C₁₋₃)alkylamino-C₁₋₅ alkyl, R₁₂-C₁₋₅ alkyl, R₁₃-C₁₋₅ alkoxy, R₁₄-C(O)-C₁₋₅ alkyl or R₁₅-C₁₋₅ alkyl(R₁₆)N;
cyclopropanyl, cyclobutanyl, cyclopentanyl, cyclohexanyl, cycloheptanyl, bicyclopentanyl, bicyclohexanyl or bicycloheptanyl, each being optionally partially or fully halogenated and optionally substituted with one to three C₁₋₃ alkyl groups, or an analog of such cycloalkyl group wherein one to three ring methylene groups are independently replaced by O, S, CHOH, >C=O, >C=S or NH;
cyclopentenyl, cyclohexenyl, cyclohexadienyl, cycloheptenyl, cycloheptadienyl, bicyclohexenyl or bicycloheptenyl, each optionally substituted with one to three C₁₋₃ alkyl groups;
C₁₋₄ alkyl-phenyl-C(O)-C₁₋₄ alkyl-, C₁₋₄ alkyl-C(O)-C₁₋₄ alkyl- or C₁₋₄ alkylphenyl-S(O)ₘ-C₁₋₄ alkyl-;
C₁₋₆ alkyl or C₁₋₆ branched or unbranched alkoxy each of which is optionally partially or fully halogenated or optionally substituted with R₁₇;
OR₁₈ or C₁₋₆ alkyl optionally substituted with OR₁₈;
amino or mono- or di-(C₁₋₅alkyl)amino optionally substituted with R₁₉;
R₂₀C(O)N(R₂₁)-, R₂₂O- or R₂₃R₂₄NC(O)-; R₂₆(CH₂)ₘC(O)N(R₂₁)- or R₂₆C(O)(CH₂)ₘN(R₂₁)-;
C₂₋₆alkenyl substituted by R₂₃R₂₄NC(O)-;
C₂₋₆ alkynyl branched or unbranched carbon chain, optionally partially or fully halogenated, wherein one or more methylene groups are optionally replaced by O, NH, S(O)ₘ and wherein said alkynyl group is optionally independently substituted with one to two oxo groups, pyrroldinyl, pyrrolyl, morpholinyl, piperidinyl, piperazinyl, imidazolyl, phenyl, pyridinyl, tetrazolyl one or more C₁₋₄ alkyl optionally substituted by one or more halogen atoms, nitrile, morpholino, piperidinyl, piperazinyl, imidazolyl, phenyl, pyridinyl, tetrazolyl, or mono- or di(C₁₋₄ alkyl)amino optionally substituted by one or more halogen atoms; or
aroyl;
R₆ is a:
C₁₋₄ alkyl optionally partially or fully halogenated and optionally substituted with R₂₆;
each R₇, R₈, R₉, R₁₀, R₁₂, R₁₃, R₁₄, R₁₅, R₁₇, R₁₉, R₂₅ and R₂₆ is independently: nitrile, phenyl, morpholino, piperidinyl, piperazinyl, imidazolyl, pyridinyl, tetrazolyl, amino or mono- or di-(C₁₋₄alkyl)amino optionally partially or fully halogenated;
each R₁₁ and R₁₆ is independently:
hydrogen or C₁₋₄ alkyl optionally partially or fully halogenated;
R₁₈ is independently:
hydrogen or a C₁₋₄ alkyl optionally independently substituted with oxo or R₂₅;
R₂₀ is independently:
C₁₋₁₀ alkyl optionally partially or fully halogenated, phenyl, or pyridinyl;
R₂₁ is independently:
hydrogen or C₁₋₃ alkyl optionally partially or fully halogenated;
each R₂₂, R₂₃ and R₂₄ is independently:
hydrogen, C₁₋₆ alkyl optionally partially or fully halogenated, said C₁₋₆ alkyl is optionally interrupted by one or more O, N or S, said C₁₋₆ alkyl also being independently optionally substituted by mono- or di-(C₁₋₃alkyl)aminocarbonyl, phenyl, pyridinyl, amino or mono- or di-(C₁₋₄alkyl)amino each of which is optionally partially or fully halogenated and optionally substituted with mono-or di-(C₁₋₃alkyl)amino;
or R₂₃ and R₂₄ taken together optionally form a heterocyclic or heteroaryl ring;
m = 0, 1 or 2;
W is O or S and
pharmaceutically acceptable derivatives thereof.

In another preferred embodiment the invention relates to pharmaceutical compositions containing **A** and **B**, characterized in that the p38 kinase inhibitor **B** is selected from the compounds of formula **6** wherein
- G is:: phenyl, naphthyl, benzocyclobutanyl, dihydronaphthyl, tetrahydronaphthyl, benzocycloheptanyl, benzocycloheptenyl, indanyl, indenyl;
pyridinyl, pyridonyl, quinolinyl, dihydroquinolinyl, tetrahydroquinoyl, isoquinolinyl, tetrahydroisoquinoyl, pyridazinyl, pyrimidinyl, pyrazinyl, benzimidazolyl, benzthiazolyl, benzoxazolyl, benzofuranyl, benzothiophenyl, benzpyrazolyl, dihydrobenzofuranyl, dihydrobenzothiophenyl, benzooxazolonyl, benzo[1,4]oxazin-3-onyl, benzodioxolyl, benzo[1,3]dioxol-2-onyl, benzofuran-3-onyl, tetrahydrobenzopyranyl, indolyl, indolinyl, indolonyl, indolinonyl, phthalimidyl;
oxetanyl, pyrrolidinyl, tetrahydrofuranyl, tetrahydrothiophenyl, piperidinyl, piperazinyl, morpholinyl, tetrahydropyranyl, dioxanyl, tetramethylene sulfonyl, tetramethylene sulfoxidyl, oxazolinyl, thiazolinyl, imidazolinyl, tertrahydropyridinyl, homopiperidinyl, pyrrolinyl, tetrahydropyrimidinyl, decahydroquinolinyl, decahydroisoquinolinyl, thiomorpholinyl, thiazolidinyl, dihydrooxazinyl, dihydropyranyl, oxocanyl, heptacanyl, thioxanyl or dithianyl;
wherein G is substituted by one or more R₁, R₂ or R₃;

In a further preferred embodiment the invention relates to pharmaceutical compositions containing **A** and **B**, characterized in that the p38 kinase inhibitor B is selected from the compounds of formula **6** wherein
- G is: phenyl, pyridinyl, pyridonyl, naphthyl, quinolinyl, isoquinolinyl, pyrazinyl, benzimidazolyl, benzoxazolyl, benzofuranyl, benzothiophenyl, benzpyrazolyl, dihydrobenzofuranyl, dihydrobenzothiophenyl, indanyl, indenyl, indolyl, indolinyl, indolonyl or indolinonyl, wherein G is substituted by one or more R₁, R₂ or R₃;
- Ar is:: naphthyl, quinolinyl, isoquinolinyl, tetrahydronaphthyl, tetrahydroquinolinyl, tetrahydroisoquinolinyl, indanyl, indenyl or indolyl each being optionally substituted by one or more R₄ or R₅ groups;
- X is:: phenyl, furanyl, thienyl, pyrrolyl, pyrazolyl, imidazolyl, pyridinyl, pyrimidinyl, pyridinonyl, dihydropyridinonyl, maleimidyl, dihydromaleimidyl, piperdinyl, piperazinyl, pyridazinyl or pyrazinyl
- Y is:: a bond or
a C₁₋₄ saturated or unsaturated carbon chain wherein one of the carbon atoms is optionally replaced by O, N, or S(O)ₘ and wherein Y is optionally independently substituted with one to two oxo groups, phenyl or one or more C₁₋₄ alkyl optionally substituted by one or more halogen atoms;
- Z is:: phenyl, pyridinyl, pyrimidinyl, pyridazinyl, pyrazinyl, imidazolyl, furanyl, thienyl, dihydrothiazolyl, dihydrothiazolyl sulfoxidyl, pyranyl, pyrrolidinyl which are optionally substituted with one to three nitrile, C₁₋₃ alkyl, C₁₋₃ alkoxy, amino, mono- or di-(C₁₋₃ alkyl)amino, CONH₂ or OH;
tetrahydropyranyl, tetrahydrofuranyl, 1,3-dioxolanonyl, 1,3-dioxanonyl, 1,4-dioxanyl, morpholinyl, thiomorpholinyl, thiomorpholino sulfoxidyl, piperidinyl, piperidinonyl, piperazinyl, tetrahydropyrimidonyl, pentamethylene sulfidyl, pentamethylene sulfoxidyl, pentamethylene sulfonyl, tetramethylene sulfidyl, tetramethylene sulfoxidyl or tetramethylene sulfonyl which are optionally substituted with one to three nitrile, C₁₋₃ alkyl, C₁₋₃ alkoxy, amino, mono- or di-(C₁₋₃ alkyl)amino, CONH₂, or OH;
nitrile, C₁₋₆ alkyl-S(O)ₘ, halogen, hydroxy, C₁₋₄ alkoxy, amino, mono- or di-(C₁₋₆ alkyl)amino, mono- or di-(C₁₋₃ alkyl)aminocarbonyl or NH₂C(O);
- each R₁: is independently:
C₃₋₆ alkyl optionally partially or fully halogenated, and optionally substituted with one to three C₃₋₆cycloalkyl, phenyl, thienyl, furyl, isoxazolyl or isothiazolyl; each of the aforementioned being optionally substituted with one to three groups selected from halogen, C₁₋₃ alkyl which is optionally partially or fully halogenated, hydroxy, nitrile or C₁₋₃alkoxy which is optionally partially or fully halogenated;
cyclopropyl, cyclobutyl, cyclopentanyl, cyclohexanyl, bicyclopentanyl or bicyclohexanyl, each being optionally partially or fully halogenated and optionally substituted with one to three C₁₋₃ alkyl groups optionally partially or fully halogenated, CN, hydroxyC₁₋₃alkyl or phenyl; or an analog of such cycloalkyl group wherein one to three ring methylene groups are independently replaced by O, S, CHOH, >C=O, >C=S or NH; or
silyl containing three C₁₋₄ alkyl groups optionally partially or fully halogenated;
- R₂: is independently:
halogen, C₁₋₃ alkoxy, C₁₋₃ alkyl-S(O)ₘ optionally partially or fully halogenated, phenylsulfonyl or nitrile;
- R₃: is independently:
phenyl, morpholino, pyridinyl, pyrimidinyl, pyrazinyl, pyrrolyl, pyrrolylidinyl, imidazolyl, pyrazolyl, each being optionally substituted with one to three phenyl, naphthyl, heterocycle or heteroaryl as hereinabove described in this paragraph, C₁₋₆ alkyl which is optionally partially or fully halogenated, cyclopropanyl, cyclobutanyl, cyclopentanyl, cyclohexanyl, cycloheptanyl, bicyclopentanyl, bicyclohexanyl, bicycloheptanyl, phenyl C₁₋₅alkyl, naphthyl C₁₋₅ alkyl, halogen, oxo, hydroxy, nitrile, C₁₋₃ alkyloxy optionally partially or fully halogenated, phenyloxy, naphthyloxy, heteroaryloxy or heterocyclicoxy
   wherein the heteroaryl or heterocyclic moiety is as hereinabove described in this paragraph, nitro, amino, mono- or di-(C₁₋₃alkyl)amino, phenylamino, naphthylamino, heteroaryl or heterocyclic amino wherein the heteroaryl or heterocyclic moiety is as hereinabove described in this paragraph, NH₂C(O), a mono- or di-(C₁₋₃alkyl)aminocarbonyl, C₁₋₅ alkyl-C(O)-C₁₋₄ alkyl, mono- or di-(C₁₋₃alkyl)amino, mono- or di-(C₁₋₃)alkylamino-C₁₋₅ alkyl, mono- or di-(C₁₋₃alkyl)amino-S(O)₂, R₇-C₁₋₅ alkyl, R₈-C₁₋₅ alkoxy, R₉-C(O)-C₁₋₅ alkyl, R₁₀-C₁₋₅ alkyl(R₁₁)N, carboxy-mono- or di-(C₁₋₅)-alkyl-amino;
C₁₋₃ alkyl or C₁₋₄ alkoxy each being optionally partially or fully halogenated or optionally substituted with R₁₇;
OR₁₈ or C₁₋₆ alkyl optionally substituted with OR₁₈;
amino or mono- or di- (C₁₋₅ alkyl)amino optionally substituted with R₁₉;
R₂₀C(O)N(R₂₁)-, R₂₂O- ; R₂₃R₂₄NC(O)-; R₂₆CH₂C(O)N(R₂₁)- or
R₂₆C(O)CH₂N(R₂₁)-;
C₂₋₄alkenyl substituted by R₂₃R₂₄NC(O)-; or
C₂₋₄ alkynyl branched or unbranched carbon chain optionally partially or fully halogenated and optionally independently substituted with one to two oxo groups, pyrroldinyl, pyrrolyl, morpholinyl, piperidinyl, piperazinyl, imidazolyl, phenyl, pyridinyl, tetrazolyl or one or more C₁₋₄ alkyl optionally substituted by one or more halogen atoms; and

R₂₃ and R₂₄ taken together optionally form imidazolyl, piperidinyl, morpholinyl, piperazinyl or a pyridinyl ring.

In yet another preferred embodiment the invention relates to pharmaceutical compositions containing **A** and **B**, characterized in that the p38 kinase inhibitor **B** is selected from the compounds of formula **6** wherein:
- G: is phenyl, pyridinyl, pyridonyl, naphthyl, quinolinyl, isoquinolinyl, pyrazinyl, benzothiophenyl, dihydrobenzofuranyl, dihydrobenzothiophenyl, indanyl, indolyl, indolinyl, indolonyl or indolinonyl, wherein G is substituted by one or more R₁, R₂ or R₃;
- Ar: is naphthyl;
- X is: phenyl, imidazolyl, pyridinyl, pyrimidinyl, piperdinyl, piperazinyl, pyridazinyl or pyrazinyl each being optionally independently substituted with one to three C₁₋₄ alkyl, C₁₋₄alkoxy, hydroxy, nitrile, amino, mono- or di-(C₁₋₃ alkyl)amino, mono-or di-(C₁₋₃ alkylamino)carbonyl, NH₂C(O), C₁₋₆ alkyl-S(O)ₘ or halogen;
- Y is:: a bond or
a C₁₋₄ saturated carbon chain wherein one of the carbon atoms is optionally replaced by O, N or S and wherein Y is optionally independently substituted with an oxo group;
- Z is:: phenyl, pyridinyl, pyrimidinyl, pyridazinyl, pyrazinyl, imidazolyl, dihydrothiazolyl, dihydrothiazolyl sulfoxide, pyranyl or pyrrolidinyl which are optionally substituted with one to two C₁₋₂ alkyl or C₁₋₂ alkoxy;
tetrahydropyranyl, morpholinyl, thiomorpholinyl, thiomorpholino sulfoxidyl, piperidinyl, piperidinonyl, piperazinyl or tetrahydropyrimidonyl which are optionally substituted with one to two C₁₋₂ alkyl or C₁₋₂ alkoxy; or
C₁₋₃ alkoxy;
each R₁ is independently:
C₃₋₅ alkyl optionally partially or fully halogenated, and optionally substituted with phenyl substituted with zero to three halogen, C₁₋₃ alkyl which is optionally partially or fully halogenated, hydroxy, nitrile or C₁₋₃alkoxy which is optionally partially or fully halogenated;
cyclopropyl, cyclobutyl, cyclopentanyl, cyclohexanyl, bicyclopentanyl or bicyclohexanyl, each being optionally partially or fully halogenated and optionally substituted with one to three C₁₋₃ alkyl groups optionally partially or fully halogenated, CN, hydroxyC₁₋₃alkyl or phenyl; and an analog of cyclopropyl, cyclobutyl, cyclopentanyl, cyclohexanyl, bicyclopentanyl or bicyclohexanyl wherein one ring methylene group is replaced by O; and
silyl containing three C₁₋₂ independently alkyl groups optionally partially or fully halogenated;
each R₂ is independently:
bromo, chloro, fluoro, methoxy, methylsulfonyl or nitrile;
each R₃ is independently:
phenyl, morpholino, pyridinyl, pyrimidinyl, pyrrolylidinyl, 2,5-pyrrolidin-dionyl, imidazolyl, pyrazolyl, each of the aforementioned is optionally substituted with one to three C₁₋₃ alkyl which is optionally partially or fully halogenated, halogen, oxo, hydroxy, nitrile and C₁₋₃ alkyloxy optionally partially or fully halogenated;
C₁₋₃ alkyl or C₁₋₃ alkoxy each being optionally partially or fully halogenated or optionally substituted with R₁₇;
OR₁₈ or C₁₋₃ alkyl optionally substituted with OR₁₈;
amino or mono- or di-(C₁₋₃ alkyl)amino optionally substituted with R₁₉;
R₂₀C(O)N(R₂₁)-, R₂₂O- ; R₂₃R₂₄NC(O)-; R₂₆CH₂C(O)N(R₂₁)- or
R₂₆C(O)CH₂N(R₂₁)-;
C₂₋₄ alkenyl substituted by R₂₃R₂₄NC(O)-; or
C₂₋₄ alkynyl substituted with pyrroldinyl or pyrrolyl;
and

R₂₃ and R₂₄ taken together optionally form morpholino.

In yet another preferred embodiment the invention relates to pharmaceutical compositions containing **A** and **B**, characterized in that the p38 kinase inhibitor **B** is selected from the compounds of formula **6** wherein
- G is: phenyl, pyridinyl, pyridonyl, naphthyl, quinolinyl, isoquinolinyl, dihydrobenzofuranyl, indanyl, indolinyl, indolonyl, or indolinonyl, wherein G is substituted by one or more R₁, R₂ or R₃;
- Ar is: 1-naphthyl;
- X is:: phenyl, imidazolyl, pyridinyl, pyrimidinyl, piperdinyl, piperazinyl, pyridazinyl or pyrazinyl;
- Y is:: a bond or
-CH₂-, -CH₂CH₂-, -C(O)-, -O-, -S-, -NH-CH₂CH₂CH₂-, -N(CH₃)-, or -NH-;
each R₁ is independently:
C₃₋₅ alkyl optionally partially or fully halogenated, and optionally substituted with phenyl;
cyclopropyl, cyclopentanyl, cyclohexanyl and bicyclopentanyl optionally substituted with one to three methyl groups optionally partially or fully halogenated, CN, hydroxymethyl or phenyl; or 2-tetrahydrofuranyl substituted by methyl; or trimethyl silyl;
each R₃ is independently:
phenyl, morpholinyl, pyridinyl, pyrimidinyl, pyrrolylidinyl, 2,5-pyrrolidin-dionyl, imidazolyl or pyrazolyl, wherein any of the aforementioned is optionally substituted with C₁₋₂ alkyl which is optionally partially or fully halogenated;
C₁₋₃ alkyl or C₁₋₃ alkoxy each being optionally partially or fully halogenated or optionally substituted with diethylamino;
OR₁₈ or C₁₋₃ alkyl optionally substituted with OR₁₈;
amino or mono- or di-(C₁₋₃ alkyl)amino optionally substituted with R₁₉;
CH₃C(O)NH-, R₂₂O- ; R₂₃R₂₄NC(O)-; R₂₆CH₂C(O)N(R₂₁)- or
R₂₆C(O)CH₂N(R₂₁)-;
C₂₋₄alkenyl substituted by R₂₃R₂₄NC(O)-; or
C₂₋₄ alkynyl substituted with pyrroldinyl or pyrrolyl;

R₂₃ and R₂₄ are H or R₂₃ and R₂₄ taken together optionally form morpholino; and R₂₆ is morpholino.

In a further preferred embodiment the invention relates to pharmaceutical compositions containing **A** and **B**, characterized in that the p38 kinase inhibitor **B** is selected from the compounds of formula **6**
- G is: phenyl, pyridinyl or naphthyl wherein G is substituted by one or more R₁, R₂ or R₃;
- X is:: imidazolyl or pyridinyl;
- Y is:: -CH₂₋, -NH-CH₂CH₂CH₂- or -NH-;
- Z: is morpholino;
- each R₁: is independently:
tert-butyl, sec-butyl, tert-amyl or phenyl;
- R₂: is chloro;
- R₃: is independently:
methyl, methoxy, methoxymethyl, hydroxypropyl, acetamide, morpholino or morpholinocarbonyl.

In yet a further preferred embodiment the invention relates to pharmaceutical compositions containing **A** and **B**, characterized in that the p38 kinase inhibitor **B** is selected from the compounds of formula **6** wherein X is pyridinyl.

In yet a still further preferred embodiment the invention relates to pharmaceutical compositions containing **A** and **B**, characterized in that the p38 kinase inhibitor **B** is selected from the compounds of formula **6** wherein the pyridinyl is attached to Ar via the 3-pyridinyl position.

Preferably the invention relates to pharmaceutical compositions containing **A** and **B**, characterized in that the p38 kinase inhibitor **B** is selected from the following compounds of formula **6**
1-(3-Cyano-phenyl)-3-[4-(6-morpholin-4-ylmethyl-pyridin-3-yl)-naphthalen-1-yl]-urea
1-(3-Fluoro-phenyl)-3-[4-(6-morpholin-4-ylmethyl-pyridin-3-yl)-naphthalen-1-yl]-urea
1-(4-Chloro-2-trifluoromethyl-phenyl)-3-[4-(6-morpholin-4-ylmethyl-pyridin-3-yl)-naphthalen-1-yl]-urea
1-(2-Chloro-5-trifluoromethyl-phenyl)-3-[4-(6-morpholin-4-ylmethyl-pyridin-3-yl)-naphthalen-1-yl]-urea
1-(3,4-Dimethyl-phenyl)-3-[4-(6-morpholin-4-ylmethyl-pyridin-3-yl)-naphthalen-1-yl]-urea
1-(3-lodo-phenyl)-3-[4-(6-morpholin-4-ylmethyl-pyridin-3-yl)-naphthalen-1-yl]-urea
1-[4-(6-Morpholin-4-ylmethyl-pyridin-3-yl)-naphthalen-1-yl]-3-m-tolyl-urea
1-(4-Methylsulfanyl-phenyl)-3-[4-(6-morpholin-4-ylmethyl-pyridin-3-yl)-naphthalen-1-yl]-urea
1-(3-Chloro-4-methyl-phenyl)-3-[4-(6-morpholin-4-ylmethyl-pyridin-3-yl)-naphthalen-1-yl]-u rea
1-(4-Chloro-3-nitro-phenyl)-3-[4-(6-morpholin-4-ylmethyl-pyrid in-3-yl)-naphthalen-1-yl]-u rea
1-(2,5-Dichloro-phenyl)-3-[4-(6-morpholin-4-ylmethyl-pyridin-3-yl)-naphthalen-1-yl]-urea
1-[4-(6-Morpholin-4-ylmethyl-pyridin-3-yl)-naphthalen-1-yl]-3-naphthalen-2-yl-urea
1-[4-(6-Morpholin-4-ylmethyl-pyridin-3-yl)-naphthalen-1-yl]-3-phenyl-urea
1-(3-Chloro-phenyl)-3-[4-(6-morpholin-4-ylmethyl-pyridin-3-yl)-naphthalen-1-yl]-urea
1-(4-Chloro-3-trifluoromethyl-phenyl)-3-[4-(6-morpholin-4-ylmethyl-pyridin-3-yl)-naphthalen-1-yl]-urea
1-[4-(6-Morpholin-4-ylmethyl-pyridin-3-yl)-naphthalen-1-yl]-3-(2,4,6-trichloro-phenyl)-urea
1-(2-Methyl-3-nitro-phenyl)-3-[4-(6-morpholin-4-ylmethyl-pyridin-3-yl)-naphthalen-1-yl]-u rea
1-(4-Methyl-2-nitro-phenyl)-3-[4-(6-morpholin-4-ylmethyl-pyridin-3-yl)-naphthalen-1-yl]-urea
1-(2,3-Dichloro-phenyl)-3-[4-(6-morpholin-4-ylmethyl-pyridin-3-yl)-naphthalen-1-yl]-urea
1-(2-Methoxy-5-methyl-phenyl)-3-[4-(6-morpholin-4-ylmethyl-pyridin-3-yl)-naphthalen-1-yl]-urea
1-(2-Chloro-6-methyl-phenyl)-3-[4-(6-morpholin-4-ylmethyl-pyrid in-3-yl)-naphthalen-1-yl]-urea
1-(2,4-Dichloro-phenyl)-3-[4-(6-morpholin-4-ylmethyl-pyridin-3-yl)-naphthalen-1-yl]-urea
1-(4-Methyl-3-nitro-phenyl)-3-[4-(6-morpholin-4-ylmethyl-pyridin-3-yl)-naphthalen-1-yl]-urea
1-(2,4-Dimethyl-phenyl)-3-[4-(6-morpholin-4-ylmethyl-pyridin-3-yl)-naphthalen-1-yl]-urea
1-(2,3-Dimethyl-phenyl)-3-[4-(6-morpholin-4-ylmethyl-pyridin-3-yl)-naphthalen-1-yl]-urea
1-(4-Cyano-phenyl)-3-[4-(6-morpholin-4-ylmethyl-pyridin-3-yl)-naphthalen-1-yl]-urea
1-[4-(6-Morpholin-4-ylmethyl-pyridin-3-yl)-naphthalen-1-yl]-3-(3,4,5-trimethoxyphenyl)-urea
1-Biphenyl-4-yl-3-[4-(6-morpholin-4-ylmethyl-pyridin-3-yl)-naphthalen-1-yl]-urea
1-(2,5-Difluoro-phenyl)-3-[4-(6-morpholin-4-ylmethyl-pyridin-3-yl)-naphthalen-1-yl]-urea
1-(3-Chloro-2-methoxy-phenyl)-3-[4-(6-morpholin-4-ylmethyl-pyridin-3-yl)-naphthalen-1-yl]-urea
1-(2-Fluoro-3-trifluoromethyl-phenyl)-3-[4-(6-morpholin-4-ylmethyl-pyridin-3-yl)-naphthalen-1-yl]-urea
1-(4-Benzyloxy-phenyl)-3-[4-(6-morpholin-4-ylmethyl-pyridin-3-yl)-naphthalen-1-yl]-urea
1-(2-Methylsulfanyl-phenyl)-3-[4-(6-morpholin-4-ylmethyl-pyridin-3-yl)-naphthalen-1-yl]-urea
1-(2-Fluoro-6-trifluoromethyl-phenyl)-3-[4-(6-morpholin-4-ylmethyl-pyridin-3-yl)-naphthalen-1-yl]-urea
1-(4-Fluoro-3-trifluoromethyl-phenyl)-3-[4-(6-morpholin-4-ylmethyl-pyridin-3-yl)-naphthalen-1-yl]-urea
1-[4-(6-Morpholin-4-ylmethyl-pyridin-3-yl)-naphthalen-1-yl]-3-(2,4,5-trimethyl-phenyl)-urea
1-[4-(6-Morpholin-4-ylmethyl-pyridin-3-yl)-naphthalen-1-yl]-3-(4-trifluoromethylphenyl)-urea
1-(3-Methylsulfanyl-phenyl)-3-[4-(6-morpholin-4-ylmethyl-pyridin-3-yl)-naphthalen-1-yl]-urea
1-(2-Methoxy-phenyl)-3-[4-(6-morpholin-4-ylmethyl-pyridin-3-yl)-naphthalen-1-yl]-urea
1-(2-Fluoro-5-trifluoromethyl-phenyl)-3-[4-(6-morpholin-4-ylmethyl-pyridin-3-yl)-naphthalen-1-yl]-u rea
1-(4-Methoxy-2-methyl-phenyl)-3-[4-(6-morpholin-4-ylmethyl-pyridin-3-yl)-naphthalen-1-yl]-urea
1-(2-Fluoro-5-nitro-phenyl)-3-[4-(6-morpholin-4-ylmethyl-pyridin-3-yl)-naphthalen-1-yl]-urea
1-(4-Ethoxy-phenyl)-3-[4-(6-morpholin-4-ylmethyl-pyridin-3-yl)-naphthalen-1-yl]-urea
1-(2,5-Dimethoxy-phenyl)-3-[4-(6-morpholin-4-ylmethyl-pyridin-3-yl)-naphthalen-1-yl]-urea
1-(4,5-Dimethyl-2-nitro-phenyl)-3-[4-(6-morpholin-4-ylmethyl-pyridin-3-yl)-naphthalen-1-yl]-urea
1-(5-Chloro-2-methyl-phenyl)-3-[4-(6-morpholin-4-ylmethyl-pyridin-3-yl)-naphthalen-1-yl]-urea
1-(2-Isopropyl-6-methyl-phenyl)-3-[4-(6-morpholin-4-ylmethyl-pyridin-3-yl)-naphthaten-1-yl]-urea
1-(2-Difluoromethoxy-phenyl)-3-[4-(6-morpholin-4-ylmethyl-pyridin-3-yl)-naphthalen-1-yl]-u rea
1-(4-Isopropyl-phenyl)-3-[4-(6-morpholin-4-ylmethyl-pyridin-3-yl)-naphthalen-1-yl]-urea
1-(4-Methoxy-phenyl)-3-[4-(6-morpholin-4-ylmethyl-pyridin-3-yl)-naphthalen-1-yl]-urea
1-(3-Ethyl-phenyl)-3-[4-(6-morpholin-4-ylmethyl-pyridin-3-yl)-naphthalen-1-yl]-urea
1-(2-Ethoxy-phenyl)-3-[4-(6-morpholin-4-ylmethyl-pyridin-3-yl)-naphthalen-1-yl]-urea
1-(4-Butoxy-phenyl)-3-[4-(6-morpholin-4-ylmethyl-pyridin-3-yl)-naphthalen-1-yl]-urea
4-{3-[4-(6-Morpholin-4-ylmethyl-pyridin-3-yl)-naphthalen-1-yl]-ureido}-benzoic acid ethyl ester
1-(4-Butyl-2-methyl-phenyl)-3-[4-(6-morpholin-4-ylmethyl-pyridin-3-yl)-naphthalen-1-yl]-urea
1-(2,6-Dibromo-4-isopropyl-phenyl)-3-[4-(6-morpholin-4-ylmethyl-pyridin-3-yl)-naphthalen-1-yl]-urea
1-(3-Methoxy-phenyl)-3-[4-(6-morpholin-4-ylmethyl-pyridin-3-yl)-naphthalen-1-yl]-urea
1-[4-(6-Morpholin-4-ylmethyl-pyridin-3-yl)-naphthalen-1-yl]-3-(4-trifluoromethylsulfanyl-phenyl)-urea
5-{3-[4-(6-Morpholin-4-ylmethyl-pyridin-3-yl)-naphthalen-1-yl]-ureido}-isophthalic acid dimethyl ester
1-(3-Cyclopentyloxy-4-methoxy-phenyl)-3-[4-(6-morpholin-4-ylmethyl-pyridin-3-yl)-naphthalen-1-yl]-urea
3-{3-[4-(6-Morpholin-4-ylmethyl-pyridin-3-yl)-naphthalen-1-yl]-ureido}-benzoic acid ethyl ester
1-(5-tert-Butyl-2-hydroxy-phenyl)-3-[4-(6-morpholin-4-ylmethyl-pyridin-3-yl)-naphthalen-1-yl]-urea
1-(2-Hydroxymethyl-4-phenyl-cyclohexyl)-3-[4-(6-morpholin-4-ylmethyl-pyridin-3-yl)-naphthalen-1-yl]-urea
1-(2-Methylsulfanyl-5-trifluoromethyl-phenyl)-3-[4-(6-morpholin-4-ylmethyl-pyridin-3-yl)-naphthalen-1-yl]-urea
1-[4-(6-Morpholin-4-ylmethyl-pyridin-3-yl)-naphthalen-1-yl]-3-(4-pentyloxy-biphenyl-3-yl)-urea
4-Methoxy-3-{3-[4-(6-morpholin-4-ylmethyl-pyridin-3-yl)-naphthalen-1-yl]-ureido}-benzoic acid methyl ester
1-(2,5-Diethoxy-phenyl)-3-[4-(6-morpholin-4-ylmethyl-pyridin-3-yl)-naphthalen-1-yl]-urea
1-Benzothiazol-6-yl-3-[4-(6-morpholin-4-ylmethyl-pyridin-3-yl)-naphthalen-1-yl]-urea
N-(2,5-Diethoxy-4-{3-[4-(6-morpholin-4-ylmethyl-pyridin-3-yl)-naphthalen-1-yl]-ureido}-phenyl)-benzamide
1-[4-(6-Morpholin-4-ylmethyl-pyridin-3-yl)-naphthalen-1-yl]-3-(3-phenoxy-phenyl)-urea
1-(5-Ethanesulfonyl-2-methoxy-phenyl)-3-[4-(6-morpholin-4-ylmethyl-pyrid in-3-yl)-naphthalen-1-yl]-urea
4-Methoxy-3-{3-[4-(6-morpholin-4-ylmethyl-pyridin-3-yl)-naphthalen-1-yl]-ureido)-N-phenyl-benzamide
1 -(2-Methyl-1,3-dioxo-2,3-dihydro-1H-isoindol-5-yl)-3-[4-(6-morpholin-4-ylmethyl-pyridin-3-yl)-naphthalen-1-yl]-urea
1-(2,3-Dimethyl-1 H-indol-5-yl)-3-[4-(6-morpholin-4-ylmethyl-pyridin-3-yl)-naphthalen-1-yl]-urea
N-Butyl-4-methoxy-3-{3-[4-(6-morpholin-4-ylmethyl-pyridin-3-yl)-naphthalen-1-yl]-ureido}-benzenesulfonamide
1-[3-(2-Methyl-[1,3]dioxolan-2-yl)-phenyl]-3-[4-(6-morpholin-4-ylmethyl-pyridin-3-yl)-naphthalen-1-yl]-urea
1-(3-Methoxy-5-trifluoromethyl-phenyl)-3-[4-(6-morpholin-4-ylmethyl-pyridin-3-yl)-naphthalen-1-yl]-urea
1-(2,4-Dimethoxy-phenyl)-3-[4-(6-morpholin-4-ylmethyl-pyrid in-3-yl)-naphthalen-1-yl]-u rea
1-(2-Methyl-4-nitro-phenyl)-3-[4-(6-morpholin-4-ylmethyl-pyridin-3-yl)-naphthalen-1-yl]-urea
1-(2-Methoxy-4-nitro-phenyl)-3-[4-(6-morpholin-4-ylmethyl-pyridin-3-yl)-naphthalen-1-yl]-urea
1-(4-Chloro-2-nitro-phenyl)-3-[4-(6-morpholin-4-ylmethyl-pyridin-3-yl)-naphthalen-1-yl]-urea
1-(5-Chloro-2-methoxy-phenyl)-3-[4-(6-morpholin-4-ylmethyl-pyridin-3-yl)-naphthalen-1-yl]-urea
1-(3,5-Dimethoxy-phenyl)-3-[4-(6-morpholin-4-ylmethyl-pyridin-3-yl)-naphthalen-1-yl]-urea
1-[4-(6-Morpholin-4-ylmethyl-pyridin-3-yl)-naphthalen-1-yl]-3-(4-trifluoromethoxyphenyl)-urea
1-[4-(6-Morpholin-4-ylmethyl-pyridin-3-yl)-naphthalen-1-yl]-3-(3-trifluoromethylsulfanyl-phenyl)-urea
1-[4-(6-Morpholin-4-ylmethyl-pyridin-3-yl)-naphthalen-1-yl]-3-(2-phenoxy-phenyl)-urea
1-(2-Methoxy-5-nitro-phenyl)-3-[4-(6-morpholin-4-ylmethyl-pyridin-3-yl)-naphthalen-1-yl]-urea
1-(5-Chloro-2,4-dimethoxy-phenyl)-3-[4-(6-morpholin-4-ylmethyl-pyridin-3-yl)-naphthalen-1-yl]-urea
1-(3,5-Bis-trifluoromethyl-phenyl)-3-[4-(6-morpholin-4-ylmethyl-pyridin-3-yl)-naphthalen-1-yl]-urea
1-(2-tert-Butyl-5-methyl-pyridin-4-yl)-3-[4-(6-morpholin-4-ylmethyl-pyridin-3-yl)-naphthalen-1-yl]-urea
1-(3-Methyl-naphthalen-2-yl)-3-[4-(6-morpholin-4-ylmethyl-pyridin-3-yl)-naphthalen-1-yl]-urea
1-(3-tert-Butyl-phenyl)-3-[4-(6-morpholin-4-ylmethyl-pyridin-3-yl)-naphthalen-1-yl]-urea
1-(4-Methyl-biphenyl-3-yl)-3-[4-(6-morpholin-4-ylmethyl-pyridin-3-yl)-naphthalen-1-yl]-urea
1-(4-tert-Butyl-biphenyl-2-yl)-3-[4-(6-morpholin-4-ylmethyl-pyridin-3-yl)-naphthalen-1-yl]-urea
1-(5-Chloro-2,4-dimethoxy-phenyl)-3-[4-(6-morpholin-4-ylmethyl-pyridin-3-yl)-naphthalen-1-yl]-urea
1-(5-Isopropyl-2-methyl-phenyl)-3-[4-(6-morpholin-4-ylmethyl-pyridin-3-yl)-naphthalen-1-yl]-urea
1-(5-sec-Butyl-2-methoxy-phenyl)-3-[4-(6-morpholin-4-ylmethyl-pyridin-3-yl)-naphthalen-1-yl]-urea
1-(5-tert-Butyl-2-methoxy-3-propyl-phenyl)-3-[4-(6-morpholin-4-ylmethyl-pyridin-3-yl)-naphthalen-1-yl]-urea
1-(5-tert-Butyl-2-methoxymethyl-phenyl)-3-[4-(6-morpholin-4-ylmethyl-pyridin-3-yl)-naphthalen-1-yl]-urea
1-(5-tert-Butyl-2-methoxy-phenyl)-3-[4-(6-morpholin-4-ylmethyl-pyridin-3-yl)-naphthalen-1-yl]-urea
1-(5-tert-Butyl-2-methyl-phenyl)-3-(4-{6-[(3-methoxy-propyl)-methyl-amino]-pyridin-3-yl}-naphthalen-1-yl)-urea
1-(5-tert-Butyl-2-methyl-phenyl)-3-[4-(4-morpholin-4-ylmethyl-imidazol-1-yl)-naphthalen-1-yl]-urea
1-(5-tert-Butyl-2-methyl-phenyl)-3-[4-(6-morpholin-4-ylmethyl-pyridin-3-yl)-naphthalen-1-yl]-urea
1-(5-tert-Butyl-2-methyl-phenyl)-3-{4-[6-(3-methoxy-propylamino)-pyridin-3-yl]-naphthalen-1-yl}-urea
1-(5-tert-Butyl-2-methyl-pyridin-3-yl)-3-[4-(6-morpholin-4-ylmethyl-pyridin-3-yl)-naphthalen-1-yl]-urea
1-(5-tert-Butyl-2-morpholin-4-yl-phenyl)-3-[4-(6-morpholin-4-ylmethyl-pyridin-3-yl)-naphthalen-1-yl]-urea
1-(6-tert-Butyl-2-chloro-3-methyl-pyridin-4-yl)-3-[4-(6-morpholin-4-ylmethyl-pyridin-3-yl)-naphthalen-1-yl]-urea
1-[4-(6-Morpholin-4-ylmethyl-pyridin-3-yl)-naphthalen-1-yl]-3-(3-trifluoromethylphenyl)-urea
1-[4-(6-Morpholin-4-ylmethyl-pyridin-3-yl)-naphthalen-1-yl]-3-(4-trifluoromethoxyphenyl)-urea
1-[5-(1,1-Dimethyl-propyl)-2-methoxy-phenyl]-3-[4-(6-morpholin-4-ylmethyl-pyridin-3-yl)-naphthalen-1-yl]-urea
1-[5-tert-Butyl-2-(1H-pyrazol-4-yl)-phenyl]-3-[4-(6-morpholin-4-ylmethyl-pyridin-3-yl)-naphthalen-1-yl]-urea
1-[5-tert-Butyl-2-(2-methyl-pyrimidin-5-yl)-phenyl]-3-[4-(6-morpholin-4-ylmethyl-pyridin-3-yl)-naphthalen-1-yl]-urea
1-[5-tert-Butyl-2-(3-hydroxy-propyl)-phenyl]-3-[4-(6-morpholin-4-ylmethyl-pyridin-3-yl)-naphthalen-1-yl]-urea
1-[5-tert-Butyl-2-(3-morpholin-4-yl-3-oxo-propyl)-phenyl]-3-[4-(6-morpholin-4-ylmethyl-pyridin-3-yl)-naphthalen-1-yl]-urea
1-[5-tert-Butyl-2-(morpholine-4-carbonyl)-phenyl]-3-[4-(6-morpholin-4-ylmethyl-pyridin-3-yl)-naphthalen-1-yl]-urea
N-(5-tert-Butyl-2-methoxy-3-{3-[4-(6-morpholin-4-ylmethyl-pyridin-3-yl)-naphthalen-1-yl]-ureido}-phenyl)-acetamide
and the pharmaceutically acceptable derivatives thereof.
1-(2-tert-Butyl-5-methyl-pyridin-4-yl)-3-[4-(6-morpholin-4-ylmethyl-pyridin-3-yl)-naphthalen-1-yl]-urea;
1-(3-Methyl-naphthalen-2-yl)-3-[4-(6-morpholin-4-ylmethyl-pyridin-3-yl)-naphthalen-1-yl]-urea;
1-(3-tert-Butyl-phenyl)-3-[4-(4-morpholin-4-ylmethyl-phenyl)-naphthalen-1-yl]-urea;
1-(3-tert-Butyl-phenyl)-3-[4-(6-morpholin-4-ylmethyl-pyridin-3-yl)-naphthalen-1-yl]-urea;
1-(4-Methyl-biphenyl-3-yl)-3-[4-(6-morpholin-4-ylmethyl-pyridin-3-yl)-naphthalen-1-yl]-urea;
1-(4-tert-Butyl-biphenyl-2-yl)-3-[4-(6-morpholin-4-ylmethyl-pyridin-3-yl)-naphthalen-1-yl]-urea;
1-(5-Chloro-2,4-dimethoxy-phenyl)-3-[4-(6-morpholin-4-ylmethyl-pyridin-3-yl)-naphthalen-1-yl]-urea;
1-(5-lsopropyl-2-methyl-phenyl)-3-[4-(6-morpholin-4-ylmethyl-pyridin-3-yl)-naphthalen-1-yl]-urea;
1-(5-sec-Butyl-2-methoxy-phenyl)-3-[4-(6-morpholin-4-ylmethyl-pyridin-3-yl)-naphthalen-1-yl]-urea;
1-(5-tert-Butyl-2-methoxy-3-propyl-phenyl)-3-[4-(6-morpholin-4-ylmethyl-pyridin-3-yl)-naphthalen-1-yl]-urea;
1-(5-tert-Butyl-2-methoxymethyl-phenyl)-3-[4-(6-morpholin-4-ylmethyl-pyridin-3-yl)-naphthalen-1-yl]-urea;
1-(5-tert-Butyl-2-methoxy-phenyl)-3-[4-(2-morpholin-4-ylmethyl-pyrimidin-5-yl)-naphthalen-1-yl]-urea;
1-(5-tert-Butyl-2-methoxy-phenyl)-3-[4-(4-thiomorpholin-4-ylmethyl-phenyl)-naphthalen-1-yl]-urea;
1-(5-tert-Butyl-2-methoxy-phenyl)-3-[4-(6-morpholin-4-ylmethyl-pyridin-3-yl)-naphthalen-1-yl]-urea;
1-(5-tert-Butyl-2-methoxy-phenyl)-3-[4-(6-morpholin-4-ylmethyl-phenyl)-naphthalen-1-yl]-urea;
1-(5-tert-Butyl-2-methoxy-phenyl)-3-{4-[4-(tetrahydro-pyran-4-ylamino)-phenyl]-naphthalen-1-yl}-urea;
1-(5-tert-Butyl-2-methoxy-phenyl)-3-{4-[6-(4-methyl-piperazin-1-ylmethyl)-pyridin-3-yl]-naphthalen-1-yl}-urea;
1-(5-tert-Butyl-2-methyl-phenyl)-3-(4-{6-[(3-methoxy-propyl)-methyl-amino]-pyridin-3-yl}-naphthalen-1-yl)-urea;
1-(5-tert-Butyl-2-methyl-phenyl)-3-[4-(4-morpholin-4-ylmethyl-imidazol-1-yl)-naphthalen-1-yl]-urea;
1-(5-tert-Butyl-2-methyl-phenyl)-3-[4-(4-morpholin-4-ylmethyl-phenyl)-naphthalen-1-yl]-urea;
1-(5-tert-Butyl-2-methyl-phenyl)-3-[4-(6-morpholin-4-ylmethyl-pyridin-3-yl)-naphthalen-1-yl]-urea;
1-(5-tert-Butyl-2-methyl-phenyl)-3-{4-[6-(3-methoxy-propylamino)-pyridin-3-yl]-naphthalen-1-yl}-urea;
1-(5-tert-Butyl-2-methyl-pyridin-3-yl)-3-[4-(6-morpholin-4-ylmethyl-pyridin-3-yl)-naphthalen-1-yl]-urea;
1-(5-tert-Butyl-2-morpholin-4-yl-phenyl)-3-[4-(6-morpholin-4-ylmethyl-pyridin-3-yl)-naphthalen-1-yl]-urea;
1-(6-tert-Butyl-2-chloro-3-methyl-pyridin-4-yl)-3-[4-(6-morpholin-4-ylmethyl-pyridin-3-yl)-naphthalen-1-yl]-urea;
1-(6-tert-Butyl-2-chloro-3-methyl-pyridin-4-yl)-3-[4-(6-thiomorpholin-4-ylmethyl-pyridin-3-yl)-naphthalen-1-yl]-urea;
1-[2-Methoxy-5-(1-methyl-cyclopropyl)-phenyl]-3-[4-(2-morpholin-4-ylmethyl-pyrimidin-5-yl)-naphthalen-1-yl]-urea;
1-[4-(6-Morpholin-4-ylmethyl-pyridin-3-yl)-naphthalen-1-yl]-3-(3-trifluoromethylphenyl)-urea;
1-[4-(6-Morpholin-4-ylmethyl-pyridin-3-yl)-naphthalen-1-yl]-3-(4-trifluoromethoxy-phenyl)-urea;
1-[5-(1,1-Dimethyl-propyl)-2-methoxy-phenyl]-3-[4-(4-thiomorpholin-4-ylmethyl-phenyl)-naphthalen-1-yl]-urea;
1-[5-(1,1-Dimethyl-propyl)-2-methoxy-phenyl]-3-[4-(6-morpholin-4-ylmethyl-pyridin-3-yl)-naphthalen-1-yl]-urea;
1-[5-(1-Cyano-cyclopropyl)-2-methoxy-phenyl]-3-[4-(2-morpholin-4-ylmethyl-pyrimidin-5-yl)-naphthalen-1-yl]-urea;
1-[5-tert-Butyl-2-(1H-pyrazol-4-yl)-phenyl]-3-[4-(6-morpholin-4-ylmethyl-pyridin-3-yl)-naphthalen-1-yl]-urea;
1-[5-tert-Butyl-2-(2-methyl-pyrimidin-5-yl)-phenyl]-3-[4-(5-pyridin-4-ylmethyl-pyridin-2-yl)-naphthalen-1-yl]-urea;
1-[5-tert-Butyl-2-(2-methyl-pyrimidin-5-yl)-phenyl]-3-[4-(6-morpholin-4-ylmethyl-pyridin-3-yl)-naphthalen-1-yl]-urea;
1-[5-tert-Butyl-2-(3-hydroxy-propyl)-phenyl]-3-[4-(6-morpholin-4-ylmethyl-pyridin-3-yl)-naphthalen-1-yl]-urea;
1-[5-tert-Butyl-2-(3-morpholin-4-yl-3-oxo-propyl)-phenyl]-3-[4-(6-morpholin-4-ylmethyl-pyridin-3-yl)-naphthalen-1-yl]-urea;
1-[5-tert-Butyl-2-(morpholine-4-carbonyl)-phenyl]-3-[4-(6-morpholin-4-ylmethyl-pyridin-3-yl)-naphthalen-1-yl]-urea;
2-[4-tert-Butyl-2-(3-{4-[6-(2,6-dimethyl-morpholin-4-ylmethyl)-pyridin-3-yl]-naphthalen-1-yl}-ureido)-phenoxy]-acetamide;
3-{4-[3-(5-tert-Butyl-2-methoxy-phenyl)-ureido]-naphthalen-1-yl}-benzamide;
4-tert-Butyl-2-{3-[4-(2-chloro-4-morpholin-4-ylmethyl-phenyl)-naphthalen-1-yl]-ureido}-benzamide;
and the pharmaceutically acceptable derivatives thereof.

More preferably the invention relates to pharmaceutical compositions containing A and B, characterized in that the p38 kinase inhibitor B is selected from the following compounds of formula **6** :
1-(2-tert-Butyl-5-methyl-pyridin-4-yl)-3-[4-(6-morpholin-4-ylmethyl-pyridin-3-yl)-naphthalen-1-yl]-urea;
1-(3-tert-Butyl-phenyl)-3-[4-(6-morpholin-4-ylmethyl-pyridin-3-yl)-naphthalen-1-yl]-urea;
1-(4-Methyl-biphenyl-3-yl)-3-[4-(6-morpholin-4-ylmethyl-pyrid in-3-yl)-naphthalen-1-yl]-urea;
1-(4-tert-Butyl-biphenyl-2-yl)-3-[4-(6-morpholin-4-ylmethyl-pyridin-3-yl)-naphthalen-1-yl]-urea;
1-(5-Isopropyl-2-methyl-phenyl)-3-[4-(6-morpholin-4-ylmethyl-pyridin-3-yl)-naphthalen-1-yl]-urea;
1-(5-sec-Butyl-2-methoxy-phenyl)-3-[4-(6-morpholin-4-ylmethyl-pyridin-3-yl)-naphthalen-1-yl]-urea;
1-(5-tert-Butyl-2-methoxymethyl-phenyl)-3-[4-(6-morpholin-4-ylmethyl-pyridin-3-yl)-naphthalen-1-yl]-urea;
1-(5-tert-Butyl-2-methoxy-phenyl)-3-[4-(6-morpholin-4-ylmethyl-pyridin-3-yl)-naphthalen-1-yl]-urea;
1-(5-tert-Butyl-2-methyl-phenyl)-3-(4-{6-[(3-methoxy-propyl)-methyl-amino]-pyridin-3-yl}-naphthalen-1-yl)-urea;
1-(5-tert-Butyl-2-methyl-phenyl)-3-[4-(6-morpholin-4-ylmethyl-pyridin-3-yl)-naphthalen-1-yl]-urea;
1-(5-tert-Butyl-2-methyl-pyridin-3-yl)-3-[4-(6-morpholin-4-ylmethyl-pyridin-3-yl)-naphthalen-1-yl]-urea;
1-[5-(1,1-Dimethyl-propyl)-2-methoxy-phenyl]-3-[4-(6-morpholin-4-ylmethyl-pyridin-3-yl)-naphthalen-1-yl]-urea;
1-[5-tert-Butyl-2-(1H-pyrazol-4-yl)-phenyl]-3-[4-(6-morpholin-4-ylmethyl-pyridin-3-yl)-naphthalen-1-yl]-urea;
1-[5-tert-Butyl-2-(2-methyl-pyrimidin-5-yl)-phenyl]-3-[4-(6-morpholin-4-ylmethyl-pyridin-3-yl)-naphthalen-1-yl]-urea;
1-[5-tert-Butyl-2-(3-hydroxy-propyl)-phenyl]-3-[4-(6-morpholin-4-ylmethyl-pyridin-3-yl)-naphthalen-1-yl]-urea;
1-[5-tert-Butyl-2-(morpholine-4-carbonyl)-phenyl]-3-[4-(6-morpholin-4-ylmethyl-pyridin-3-yl)-naphthalen-1-yl]-urea;
N-(5-tert-Butyl-2-methoxy-3-{3-[4-(6-morpholin-4-ylmethyl-pyridin-3-yl)-naphthalen-1-yl]-ureido}-phenyl)-acetamide
and the pharmaceutically acceptable derivatives thereof.

In another preferred embodiment the invention relates to pharmaceutical compositions containing A and B, characterized in that the p38 kinase inhibitor B is selected from the compounds of formula 7 as disclosed in WO 00/55139 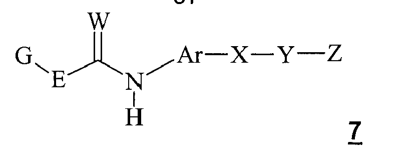 wherein:
- E is: carbon or a heteroatom group chosen from -O-, -NH- and -S-;
- G is :: an aromatic C₆₋₁₀ carbocycle or a nonaromatic C₃₋₁₀carbocycle saturated or unsaturated;
a 6-14 membered monocyclic, bicyclic or tricyclic heteroaryl containing 1 or more heteroatoms chosen from O, N and S;
a 6-8 membered monocyclic heterocycle containing one or more heteroatoms chosen from O, N and S;
or
an 8-11 membered bicyclic heterocycle, containing one or more heteroatoms chosen from O, N and S;
wherein G is optionally substituted by one or more R₁, R₂ or R₃;
- Ar is:: phenyl, naphthyl, quinolinyl, isoquinolinyl, tetrahydronaphthyl, tetrahydroquinolinyl, tetrahydroisoquinolinyl, benzimidazolyl, benzofuranyl, dihydrobenzofuranyl, indolinyl, benzothienyl, dihydrobenzothienyl, indanyl, indenyl or indolyl each being optionally substituted by one or more R₄ or R₅;
- X is:: a C₅₋₈ cycloalkyl or cycloalkenyl optionally substituted with one to two oxo groups or one to three C₁₋₄ alkyl, C₁₋₄ alkoxy or C₁₋₄ alkylamino chains each being branched or unbranched;
aryl, furanyl, thienyl, pyrrolyl, pyrazolyl, imidazolyl, pyridinyl, pyrimidinyl, pyridinonyl, dihydropyridinonyl, maleimidyl, dihydromaleimidyl, piperdinyl, benzimidazole, 3H-imidazo[4,5-b]pyridine, piperazinyl, pyridazinyl or pyrazinyl; each being optionally independently substituted with one to three C₁₋₄ alkyl, C₁₋₄alkoxy, hydroxy, nitrile, amino, mono- or di-(C₁₋₃ alkyl)amino, mono- or di-(C₁₋₃ alkylamino)carbonyl, NH₂C(O), C₁₋₆ alkyl-S(O)ₘ or halogen;
- Y is:: a bond or a C₁₋₄ saturated or unsaturated branched or unbranched carbon chain optionally partially or fully halogenated, wherein one or more C atoms are optionally replaced by O, N, or S(O)ₘ and wherein Y is optionally independently substituted with one to two oxo groups, nitrile, phenyl or one or more C₁₋₄ alkyl optionally substituted by one or more halogen atoms;
- Z is:: aryl, heteroaryl selected from pyridinyl, piperazinyl, pyrimidinyl, pyridazinyl, pyrazinyl, imidazolyl, pyrazolyl, triazolyl, tetrazolyl, furanyl, thienyl and pyranyl, heterocycle selected from tetrahydropyrimidonyl, cyclohexanonyl, cyclohexanolyl, 2-oxa- or 2-thia-5-aza-bicyclo[2.2.1]heptanyl, pentamethylene sulfidyl, pentamethylene sulfoxidyl, pentamethylene sulfonyl, tetramethylene sulfidyl, tetramethylene sulfoxidyl or tetramethylene sulfonyl, tetrahydropyranyl, tetrahydrofuranyl, 1,3-dioxolanonyl, 1,3-dioxanonyl, 1,4-dioxanyl, morpholino, thiomorpholino, thiomorpholino sulfoxidyl, thiomorpholino sulfonyl, piperidinyl, piperidinonyl, pyrrolidinyl and dioxolanyl, each of the aforementioned Z are optionally substituted with one to three halogen, C₁₋₆ alkyl, C₁₋₆ alkoxy, C₁₋₃ alkoxy-C₁₋₃ alkyl, C₁₋₆ alkoxycarbonyl, aroyl, C₁₋₃acyl, oxo, hydroxy, pyridinyl-C₁₋₃ alkyl, imidazolyl-C₁₋₃ alkyl, tetrahydrofuranyl-C₁₋₃ alkyl, nitrile-C₁₋₃ alkyl, nitrile, carboxy, phenyl wherein the phenyl ring is optionally substituted with one to two halogen, C₁₋₆ alkoxy, hydroxy or mono- or di-(C₁₋₃ alkyl)amino, C₁₋₆ alkyl-S(O)ₘ, or phenyl-S(O)ₘ wherein the phenyl ring is optionally substituted with one to two halogen, C₁₋₆ alkoxy, hydroxy, halogen or mono- or di-(C₁₋₃ alkyl)amino;
or Z is optionally substituted with one to three amino or amino-C₁₋₃ alkyl wherein the N atom is optionally independently mono- or di-substituted by aminoC₁₋₆alkyl, C₁₋₃alkyl, arylC₀₋₃alkyl, C₁₋₅ alkoxyC₁₋₃ alkyl, C₁₋₅ alkoxy, aroyl, C₁₋₃acyl, C₁₋₃alkyl-S(O)ₘ- or arylC₀₋₃alkyl-S(O)ₘ- each of the aforementioned alkyl and aryl attached to the amino group is optionally substituted with one to two halogen, C₁₋₆ alkyl or C₁₋₆ alkoxy;
or Z is optionally substituted with one to three aryl, heterocycle or heteroaryl as hereinabove described in this paragraph each in turn is optionally substituted by halogen, C₁₋₆ alkyl or C₁₋₆ alkoxy;
or Z is hydroxy, halogen, nitrile, amino wherein the N atom is optionally independently mono- or di-substituted by C₁₋₃acyl, C₁₋₆alkyl or C₁₋₃alkoxyC₁₋₃alkyl, C₁₋₆alkyl branched or unbranched, C₁₋₆alkoxy, C₁₋₃acylamino, nitrileC₁₋₄alkyl, C₁₋₆ alkyl-S(O)ₘ, and phenyl-S(O)ₘ, wherein the phenyl ring is optionally substituted with one to two halogen, C₁₋₆ alkoxy, hydroxy or mono- or di-(C₁₋₃ alkyl)amino;
each R₁ is independently:
C₁₋₁₀ alkyl branched or unbranched optionally partially or fully halogenated, wherein one or more C atoms are optionally independently replaced by O, N or S(O)ₘ, and wherein said C₁₋₁₀ alkyl is optionally substituted with one to three C₃₋₁₀ cycloalkyl, hydroxy, oxo, phenyl, naphthyl, pyridinyl, pyrimidinyl, pyrazinyl, pyridazinyl, pyrrolyl, pyrrolidinyl, imidazolyl, pyrazolyl, thienyl, furyl, dioxolanyl, isoxazolyl or isothiazolyl; each of the aforementioned being optionally substituted with one to five groups selected from halogen, C₁₋₆ alkyl which is optionally partially or fully halogenated, C₃-₈ cycloalkanyl, C₅₋₈ cycloalkenyl, hydroxy, nitrile, C₁₋₃ alkoxy which is optionally partially or fully halogenated or NH₂C(O), mono- or di(C₁₋₃alkyl)amino, and mono- or di(C₁₋₃alkyl)aminocarbonyl;
or R₁ is
cyclopropyloxy, cyclobutyloxy, cyclopentyloxy, cyclohexyloxy, or cycloheptyloxy each being optionally partially or fully halogenated and optionally substituted with one to three C₁₋₃ alkyl groups optionally partially or fully halogenated, nitrile, hydroxyC₁₋₃alkyl or aryl; or an analog of such cycloalkyl group wherein one to three ring methylene groups are independently replaced by O, S(O)ₘ, CHOH, >C=O, >C=S or NH;
phenyloxy or benzyloxy each being optionally partially or fully halogenated and optionally substituted with one to three C₁₋₃ alkyl groups optionally partially or fully halogenated, nitrile, hydroxyC₁₋₃alkyl or aryl; or an analog of such cycloaryl group wherein one to two ring methyne groups are independently replaced by N;
cyclopropyl, cyclobutyl, cyclopentyl, cyclohexyl, cycloheptyl, bicyclopentanyl, bicyclohexanyl or bicycloheptanyl, each being optionally partially or fully halogenated and optionally substituted with one to three C₁₋₃ alkyl optionally partially or fully halogenated, nitrile, hydroxyC₁₋₃alkyl or aryl; or an analog of such cycloalkyl group wherein one to three ring methylene groups are independently replaced by O, S(O)ₘ, CHOH, >C=O, >C=S or NH;
C₃₋₁₀ branched or unbranced alkenyl each being optionally partially or fully halogenated, and optionally substituted with one to three C₁₋₅ branched or unbranched alkyl, phenyl, naphthyl, pyridinyl, pyrimidinyl, pyrazinyl, pyridazinyl, pyrrolyl, imidazolyl, pyrazolyl, thienyl, furyl, isoxazolyl or isothiazolyl, each of the aforementioned being substituted with one to five halogen, C₁₋₆ alkyl which is optionally partially or fully halogenated, cyclopropanyl, cyclobutanyl, cyclopentanyl, cyclohexanyl, cycloheptanyl, bicyclopentanyl, bicyclohexanyl and bicycloheptanyl, hydroxy, nitrile, C₁₋₃ alkyloxy which is optionally partially or fully halogenated, NH₂C(O), mono- or di(C₁₋₃alkyl)aminocarbonyl; the C₃₋₁₀ branched or unbranced alkenyl being optionally interrupted by one or more heteroatoms chosen from O, N and S(O)ₘ;
cyclopentenyl, cyclohexenyl, cyclohexadienyl, cycloheptenyl, cycloheptadienyl, bicyclohexenyl or bicycloheptenyl, wherein such cycloalkenyl group is optionally substituted with one to three C₁₋₃ alkyl groups;
oxo, nitrile, halogen;
silyl containing three C₁₋₄ alkyl groups optionally partially or fully halogenated; or
C₃₋₆ alkynyl branched or unbranched carbon chain optionally partially or fully halogenated, wherein one or more methylene groups are optionally replaced by O, NH or S(O)ₘ and wherein said alkynyl group is optionally independently substituted with one to two oxo groups, hydroxy, pyrroldinyl, pyrrolyl, tetrahydropyranyl, one or more C₁₋₄ alkyl optionally substituted by one or more halogen atoms, nitrile, morpholino, piperidinyl, piperazinyl, imidazolyl, phenyl, pyridinyl, tetrazolyl, or mono- or di(C₁₋₃alkyl)amino optionally substituted by one or more halogen atoms;
each R₂, R₄, and R₅ is
a C₁₋₆ branched or unbranched alkyl optionally partially or fully halogenated, C₁₋₆acyl, aroyl, C₁₋₄ branched or unbranched alkoxy, each being optionally partially or fully halogenated, halogen, methoxycarbonyl, C₁₋₃ alkyl-S(O)ₘ optionally partially or fully halogenated, or phenyl-S(O)ₘ;
OR₆, C₁₋₆ alkoxy, hydroxy, nitrile, nitro, halogen;
or amino-S(O)ₘ- wherein the N atom is optionally independently mono- or di-substituted by C₁₋₆alkyl or arylC₀₋₃alkyl, or amino wherein the N atom is optionally independently mono- or di-substituted by C₁₋₃alkyl, arylC₀₋₃alkyl, C₁₋₆acyl, C₁₋₆alkyl-S(O)ₘ- or arylC₀₋₃alkyl-S(O)ₘ-, each of the aforementioned alkyl and aryl in this subparagraph are optionally partially or fully halogenated and optionally substituted with one to two C₁₋₆ alkyl or C₁₋₆ alkoxy;
each R₃ is independently:
phenyl, naphthyl, morpholino, pyridinyl, pyrimidinyl, pyrazinyl, pyridazinyl, pyrrolyl, pyrrolidinyl, imidazolyl, pyrazolyl, thiazolyl, oxazoyl, [1,3,4]oxadiazol, triazolyl, tetrazolyl, thienyl, furyl, tetrahydrofuryl, isoxazolyl, isothiazolyl, quinolinyl, isoquinolinyl, indolyl, benzimidazolyl, benzofuranyl, benzoxazolyl, benzisoxazolyl, benzpyrazolyl, benzothiofuranyl, cinnolinyl, pterindinyl, phthalazinyl, naphthypyridinyl, quinoxalinyl, quinazolinyl, purinyl or indazolyl, each of the aforementioned is optionally substituted with one to three phenyl, naphthyl, heterocycle or heteroaryl as hereinabove described in this paragraph, C₁₋₆ branched or unbranched alkyl which is optionally partially or fully halogenated, cyclopropanyl, cyclobutanyl, cyclopentanyl, cyclohexanyl, cycloheptanyl, bicyclopentanyl, bicyclohexanyl, bicycloheptanyl, phenyl C₁₋₅ alkyl, naphthyl C₁₋₅ alkyl, halogen, hydroxy, oxo, nitrile, C₁₋₃ alkoxy optionally partially or fully halogenated, phenyloxy, naphthyloxy, heteroaryloxy or heterocyclicoxy wherein the heterocyclic or heteroaryl moiety is as hereinabove described in this paragraph, nitro, amino, mono- or di-(C₁₋₃alky)lamino, phenylamino, naphthylamino, heteroaryl or heterocyclic amino wherein the heteroaryl heterocyclic moiety is as hereinabove described in this paragraph, NH₂C(O), a mono- or di-(C₁₋₃alkyl) aminocarbonyl, C₁₋₅ alkyl-C(O)-C₁₋₄ alkyl, amino-C₁₋₅ alkyl, mono- or di-(C₁₋₅alkyl)amino, mono- or di-(C₁₋₃alkyl)amino-C₁₋₅ alkyl, amino-S(O)₂, di-(C₁₋₃alkyl)amino-S(O)₂, R₇-C₁₋₅ alkyl, R₈-C₁₋₅ alkoxy, R₉-C(O)-C₁₋₅ alkyl, R₁₀-C₁₋₅ alkyl(R₁₁)N, carboxy-mono- or di-(C₁₋₅alkyl)-amino;
a fused aryl selected from benzocyclobutanyl, indanyl, indenyl, dihydronaphthyl, tetrahydronaphthyl, benzocycloheptanyl and benzocycloheptenyl, or a fused heteroaryl selected from cyclopentenopyridinyl, cyclohexanopyridinyl, cyclopentanopyrimidinyl, cyclohexanopyrimidinyl, cyclopentanopyrazinyl, cyclohexanopyrazinyl, cyclopentanopyridazinyl, cyclohexanopyridazinyl, cyclopentanoquinolinyl, cyclohexanoquinolinyl, cyclopentanoisoquinolinyl, cyclohexanoisoquinolinyl, cyclopentanoindolyl, cyclohexanoindolyl, cyclopentanobenzimidazolyl, cyclohexanobenzimidazolyl, cyclopentanobenzoxazolyl, cyclohexanobenzoxazolyl, cyclopentanoimidazolyl, cyclohexanoimidazolyl, cyclopentanothienyl and cyclohexanothienyl; wherein the fused aryl or fused heteroaryl ring is independently substituted with zero to three phenyl, naphthyl, pyridinyl, pyrimidinyl, pyrazinyl, pyridazinyl, pyrrolyl, imidazolyl, pyrazolyl, thienyl, furyl, isoxazolyl, isothiazolyl, C₁₋₆ alkyl which is optionally partially or fully halogenated, halogen, nitrile, C₁₋₃ alkyloxy which is optionally partially or fully halogenated, phenyloxy, naphthyloxy, heteroaryloxy or heterocyclicoxy wherein the heteroaryl or heterocyclic moiety is as hereinabove described in this paragraph, nitro, amino, mono- or di-(C₁₋₃alkyl)amino, phenylamino, naphthylamino, heteroaryl or heterocyclic amino wherein the heteroaryl or heterocyclic moiety is as hereinabove described in this paragraph, NH₂C(O), mono- or di-(C₁₋₃alkyl)aminocarbonyl, C₁₋₄ alkyl-OC(O), C₁₋₅ alkyl-C(O)-C₁₋₄ alkyl, amino-C₁₋₅ alkyl, mono- or di-(C₁₋₃)alkylamino-C₁₋₅ alkyl, R₁₂-C₁₋₅ alkyl, R₁₃-C₁₋₅ alkoxy, R₁₄-C(O)-C₁₋₅ alkyl or R₁₅-C₁₋₅ alkyl(R₁₆)N;
cyclopropanyl, cyclobutanyl, cyclopentanyl, cyclohexanyl, cycloheptanyl, bicyclopentanyl, bicyclohexanyl or bicycloheptanyl, each being optionally be partially or fully halogenated and optionally substituted with one to three C₁₋₃ alkyl groups, or an analog of such cycloalkyl group wherein one to three ring methylene groups are independently replaced by O, S, CHOH, >C=O, >C=S or NH;
cyclopentenyl, cyclohexenyl, cyclohexadienyl, cycloheptenyl, cycloheptadienyl, bicyclohexenyl or bicycloheptenyl, each optionally substituted with one to three C₁₋₃ alkyl groups;
C₁₋₄ alkyl-phenyl-C(O)-C₁₋₄ alkyl-, C₁₋₄ alkyl-C(O)-C₁₋₄ alkyl- or C₁₋₄ alkylphenyl-S(O)ₘ-C₁₋₄ alkyl-;
C₁₋₆ alkyl or C₁₋₆ branched or unbranched alkoxy each of which is optionally partially or fully halogenated or optionally substituted with R₁₇;
OR₁₈ or C₁₋₆ alkyl optionally substituted with OR₁₈;
amino or mono- or di-(C₁₋₅alkyl)amino optionally substituted with R₁₉;
R₂₀C(O)N(R₂₁)-, R₂₂O- or R₂₃R₂₄NC(O)-; R₂₆(CH₂)ₘC(O)N(R₂₁)-, R₂₃R₂₄NC(O)-C₁₋₃alkoxy or R₂₆C(O)(CH₂)ₘN(R₂₁)-;
C₂₋₆alkenyl substituted by R₂₃R₂₄NC(O)-;
C₂₋₆ alkynyl branched or unbranched carbon chain, optionally partially or fully halogenated, wherein one or more methylene groups are optionally replaced by O, NH, S(O)ₘ and wherein said alkynyl group is optionally independently substituted with one to two oxo groups, pyrroldinyl, pyrrolyl, morpholino, piperidinyl, piperazinyl, imidazolyl, phenyl, pyridinyl, tetrazolyl one or more C₁₋₄ alkyl optionally substituted by one or more halogen atoms, nitrile, morpholino, piperidinyl, piperazinyl, imidazolyl, phenyl, pyridinyl, tetrazolyl, or mono- or di(C₁₋₄ alkyl)amino optionally substituted by one or more halogen atoms;
C₁₋₆acyl or aroyl;

- R₆: is a:
C₁₋₄ alkyl optionally partially or fully halogenated and optionally substituted with R₂₆;
each R₇, R₈, R₉, R₁₀, R₁₂, R₁₃, R₁₄, R₁₅, R₁₇, R₁₉, R₂₅ and R₂₆ is independently:
nitrile, phenyl, morpholino, piperidinyl, piperazinyl, imidazolyl, pyridinyl, tetrazolyl, amino or mono- or di-(C₁₋₄alkyl)amino optionally partially or fully halogenated;
each R₁₁ and R₁₆ is independently:
hydrogen or C₁₋₄ alkyl optionally partially or fully halogenated;
R₁₈ is independently:
hydrogen or a C₁₋₄ alkyl optionally independently substituted with oxo or R₂₅; R₂₀ is independently:
   C₁₋₁₀ alkyl optionally partially or fully halogenated, phenyl, or pyridinyl;
R₂₁ is independently:
hydrogen or C₁₋₃ alkyl optionally partially or fully halogenated;
each R₂₂, R₂₃ and R₂₄ is independently:
hydrogen, C₁₋₆ alkyl optionally partially or fully halogenated, said C₁₋₆ alkyl is optionally interrupted by one or more O, N or S, said C₁₋₆ alkyl also being independently optionally substituted by mono- or di-(C₁₋₃alkyl)aminocarbonyl, phenyl, pyridinyl, amino or mono- or di-(C₁₋₄alkyl)amino each of which is optionally partially or fully halogenated and optionally substituted with mono-or di-(C₁₋₃alkyl)amino;
or R₂₃ and R₂₄ taken together optionally form a heterocyclic or heteroaryl ring;
m = 0, 1 or 2;
W is O or S and
pharmaceutically acceptable derivatives thereof.

In a preferred embodiment the invention relates to pharmaceutical compositions containing **A** and **B**, characterized in that the p38 kinase inhibitor **B** is selected from the compounds of formula **7** wherein:
- E is: -CH₂-, -NH- or -O-;
- W is: O;
and
- G is:: phenyl, naphthyl, benzocyclobutanyl, dihydronaphthyl, tetrahydronaphthyl, benzocycloheptanyl, benzocycloheptenyl, indanyl, indenyl;
pyridinyl, pyridonyl, quinolinyl, dihydroquinolinyl, tetrahydroquinoyl, isoquinolinyl, tetrahydroisoquinoyl, pyridazinyl, pyrimidinyl, pyrazinyl, benzimidazolyl, benzthiazolyl, benzooxazolyl, benzofuranyl, benzothiophenyl, benzpyrazolyl, dihydrobenzofuranyl, dibenzofuranyl, dihydrobenzothiophenyl, benzooxazolonyl, benzo[1,4]oxazin-3-onyl, benzodioxolyl, benzo[1,3]dioxol-2-onyl, benzofuran-3-onyl, tetrahydrobenzopyranyl, indolyl, 2,3-dihydro-1 H-indolyl, indolinyl, indolonyl, indolinonyl, phthalimidyl, chromoyl;
oxetanyl, pyrrolidinyl, tetrahydrofuranyl, tetrahydrothiophenyl, piperidinyl, piperazinyl, morpholino, tetrahydropyranyl, dioxanyl, tetramethylene sulfonyl, tetramethylene sulfoxidyl, oxazolinyl, 3,4-dihydro-2H-benzo[1,4]oxazinyl, thiazolinyl, imidazolinyl, tertrahydropyridinyl, homopiperidinyl, pyrrolinyl, tetrahydropyrimidinyl, decahydroquinolinyl, decahydroisoquinolinyl, thiomorpholino, thiazolidinyl, dihydrooxazinyl, dihydropyranyl, oxocanyl, heptacanyl, thioxanyl or dithianyl;
wherein G is optionally substituted by one or more R₁, R₂ or R₃.

In yet another preferred embodiment the invention relates to pharmaceutical compositions containing **A** and **B**, characterized in that the p38 kinase inhibitor **B** is selected from the compounds of formula **7** wherein:
- E is: -NH-;
- G is: phenyl, pyridinyl, pyridonyl, naphthyl, quinolinyl, isoquinolinyl, pyrazinyl, benzimidazolyl, benzooxazolyl, benzooxazolonyl, benzofuranyl, benzothiophenyl, benzpyrazolyl, dihydrobenzofuranyl, dihydrobenzothiophenyl, 3,4-dihydro-2H-benzo[1,4]oxazinyl, indanyl, indenyl, indolyl, indolinyl, indolonyl, 2,3-dihydro-1H-indolyl or indolinonyl, wherein G is optionally substituted by one or more R₁, R₂ or R₃;
- Ar is:: naphthyl, quinolinyl, isoquinolinyl, tetrahydronaphthyl, tetrahydroquinolinyl, tetrahydroisoquinolinyl, indanyl, indenyl or indolyl each being optionally substituted by one or more R₄ or R₅ groups;
- X is:: phenyl, furanyl, thienyl, pyrrolyl, pyrazolyl, imidazolyl, pyridinyl, pyrimidinyl, pyridinonyl, dihydropyridinonyl, maleimidyl, dihydromaleimidyl, piperdinyl, piperazinyl, pyridazinyl or pyrazinyl; each being optionally independently substituted with one to three C₁₋₄ alkyl, C₁₋₄alkoxy, hydroxy, nitrile, amino, mono- or di-(C₁₋₃ alkyl)amino, mono- or di-(C₁₋₃ alkylamino)carbonyl, NH₂C(O), C₁₋₆ alkyl-S(O)ₘ or halogen;
- Y is:: a bond or
a C₁₋₄ saturated or unsaturated carbon chain wherein one or more of the C atoms is optionally replaced by O, N, or S(O)ₘ and wherein Y is optionally independently substituted with one to two oxo groups, nitrile, phenyl or one or more C₁₋₄ alkyl optionally substituted by one or more halogen atoms;
- Z is:: phenyl, heteroaryl selected from pyridinyl, piperazinyl, pyrimidinyl, pyridazinyl, pyrazinyl, imidazolyl, furanyl, thienyl and pyranyl, heterocycle selected from 2-oxa-5-aza-bicyclo[2.2.1]heptanyl, tetrahydropyrimidonyl, pentamethylene sulfidyl, pentamethylene sulfoxidyl, pentamethylene sulfonyl, tetramethylene sulfidyl, tetramethylene sulfoxidyl tetramethylene sulfonyl, tetrahydropyranyl, tetrahydrofuranyl, 1,3-dioxolanonyl, 1,3-dioxanonyl, 1,4-dioxanyl, morpholino, thiomorpholino, thiomorpholino sulfoxidyl, piperidinyl, piperidinonyl, dihydrothiazolyl, dihydrothiazolyl sulfoxidyl, pyrrolidinyl and dioxolanyl which are optionally substituted with one to three nitrile, C₁₋₃ alkyl, C₁₋₃ alkoxy, amino, mono- or di-(C₁₋₃ alkyl)amino, CONH₂ or OH;
or Z is optionally substituted by phenyl, heterocycle or heteroaryl as hereinabove described in this paragraph each in turn is optionally substituted by halogen, C₁₋₃ alkyl or C₁₋₃ alkoxy;
or Z is nitrile, nitrileC₁₋₃ alkyl, C₁₋₆ alkyl-S(O)ₘ, halogen, hydroxy, C₁₋₃ alkyl, C₁₋₃ acylamino, C₁₋₄ alkoxy, amino, mono- or di-(C₁₋₃ alkyl)aminocarbonyl, or amino mono or di-substituted by aminoC₁₋₆ alkyl or C₁₋₃alkoxyC₁₋₃alkyl;
each R₁ is independently:
C₁₋₆ alkyl branched or unbranched optionally partially or fully halogenated, wherein one or more C atoms are optionally independently replaced by O, N or S(O)ₘ, and wherein said C₁₋₆ alkyl is optionally substituted with one to three C₃₋₆cycloalkyl, oxo, phenyl, dioxolanyl, pyrrolidinyl, furyl, isoxazolyl or isothiazolyl; each of the aforementioned being optionally substituted with one to three groups selected from halogen, C₁₋₃ alkyl which is optionally partially or fully halogenated, hydroxy, nitrile and C₁₋₃alkoxy which is optionally partially or fully halogenated;
cyclopropyl, cyclobutyl, cyclopentanyl, cyclohexanyl, bicyclopentanyl or bicyclohexanyl, each being optionally partially or fully halogenated and optionally substituted with one to three C₁₋₃ alkyl groups optionally partially or fully halogenated, nitrile, hydroxyC₁₋₃alkyl or phenyl; or an analog of such cycloalkyl group wherein one to three ring methylene groups are independently replaced by O, S, CHOH, >C=O, >C=S or NH;
oxo;
C₃₋₆ alkynyl branched or unbranched carbon chain optionally partially or fully halogenated, wherein one or more methylene groups are optionally replaced by O, NH or S(O)ₘ and wherein said alkynyl group is optionally independently substituted with one to two oxo groups, hydroxy, pyrroldinyl, pyrrolyl, tetrahydropyranyl, C₁₋₄ alkyl optionally substituted by one or more halogen atoms, nitrile, morpholino, piperidinyl, piperazinyl, imidazolyl, phenyl, pyridinyl, tetrazolyl, or mono- or di(C₁₋₃alkyl)amino optionally substituted by one or more halogen atoms;
or
silyl containing three C₁₋₄ alkyl groups optionally partially or fully halogenated;

- R₂: is independently:
- R₃: a C₁₋₅ branched or unbranched alkyl optionally partially or fully halogenated, acetyl, aroyl, C₁₋₄ branched or unbranched alkoxy, each being optionally partially or fully halogenated, halogen, methoxycarbonyl, C₁₋₂ alkyl-S(O)ₘ optionally partially or fully halogenated, or phenyl-S(O)ₘ;
C₁₋₃ alkoxy, hydroxy, nitrile, nitro, halogen;
or amino-S(O)ₘ- wherein the N atom is optionally independently mono- or di-substituted by C₁₋₃alkyl or arylC₀₋₃alkyl, or amino wherein the N atom is optionally independently mono- or di-substituted by C₁₋₃alkyl, arylC₀₋₃alkyl, C₁₋₃acyl, C₁₋₄alkyl-S(O)ₘ- or arylC₀₋₃alkyl-S(O)ₘ-, each of the aforementioned alkyl and aryl in this subparagraph are optionally partially or fully halogenated and optionally substituted with one to two C₁₋₃ alkyl or C₁₋₃ alkoxy;
- R₃ is: independently:
phenyl, morpholino, pyridinyl, pyrimidinyl, pyrazinyl, pyrrolyl, pyrrolidinyl, imidazolyl, [1,3,4]oxadiazol, pyrazolyl, each is optionally substituted with one to three phenyl, naphthyl, heterocycle or heteroaryl as hereinabove described in this paragraph, C₁₋₆ alkyl which is optionally partially or fully halogenated, cyclopropanyl, cyclobutanyl, cyclopentanyl, cyclohexanyl, cycloheptanyl, bicyclopentanyl, bicyclohexanyl, bicycloheptanyl, phenyl C₁₋₅ alkyl, naphthyl C₁₋₅ alkyl, halogen, oxo, hydroxy, nitrile, C₁₋₃ alkoxy optionally partially or fully halogenated, phenyloxy, naphthyloxy, heteroaryloxy or heterocyclicoxy wherein the heteroaryl or heterocyclic moiety is as hereinabove described in this paragraph, nitro, amino, mono- or di-(C₁₋₃alkyl)amino, phenylamino, naphthylamino, heteroaryl or heterocyclic amino wherein the heteroaryl or heterocyclic moiety is as hereinabove described in this paragraph, NH₂C(O), a mono- or di-(C₁₋₃alkyl)aminocarbonyl, C₁₋₅ alkyl-C(O)-C₁₋₄ alkyl, mono- or di-(C₁₋₃alkyl)amino, mono- or di-(C₁₋₃)alkylamino-C₁₋₅ alkyl, mono- or di-(C₁₋₃alkyl)amino-S(O)₂, R₇-C₁₋₅alkyl, R₈-C₁₋₅ alkoxy, R₉-C(O)-C₁₋₅ alkyl, R₁₀-C₁₋₅ alkyl(R₁₁)N, carboxy-mono- or di-(C₁₋₅)-alkyl-amino;
C₁₋₃ alkyl or C₁₋₄ alkoxy each being optionally partially or fully halogenated or optionally substituted with R₁₇;
OR₁₈ or C₁₋₆ alkyl optionally substituted with OR₁₈;
amino or mono- or di- (C₁₋₅ alkyl)amino optionally substituted with R₁₉;
R₂₀C(O)N(R₂₁)-, R₂₂O- ; R₂₃R₂₄NC(O)-; R₂₆CH₂C(O)N(R₂₁)-,
R₂₃R₂₄NC(O)-C₁₋₂alkoxy or R₂₆C(O)CH₂N(R₂₁)-;
C₂₋₄alkenyl substituted by R₂₃R₂₄NC(O)-; or
C₂₋₄ alkynyl branched or unbranched carbon chain optionally partially or fully halogenated wherein one of the methylene groups is optionally replaced by O, and optionally independently substituted with one to two oxo groups, pyrroldinyl, pyrrolyl, morpholino, piperidinyl, piperazinyl, imidazolyl, phenyl, pyridinyl, tetrazolyl or one or more C₁₋₄ alkyl optionally substituted by one or more halogen atoms;
C₁₋₃acyl; and
R₂₃ and R₂₄ taken together optionally form imidazolyl, piperidinyl, morpholino, piperazinyl or a pyridinyl ring.

In yet another preferred embodiment the invention relates to pharmaceutical compositions containing **A** and **B**, characterized in that the p38 kinase inhibitor **B** is selected from the compounds of formula **7** wherein:
- G: is phenyl, pyridinyl, pyridonyl, naphthyl, quinolinyl, isoquinolinyl, pyrazinyl, 3,4-dihydro-2H-benzo[1,4]oxazinyl, benzothiophenyl, dihydrobenzofuranyl, dihydrobenzothiophenyl, benzooxazolyl, indanyl, indolyl, indolinyl, indolonyl or indolinonyl, wherein G is optionally substituted by one or more R₁, R₂ or R₃;

Ar is naphthyl;
- X is: phenyl, imidazolyl, pyridinyl, pyrimidinyl, piperdinyl, piperazinyl, pyridazinyl or pyrazinyl each being optionally independently substituted with one to three C₁₋₄ alkyl, C₁₋₄alkoxy, hydroxy, nitrile, amino, mono- or di-(C₁₋₃ alkyl)amino, mono- or di-(C₁₋₃ alkylamino)carbonyl, NH₂C(O), C₁₋₆ alkyl-S(O)ₘ or halogen;
- Y is:: a bond or
a C₁₋₄ saturated carbon chain wherein one or more of the C atoms is optionally replaced by O, N or S and wherein Y is optionally independently substituted with nitrile or oxo;
- Z is:: phenyl, pyridinyl, pyrimidinyl, pyridazinyl, pyrazinyl, imidazolyl, dihydrothiazolyl, dihydrothiazolyl sulfoxide, pyranyl, pyrrolidinyl, phenylpiperazinyl, tetrahydropyranyl, tetrahydrofuranyl, dioxolanyl, 2-oxa-5-aza-bicyclo[2.2.1]heptanyl, morpholino, thiomorpholino, thiomorpholino sulfoxidyl, piperidinyl, piperidinonyl, piperazinyl or tetrahydropyrimidonyl each of which are optionally substituted with one to two C₁₋₂ alkyl or C₁₋₂ alkoxy;
or Z is hydroxy, C₁₋₃ alkyl, C₁₋₃ alkoxy, C₁₋₃ acylamino, C₁₋₃ alkylsulfonyl, nitrile C₁₋₃ alkyl or amino mono or di-substituted by C₁₋₃ alkoxyC₁₋₃ alkyl;
each R₁ is independently:
C₁₋₅ alkyl branched or unbranched optionally partially or fully halogenated, wherein one or more C atoms are optionally independently replaced by O, N or S(O)ₘ, and wherein said C₁₋₅ alkyl is optionally substituted with oxo, dioxolanyl, pyrrolidinyl, furyl or phenyl each optionally substituted with one to three halogen, C₁₋₃ alkyl which is optionally partially or fully halogenated, hydroxy, nitrile and C₁₋₃alkoxy which is optionally partially or fully halogenated;
cyclopropyl, cyclobutyl, cyclopentanyl, cyclohexanyl, bicyclopentanyl or bicyclohexanyl, each being optionally partially or fully halogenated and optionally substituted with one to three C₁₋₃ alkyl groups optionally partially or fully halogenated, nitrile, hydroxyC₁₋₃alkyl or phenyl; and an analog of cyclopropyl, cyclobutyl, cyclopentanyl, cyclohexanyl, bicyclopentanyl or bicyclohexanyl wherein one ring methylene group is replaced by O;
oxo;
C₂₋₄ alkynyl optionally partially or fully halogenated wherein one or more methylene groups are optionally replaced by O, and optionally independently substituted with one to two oxo groups, hydroxy, pyrroldinyl, pyrrolyl, tetrahydropyranyl, C₁₋₄ alkyl optionally substituted by one or more halogen atoms, nitrile, morpholino, piperidinyl, piperazinyl, imidazolyl, phenyl, pyridinyl, tetrazolyl, or mono- or di(C₁₋₃alkyl)amino optionally substituted by one or more halogen atoms;
or
silyl containing three C₁₋₂ alkyl groups optionally partially or fully halogenated;
each R₂ is independently:
a C₁₋₄ alkyl optionally partially or fully halogenated, C₁₋₄ alkoxy optionally partially or fully halogenated, bromo, chloro, fluoro, methoxycarbonyl, methyl-S(O)ₘ , ethyl-S(O)ₘ each optionally partially or fully halogenated or phenyl-S(O)ₘ;
or R₂ is mono- or di-C₁₋₃acylamino, amino-S(O)ₘ or S(O)ₘamino wherein the N atom is mono- or di-substituted by C₁₋₃alkyl or phenyl, nitrile, nitro or amino;
each R₃ is independently:
phenyl, morpholino, pyridinyl, pyrimidinyl, pyrrolidinyl, 2,5-pyrrolidin-dionyl, imidazolyl, [1,3,4]oxadiazol, pyrazolyl, each of the aforementioned is optionally substituted with one to three C₁₋₃ alkyl which is optionally partially or fully halogenated, halogen, oxo, hydroxy, nitrile and C₁₋₃ alkoxy optionally partially or fully halogenated;
C₁₋₃ alkyl or C₁₋₃ alkoxy optionally partially or fully halogenated or optionally substituted with R₁₇;
OR₁₈ or C₁₋₃ alkyl optionally substituted with OR₁₈;
amino or mono- or di-(C₁₋₃ alkyl)amino optionally substituted with R₁₉;
R₂₀C(O)N(R₂₁)-, R₂₂O- ; R₂₃R₂₄NC(O)-; R₂₆CH₂C(O)N(R₂₁)-, NH₂C(O)methoxy or R₂₆C(O)CH₂N(R₂₁)-;
C₂₋₄ alkenyl substituted by R₂₃R₂₄NC(O)-; or
C₂₋₄ alkynyl substituted with pyrroldinyl or pyrrolyl;
C₁₋₃acyl and
R₂₃ and R₂₄ taken together optionally form morpholino.

In another preferred embodiment the invention relates to pharmaceutical compositions containing **A** and **B**, characterized in that the p38 kinase inhibitor **B** is selected from the compounds of formula **7** wherein:
- G: is phenyl, pyridinyl, pyridonyl, 2-naphthyl, quinolinyl, isoquinolinyl, dihydrobenzofuranyl, indanyl, 5-indolyl, 3-oxo-3,4-dihydro-2H-benzo[1,4]oxazin-8-yl, benzooxalolyl, 2,3-dihydrobenzooxazol-7-yl, 2-oxo-2,3-dihydro-1 H-indol-5-yl, indolinyl, indolonyl, or indolinonyl , wherein G is optionally substituted by one or more R₁, R₂ or R₃;
- Ar: is 1-naphthyl;
- X is:: phenyl, imidazolyl, pyridinyl, pyrimidinyl, piperdinyl, piperazinyl, pyridazinyl or pyrazinyl;
- Y is:: a bond or
-CH₂-, -CH₂CH₂-, -C(O)-, -O-, -S-, -NH-CH₂CH₂CH₂- , -N(CH₃)-,
CH₂(CN)CH₂-NH-CH₂ or -NH-;
- Z is: morpholino, dioxolanyl, tetrahydrofuranyl, pyridinyl, 2-oxa-5-aza-bicyclo[2.2.1]heptanyl, C₁₋₃alkoxyphenylpiperazinyl, hydroxy, C₁₋₃alkyl, N,N-diC₁₋₃alkoxyC₁₋₃alkylamino, C₁₋₃acylamino, C₁₋₃alkylsulfonyl or nitrileC₁₋₃alkyl;
each R₁ is independently:
C₁₋₅ alkyl optionally partially or fully halogenated wherein one or more C atoms are optionally independently replaced by O or N, and wherein said C₁₋₅ alkyl is optionally substituted with oxo, dioxolanyl, pyrrolidinyl, furyl or phenyl optionally substituted by C₁₋₃alkoxy;
cyclopropyl, cyclopentanyl, cyclohexanyl and bicyclopentanyl optionally substituted with one to three methyl groups optionally partially or fully halogenated, nitrile, hydroxymethyl or phenyl; or 2-tetrahydrofuranyl substituted by methyl; or
trimethyl silyl;
propynyl substituted hydroxy or tetrahydropyran-2-yloxy;

- R₂: is
is mono- or di-C₁₋₃acylamino, amino-S(O)ₘ or S(O)ₘ amino wherein the N atom is mono- or di-substituted by C₁₋₃alkyl or phenyl, bromo, chloro, fluoro, nitrile, nitro, amino, _methylsulfonyl optionally partially or fully halogenated or phenylsulfonyl;
each R₃ is independently:
phenyl, morpholino, pyridinyl, pyrimidinyl, pyrrolidinyl, 2,5-pyrrolidin-dionyl, imidazolyl, [1,3,4]oxadiazol or pyrazolyl, each is optionally substituted with C₁₋₂ alkyl which is optionally partially or fully halogenated;
C₁₋₃ alkyl or C₁₋₃ alkoxy each being optionally partially or fully halogenated or optionally substituted with diethylamino;
OR₁₈ or C₁₋₃ alkyl optionally substituted with OR₁₈;
amino or mono- or di-(C₁₋₃ alkyl)amino optionally substituted with R₁₉;
CH₃C(O)NH-, R₂₂O- ; R₂₃R₂₄NC(O)-; R₂₆CH₂C(O)N(R₂₁)-, NH₂C(O)methoxy or R₂₆C(O)CH₂N(R₂₁)-;
C₂₋₄alkenyl substituted by R₂₃R₂₄NC(O)-; or
C₂₋₄ alkynyl substituted with pyrroldinyl or pyrrolyl;
C₁₋₂acyl; and
R₂₃ and R₂₄ are H or R₂₃ and R₂₄ taken together optionally form morpholino; and
R₂₆ is morpholino.

In another preferred embodiment the invention relates to pharmaceutical compositions containing **A** and **B**, characterized in that the p38 kinase inhibitor **B** is selected from the compounds of formula **7** wherein:
- G is: phenyl, pyridinyl, 5-indolyl, 3-oxo-3,4-dihydro-2H-benzo[1,4]oxazin-8-yl, benzooxalolyl, 2,3-dihydrobenzooxazol-7-yl, 2-oxo-2,3-dihydro-1H-indol-5-yl or 2-naphthyl wherein G is optionally substituted by one or more R₁, R₂ or R₃;
- X is:: imidazolyl, pyridinyl, pyrimidinyl or pyrazinyl;
- Y is:: a bond, CH₂(CN)CH₂-NH-CH₂, -CH₂-, -NH-CH₂CH₂CH₂- or -NH-;
- Z: is morpholin-4yl, dioxolan-2yl, tetrahydrofuranyl, pyridinyl, 2-oxa-5-aza-bicyclo[2.2.1]hept-5yl, methoxyphenylpiperazinyl, hydroxy, methyl, N,N-dimethoxyethylamino, acetylamino, methylsulfonyl or cyanoethyl;
each R₁ is independently:
tert-butyl, sec-butyl, tert-amyl, phenyl, tetrahydropyran-2-yloxypropynyl, hydroxypropynyl, trihalomethyl, 2,2-diethylpropionyl or cyclohexanyl;

- R₂: is chloro, nitro, amino, nitrile, methylsulfonylamino, diacetylamino, phenylsulfonylamino, N,N-di(methylsulfonyl)amino, methylsulfonyl or trihalomethylsulfonyl;
- R₃: is independently:
methyl, C₁₋₃ alkoxy, methoxymethyl, hydroxypropyl, dimethylamino, C₁₋₄alkylamino, NH₂C(O)methoxy, acetyl, pyrrolidinyl, imidazolyl, pyrazolyl, morpholino or morpholinocarbonyl.

In yet another preferred embodiment the invention relates to pharmaceutical compositions containing **A** and **B,** characterized in that the p38 kinase inhibitor **B** is selected from the compounds of formula **7** wherein X is pyridinyl.

In still another preferred embodiment the invention relates to pharmaceutical compositions containing **A** and **B,** characterized in that the p38 kinase inhibitor **B** is selected from the compounds of formula **7** wherein the pyridinyl is attached to Ar via the 3-pyridinyl position.

Preferably the invention relates to pharmaceutical compositions containing **A** and **B,** characterized in that the p38 kinase inhibitor **B** is selected from the following compounds of formula **7 :**
1-(4-tert-Butyl-phenyl)-3-[4-(6-morpholin-4-ylmethyl-pyridin-3-yl)-naphthalen-1-yl]-urea;
1-(5-tert-Butyl-2-methyl-phenyl)-3-[4-(4-morpholin-4-ylmethyl-piperidin-1-yl)-naphthalen-1-yl]-urea;
1-(6-Chloro-4-trifluoromethyl-pyridin-2-yl)-3-[4-(6-morpholin-4-ylmethyl-pyridin-3-yl)-naphthalen-1-yl]-urea;
1-(4-Difluoromethoxy-phenyl)-3-[4-(6-morpholin-4-ylmethyl-pyridin-3-yl)-naphthalen-1-yl]-urea;
1-(3-Methyl-naphthalen-2-yl)-3-[4-(6-morpholin-4-ylmethyl-pyridin-3-yl)-naphthalen-1-yl]-u rea;
1-[2-Methoxy-5-(1-methyl-1-phenyl-ethyl)-phenyl]-3-[4-(6-morpholin-4-ylmethyl-pyridin-3-yl)-naphthalen-1-yl]-urea;
(5-tert-Butyl-2-methyl-phenyl)-carbamic acid 3-(5-{4-[3-(5-tert-butyl-2-methyl-phenyl)-ureido]-naphthalen-1-yl}-pyridin-2-ylamino)-propyl ester;
1-(6-tert-Butyl-benzo[1,3]dioxol-5-yl)-3-[4-(6-morpholin-4-ylmethyl-pyridin-3-yl)-naphthalen-1-yl]-urea;
N-(5-tert-Butyl-2-methoxy-3-{3-[4-(6-morpholin-4-ylmethyl-pyridin-3-yl)-naphthalen-1-yl]-ureido}-phenyl)-acetamide;
1,3-Bis-[4-(6-morpholin-4-ylmethyl-pyridin-3-yl)-naphthalen-1-yl]-urea;
1-[5-tert-Butyl-3-(2,2-dimethyl-[1,3]dioxolan-4-ylmethyl)-2-hydroxy-phenyl]-3-[4-(6-morpholin-4-ylmethyl-pyridin-3-yl)-naphthalen-1-yl]-urea;
1-[5-tert-Butyl-2-(2-pyrrolidin-1-yl-ethoxy)-phenyl]-3-[4-(6-morpholin-4-ylmethyl-pyridin-3-yl)-naphthalen-1-yl]-urea;
1-[5-tert-Butyl-3-(2,3-dihydroxy-propyl)-2-hydroxy-phenyl]-3-[4-(6-morpholin-4-ylmethyl-pyridin-3-yl)-naphthalen-1-yl]-urea;
1-(2,3-Dimethyl-1 H-indol-5-yl)-3-[4-(6-morpholin-4-ylmethyl-pyridin-3-yl)-naphthalen-1-yl]-urea;
1-[4-(6-Morpholin-4-ylmethyl-pyridin-3-yl)-naphthalen-1-yl]-3-(2-p-tolyloxy-5-trifluoromethyl-phenyl)-urea;
1-[2-(2-Methoxy-phenoxy)-5-trifluoromethyl-phenyl]-3-[4-(6-morpholin-4-ylmethyl-pyridin-3-yl)-naphthalen-1-yl]-urea;
1-[4-(6-Morpholin-4-ylmethyl-pyridin-3-yl)-naphthalen-1-yl]-3-naphthalen-1-yl-urea;
1-{5-tert-Butyl-2-methyl-3-[3-(tetrahydro-pyran-2-yloxy)-prop-1-ynyl]-phenyl}-3-[4-(6-morpholin-4-ylmethyl-pyridin-3-yl)-naphthalen-1-yl]-urea;
1-{5-tert-Butyl-2-[3-(tetrahydro-pyran-2-yloxy)-prop-1-ynyl]-phenyl}-3-[4-(6-morpholin-4-ylmethyl-pyridin-3-yl)-naphthalen-1-yl]-urea;
1-(5-Hydroxymethyl-2-methyl-phenyl)-3-[4-(6-morpholin-4-ylmethyl-pyridin-3-yl)-naphthalen-1-yl]-urea;
1-(2-Methoxy-dibenzofuran-3-yl)-3-[4-(6-morpholin-4-ylmethyl-pyridin-3-yl)-naphthalen-1-yl]-urea;
1-(2,5-Di-tert-butyl-phenyl)-3-[4-(6-morpholin-4-ylmethyl-pyridin-3-yl)-naphthalen-1-yl]-urea;
1-[3-(4-Bromo-1-methyl-1H-pyrazol-3-yl)-phenyl]-3-[4-(6-morpholin-4-ylmethyl-pyridin-3-yl)-naphthalen-1-yl]-urea;
1-(3-Hydroxy-5,6,7,8-tetrahydro-naphthalen-2-yl)-3-[4-(6-morpholin-4-ylmethyl-pyridin-3-yl)-naphthalen-1-yl]-urea;
1-(1-Acetyl-2,3-dihydro-1H-indol-5-yl)-3-[4-(6-morpholin-4-ylmethyl-pyridin-3-yl)-naphthalen-1-yl]-urea;
1-[4-(6-Morpholin-4-ylmethyl-pyridin-3-yl)-naphthalen-1-yl]-3-(3-oxazol-5-yl-phenyl)-urea;
1-[4-(6-Morpholin-4-ylmethyl-pyridin-3-yl)-naphthalen-1-yl]-3-(3-[1,3,4]oxadiazol-2-yl-phenyl)-urea;
1-(2-Methoxy-5-trifluoromethyl-phenyl)-3-[4-(6-morpholin-4-ylmethyl-pyridin-3-yl)-naphthalen-1-yl]-urea;
Furan-2-carboxylic acid (4-tert-butyl-2-{3-[4-(6-morpholin-4-ylmethyl-pyridin-3-yl)-naphthalen-1-yl]-ureido}-phenyl)-amide;
1-(2-Methoxy-4-phenylamino-phenyl)-3-[4-(6-morpholin-4-ylmethyl-pyridin-3-yl)-naphthalen-1-yl]-urea;
1-(5-Methoxy-2-methyl-phenyl)-3-[4-(6-morpholin-4-ylmethyl-pyridin-3-yl)-naphthalen-1-yl]-urea;
1-(3-Hydroxy-naphthalen-2-yl)-3-[4-(6-morpholin-4-ylmethyl-pyridin-3-yl)-naphthalen-1-yl]-urea;
N, N-Diethyl-4-methoxy-3-{3-[4-(6-morpholin-4-ylmethyl-pyridin-3-yl)-naphthalen-1-yl]-ureido}-benzenesulfonamide;
1-(2,2-Difluoro-benzo[1,3]dioxol-5-yl)-3-[4-(6-morpholin-4-ylmethyl-pyridin-3-yl)-naphthalen-1-yl]-urea;
1-[5-(1,1-Dimethyl-propyl)-2-phenoxy-phenyl]-3-[4-(6-morpholin-4-ylmethyl-pyridin-3-yl)-naphthalen-1-yl]-urea;
1-[5-(2,2-Dimethyl-propionyl)-2-methyl-phenyl]-3-[4-(6-morpholin-4-ylmethyl-pyridin-3-yl)-naphthalen-1-yl]-urea;
2-Chloro-5-{3-[4-(6-morpholin-4-ylmethyl-pyridin-3-yl)-naphthalen-1-yl]-ureido}-benzoic acid isopropyl ester;
1-(4-Amino-3,5-dibromo-phenyl)-3-[4-(6-morpholin-4-ylmethyl-pyridin-3-yl)-naphthalen-1-yl]-urea;
1-[5-tert-Butyl-3-(3-hydroxy-prop-1-ynyl)-2-methyl-phenyl]-3-[4-(6-morpholin-4-ylmethyl-pyridin-3-yl)-naphthalen-1-yl]-urea;
1-[5-tert-Butyl-2-(3-hydroxy-prop-1-ynyl)-phenyl]-3-[4-(6-morpholin-4-ylmethyl-pyridin-3-yl)-naphthalen-1-yl]-urea;
1-[5-tert-Butyl-3-(2,2-dimethyl-[1,3]dioxolan-4-ylmethyl)-2-methoxy-phenyl]-3-[4-(6-morpholin-4-ylmethyl-pyridin-3-yl)-naphthalen-1-yl]-urea;
1-[5-tert-Butyl-3-(2,3-dihydroxy-propyl)-2-methoxy-phenyl]-3-[4-(6-morpholin-4-ylmethyl-pyridin-3-yl)-naphthalen-1-yl]-urea;
1-(5-tert-Butoxy-2-methoxy-phenyl)-3-[4-(6-morpholin-4-ylmethyl-pyridin-3-yl)-naphthalen-1-yl]-urea;
1-[5-(1-Cyano-cyclopropyl)-2-methoxy-phenyl]-3-[4-(6-morpholin-4-ylmethyl-pyridin-3-yl)-naphthalen-1-yl]-urea;
1-[5-tert-Butyl-3-(2-d iethylamino-ethyl)-2-methoxy-phenyl]-3-[4-(6-morpholin-4-ylmethyl-pyridin-3-yl)-naphthalen-1-yl]-urea;
1-(5-tert-Butyl-2-methoxy-phenyl)-3-[4-(6-[1,3]dioxolan-2-yl-pyridin-3-yl)-naphthalen-1-yl]-urea;
1-(5-tert-Butyl-2-pyrrolid in-1-yl-phenyl)-3-[4-(6-morpholin-4-ylmethyl-pyridin-3-yl)-naphthalen-1-yl]-urea;
1-(5-tert-Butyl-2-dimethylamino-phenyl)-3-[4-(6-morpholin-4-ylmethyl-pyridin-3-yl)-naphthalen-1-yl]-urea;
1-(5-tert-Butyl-2-propoxy-phenyl)-3-[4-(6-morpholin-4-ylmethyl-pyridin-3-yl)-naphthalen-1-yl]-urea;
1-(5-tert-Butyl-2-methoxy-phenyl)-3-[4-(6-hydroxymethyl-pyridin-3-yl)-naphthalen-1-yl]-urea;
1-(5-tert-Butyl-2-methoxy-phenyl)-3-{4-[6-(2,6-dimethyl-morpholin-4-ylmethyl)-pyridin-3-yl]-naphthalen-1-yl}-urea;
2-(5-tert-Butyl-2-methoxy-phenyl)-N-[4-(6-morpholin-4-ylmethyl-pyridin-3-yl)-naphthalen-1-yl]-acetamide;
1-(2-Methoxy-5-phenoxy-phenyl)-3-[4-(6-morpholin-4-ylmethyl-pyridin-3-yl)-naphthalen-1-yl]-urea;
1-(3,3-Dimethyl-2-oxo-2,3-dihydro-1H-indol-7-yl)-3-[4-(6-morpholin-4-ylmethyl-pyridin-3-yl)-naphthalen-1-yl]-urea;
1-(5-tert-Butyl-2-cyclopentyloxy-phenyl)-3-[4-(6-morpholin-4-ylmethyl-pyridin-3-yl)-naphthalen-1-yl]-urea;
1-(5-tert-Butyl-2-methoxy-phenyl)-3-{4-[6-(3-pyridin-3-yl-pyrrolidin-1-ylmethyl)-pyridin-3-yl]-naphthalen-1-yl}-urea;
1-(5-Cyclohexyl-2-methoxy-phenyl)-3-[4-(6-morpholin-4-ylmethyl-pyridin-3-yl)-naphthalen-1-yl]-urea;
1-(2,4-Dimethoxy-5-trifluoromethyl-phenyl)-3-[4-(6-morpholin-4-ylmethyl-pyridin-3-yl)-naphthalen-1-yl]-urea;
1-(6-tert-Butyl-3-oxo-3,4-dihydro-2H-benzo[1,4]oxazin-7-yl)-3-[4-(6-morpholin-4-ylmethyl-pyridin-3-yl)-naphthalen-1-yl]-urea;
1-(5-tert-Butyl-2-methoxy-3-nitro-phenyl)-3-[4-(6-morpholin-4-ylmethyl-pyridin-3-yl)-naphthalen-1-yl]-urea;
1-(3-Amino-5-tert-butyl-2-methoxy-phenyl)-3-[4-(6-methyl-pyridin-3-yl)-naphthalen-1-yl]-u rea;
N-Acetyl-N-(5-tert-butyl-2-methoxy-3-{3-[4-(6-morpholin-4-ylmethyl-pyridin-3-yl)-naphthalen-1-yl]-ureido}-phenyl)-acetamide;
1-(6-tert-Butyl-4-methyl-3-oxo-3,4-dihydro-2H-benzo[1,4]oxazin-8-yl)-3-[4-(6-morpholin-4-ylmethyl-pyridin-3-yl)-naphthalen-1-yl]-urea;
1-[6-tert-Butyl-4-(2-morpholin-4-yl-ethyl)-3-oxo-3,4-dihydro-2H-benzo[1,4]oxazin-8-yl]-3-[4-(6-morpholin-4-ylmethyl-pyridin-3-yl)-naphthalen-1-yl]-urea;
1-(5-tert-Butyl-2-ethoxy-phenyl)-3-[4-(6-morpholin-4-ylmethyl-pyridin-3-yl)-naphthalen-1-yl]-urea;
1-(5-tert-Butyl-2-isopropoxy-phenyl)-3-[4-(6-morpholin-4-ylmethyl-pyridin-3-yl)-naphthalen-1-yl]-urea;
1-(5-tert-Butyl-2-imidazol-1-yl-phenyl)-3-[4-(6-morpholin-4-ylmethyl-pyridin-3-yl)-naphthalen-1-yl]-urea;
N-(5-tert-Butyl-2-methoxy-4-{3-[4-(6-morpholin-4-ylmethyl-pyridin-3-yl)-naphthalen-1-yl]-ureido}-phenyl)-methanesulfonamide;
1-(5-tert-Butyl-3-ethylamino-2-methoxy-phenyl)-3-[4-(6-morpholin-4-ylmethyl-pyridin-3-yl)-naphthalen-1-yl]-urea;
N-(5-tert-Butyl-2-methoxy-3-{3-[4-(6-morpholin-4-ylmethyl-pyridin-3-yl)-naphthalen-1-yl]-ureido}-phenyl)-bis(methanesulfon)amide;
1-[5-tert-Butyl-2-(1-methyl-1H-pyrazol-4-yl)-phenyl]-3-[4-(6-morpholin-4-ylmethyl-pyridin-3-yl)-naphthalen-1-yl]-urea;
1-(2-Methanesulfinyl-5-trifluoromethyl-phenyl)-3-[4-(6-morpholin-4-ylmethyl-pyridin-3-yl)-naphthalen-1-yl]-urea;
1-(2-Ethanesulfonyl-5-trifluoromethyl-phenyl)-3-[4-(6-morpholin-4-ylmethyl-pyridin-3-yl)-naphthalen-1-yl]-urea;
1-[4-(6-{[Bis-(2-methoxy-ethyl)-amino]-methyl}-pyridin-3-yl)-naphthalen-1-yl]-3-(5-tert-butyl-2-methoxy-phenyl)-urea;
1-(5-tert-Butyl-2-methoxy-phenyl)-3-{4-[6-(3-dimethylamino-pyrrolidin-1-ylmethyl)-pyridin-3-yl]-naphthalen-1-yl}-urea;
N-[1-(5-{4-[3-(5-tert-Butyl-2-methoxy-phenyl)-ureido]-naphthalen-1-yl}-pyridin-2-ylmethyl)-pyrrolidin-3-yl]-acetamide;
1-(1-Acetyl-3,3-dimethyl-2,3-dihydro-1H-indol-5-yl)-3-[4-(6-morpholin-4-ylmethyl-pyridin-3-yl)-naphthalen-1-yl]-urea;
N-(5-tert-Butyl-2-methoxy-3-{3-[4-(6-morpholin-4-ylmethyl-pyridin-3-yl)-naphthalen-1-yl]-ureido}-phenyl)-propionamide;
1-(5-tert-Butyl-2-methyl-benzooxazol-7-yl)-3-[4-(6-morpholin-4-ylmethyl-pyridin-3-yl)-naphthalen-1-yl]-urea;
1-[4-(6-Morpholin-4-ylmethyl-pyridin-3-yl)-naphthalen-1-yl]-3-(3-trifluoromethanesulfonyl-phenyl)-urea;
N-(5-tert-Butyl-2-methoxy-3-{3-[4-(6-morpholin-4-ylmethyl-pyridin-3-yl)-naphthalen-1-yl]-ureido}-phenyl)-isobutyramide;
2-(4-tert-Butyl-2-{3-[4-(6-morpholin-4-ylmethyl-pyridin-3-yl)-naphthalen-1-yl]-ureido}-phenoxy)-acetamide;
1-(5-tert-Butyl-2-oxo-2,3-dihydro-benzooxazol-7-yl)-3-[4-(6-morpholin-4-ylmethyl-pyridin-3-yl)-naphthalen-1-yl]-urea;
1-(6-tert-Butyl-3-cyano-2-methoxymethoxy-pyridin-4-yl)-3-[4-(6-morpholin-4-ylmethyl-pyridin-3-yl)-naphthalen-1-yl]-urea;
1-(6-tert-Butyl-3-cyano-2-hydroxy-pyridin-4-yl)-3-[4-(6-morpholin-4-ylmethyl-pyridin-3-yl)-naphthalen-1-yl]-urea;
1-(5-tert-Butyl-3-cyano-2-methoxy-phenyl)-3-[4-(6-morpholin-4-ylmethyl-pyridin-3-yl)-naphthalen-1-yl]-urea;
1-[4-(6-Morpholin-4-ylmethyl-pyridin-3-yl)-naphthalen-1-yl]-3-(1,3,3-trimethyl-2,3-dihydro-1 H-indol-5-yl)-urea;
1-(5-tert-Butyl-benzooxazol-7-yl)-3-[4-(6-morpholin-4-ylmethyl-pyridin-3-yl)-naphthalen-1-yl]-urea;
N-(5-tert-Butyl-2-methoxy-3-{3-[4-(6-morpholin-4-ylmethyl-pyridin-3-yl)-naphthalen-1-yl]-ureidol-phenyl)-benzenesulfonamide;
Ethanesulfonic acid (5-tert-butyl-2-methoxy-3-f3-[4-(6-morpholin-4-ylmethyl-pyridin-3-yl)-naphthalen-1-yl]-ureido}-phenyl)-amide;
1-(5-tert-Butyl-2-methoxy-phenyl)-3-[4-(4-morpholin-4-ylmethyl-piperidin-1-yl)-naphthalen-1-yl]-urea;
1-[5-tert-Butyl-2-(1-methyl-1H-pyrazol-4-yl)-phenyl]-3-[4-(4-morpholin-4-ylmethyl-piperidin-1-yl)-naphthalen-1-yl]-urea;
1-(5-tert-Butyl-2-methoxy-phenyl)-3-[4-(2-morpholin-4-ylmethyl-pyrimidin-5-yl)-naphthalen-1-yl]-urea;
1-(5-tert-Butyl-2-methylsulfanyl-phenyl)-3-[4-(6-morpholin-4-ylmethyl-pyridin-3-yl)-naphthalen-1-yl]-urea;
1-(5-tert-Butyl-2-methoxy-pyridin-3-yl)-3-[4-(6-morpholin-4-ylmethyl-pyridin-3-yl)-naphthalen-1-yl]-urea;
2,2,2-Trifluoro-ethanesulfonic acid (5-tert-butyl-2-methoxy-3-{3-[4-(6-morpholin-4-ylmethyl-pyridin-3-yl)-naphthalen-1-yl]-ureido}-phenyl)-amide;
N-(5-{4-[3-(5-tert-Butyl-2-methyl-phenyl)-ureido]-naphthalen-1-yl}-pyrazin-2-yl)-methanesulfonamide;
1-[4-(6-{[Bis-(2-cyano-ethyl)-amino]-methyl}-pyridin-3-yl)-naphthalen-1-yl]-3-(5-tert-butyl-2-methoxy-phenyl)-urea;
1-(5-tert-Butyl-2-methoxy-phenyl)-3-{4-[6-(4-methyl-piperazin-1-ylmethyl)-pyridin-3-yl]-naphthalen-1-yl}-urea;
1-(5-tert-Butyl-2-methoxy-phenyl)-3-[4-(6-thiomorpholin-4-ylmethyl-pyridin-3-yl)-naphthalen-1-yl]-urea;
1-(5-tert-Butyl-2-methoxy-phenyl)-3-{4-[6-(2,6-dimethyl-piperidin-1-ylmethyl)-pyridin-3-yl]-naphthalen-1-yl}-urea;
1-(5-tert-Butyl-2-methoxy-phenyl)-3-{4-[6-(1-oxo-tetrahydro-thiopyran-4-ylamino)-pyridin-3-yl]-naphthalen-1-yl}-urea;
1-(5-tert-Butyl-2-methoxy-phenyl)-3-{4-[6-(tetrahydro-pyran-4-ylamino)-pyridin-3-yl]-naphthalen-1-yl}-urea;
1-(5-tert-Butyl-2-methoxy-phenyl)-3-[4-(6-{[(2-cyano-ethyl)-(tetrahydro-furan-2-ylmethyl)-amino]-methyl}-pyridin-3-yl)-naphthalen-1-yl]-urea;
1-(5-tert-Butyl-2-methoxy-phenyl)-3-{4-[6-(2-methoxymethyl-morpholin-4-ylmethyl)-pyridin-3-yl]-naphthalen-1-yl}-urea;
1-(5-tert-Butyl-2-methoxy-phenyl)-3-(4-{6-[(2-morpholin-4-yl-ethylamino)-methyl]-pyridin-3-yl}-naphthalen-1-yl)-urea;
1-(5-tert-Butyl-2-methoxy-phenyl)-3-{4-[6-(2-methyl-3-oxo-piperazin-1-ylmethyl)-pyridin-3-yl]-naphthalen-1-yl}-urea;
1-(5-{4-[3-(5-tert-Butyl-2-methoxy-phenyl)-ureido]-naphthalen-1-yl}-pyridin-2-ylmethyl)-piperidine-3-carboxylic acid amide;
1-(5-{4-[3-(5-tert-Butyl-2-methoxy-phenyl)-ureido]-naphthalen-1-yl}-pyridin-2-ylmethyl)-piperidine-4-carboxylic acid amide;
1-(5-tert-Butyl-2-methoxy-phenyl)-3-(4-[6-(1-oxo-1l4-thiomorpholin-4-ylmethyl)-pyridin-3-yl]-naphthalen-1-yl}-urea;
1-(3,3-Dimethyl-2-oxo-2,3-dihydro-1H-indol-5-yl)-3-[4-(6-morpholin-4-ylmethyl-pyridin-3-yl)-naphthalen-1-yl]-urea;
1-(5-tert-Butyl-2-methoxy-phenyl)-3-{4-[6-(3-oxo-piperazin-1-ylmethyl)-pyridin-3-yl]-naphthalen-1-yl}-urea;
1-{4-[6-(4-Acetyl-piperazin-1-ylmethyl)-pyridin-3-yl]-naphthalen-1-yl}-3-(5-tert-butyl-2-methoxy-phenyl)-urea;
4-(5-{4-[3-(5-tert-Butyl-2-methoxy-phenyl)-ureido]-naphthalen-1-yl}-pyridin-2-ylmethyl)-piperazine-1-carboxylic acid ethyl ester;
1-(5-tert-Butyl-2-methoxy-phenyl)-3-(4-{6-[(2-pyridin-3-yl-ethylamino)-methyl]-pyridin-3-yl}-naphthalen-1-yl)-urea;
1-(5-tert-Butyl-2-methoxy-phenyl)-3-(4-{6-[(tetrahydro-furan-3-ylamino)-methyl]-pyridin-3-yl}-naphthalen-1-yl)-urea;
1-(5-tert-Butyl-2-methoxy-phenyl)-3-[4-(6-{[(2-cyano-ethyl)-pyridin-3-ylmethyl-amino]-methyl}-pyridin-3-yl)-naphthalen-1-yl]-urea;
1-(5-tert-Butyl-2-methoxy-phenyl)-3-(4-{6-[(2-methylsulfanyl-ethylamino)-methyl]-pyridin-3-yl}-naphthalen-1-yl)-urea;
1-(5-tert-Butyl-2-methoxy-phenyl)-3-{4-[6-(2-oxa-5-aza-bicyclo[2.2.1]hept-5-ylmethyl)-pyridin-3-yl]-naphthalen-1-yl}-urea;
1-(5-tert-Butyl-2-methoxy-phenyl)-3-{4-[6-(2,6-dimethyl-morpholin-4-ylmethyl)-pyridin-3-yl]-naphthalen-1-yl}-urea;
1-(5-tert-Butyl-2-methoxy-phenyl)-3-(4-{6-[(2-piperazin-1-yl-ethylamino)-methyl]-pyridin-3-yl}-naphthalen-1-yl)-urea;
1-(5-tert-Butyl-2-methoxy-phenyl)-3-{4-[6-(4-pyrimidin-2-yl-piperazin-1-ylmethyl)-pyridin-3-yl]-naphthalen-1-yl}-urea;
1-(5-tert-Butyl-2-methoxy-phenyl)-3-{4-[6-(4-pyridin-2-yl-piperazin-1-ylmethyl)-pyridin-3-yl]-naphthalen-1-yl}-urea;
1-(5-tert-Butyl-2-methoxy-phenyl)-3-(4-{6-[4-(3-methoxy-phenyl)-piperazin-1-ylmethyl]-pyridin-3-yl}-naphthalen-1-yl)-urea;
1-(5-tert-Butyl-2-methoxy-phenyl)-3-{4-[6-(morpholine-4-carbonyl)-pyridin-3-yl]-naphthalen-1-yl}-urea;
1-(5-tert-Butyl-2-methoxy-phenyl)-3-{4-[6-(2-thia-5-aza-bicyclo[2.2.1]hept-5-ylmethyl)-pyridin-3-yl]-naphthalen-1-yl}-urea;
1-(5-tert-Butyl-2-methoxy-phenyl)-3-[4-(5-morpholin-4-ylmethyl-pyrazin-2-yl)-naphthalen-1-yl]-urea;
1-(6-tert-Butyl-3-oxo-3,4-dihydro-2H-benzo[1,4]oxazin-8-yl)-3-[4-(6-morpholin-4-ylmethyl-pyridin-3-yl)-naphthalen-1-yl]-urea;
1-(3-Amino-5-tert-butyl-2-methoxy-phenyl)-3-[4-(6-morpholin-4-ylmethyl-pyridin-3-yl)-naphthalen-1-yl]-urea;
N-(5-{4-[3-(5-tert-Butyl-2-methoxy-phenyl)-ureido]-naphthalen-1-yl}-pyridin-2-yl)-acetamide;
N-(5-tert-Butyl-2-methoxy-3-{3-[4-(6-morpholin-4-ylmethyl-pyridin-3-yl)-naphthalen-1-yl]-ureido}-phenyl)-N-methyl-acetamide;
N-(5-tert-Butyl-2-methoxy-3-{3-[4-(6-morpholin-4-ylmethyl-pyridin-3-yl)-naphthalen-1-yl]-ureido}-phenyl)-2,2,2-trifluoro-acetamide;
1-(5-tert-Butyl-2-methoxy-phenyl)-3-{4-[6-(pyridin-3-yloxy)-pyridin-3-yl]-naphthalen-1-yl}-urea;
1-(5-tert-Butyl-2-methoxy-phenyl)-3-{4-[6-(pyridin-3-ylamino)-pyridin-3-yl]-naphthalen-1-yl}-urea;
[4-(6-Morpholin-4-ylmethyl-pyridin-3-yl)-naphthalen-1-yl]-carbamic acid 3-tert-butylphenyl ester;
N-(5-tert-Butyl-2-methoxy-3-{3-[4-(6-morpholin-4-ylmethyl-pyridin-3-yl)-naphthalen-1-yl]-ureido}-phenyl)-methanesulfonamide and
and the pharmaceutically acceptable derivatives thereof.

In another preferred embodiment the invention relates to pharmaceutical compositions containing **A** and **B**, characterized in that the p38 kinase inhibitor **B** is selected from the following compounds of formula **7**
1-(3-Methyl-naphthalen-2-yl)-3-[4-(6-morpholin-4-ylmethyl-pyridin-3-yl)-naphthalen-1-yl]-urea;
N-(5-tert-Butyl-2-methoxy-3-{3-[4-(6-morpholin-4-ylmethyl-pyridin-3-yl)-naphthalen-1-yl]-ureido}-phenyl)-acetamide;
1-[5-tert-Butyl-3-(2,3-dihydroxy-propyl)-2-hydroxy-phenyl]-3-[4-(6-morpholin-4-ylmethyl-pyridin-3-yl)-naphthalen-1-yl]-urea;
1-(2,3-Dimethyl-1 H-indol-5-yl)-3-[4-(6-morpholin-4-ylmethyl-pyridin-3-yl)-naphthalen-1-yl]-urea;
1-{5-tert-Butyl-2-methyl-3-[3-(tetrahydro-pyran-2-yloxy)-prop-1-ynyl]-phenyl}-3-[4-(6-morpholin-4-ylmethyl-pyridin-3-yl)-naphthalen-1-yl]-urea;
1-(2-Methoxy-5-trifluoromethyl-phenyl)-3-[4-(6-morpholin-4-ylmethyl-pyridin-3-yl)-naphthalen-1-yl]-urea;
1-[5-(2,2-Dimethyl-propionyl)-2-methyl-phenyl]-3-[4-(6-morpholin-4-ylmethyl-pyridin-3-yl)-naphthalen-1-yl]-urea;
1-[5-tert-Butyl-3-(3-hyd roxy-prop-1-ynyl)-2-methyl-phenyl]-3-[4-(6-morphol in-4-ylmethyl-pyridin-3-yl)-naphthalen-1-yl]-urea;
1-[5-tert-Butyl-2-(3-hydroxy-prop-1-ynyl)-phenyl]-3-[4-(6-morpholin-4-ylmethyl-pyridin-3-yl)-naphthalen-1-yl]-urea;
1-[5-tert-Butyl-3-(2,2-d imethyl-[1,3]dioxolan-4-ylmethyl)-2-methoxy-phenyl]-3-[4-(6-morpholin-4-ylmethyl-pyridin-3-yl)-naphthalen-1-yl]-urea;
1-[5-tert-Butyl-3-(2,3-dihydroxy-propyl)-2-methoxy-phenyl]-3-[4-(6-morpholin-4-ylmethyl-pyridin-3-yl)-naphthalen-1-yl]-urea;
1-(5-tert-Butoxy-2-methoxy-phenyl)-3-[4-(6-morpholin-4-ylmethyl-pyridin-3-yl)-naphthalen-1-yl]-urea;
1-[5-(1-Cyano-cyclopropyl)-2-methoxy-phenyl]-3-[4-(6-morpholin-4-ylmethyl-pyridin-3-yl)-naphthalen-1-yl]-urea;
1-[5-tert-Butyl-3-(2-diethylamino-ethyl)-2-methoxy-phenyl]-3-[4-(6-morpholin-4-ylmethyl-pyridin-3-yl)-naphthalen-1-yl]-urea;
1-(5-tert-Butyl-2-methoxy-phenyl)-3-[4-(6-[1,3]dioxolan-2-yl-pyridin-3-yl)-naphthalen-1-yl]-urea;
1-(5-tert-Butyl-2-pyrrolidin-1-yl-phenyl)-3-[4-(6-morpholin-4-ylmethyl-pyridin-3-yl)-naphthalen-1-yl]-urea;
1-(5-tert-Butyl-2-dimethylamino-phenyl)-3-[4-(6-morpholin-4-ylmethyl-pyridin-3-yl)-naphthalen-1-yl]-urea;
1-(5-tert-Butyl-2-propoxy-phenyl)-3-[4-(6-morpholin-4-ylmethyl-pyridin-3-yl)-naphthalen-1-yl]-urea;
1-(5-tert-Butyl-2-methoxy-phenyl)-3-[4-(6-hydroxymethyl-pyridin-3-yl)-naphthalen-1-yl]-urea;
1-(5-tert-Butyl-2-methoxy-phenyl)-3-{4-[6-(2,6-dimethyl-morpholin-4-ylmethyl)-pyridin-3-yl]-naphthalen-1-yl}-urea;
1-(5-Cyclohexyl-2-methoxy-phenyl)-3-[4-(6-morpholin-4-ylmethyl-pyridin-3-yl)-naphthalen-1-yl]-urea;
1-(2,4-Dimethoxy-5-trifluoromethyl-phenyl)-3-[4-(6-morpholin-4-ylmethyl-pyridin-3-yl)-naphthalen-1-yl]-urea;
1-(5-tert-Butyl-2-methoxy-3-nitro-phenyl)-3-[4-(6-morpholin-4-ylmethyl-pyridin-3-yl)-naphthalen-1-yl]-urea;
1-(3-Amino-5-tert-butyl-2-methoxy-phenyl)-3-[4-(6-methyl-pyridin-3-yl)-naphthalen-1-yl]-urea;
N-Acetyl-N-(5-tert-butyl-2-methoxy-3-{3-[4-(6-morpholin-4-ylmethyl-pyridin-3-yl)-naphthalen-1-yl]-ureido}-phenyl)-acetamide;
1-(6-tert-Butyl-4-methyl-3-oxo-3,4-dihydro-2H-benzo[1,4]oxazin-8-yl)-3-[4-(6-morpholin-4-ylmethyl-pyridin-3-yl)-naphthalen-1-yl]-urea;
1-(5-tert-Butyl-2-ethoxy-phenyl)-3-[4-(6-morpholin-4-ylmethyl-pyridin-3-yl)-naphthalen-1-yl]-urea;
1-(5-tert-Butyl-2-isopropoxy-phenyl)-3-[4-(6-morpholin-4-ylmethyl-pyridin-3-yl)-naphthalen-1-yl]-urea;
1-(5-tert-Butyl-2-imidazol-1-yl-phenyl)-3-[4-(6-morpholin-4-ylmethyl-pyridin-3-yl)-naphthalen-1-yl]-urea;
1-(5-tert-Butyl-3-ethylamino-2-methoxy-phenyl)-3-[4-(6-morpholin-4-ylmethyl-pyridin-3-yl)-naphthalen-1-yl]-urea;
N-(5-tert-Butyl-2-methoxy-3-{3-[4-(6-morpholin-4-ylmethyl-pyridin-3-yl)-naphthalen-1-yl]-ureido}-phenyl)-bis(methanesulfon)amide;
1-[5-tert-Butyl-2-(1-methyl-1H-pyrazol-4-yl)-phenyl]-3-[4-(6-morpholin-4-ylmethyl-pyridin-3-yl)-naphthalen-1-yl]-urea;
1-(2-Methanesulfinyl-5-trifluoromethyl-phenyl)-3-[4-(6-morpholin-4-ylmethyl-pyridin-3-yl)-naphthalen-1-yl]-urea;
1-[4-(6-{[Bis-(2-methoxy-ethyl)-amino]-methyl}-pyridin-3-yl)-naphthalen-1-yl]-3-(5-tert-butyl-2-methoxy-phenyl)-urea;
N-[1-(5-{4-[3-(5-tert-Butyl-2-methoxy-phenyl)-ureido]-naphthalen-1-yl}-pyridin-2-ylmethyl)-pyrrolidin-3-yl]-acetamide;
1-(1-Acetyl-3,3-dimethyl-2,3-dihydro-1H-indol-5-yl)-3-[4-(6-morpholin-4-ylmethyl-pyridin-3-yl)-naphthalen-1-yl]-urea;
N-(5-tert-Butyl-2-methoxy-3-{3-[4-(6-morpholin-4-ylmethyl-pyridin-3-yl)-naphthalen-1-yl]-ureido}-phenyl)-propionamide;
1-(5-tert-Butyl-2-methyl-benzooxazol-7-yl)-3-[4-(6-morpholin-4-ylmethyl-pyridin-3-yl)-naphthalen-1-yl]-urea;
1-[4-(6-Morpholin-4-ylmethyl-pyridin-3-yl)-naphthalen-1-yl]-3-(3-trifluoromethanesulfonyl-phenyl)-urea;
N-(5-tert-Butyl-2-methoxy-3-{3-[4-(6-morpholin-4-ylmethyl-pyridin-3-yl)-naphthalen-1-yl]-ureido}-phenyl)-isobutyramide;
2-(4-tert-Butyl-2-{3-[4-(6-morpholin-4-ylmethyl-pyridin-3-yl)-naphthalen-1-yl]-ureido}-phenoxy)-acetamide;
1-(5-tert-Butyl-2-oxo-2,3-dihydro-benzooxazol-7-yl)-3-[4-(6-morpholin-4-ylmethyl-pyridin-3-yl)-naphthalen-1-yl]-urea;
1-(5-tert-Butyl-3-cyano-2-methoxy-phenyl)-3-[4-(6-morpholin-4-ylmethyl-pyridin-3-yl)-naphthalen-1-yl]-urea;
1-(5-tert-Butyl-benzooxazol-7-yl)-3-[4-(6-morpholin-4-ylmethyl-pyridin-3-yl)-naphthalen-1-yl]-urea;
N-(5-tert-Butyl-2-methoxy-3-{3-[4-(6-morpholin-4-ylmethyl-pyridin-3-yl)-naphthalen-1-yl]-ureidol}-phenyl)-benzenesulfonamide;
Ethanesulfonic acid (5-tert-butyl-2-methoxy-3-{3-[4-(6-morpholin-4-ylmethyl-pyridin-3-yl)-naphthalen-1-yl]-ureido}-phenyl)-amide;
1-(5-tert-Butyl-2-methoxy-phenyl)-3-[4-(2-morpholin-4-ylmethyl-pyrimidin-5-yl)-naphthalen-1-yl]-urea;
1-(5-tert-Butyl-2-methylsulfanyl-phenyl)-3-[4-(6-morpholin-4-ylmethyl-pyridin-3-yl)-naphthalen-1-yl]-urea;
1-(5-tert-Butyl-2-methoxy-pyridin-3-yl)-3-[4-(6-morpholin-4-ylmethyl-pyrid in-3-yl)-naphthalen-1-yl]-urea;
2,2,2-Trifluoro-ethanesulfonic acid (5-tert-butyl-2-methoxy-3-{3-[4-(6-morpholin-4-ylmethyl-pyridin-3-yl)-naphthalen-1-yl]-ureido}-phenyl)-amide;
N-(5-{4-[3-(5-tert-Butyl-2-methyl-phenyl)-ureido]-naphthalen-1-yl}-pyrazin-2-yl)-methanesulfonamide;
1-(4-(6-{[Bis-(2-cyano-ethyl)-amino]-methyl}-pyridin-3-yl)-naphthalen-1-yl]-3-(5-tert-butyl-2-methoxy-phenyl)-urea;
1-(5-tert-Butyl-2-methoxy-phenyl)-3-{4-[6-(4-methyl-piperazin-1-ylmethyl)-pyridin-3-yl]-naphthalen-1-yl}-urea;
1-(5-tert-Butyl-2-methoxy-phenyl)-3-[4-(6-thiomorpholin-4-ylmethyl-pyridin-3-yl)-naphthalen-1-yl]-urea;
1-(5-tert-Butyl-2-methoxy-phenyl)-3-{4-[6-(2,6-dimethyl-piperidin-1-ylmethyl)-pyridin-3-yl]-naphthalen-1-yl}-urea;
1-(5-tert-Butyl-2-methoxy-phenyl)-3-{4-[6-(1-oxo-tetrahydro-thiopyran-4-ylamino)-pyridin-3-yl]-naphthalen-1-yl}-urea;
1-(5-tert-Butyl-2-methoxy-phenyl)-3-{4-[6-(tetrahydro-pyran-4-ylamino)-pyridin-3-yl]-naphthalen-1-yl}-urea;
1-(5-tert-Butyl-2-methoxy-phenyl)-3-[4-(6-{[(2-cyano-ethyl)-(tetrahydro-furan-2-ylmethyl)-amino]-methyl}-pyridin-3-yl)-naphthalen-1-yl]-urea;
1-(5-tert-Butyl-2-methoxy-phenyl)-3-{4-[6-(2-methoxymethyl-morpholin-4-ylmethyl)-pyridin-3-yl]-naphthalen-1-yl}-urea;
1-(5-tert-Butyl-2-methoxy-phenyl)-3-{4-[6-(2-methyl-3-oxo-piperazin-1-ylmethyl)-pyridin-3-yl]-naphthalen-1-yl}-urea;
1-(5-{4-[3-(5-tert-Butyl-2-methoxy-phenyl)-ureido]-naphthalen-1-yl}-pyridin-2-ylmethyl)-piperidine-3-carboxylic acid amide;
1-(5-tert-Butyl-2-methoxy-phenyl)-3-{4-[6-(1-oxo-1l4-thiomorpholin-4-ylmethyl)-pyridin-3-yl]-naphthalen-1-yl}-urea;
1-(3,3-Dimethyl-2-oxo-2,3-dihydro-1 H-indol-5-yl)-3-[4-(6-morpholin-4-ylmethyl-pyridin-3-yl)-naphthalen-1-yl]-urea;
1-(5-tert-Butyl-2-methoxy-phenyl)-3-{4-[6-(3-oxo-piperazin-1-ylmethyl)-pyridin-3-yl]-naphthalen-1-yl}-urea;
1-(5-tert-Butyl-2-methoxy-phenyl)-3-(4-{6-[(tetrahydro-furan-3-ylamino)-methyl]-pyridin-3-yl}-naphthalen-1-yl)-urea;
1-(5-tert-Butyl-2-methoxy-phenyl)-3-[4-(6-{[(2-cyano-ethyl)-pyridin-3-ylmethyl-amino]-methyl}-pyridin-3-yl)-naphthalen-1-yl]-urea;
1-(5-tert-Butyl-2-methoxy-phenyl)-3-{4-[6-(2-oxa-5-aza-bicyclo[2.2.1]hept-5-ylmethyl)-pyridin-3-yl]-naphthalen-1-yl}-urea;
1-(5-tert-Butyl-2-methoxy-phenyl)-3-{4-[6-(2,6-dimethyl-morpholin-4-ylmethyl)-pyridin-3-yl]-naphthalen-1-yl}-urea;
1-(5-tert-Butyl-2-methoxy-phenyl)-3-(4-{6-[4-(3-methoxy-phenyl)-piperazin-1-ylmethyl]-pyridin-3-yl}-naphthalen-1-yl)-urea;
1-(5-tert-Butyl-2-methoxy-phenyl)-3-{4-[6-(morpholine-4-carbonyl)-pyridin-3-yl]-naphthalen-1-yl}-urea;
1-(5-tert-Butyl-2-methoxy-phenyl)-3-[4-(5-morpholin-4-ylmethyl-pyrazin-2-yl)-naphthalen-1-yl]-urea;
1-(6-tert-Butyl-3-oxo-3,4-dihydro-2H-benzo[1,4]oxazin-8-yl)-3-[4-(6-morpholin-4-ylmethyl-pyridin-3-yl)-naphthalen-1-yl]-urea;
1-(3-Amino-5-tert-butyl-2-methoxy-phenyl)-3-[4-(6-morpholin-4-ylmethyl-pyridin-3-yl)-naphthalen-1-yl]-urea;
N-(5-{4-[3-(5-tert-Butyl-2-methoxy-phenyl)-ureido]-naphthalen-1-yl}-pyridin-2-yl)-acetamide;
N-(5-tert-Butyl-2-methoxy-3-{3-[4-(6-morpholin-4-ylmethyl-pyridin-3-yl)-naphthalen-1-yl]-ureido}-phenyl)-N-methyl-acetamide;
N-(5-tert-Butyl-2-methoxy-3-{3-[4-(6-morpholin-4-ylmethyl-pyridin-3-yl)-naphthalen-1-yl]-ureido}-phenyl)-2,2,2-trifluoro-acetamide;
1-(5-tert-Butyl-2-methoxy-phenyl)-3-{4-[6-(pyridin-3-yloxy)-pyridin-3-yl]-naphthalen-1-yl}-urea;
[4-(6-Morpholin-4-ylmethyl-pyridin-3-yl)-naphthalen-1-yl]-carbamic acid 3-tert-butylphenyl ester;
N-(5-tert-Butyl-2-methoxy-3-{3-[4-(6-morpholin-4-ylmethyl-pyridin-3-yl)-naphthalen-1-yl]-ureido}-phenyl)-methanesulfonamide and
and the pharmaceutically acceptable derivatives thereof.

Particularily preferred the invention relates to pharmaceutical compositions containing **A** and **B,** characterized in that the p38 kinase inhibitor **B** is selected from the following compounds:

### Example 1:

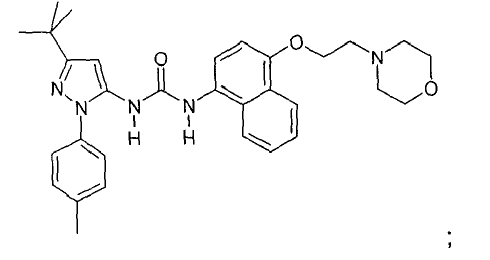

### Example 2:

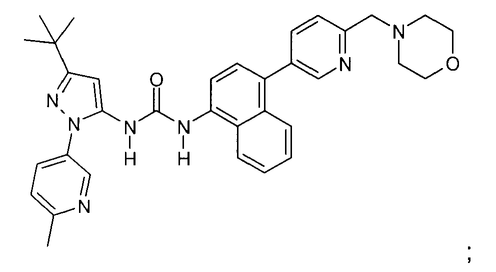

### Example 3:

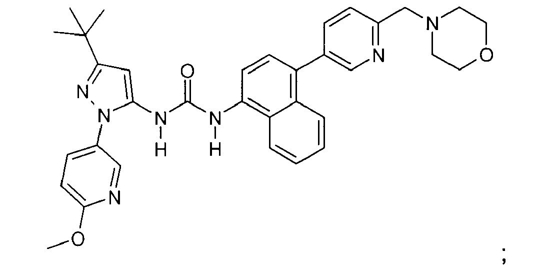

### Example 4:

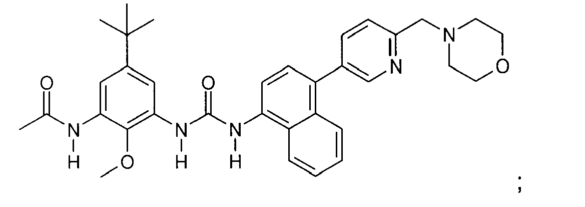

### Example 5:

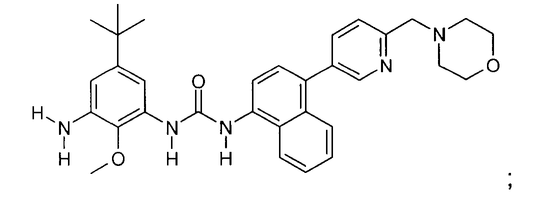

### Example 6:

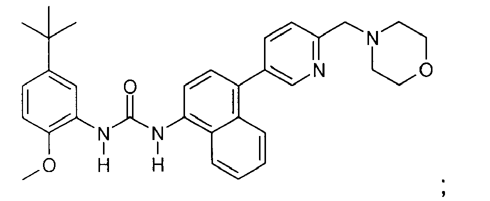

### Example 7:

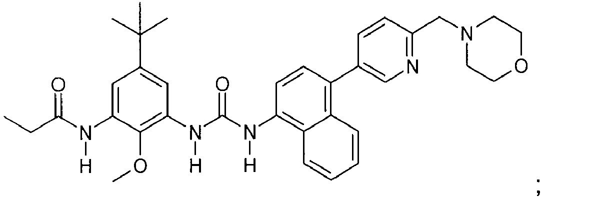

### Example 8:

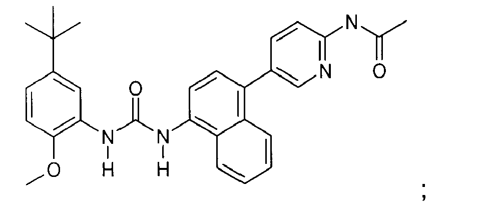

and the pharmaceutically acceptable derivatives thereof.

Any reference to the abovementioned p38 kinase inhibitors **B** within the scope of the present invention includes a reference to any pharmaceutically acceptable acid addition salts thereof which may exist. By the physiologically or pharmaceutically acceptable acid addition salts which may be formed from **B** are meant, according to the invention, pharmaceutically acceptable salts selected from among the salts of hydrochloric, hydrobromic, sulfuric, nitric, perchloric, fumaric, maleic, phosphoric, glycolic, lactic, salicylic, succinic, toluene-p-sulfuric, tartaric, acetic, citric, methanesulfonic, formic, benzoic, malonic, naphthalene-2-sulfuric and benzenesulfonic acids.

Any reference to the abovementioned p38 kinase inhibitors **B** within the scope of the present invention includes a reference to any alkali metal and alkaline earth metal salts thereof which may exist. If the compounds of formula **B** are present in the form of their basic salts, the sodium or potassium salts are particularly preferred.

The pharmaceutical combinations of **A** and **B** according to the invention are preferably administered by parenteral or oral route or by inhalation, the latter being particularly preferred. For oral or parenteral administration the pharmaceutical compositions according to the invention may be administered in the form of solutions and tablets. For inhalation, as preferred according to the invention, suitable inhalable powders may be used which are packed into suitable capsules (inhalettes) and administered using suitable powder inhalers. Alternatively, the drug may be inhaled by the application of suitable inhalation aerosols. These include inhalation aerosols which contain HFA134a, HFA227 or a mixture thereof as propellant gas. The drug may also be inhaled using suitable solutions of the pharmaceutical combination consisting of **A** and **B.**

Within the scope of the present invention references to the term physiologically acceptable salts are to be understood as references to the term pharmaceutically acceptable salts.

In one aspect, therefore, the invention relates to a pharmaceutical composition which contains a combination of **A** and **B.**

In another aspect the present invention relates to a pharmaceutical composition suitable for inhalation which contains one or more salts **A** and one or more compounds **B,** optionally in the form of their solvates or hydrates. The active substances may either be combined in a single preparation or contained in two separate formulations. Pharmaceutical compositions which contain the active substances **A** and **B** in a single preparation are preferred according to the invention. In another aspect the present invention relates to a pharmaceutical composition which contains, in addition to therapeutically effective quantities of **A** and **B,** a pharmaceutically acceptable carrier or excipient. In another aspect the present invention relates to a pharmaceutical composition which does not contain any pharmaceutically acceptable carrier or excipient in addition to therapeutically effective quantities of **A** and **B.**
The present invention also relates to the use of **A** and **B** for preparing a pharmaceutical composition containing therapeutically effective quantities of **A** and **B** for treating diseases of the upper or lower respiratory tract, particularly for treating asthma, chronic obstructive pulmonary diseases (COPD) and/or pulmonary hypertension, provided that treatment with p38 kinase inhibitors is not contraindicated from a therapeutic point of view, by simultaneous or successive administration. The present invention preferably relates to the abovementioned use of **A** and **B** for preparing a pharmaceutical composition containing therapeutically effective quantities of **A** and **B** for treating asthma and/or chronic obstructive pulmonary diseases (COPD), which may possibly be associated with pulmonary hypertension, provided that treatment with p38 kinase inhibitors is not contraindicated from a therapeutic point of view, by simultaneous or successive administration. Of equal importance is the abovementioned use of **A** and **B** for preparing a pharmaceutical composition containing therapeutically effective quantities of **A** and **B** for treating pulmonary hypertension.

The present invention further relates to the simultaneous or successive use of therapeutically effective doses of the combination of the above pharmaceutical compositions **A** and **B** for treating inflammatory or obstructive diseases of the respiratory tract, particularly asthma, chronic obstructive pulmonary diseases (COPD) and/or pulmonary hypertension, provided that treatment with p38 kinase inhibitors is not contraindicated from a therapeutic point of view, by simultaneous or successive administration. The present invention preferably relates to the abovementioned use of therapeutically effective doses of the combination of the abovementioned pharmaceutical compositions **A** and **B** for treating asthma and/or chronic obstructive pulmonary diseases (COPD), which may possibly be associated with pulmonary hypertension, provided that treatment with p38 kinase inhibitors is not contraindicated from a therapeutic point of view, by simultaneous or successive administration. Of equal importance is the abovementioned use of therapeutically effective doses of the combination of the abovementioned pharmaceutical compositions **A** and **B** for treating pulmonary hypertension.

In the active substance combinations of **A** and **B** according to the invention, ingredients **A** and **B** may be present in the form of their enantiomers, mixtures of enantiomers or in the form of racemates.
The proportions in which the two active substances **A** and **B** may be used in the active substance combinations according to the invention are variable. Active substances **A** and **B** may possibly be present in the form of their solvates or hydrates. Depending on the choice of the compounds **A** and **B,** the weight ratios which may be used within the scope of the present invention vary on the basis of the different molecular weights of the various compounds and their different potencies. As a rule, the pharmaceutical combinations according to the invention may contain compounds **A** and **B** in ratios by weight ranging from 1:300 to 20:1, preferably from 1:200 to 10:1. In the particularly preferred pharmaceutical combinations which contain compound **A** and a compound selected from the compounds of formula **1**, **2**, **3a, 3b, 3c, 3d, 4, 5, 5a, 6,** or **7** as p38 kinase inhibitor **B,** the weight ratios of **A** to **B** are most preferably in a range in which A' and B are present in proportions of 1:100 to 5:1, more preferably from 1:80 to 1:1.

The pharmaceutical compositions according to the invention containing the combinations of **A** and **B** are normally administered so that **A** and **B** are present together in doses of about 100 to 10000 µg, preferably 1000 to 9000 µg, more preferably 1500 to 8000µg, better still from about 2000 to about 7000 µg, more preferably 2500 to 6000µg per single dose. For example about 3000 to about 5500 µg of the combination of **A** and **B** according to the invention may be administered once or twice daily to the patient in need thereof.

For example, combinations of **A** and **B** according to the invention contain a quantity of **A'** and p38 kinase inhibitor **B** such that the total dosage per single dose is about
2500µg, 2550µg, 2600µg, 2650µg, 2700µg, 2750µg, 2800µg, 2850µg, 2900µg, 2950µg,3000µg,3050µg,3100µg,3150µg,3200µg,3250µg,3300µg,3350µg, 3400µg, 3450µg, 3500µg, 3550µg, 3600µg, 3650µg, 3700µg, 3750µg, 3800µg, 3850µg,3900µg,3950µg,4000µg,4050µg,4100µg,4150µg,4200µg,4250µg, 4300µg, 4350µg, 4400µg, 4450µg, 4500µg, 4550µg, 4600µg, 4650µg, 4700µg, 4750µg,4800µg,4850µg,4900µg,4950µg,5000µg,5050µg,5100µg,5150µg, 5200µg, 5250µg, 5300µg, 5350µg, 5400µg, 5450µg, 5500µg, 5550µg, 5600µg, 5650µg, 5700µg, 5750µg, 5800µg, 5850µg, 5900µg, 5950µg, 6000µg, 6050µg, 6100µg,6150µg,6200µg,6250µg,6300µg,6350µg,6400µg,6450µg,6500µg, 6550µg, 6600µg, 6650µg, 6700µg, 6750µg, 6800µg, 6850µg, 6900µg, 6950µg, 7000µg, 7050µg, 7100µg, 7150µg, 7200µg, 7250µg, 7300µg, 7350µg, 7400µg, 7450µg, 7500µg or the like. The proposed dosages per single dose suggested above are not to be regarded as being restricted to the numerical values actually stated, but are intended only as examples of dosages. Of course, dosages which fluctuate around the above values in a range of about +/- 25µg are also covered by the values given above by way of example. In these dosage ranges the active substances **A'** and **B** may be present in the weight ratios specified above.

For example, without restricting the scope of the invention thereto, the combinations of **A** and **B** according to the invention may contain a quantity of **A'** and p38 kinase inhibitor **B** such that, in each individual dose,
16,5µg of **A'** and 2500µg of **B,** 16,5µg of **A'** and 3000µg of **B,** 16,5µg of **A'** and 3500µg of **B,** 16,5µg of **A'** and 4000µg of **B,** 16,5µg of **A'** and 4500µg of **B*,*** 16,5µg of **A'** and 5000µg of **B,** 16,5µg of **A'** and 5500µg of **B,** 16,5µg of **A'** and 6000µg of **B,** 16,5µg of **A'** and 6500µg of **B,** 16,5µg of **A'** and 7000µg of **B,** 33,1 µg of **A'** and 2500µg of **B,** 33,1 µg of **A'** and 3000µg of **B,** 33,1 µg of **A'** and 3500µg of **B,** 33,1 µg of **A'** and 4000µg of **B,** 33,1 µg of **A'** and 4500µg of **B,** 33,1 µg of **A'** and 5000µg of **B,** 33,1 µg of **A'** and 5500µg of **B,** 33,1 µg of **A'** and 6000µg of **B,** 33,1 µg of **A'** and 6500µg of **B,** 33,1 µg of **A'** and 7000µg of **B,** 49,5µg of **A'** and 2500µg of **B,** 49,5µg of **A'** and 3000µg of **B,** 49,5µg of **A'** and 3500µg of **B,** 49,5µg of **A'** and 4000µg of **B,** 49,5µg of **A'** and 4500µg of **B,** 49,5µg of **A'** and 5000µg of **B,** 49,5µg of **A'** and 5500µg of **B,** 49,5µg of **A**' and 6000µg of **B**, 49,5µg of **A'** and 6500µg of **B**, 49,5µg of **A'** and 7000µg of **B**, 82,6µg of **A**' and 2500µg of **B**, 82,6µg of **A**' and 3000µg of **B**, 82,6µg of **A'** and 3500µg of **B,** 82,6µg of **A'** and 4000µg of **B,** 82,6µg of **A'** and 4500µg of **B,** 82,6µg of **A'** and 5000µg of **B,** 82,6µg of **A'** and 5500µg of **B,** 82,6µg of **A'** and 6000µg of **B,** 82,6µg of **A'** and 6500µg of **B,** 82,6µg of **A'** and 7000µg of **B,** 165,1µg of **A'** and 2500µg of **B,** 165,1µg of **A'** and 3000µg of **B,** 165,1µg of **A'** and 3500µg of **B,** 165,1µg of **A'** and 4000µg of **B,** 165,1µg of **A'** and 4500µg of **B,** 165,1µg of **A'** and 5000µg of **B,** 165,1 µg of **A'** and 5500µg of **B,** 165,1 µg of **A'** and 6000µg of **B,** 165,1µg of **A'** and 6500µg of **B,** 165,1µg of **A'** and 7000µg of **B**, 206,4µg of **A'** and 2500µg of **B,** 206,4µg of **A'** and 3000µg of **B,** 206,4µg of **A'** and 3500µg of **B,** 206,4µg of **A'** and 4000µg of **B,** 206,4µg of **A'** and 4500µg of **B,** 206,4µg of **A'** and 5000µg of **B,** 206,4µg of **A'** and 5500µg of **B** oder 206,4µg of **A**' and 6000µg of **B,** 206,4µg of **A'** and 6500µg of **B,** 206,4µg of **1'** and 7000µg of **B ,** 412,8µg of **A'** and 2500µg of **B,** 412,8µg of **A'** and 3000µg of **B,** 412,8µg of **A'** and 3500µg of **B,** 412,8µg of **A'** and 4000µg of **B,** 412,8µg of **A'** and 4500µg of **B,** 412,8µg of **A'** and 5000µg of **B,** 412,8µg of **A'** and 5500µg of **B** oder 412,8µg of **A'** and 6000µg of **B,** 412,8µg of **A'** and 6500µg of **B,** 412,8µg of **1'** and 7000µg of **B** are administered.

If the active substance combination in which **A** denotes the bromide is used as the preferred combination of **A** and **B** according to the invention, the quantities of active substance **A'** and **B** administered per single dose mentioned by way of example correspond to the following quantities of **A** and **B** administered per single dose: 20µg of **A** and 2500µg of **B,** 20µg of **A** and 3000µg of **B,** 20µg of **A** and 3500µg of **B,** 20µg of **A** and 4000µg of **B,** 20µg of **A** and 4500µg of **B,** 20µg of **A** and 5000µg of **B,** 20µg of **A** and 5500µg of **B,** 20µg of **A** and 6000µg of **B,** 20µg of **A** and 6500µg of **B,** 20µg of **A** and 7000µg of **B,** 40µg of **A** and 2500µg of **B,** 40µg of **A** and 3000µg of **B,** 40µg of **A** and 3500µg of **B,** 40µg of **A** and 4000µg of **B,** 40µg of **A** and 4500µg of **B,** 40µg of **A** and 5000µg of **B,** 40µg of **A** and 5500µg of **B,** 40µg of **A** and 6000µg of **B,** 40µg of **A** and 6500µg of **B,** 40µg of **A** and 7000µg of **B,** 60µg of **A** and 2500µg of **B,** 60µg of **A** and 3000µg of **B,** 60µg of **A** and 3500µg of **B,** 60µg of **A** and 4000µg of **B,** 60µg of **A** and 4500µg of **B,** 60µg of **A** and 5000µg of **B,** 60µg of **A** and 5500µg of **B,** 60µg of **A** and 6000µg of **B,** 60µg of **A** and 6500µg of **B,** 60µg of **A** and 7000µg of **B,** 100µg of **A** and 2500µg of **B,** 100µg of **A** and 3000µg of **B,** 100µg of **A** and 3500µg of **B,** 100µg of **A** and 4000µg of **B,** 100µg of **A** and 4500µg of **B,** 100µg of **A** and 5000µg of **B,** 100µg of **A** and 5500µg of **B,** 100µg of **A** and 6000µg of **B,** 100µg of **A** and 6500µg of **B,** 100µg of **A** and 7000µg of **B,** 200µg of **A** and 2500µg of **B,** 200µg of **A** and 3000µg of **B,** 200µg of **A** and 3500µg of **B,** 200µg of **A** and 4000µg of **B,** 200µg of **A** and 4500µg of **B,** 200µg of **A** and 5000µg of **B,** 200µg of **A** and 5500µg of **B,** 200µg of **A** and 6000µg of **B,** 200µg of **A** and 6500µg of **B,** 200µg of **A** and 7000µg of **B,** 250µg of **A** and 2500µg of **B,** 250µg of **A** and 3000µg of **B,** 250µg of **A** and 3500µg of **B,** 250µg of **A** and 4000µg of **B,** 250µg of **A** and 4500µg of **B,** 250µg of **A** and 5000µg of **B,** 250µg of **A** and 5500µg of **B,** 250µg of **A** and 6000ug of **B,** 250µg of **A** and 6500µg of **B** oder 250µg of **A** and 7000µg of **B,** 500µg of **A** and 2500µg of **B,** 500µg of **A** and 3000µg of **B,** 500µg of **A** and 3500µg of **B,** 500µg of **A** and 4000µg of **B,** 500µg of **A** and 4500µg of **B,** 500µg of **A** and 5000µg of **B,** 500µg of **A** and 5500µg of **B,** 500µg of **A** and 6000µg of **B,** 500µg of **A** and 6500µg of **B** oder 500µg of **A** and 7000µg of **B**

The active substance combinations of **A** and **B** according to the invention are preferably administered by inhalation or by nasal application. For this purpose, ingredients **A** and **B** have to be made available in inhalable forms. Inhalable preparations include inhalable powders, propellant-containing metering aerosols or propellant-free inhalable solutions. Inhalable powders according to the invention containing the combination of active substances **A** and **B** may consist of the active substances on their own or of a mixture of the active substances with physiologically acceptable excipients. Within the scope of the present invention, the term propellant-free inhalable solutions also includes concentrates or sterile inhalable solutions ready for use. The preparations according to the invention may contain the combination of active substances **A** and **B** either together in one formulation or in two separate formulations. These formulations which may be used within the scope of the present invention are described in more detail in the next part of the specification.

### A) Inhalable powder containing the combinations of active substances A and B according to the invention:

The inhalable powders according to the invention may contain **A** and **B** either on their own or in admixture with suitable physiologically acceptable excipients.

If the active substances **A** and **B** are present in admixture with physiologically acceptable excipients, the following physiologically acceptable excipients may be used to prepare these inhalable powders according to the invention:
monosaccharides (e.g. glucose or arabinose), disaccharides (e.g. lactose, saccharose, maltose), oligo- and polysaccharides (e.g. dextrane), polyalcohols (e.g. sorbitol, mannitol, xylitol), salts (e.g. sodium chloride, calcium carbonate) or mixtures of these excipients with one another. Preferably, mono- or disaccharides are used, while the use of lactose or glucose is preferred, particularly, but not exclusively, in the form of their hydrates. For the purposes of the invention, lactose is the particularly preferred excipient, while lactose monohydrate is most particularly preferred.

Within the scope of the inhalable powders according to the invention the excipients have a maximum average particle size of up to 250µm, preferably between 10 and 150µm, most preferably between 15 and 80µm. It may sometimes seem appropriate to add finer excipient fractions with an average particle size of 1 to 9µm to the excipients mentioned above. These finer excipients are also selected from the group of possible excipients listed hereinbefore. Finally, in order to prepare the inhalable powders according to the invention, micronised active substance **A** and **B,** preferably with an average particle size of 0.5 to 10µm, more preferably from 1 to 5µm, is added to the excipient mixture. Processes for producing the inhalable powders according to the invention by grinding and micronising and by finally mixing the ingredients together are known from the prior art. The inhalable powders according to the invention may be prepared and administered either in the form of a single powder mixture which contains both **A** and **B** or in the form of separate inhalable powders which contain only **A** or **B.**

The inhalable powders according to the invention may be administered using inhalers known from the prior art. Inhalable powders according to the invention which contain a physiologically acceptable excipient in addition to **A** and **B** may be administered, for example, by means of inhalers which deliver a single dose from a supply using a measuring chamber as described in US 4570630A, or by other means as described in DE 36 25 685 A. The inhalable powders according to the invention which contain **A** and **B** optionally combined with a physiologically acceptable excipient may be administered for example with an inhaler known by the name Turbuhaler® , for example with inhalers as disclosed in EP 237507 A, for example. Preferably, the inhalable powders according to the invention which contain physiologically acceptable excipient in addition to **A** and **B** are packed into capsules (to produce so-called inhalettes) which are used in inhalers as described, for example, in WO 94/28958.

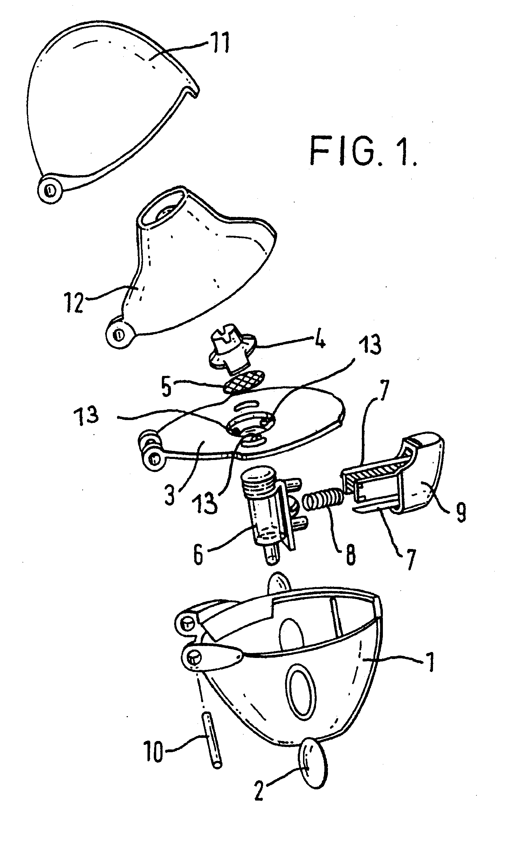

A particularly preferred inhaler for administering the pharmaceutical combination according to the invention in inhalettes is shown in Figure 1.

The inhaler according to figure 1 is characterised by a housing 1 containing two windows 2, a deck 3 in which there are air inlet ports and which is provided with a screen 5 secured via a screen housing 4, an inhalation chamber 6 connected to the deck 3 on which there is a push button 9 provided with two sharpened pins 7 and movable counter to a spring 8, a mouthpiece 12 which is connected to the housing 1, the deck 3 and a cover 11 via a spindle 10 to enable it to be flipped open or shut and three holes 13 with diameters below 1 mm in the central region around the capsule chamber 6 and underneath the screen housing 4 and screen 5.

The main air flow enters the inhaler between deck 3 and base 1 near to the hinge. The deck has in this range a reduced width, which forms the entrance slit for the air. Then the flow reverses and enters the capsule chamber 6 through the inlet tube. The flow is then further conducted through the filter and filter holder to the mouthpiece. A small portion of the flow enters the device between mouthpiece and deck and flows then between filterholder and deck into the main stream. Due to production tolerances there is some uncertainty in this flow because of the actual width of the slit between filterholder and deck. In case of new or reworked tools the flow resistance of the inhaler may therefore be a little off the target value. To correct this deviation the deck has in the central region around the capsule chamber 6 and underneath the screen housing 4 and screen 5 three holes 13 with diameters below 1 mm. Through these holes 13 flows air from the base into the main air stream and reduces such slightly the flow resistance of the inhaler. The actual diameter of these holes 13 can be chosen by proper inserts in the tools so that the mean flow resistance can be made equal to the target value.

If the inhalable powders according to the invention are packed into capsules (inhalers) for the preferred use described above, the quantities packed into each capsule should be 1 to 30mg, preferably 3 to 20mg, more particularly 5 to 10mg of inhalable powder per capsule. These capsules contain, according to the invention, either together or separately, the doses of **A'** and **B** mentioned hereinbefore for each single dose.

### B) Propellant gas-driven inhalation aerosols containing the combinations of active substances A and B according to the invention:

Inhalation aerosols containing propellant gas according to the invention may contain substances **A** and **B** dissolved in the propellant gas or in dispersed form. **A** and **B** may be present in separate formulations or in a single preparation, in which **A** and **B** are either both dissolved, both dispersed or only one component is dissolved and the other is dispersed. The propellant gases which may be used to prepare the inhalation aerosols according to the invention are known from the prior art. Suitable propellant gases are selected from among hydrocarbons such as n-propane, n-butane or isobutane and halohydrocarbons such as fluorinated derivatives of methane, ethane, propane, butane, cyclopropane or cyclobutane. The propellant gases mentioned above may be used on their own or in mixtures thereof.
Particularly preferred propellant gases are halogenated alkane derivatives selected from TG134a (1,1,1,2-tetrafluoroethane) and TG227(1,1,1,2,3,3,3-heptafluoropropane) and mixtures thereof.

The propellant-driven inhalation aerosols according to the invention may also contain other ingredients such as co-solvents, stabilisers, surfactants, antioxidants, lubricants and pH adjusters. All these ingredients are known in the art.

The inhalation aerosols containing propellant gas according to the invention may contain up to 5 wt.-% of active substance **A** and/or **B**. Aerosols according to the invention contain, for example, 0.002 to 5 wt.-%, 0.01 to 3 wt.-%, 0.015 to 2 wt.-%, 0.1 to 2 wt.-%, 0.5 to 2 wt.-% or 0.5 to 1.5 wt.-% of active substance **A** and/or **B.** If the active substances **A** and/or **B** are present in dispersed form, the particles of active substance preferably have an average particle size of up to 10µm, preferably from 0.1 to 5µm, more preferably from 1 to 5µm.

The propellant-driven inhalation aerosols according to the invention mentioned above may be administered using inhalers known in the art (MDIs = metered dose inhalers). Accordingly, in another aspect, the present invention relates to pharmaceutical compositions in the form of propellant-driven aerosols as hereinbefore described combined with one or more inhalers suitable for administering these aerosols. In addition, the present invention relates to inhalers which are characterised in that they contain the propellant gas-containing aerosols described above according to the invention. The present invention also relates to cartridges which when fitted with a suitable valve can be used in a suitable inhaler and which contain one of the above-mentioned propellant gas-containing inhalation aerosols according to the invention. Suitable cartridges and methods of filling these cartridges with the inhalable aerosols containing propellant gas according to the invention are known from the prior art.

### C) Propellant-free inhalable solutions or suspensions containing the combinations of active substances A and B according to the invention:

It is particularly preferred to use the active substance combination according to the invention in the form of propellant-free inhalable solutions and suspensions. The solvent used may be an aqueous or alcoholic, preferably an ethanolic solution. The solvent may be water on its own or a mixture of water and ethanol. The relative proportion of ethanol compared with water is not limited but the maximum is up to 70 percent by volume, more particularly up to 60 percent by volume and most preferably up to 30 percent by volume. The remainder of the volume is made up of water. The solutions or suspensions containing **A** and **B**, separately or together, are adjusted to a pH of 2 to 7, preferably 2 to 5, using suitable acids. The pH may be adjusted using acids selected from inorganic or organic acids. Examples of suitable inorganic acids include hydrochloric acid, hydrobromic acid, nitric acid, sulphuric acid and/or phosphoric acid. Examples of particularly suitable organic acids include ascorbic acid, citric acid, malic acid, tartaric acid, maleic acid, succinic acid, fumaric acid, acetic acid, formic acid and/or propionic acid etc. Preferred inorganic acids are hydrochloric and sulphuric acids. It is also possible to use the acids which have already formed an acid addition salt with one of the active substances. Of the organic acids, ascorbic acid, fumaric acid and citric acid are preferred. If desired, mixtures of the above acids may be used, particularly in the case of acids which have other properties in addition to their acidifying qualities, e.g. as flavourings, antioxidants or complexing agents, such as citric acid or ascorbic acid, for example. According to the invention, it is particularly preferred to use hydrochloric acid to adjust the pH.

According to the invention, the addition of editic acid (EDTA) or one of the known salts thereof, sodium edetate, as stabiliser or complexing agent is unnecessary in the present formulation. Other embodiments may contain this compound or these compounds. In a preferred embodiment the content based on sodium edetate is less than 100mg/100ml, preferably less than 50mg/100 ml, more preferably less than 20mg/100 ml. Generally, inhalable solutions in which the content of sodium edetate is from 0 to 10mg/100ml are preferred.

Co-solvents and/or other excipients may be added to the propellant-free inhalable solutions according to the invention. Preferred co-solvents are those which contain hydroxyl groups or other polar groups, e.g. alcohols ― particularly isopropyl alcohol, glycols ― particularly propyleneglycol, polyethyleneglycol, polypropyleneglycol, glycolether, glycerol, polyoxyethylene alcohols and polyoxyethylene fatty acid esters. The terms excipients and additives in this context denote any pharmacologically acceptable substance which is not an active substance but which can be formulated with the active substance or substances in the pharmacologically suitable solvent in order to improve the qualitative properties of the active substance formulation. Preferably, these substances have no pharmacological effect or, in connection with the desired therapy, no appreciable or at least no undesirable pharmacological effect. The excipients and additives include, for example, surfactants such as soya lecithin, oleic acid, sorbitan esters, such as polysorbates, polyvinylpyrrolidone, other stabilisers, complexing agents, antioxidants and/or preservatives which guarantee or prolong the shelf life of the finished pharmaceutical formulation, flavourings, vitamins and/or other additives known in the art. The additives also include pharmacologically acceptable salts such as sodium chloride as isotonic agents.

The preferred excipients include antioxidants such as ascorbic acid, for example, provided that it has not already been used to adjust the pH, vitamin A, vitamin E, tocopherols and similar vitamins and provitamins occurring in the human body. Preservatives may be used to protect the formulation from contamination with pathogens. Suitable preservatives are those which are known in the art, particularly cetyl pyridinium chloride, benzalkonium chloride or benzoic acid or benzoates such as sodium benzoate in the concentration known from the prior art. The preservatives mentioned above are preferably present in concentrations of up to 50mg/100ml, more preferably between 5 and 20mg/100ml.

Preferred formulations contain, in addition to the solvent water and the combination of active substances **A** and **B,** only benzalkonium chloride and sodium edetate. In another preferred embodiment, no sodium edetate is present.

The propellant-free inhalable solutions according to the invention are administered in particular using inhalers of the kind which are capable of nebulising a small amount of a liquid formulation in the therapeutic dose within a few seconds to produce an aerosol suitable for therapeutic inhalation. Within the scope of the present invention, preferred inhalers are those in which a quantity of less than 100µL, preferably less than 50µL, more preferably between 10 and 30µL of active substance solution can be nebulised in preferably one spray action to form an aerosol with an average particle size of less than 20µm, preferably less than 10µm, in such a way that the inhalable part of the aerosol corresponds to the therapeutically effective quantity.

An apparatus of this kind for propellant-free delivery of a metered quantity of a liquid pharmaceutical composition for inhalation is described for example in International Patent Application WO 91/14468 and also in WO 97/12687 (cf. in particular Figures 6a and 6b). The nebulisers (devices) described therein are known by the name Respimat®.

This nebuliser (Respimat®) can advantageously be used to produce the inhalable aerosols according to the invention containing the combination of active substances **A** and **B**. Because of its cylindrical shape and handy size of less than 9 to 15 cm long and 2 to 4 cm wide, this device can be carried at all times by the patient. The nebuliser sprays a defined volume of pharmaceutical formulation using high pressures through small nozzles so as to produce inhalable aerosols.

The preferred atomiser essentially consists of an upper housing part, a pump housing, a nozzle, a locking mechanism, a spring housing, a spring and a storage container, characterised by
- a pump housing which is secured in the upper housing part and which comprises at one end a nozzle body with the nozzle or nozzle arrangement,
- a hollow plunger with valve body,
- a power takeoff flange in which the hollow plunger is secured and which is located in the upper housing part,
- a locking mechanism situated in the upper housing part,
- a spring housing with the spring contained therein, which is rotatably mounted on the upper housing part by means of a rotary bearing,
- a lower housing part which is fitted onto the spring housing in the axial direction.

The hollow plunger with valve body corresponds to a device disclosed in WO 97/12687. It projects partially into the cylinder of the pump housing and is axially movable within the cylinder. Reference is made in particular to Figures 1 to 4, especially Figure 3, and the relevant parts of the description. The hollow plunger with valve body exerts a pressure of 5 to 60 Mpa (about 50 to 600 bar), preferably 10 to 60 Mpa (about 100 to 600 bar) on the fluid, the measured amount of active substance solution, at its high pressure end at the moment when the spring is actuated. Volumes of 10 to 50 microlitres are preferred, while volumes of 10 to 20 microlitres are particularly preferred and a volume of 15 microlitres per spray is most particularly preferred.

The valve body is preferably mounted at the end of the hollow plunger facing the valve body.

The nozzle in the nozzle body is preferably microstructured, i.e. produced by microtechnology. Microstructured valve bodies are disclosed for example in WO-94/07607; reference is hereby made to the contents of this specification, particularly Figure 1 therein and the associated description.

The valve body consists for example of two sheets of glass and/or silicon firmly joined together, at least one of which has one or more microstructured channels which connect the nozzle inlet end to the nozzle outlet end. At the nozzle outlet end there is at least one round or non-round opening 2 to 10 microns deep and 5 to 15 microns wide, the depth preferably being 4.5 to 6.5 microns while the length is preferably 7 to 9 microns.

In the case of a plurality of nozzle openings, preferably two, the directions of spraying of the nozzles in the nozzle body may extend parallel to one another or may be inclined relative to one another in the direction of the nozzle opening. In a nozzle body with at least two nozzle openings at the outlet end the directions of spraying may be at an angle of 20 to 160° to one another, preferably 60 to 150°, most preferably 80 to 100°. The nozzle openings are preferably arranged at a spacing of 10 to 200 microns, more preferably at a spacing of 10 to 100 microns, most preferably 30 to 70 microns. Spacings of 50 microns are most preferred. The directions of spraying will therefore meet in the vicinity of the nozzle openings.

The liquid pharmaceutical preparation strikes the nozzle body with an entry pressure of up to 600 bar, preferably 200 to 300 bar, and is atomised into an inhalable aerosol through the nozzle openings. The preferred particle or droplet sizes of the aerosol are up to 20 microns, preferably 3 to 10 microns.

The locking mechanism contains a spring, preferably a cylindrical helical compression spring, as a store for the mechanical energy. The spring acts on the power takeoff flange as an actuating member the movement of which is determined by the position of a locking member. The travel of the power takeoff flange is precisely limited by an upper and lower stop. The spring is preferably biased, via a power step-up gear, e.g. a helical thrust gear, by an external torque which is produced when the upper housing part is rotated counter to the spring housing in the lower housing part. In this case, the upper housing part and the power takeoff flange have a single or multiple V-shaped gear.

The locking member with engaging locking surfaces is arranged in a ring around the power takeoff flange. It consists, for example, of a ring of plastic or metal which is inherently radially elastically deformable. The ring is arranged in a plane at right angles to the atomiser axis. After the biasing of the spring, the locking surfaces of the locking member move into the path of the power takeoff flange and prevent the spring from relaxing. The locking member is actuated by means of a button. The actuating button is connected or coupled to the locking member. In order to actuate the locking mechanism, the actuating button is moved parallel to the annular plane, preferably into the atomiser; this causes the deformable ring to deform in the annual plane. Details of the construction of the locking mechanism are given in WO 97/20590.

The lower housing part is pushed axially over the spring housing and covers the mounting, the drive of the spindle and the storage container for the fluid.

When the atomiser is actuated the upper housing part is rotated relative to the lower housing part, the lower housing part taking the spring housing with it. The spring is thereby compressed and biased by means of the helical thrust gear and the locking mechanism engages automatically. The angle of rotation is preferably a whole-number fraction of 360 degrees, e.g. 180 degrees. At the same time as the spring is biased, the power takeoff part in the upper housing part is moved along by a given distance, the hollow plunger is withdrawn inside the cylinder in the pump housing, as a result of which some of the fluid is sucked out of the storage container and into the high pressure chamber in front of the nozzle.

If desired, a number of exchangeable storage containers which contain the fluid to be atomised may be pushed into the atomiser one after another and used in succession. The storage container contains the aqueous aerosol preparation according to the invention.

The atomising process is initiated by pressing gently on the actuating button. As a result, the locking mechanism opens up the path for the power takeoff member. The biased spring pushes the plunger into the cylinder of the pump housing. The fluid leaves the nozzle of the atomiser in atomised form.

Further details of construction are disclosed in PCT Applications WO 97/12683 and WO 97/20590, to which reference is hereby made.

The components of the atomiser (nebuliser) are made of a material which is suitable for its purpose. The housing of the atomiser and, if its operation permits, other parts as well are preferably made of plastics, e.g. by injection moulding. For medicinal purposes, physiologically safe materials are used.

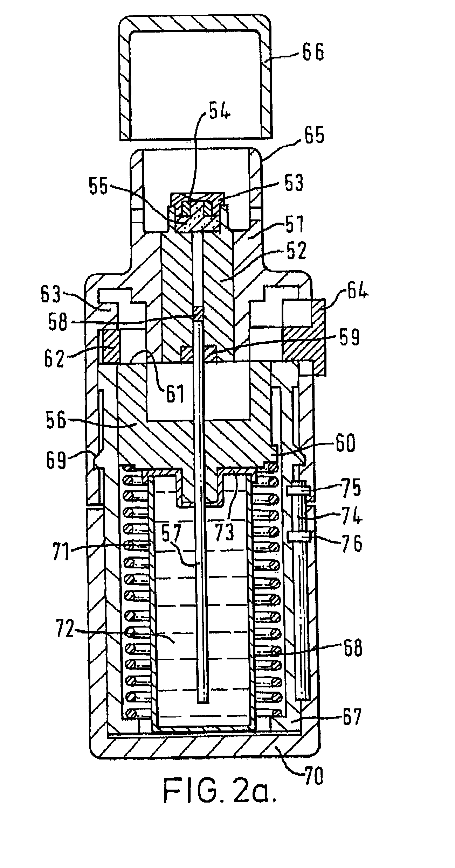

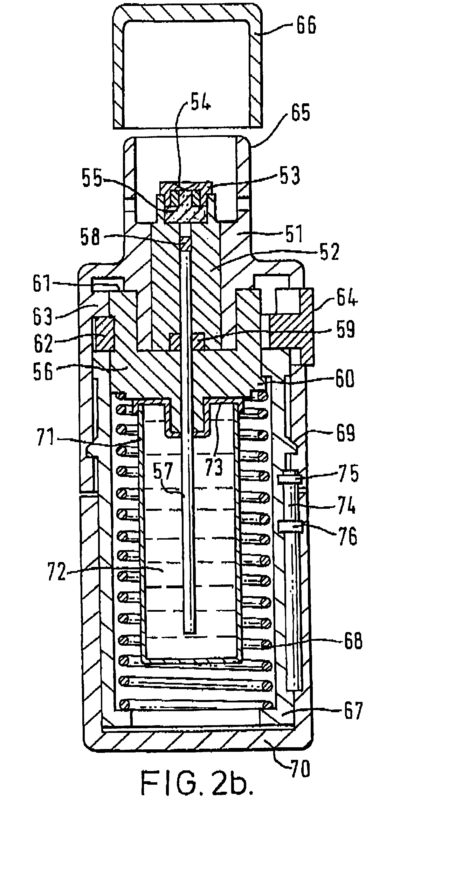

Figures 2a/b attached to this patent application, which are identical to Figures 6a/b of WO 97/12687, show the nebuliser (Respimat®) which can advantageously be used for inhaling the aqueous aerosol preparations according to the invention.

Figure 2a shows a longitudinal section through the atomiser with the spring biased while Figure 2b shows a longitudinal section through the atomiser with the spring relaxed.

The upper housing part (51) contains the pump housing (52) on the end of which is mounted the holder (53) for the atomiser nozzle. In the holder is the nozzle body (54) and a filter (55). The hollow plunger (57) fixed in the power takeoff flange (56) of the locking mechanism projects partially into the cylinder of the pump housing. At its end the hollow plunger carries the valve body (58). The hollow plunger is sealed off by means of the seal (59). Inside the upper housing part is the stop (60) on which the power takeoff flange abuts when the spring is relaxed. On the power takeoff flange is the stop (61) on which the power takeoff flange abuts when the spring is biased. After the biasing of the spring the locking member (62) moves between the stop (61) and a support (63) in the upper housing part. The actuating button (64) is connected to the locking member. The upper housing part ends in the mouthpiece (65) and is sealed off by means of the protective cover (66) which can be placed thereon.

The spring housing (67) with compression spring (68) is rotatably mounted on the upper housing part by means of the snap-in lugs (69) and rotary bearing. The lower housing part (70) is pushed over the spring housing. Inside the spring housing is the exchangeable storage container (71) for the fluid (72) which is to be atomised. The storage container is sealed off by the stopper (73) through which the hollow plunger projects into the storage container and is immersed at its end in the fluid (supply of active substance solution).

The spindle (74) for the mechanical counter is mounted in the covering of the spring housing. At the end of the spindle facing the upper housing part is the drive pinion (75). The slider (76) sits on the spindle.

The nebuliser described above is suitable for nebulising the aerosol preparations according to the invention to produce an aerosol suitable for inhalation.

If the formulation according to the invention is nebulised using the method described above (Respimat®) the quantity delivered should correspond to a defined quantity with a tolerance of not more than 25%, preferably 20% of this amount in at least 97%, preferably at least 98% of all operations of the inhaler (spray actuations). Preferably, between 5 and 30 mg of formulation, most preferably between 5 and 20 mg of formulation are delivered as a defined mass on each actuation.

However, the formulation according to the invention may also be nebulised by means of inhalers other than those described above, e.g. jet stream inhalers or other stationary nebulisers.

Accordingly, in a further aspect, the invention relates to pharmaceutical formulations in the form of propellant-free inhalable solutions or suspensions as described above combined with a device suitable for administering these formulations, preferably in conjunction with the Respimat®. Preferably, the invention relates to propellant-free inhalable solutions or suspensions characterised by the combination of active substances A and B according to the invention in conjunction with the device known by the name Respimat®. In addition, the present invention relates to the above-mentioned devices for inhalation, preferably the Respimat®, characterised in that they contain the propellant-free inhalable solutions or suspensions according to the invention as described hereinbefore.

Inhalable solutions which contain the active substances **A** and **B** in a single preparation are preferred according to the invention. The term preparation also includes those which contain both ingredients A and B in two-chamber cartridges as disclosed for example in WO 00/23037. Reference is hereby made to this publication in its entirety.

The propellant-free inhalable solutions or suspensions according to the invention may take the form of concentrates or sterile inhalable solutions or suspensions ready for use, as well as the above-mentioned solutions and suspensions designed for use in a Respimat®. Formulations ready for use may be produced from the concentrates, for example, by the addition of isotonic saline solutions. Sterile formulations ready for use may be administered using energy-operated fixed or portable nebulisers which produce inhalable aerosols by means of ultrasound or compressed air by the Venturi principle or other principles.
Accordingly, in another aspect, the present invention relates to pharmaceutical compositions in the form of propellant-free inhalable solutions or suspensions as described hereinbefore which take the form of concentrates or sterile formulations ready for use, combined with a device suitable for administering these solutions, characterised in that the device is an energy-operated free-standing or portable nebuliser which produces inhalable aerosols by means of ultrasound or compressed air by the Venturi principle or other methods.

The Examples which follow serve to illustrate the present invention in more detail without restricting the scope of the invention to the following embodiments by way of example.

### Examples of Formulations

### Inhalable powders:

1)

| **Ingredients** | **µg per capsule** |
|---|---|
| **A'**-bromide | 20 |
| component **B** (example 1) | 3500 |
| Lactose | 3480 |
| **Total** | 7000 |

2)

| **Ingredients** | **µg per capsule** |
|---|---|
| **A'**-bromide | 60 |
| component **B** (example 1) | 3000 |
| Lactose | 3940 |
| **Total** | 7000 |

3)

| **Ingredients** | **µg per capsule** |
|---|---|
| **A'**-bromide | 100 |
| component **B** (example 1) | 5000 |
| Lactose | 4900 |
| **Total** | 10000 |

4)

| **Ingredients** | **µg per capsule** |
|---|---|
| **A'**-bromide | 125 |
| component **B** (example 2) | 5000 |
| Lactose | 1875 |
| **Total** | 7000 |

5)

| **Ingredients** | **µg per capsule** |
|---|---|
| **A'**-bromide | 200 |
| component **B** (example 1) | 5000 |
| **Total** | 5200 |

6)

| **Ingredients** | **µg per capsule** |
|---|---|
| **A'**-bromide | 150 |
| component **B** (example 2) | 5000 |
| **Total** | 5150 |

7)

| **Ingredients** | **µg per capsule** |
|---|---|
| **A'**-bromide | 100 |
| component **B** (example 2) | 3500 |
| Lactose | 3400 |
| **Total** | 7000 |

8)

| **Ingredients** | **µg per capsule** |
|---|---|
| **A'**-bromide | 150 |
| component **B** (example 2) | 3000 |
| Lactose | 3850 |
| **Total** | 7000 |

9)

| **Ingredients** | **µg per capsule** |
|---|---|
| **A'**-bromide | 150 |
| component **B** (example 3) | 5000 |
| **Total** | 5150 |

## Claims

1. Pharmaceutical compositions **characterised in that** they contain one or more anticholinergics of formula **A** 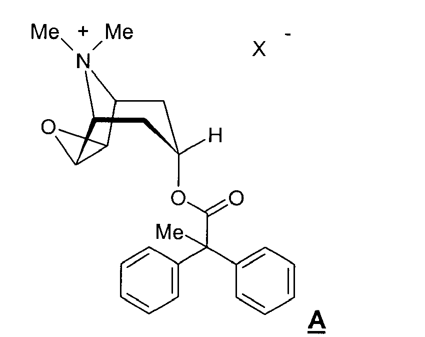 wherein
X - denotes an anion (counter-ion), preferably an anion selected from the group consisting of chloride, bromide, iodide, sulphate, phosphate, methansulphonate, nitrate, maleate, acetate, citrate, fumarate, tartrate, oxalate, succinate, benzoate and p-toluenesulphonate
combined with one or more p38 kinase inhibitors **(B),** optionally in the form of the enantiomers, mixtures of the enantiomers or in the form of the racemates thereof, optionally in the form of the solvates or hydrates and optionally together with a pharmaceutically acceptable excipient.

2. Pharmaceutical composition according to claim 1, **characterised in that** the active substances **A** and **B** are present either together in a single formulation or in two separate formulations.

3. Pharmaceutical composition according to one of claims 1 and 2, **characterised in that** in **A** X is selected from among chloride, bromide, methansulphonate and p-toluenesulphonate.

4. Pharmaceutical composition according to one of claims 1 to 3, **characterised in that** in **A** X⁻ denotes bromide.

5. Pharmaceutical composition according to one of claims 1 to 4, **characterized in that** the p38 kinase inhibitor **B** is selected from the group of compounds disclosed in US Patents 5,716,972, US 5,686,455, US 5,656,644, US 5,593,992, US 5,593,991, US 5,663,334, US 5,670,527, US 5,559,137, 5,658,903, US 5,739,143, US 5,756,499, US 6,277,989, US 6,340,685, and US 5,716,955 and PCT applications WO 92/12154, WO 94/19350, WO 95/09853, WO 95/09851, WO 95/09847, WO 95/09852, WO 97/25048, WO 97/25047, WO 97/33883, WO 97/35856, WO 97/35855, WO 97/36587, WO 97/47618, WO 97/16442, WO 97/16441, WO 97/12876, WO 98/25619, WO 98/06715, WO 98/07425, WO 98/28292, WO 98/56377, WO 98/07966, WO 98/56377, WO 98/22109, WO 98/24782, WO 98/24780, WO 98/22457, WO 98/52558, WO 98/52559, WO 98/52941, WO 98/52937, WO 98/52940, WO 98/56788, WO 98/27098, WO 98/47892, WO 98/47899, WO 98/50356, WO 98/32733, WO 99/58523, WO 99/01452, WO 99/01131, WO 99/01130, WO 99/01136, WO 99/17776, WO 99/32121, WO 99/58502, WO 99/58523, WO 99/57101, WO 99/61426, WO 99/59960, WO 99/59959, WO 99/00357, WO 99/03837, WO 99/01441, WO 99/01449, WO 99/03484, WO 99/15164, WO 99/32110, WO 99/32111, WO 99/32463, WO 99/64400, WO 99/43680, WO 99/17204, WO 99/25717, WO 99/50238, WO 99/61437, WO 99/61440, WO 00/26209, WO 00/18738, WO 00/17175, WO 00/20402, WO 00/01688, WO 00/07980, WO 00/07991, WO 00/06563, WO 00/12074, WO 00/12497, WO 00/31072, WO 00/31063, WO 00/23072, WO 00/31065, WO 00/35911, WO 00/39116, WO 00/43384, WO 00/41698, WO 00/69848, WO 00/26209, WO 00/63204, WO 00/07985, WO 00/59904, WO 00/71535, WO 00/10563, WO 00/25791, WO 00/55152, WO 00/55139, WO 00/17204, WO 00/36096, WO 00/55120, WO 00/55153, WO 00/56738, WO 01/21591, WO 01/29041, WO 01/29042, WO 01/62731, WO 01/05744, WO 01/05745, WO 01/05746, WO 01/05749, WO 01/05751, WO 01/27315, WO 01/42189, WO 01/00208, WO 01/42241, WO 01/34605, WO 01/47897, WO 01/64676, WO 01/37837, WO 01/38312, WO 01/38313, WO 01/36403, WO 01/38314, WO 01/47921, WO 01/27089, DE 19842833, and JP 2000 86657.

6. Pharmaceutical composition according to claim 5, **characterized in that** the p38 kinase inhibitor **B** is selected from the group of compounds disclosed in US 6,277,989, US 6,340,685, WO 00/12074, WO 00/12497, WO 00/59904, WO 00/71535, WO 01/64676, WO 99/61426, WO 00/10563, WO 00/25791, WO 01/37837, WO 01/38312, WO 01/38313, WO 01/38314, WO 01/47921, WO 99/61437, WO 99/61440, WO 00/17175, WO 00/17204, WO 00/36096, WO 98/27098, WO 99/00357, WO 99/58502, WO 99/64400, WO 99/01131, WO 00/43384, WO 00/55152, WO 00/55139, and WO 01/36403.

7. Pharmaceutical composition according to claim 6, **characterized in that** the p38 kinase inhibitor **B** is a compound of formula **1** 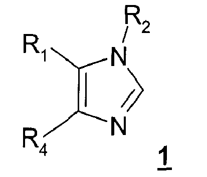 wherein
R₁ is 4-pyridyl, pyrimidinyl, 4-pyridazinyl, 1,2,4-triazin-5-yl, quinolyl, isoquinolinyl, or quinazolin-4-yl ring, which ring is substituted with Y-Rₐ and optionally with an additional independent substituent selected from C₁₋₄ alkyl, halogen, hydroxyl, C₁₋₄ alkoxy, C₁₋₄ akylthio, C₁₋₄ aklylsulfinyl, CH₂OR₁₂, amino, mono and di- C₁₋₆ alkyl substituted amino, an N-heterocyclyl ring which ring has from 5 to 7 members and optionally contains an additional heteroatom selected from oxygen, sulfur or NR₁₅, N(R₁₀)C(O)R_{b} or NHRₐ;
Y is oxygen or sulfur;
R₄ is phenyl, naphth-1-yl or naphth―yl, or a heteroaryl, which is optionally substituted by one or two substituents, each of which is independently selected, and which, for a 4-phenyl, 4naphth-1-yl, 5-naphth-2-yl or 6-naphth-2-yl substituent, is halogen, cyano, nitro, C(Z)NR₇R₁₇, C(Z)OR₁₆, (CR₁₀R₂₀)ᵥCOR₁₂, SR₅, SOR₅, OR₁₂, halo-substituted-C₁₋₄ alkyl, C₁₋₄ alkyl, ZC(Z)R₁₂, NR₁₀C(Z)R₁₆, or (CR₁₀R₂₀)ᵥNR₁₀R₂₀ and which, for other positions of substitution, is halogen, cyano, C(Z)NR₁₃R₁₄, C(Z)OR₃, (CR₁₀R₂₀)_{m"}COR₃, S(O)ₘR₃, OR₃, halo-substituted-C₁₋₄ alkyl, C₁₋₄ alkyl, (CR₁₀R₂₀)_{m"}R₁₀C(Z)R₃, NR₁₀S(O)_{m'}R₈, NR₁₀S(O)_{m'}NR₇R₁₇, ZC(Z)R₃ or (CR₁₀R₂₀)_{m"}NR₁₃R₁₄;
Z is oxygen or sulfur;
n is an integer having a value of 1 to 10;
m is 0, or integer 1 or 2;
m' is an integer having a value of 1 or 2;
m" is 0, or an integer having a value of 1 to 5;
v is 0, or an integer having a value of 1 to 2;
R₂ is -C(H) (A) (R₂₂);
A is optionally substituted aryl, heterocyclyl, or heteroaryl ring, or A is substituted C₁₋₁₀ alkyl;
R₂₂ is an optionally substituted C₁₋₁₀ alkyl;
Rₐ is aryl, arylC₁₋₆ alkyl, heterocyclic, heterocyclylC₁₋₆ alkyl, heteroaryl, heteroarylC₁₋₆alkyl, wherein each of these moieties may be optionally substituted;
R_{b} is hydrogen, C₁₋₆ alkyl, C₃₋₇ cycloalkyl, aryl, aryl C₁₋₄ alkyl, heteroaryl, heteroarylC₁₋₄ alkyl, heterocyclyl, or heterocyclylC₁₋₄ alkyl, wherein each of these moieties may be optionally substituted;
R₃ is heterocyclyl, heterocyclyl C₁₋₁₀ alkyl or R₈;
R₅ is hydrogen, C₁₋₄ alkyl, C₂₋₄ alkenyl, C₂₋₄ alkynyl or NR₇R₁₇, excluding the moieties SR₅ being SNR₇R₁₇and SOR₅ being SOH;
R₆ is hydrogen, a pharmaceutically acceptable cation, C₁₋₁₀ alkyl, C₃₋₇ cycloalkyl, aryl, aryl C₁₋₄ alkyl, heteroaryl, heteroaryl C₁₋₄ alkyl, heterocyclyl, aryl, or C₁₋₁₀ alkanoyl;
R₇ and R₁₇ is each independently selected from hydrogen or C₁₋₄ alkyl or R₇ and R₁₇ together with the nitrogen to which they are attached form a heterocyclic ring of 5 to 7 members which ring optionally contains an additional heteroatom selected from oxygen, sulfur or NR₁₅;
R₈ is C₁₋₁₀ alkyl, halo-substituted C₁₋₁₀ alkyl, C₂₋₁₀ alkenyl, C₂₋₁₀ alkynyl, C₃₋₇ cycloalkyl, C₅₋₇ cycloalkenyl, aryl, aryl C₁₋₁₀ alkyl, heteroaryl, heteroaryl C₁₋₁₀ alkyl, (CR₁₀R₂₀)ₙOR₁₁, (CR₁₀R₂₀)ₙS(O)ₘR₁₈, (CR₁₀R₂₀)ₙNHS(O)₂R₁₈, (CR₁₀R₂₀)ₙNR₁₃R₁₄; wherein the aryl, arylalkyl, heteroaryl, heteroaryl alkyl may be optionally substituted;
R₉ is hydrogen, C(Z) R₁₁ or optionally substituted C₁₋₁₀ alkyl, S(O)₂R₁₈, optionally substituted aryl or optionally substituted aryl C₁₋₄ alkyl;
R₁₀ and R₂₀ is each independently selected from hydrogen or C₁₋₄ alkyl;
R₁₁ is hydrogen, C₁₋₁₀ alkyl, C₃₋₇ cycloalkyl, heterocyclyl, heterocyclyl C₁₋₁₀ alkyl, aryl, arylC₁₋₁₀ alkyl, heteroaryl or heteroaryl C₁₋₁₀ alkyl, wherein these moieties may be optionally substituted;
R₁₂ is hydrogen or R_{16;}
R₁₃ an R₁₄ is each independently selected from hydrogen or optionally substituted C₁₋₄ alkyl, optionally substituted aryl or optionally substituted arylC₁₋₄ alkyl, or together with the nitrogen which they are attached form a heterocyclic ring of 5 to 7 members which ring optionally contains an additional heteroatom selected from oxygen, sulfur or NR₉;
R₁₅ is Rio or C(Z)-C₁₋₄ alkyl;
R₁₆ is C₁₋₄ alkyl, halo-substituted-C₁₋₄ alkyl, or C₃₋₇ cycloalkyl;
R₁₈ is C₁₋₁₀ alkyl, C₃₋₇ cycloalkyl, heterocyclyl, aryl, aryl₁₋₁₀ alkyl, heterocyclyl, heterocyclyl- C₁₋₁₀alkyl, heteroaryl or heteroaryl₁₋₁₀ alkyl;
or a pharmaceutically acceptable salt thereof.

8. Pharmaceutical composition according to claim 6, **characterized in that** the p38 kinase inhibitor **B** is a compound of formula **2** 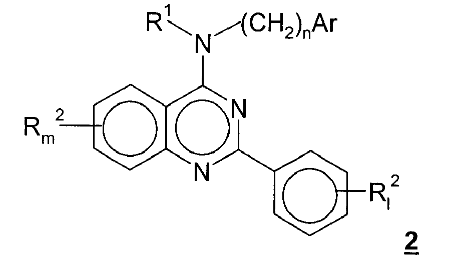 wherein
R¹ is H, alkyl(1-6C) or arylalkyl optionally substituted on the aryl group with 1-3 substituents independently selected from alkyl (1-6C), halo, OR, NR₂, SR, -OOCR, -NROCR, RCO, -COOR, -CONR₂, -SO₂NR₂, CN, CF₃, and NO₂, wherein each R is independently H or lower alkyl (1-4C);
each R² is independently alkyl (1-6C), halo, OR, SR, OOCR, NROCR, COOR, RCO, CONR₂, SO₂NR₂, CN, CF₃ or NO₂, wherein each R is independently H or lower alkyl (1-4C);
each of I, m, and n is independently 0, 1 or 2; and
Ar is phenyl, 2-, 3- or 4-pyridyl, indolyl, 2- or 4-pyrimidyl, or benzimidazolyl, each optionally substituted with optionally substituted alkyl, alkenyl, alkynyl, aryl, N-aryl, NH-aroyl, halo, OR, NR₂, SR, -OOCR, -NROCR, RCO, -COOR,-CONR₂, SO₂NR₂, CN, CF₃, or NO₂, wherein each R is independently H or alkyl (1-4C), or the pharmaceutically acceptable salts thereof.

9. Pharmaceutical composition according to claim 6, **characterized in that** the p38 kinase inhibitor **B** is a compound of formula **3a**, **3b**, **3c**, or **3d** 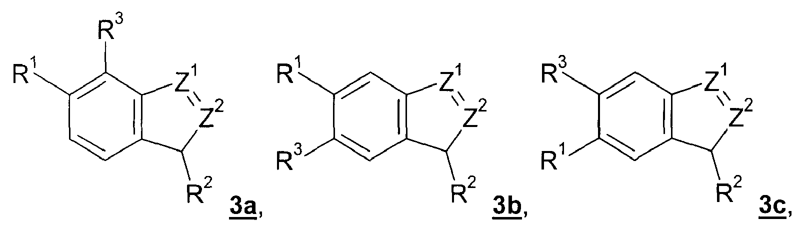 or 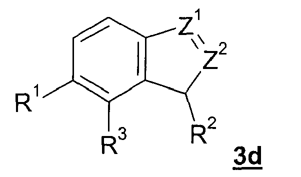 and the pharmaceutically acceptable salts thereof,
wherein each of Z¹ and Z² is independently CR⁴ or N;
where each R⁴ is independently selected from H and alkyl(1-6C);
wherein said alkyl optionally includes one or more heteroatoms selected from O, S and N, and wherein said alkyl is optionally substituted by one or more substituents selected from halo, OR, SR, NR₂, RCO, COOR, CONR₂, OOCR, NROCR, CN, =O, a 5 or 6 membered saturated carbocyclic ring or heterocyclic ring containing 1-2 N, and a 6-membered aromatic ring optionally containing 1-2 N heteroatoms, wherein R in the foregoing optional substituents is H or alkyl (1-6C);
R¹ is 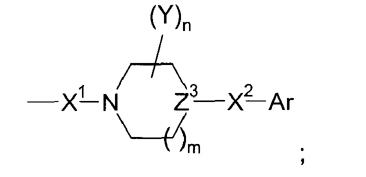
wherein
X¹ is CO, SO, CHOH or SO₂ ;
m is 1;
Y is optionally substituted alkyl, optionally substituted aryl, or optionally substituted arylalkyl;
n is 0, 1 or 2;
Z³ is N;
X² is CH or CH₂ ; and
Ar consists of one or two phenyl moieties directly coupled to X², said one or two phenyl moieties being optionally substituted by a substituent selected from halo, nitro, alkyl (1-6C), alkenyl (1-6C), CN, CF₃, RCO, COOR, CONR₂, NR₂, OR, SR, OOCR, NROCR, (wherein R in the foregoing is H or 1-6C alkyl), and phenyl, itself optionally substituted by the foregoing substituents;
R² is selected from H, and alkyl (1-6C);
wherein said alkyl optionally includes one or more heteroatoms which are selected from O, S and N, and wherein said alkyl is optionally substituted by one or more substituents selected from halo, OR, SR, NR₂, RCO, COOR, CONR₂, OOCR, NROCR, (where R in the foregoing is H or 1-6C alkyl) CN, =O, a 5 or 6 membered saturated carbocyclic ring or heterocyclic ring containing 1-2 N, and a 6-membered aromatic ring optionally containing 1-2 N heteroatoms;
R³ is H, halo, NO₂, alkyl (1-6C), alkenyl (1-6C), CN, OR, SR, NR₂, RCO, COOR, CONR₂, OOCR, or NROCR where R is H or alkyl (1-6C).

10. Pharmaceutical composition according to claim 6, **characterized in that** the p38 kinase inhibitor **B** is a compound of formula **4** 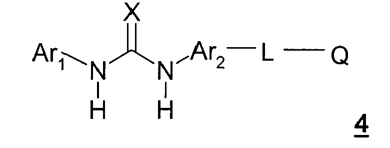 wherein
Ar₁ is a heterocyclic group selected from the group consisting of pyrrole, pyrrolidine, pyrazole, imidazole, oxazole, thiazole, furan and thiophene; and wherein Ar₁ may be substituted by one or more R₁,R₂ or R₃;
Ar₂ is phenyl, naphthyl, quinoline, isoquinoline, tetrahydronaphthyl, tetrahydroquinoline, tetrahydroisoquinoline, benzimidazole, benzofuran, indanyl, indenyl or indole each being optionally substituted with one to three R₂ groups;
L, a linking group, is a
C₁₋₁₀ saturated or unsaturated branched or unbranched carbon chain;
wherein one or more methylene groups are optionally independently replaced by O,N or S; and
wherein said linking group is optionally substituted with 0-2 oxo groups and one or more C₁₋₄ branched or unbranched alkyl which may be substituted by one or more halogen atoms;
Q is selected from the group consisting of:
d) phenyl, naphthyl, pyridine, pyrimidine, pyridazine, imidazole, benzimidazole, furan, thiophene, pyran, naphthyridine, oxazo[4,5-b]pyridine and imidazo[4,5-*b*]pyridine, which are optionally substituted with one to three groups selected from the group consisting of halogen, C₁₋₆ alkyl, C₁₋₆ alkoxy, hydroxy, mono- or di-(C₁₋₃ alkyl)amino, C₁₋₆ alkyl-S(O)ₘ and phenylamino wherein the phenyl ring is optionally substituted with one to two groups consisting of halogen, C₁₋₆ alkyl and C₁₋₆ alkoxy;
e) tetrahydropyran, tetrahydrofuran, 1,3-dioxolanone, 1,3-dioxanone, 1,4-dioxane, morpholine, thiomorpholine, thiomorpholine sulfoxide, thiomorpholine sulfone, piperidine, piperidinone, tetrahydropyrimidone, cyclohexanone, cyclohexanol, pentamethylene sulfide, pentamethylene sulfoxide, pentamethylene sulfone, tetramethylene sulfide, tetramethylene sulfoxide and tetramethylene sulfone which are optionally substituted with one to three groups selected from the group consisting of C₁₋₆ alkyl, C₁₋₆ alkoxy, hydroxy, mono- or di-(C₁₋₃ alkyl)amino-C₁₋₃ alkyl, phenylamino-C₁₋₃ alkyl and C₁₋₃ alkoxy-C₁₋₃ alkyl;
f) C₁₋₆ alkoxy, secondary or tertiary amine wherein the amino nitrogen is covalently bonded to groups selected from the group consisting of C₁₋₃ alkyl and C₁₋₅ alkoxyalkyl and phenyl wherein the phenyl ring is optionally substituted with one to two groups consisting of halogen, C₁₋₆ alkoxy, hydroxy or mono- or di-(C₁₋₃ alkyl)amino, C₁₋₆ alkyl-S(O)ᵣ, phenyl-S(O)ₜ, wherein the phenyl ring is optionally substituted with one to two groups consisting of halogen, C₁₋₆ alkoxy, hydroxy or mono- or di-(C₁₋₃ alkyl)amino;
R₁ is selected from the group consisting of:
(g) C₃₋₁₀ branched or unbranched alkyl, which may optionally be partially or fully halogenated, and optionally substituted with one to three phenyl, naphthyl or heterocyclic groups selected from the group consisting of pyridinyl, pyrimidinyl, pyrazinyl, pyridazinyl, pyrrolyl, imidazolyl, pyrazolyl, thienyl, furyl, isoxazolyl and isothiazolyl; each such phenyl, naphthyl or heterocycle selected from the group hereinabove described, being substituted with 0 to 5 groups selected from the group consisting of halogen, C₁₋₆ branched or unbranched alkyl which is optionally partially or fully halogenated, C₃₋₈ cycloalkyl, C₅₋₈ cycloalkenyl, hydroxy, cyano, C₁₋₃ alkyloxy which is optionally partially or fully halogenated, NH₂C(O) and di(C₁₋₃)alkylaminocarbonyl;
(h) C₃₋₇ cycloalkyl selected from the group consisting of cyclopropyl, cyclobutyl, cyclopentanyl, cyclohexanyl, cycloheptanyl, bicyclopentanyl, bicyclohexanyl and bicycloheptanyl, which may optionally be partially or fully halogenated and which may optionally be substituted with one to three C₁₋₃ alkyl groups, or an analog of such cycloalkyl group wherein one to three ring methylene groups are replaced by groups independently selected from O, S, CHOH, >C=O, >C=S and NH;
(i) C₃₋₁₀ branched alkenyl which may optionally be partially or fully halogenated, and which is optionally substituted with one to three C₁₋₅ branched or unbranched alkyl, phenyl, naphthyl or heterocyclic groups, with each such heterocyclic group being independently selected from the group consisting of pyridinyl, pyrimidinyl, pyrazinyl, pyridazinyl, pyrrolyl, imidazolyl, pyrazolyl, thienyl, furyl, isoxazolyl and isothiazolyl, and each such phenyl, naphthyl or heterocyclic group being substituted with 0 to 5 groups selected from halogen, C₁₋₆ branched or unbranched alkyl which is optionally partially or fully halogenated, cyclopropyl, cyclobutyl, cyclopentanyl, cyclohexanyl, cycloheptanyl, bicyclopentanyl, bicyclohexanyl and bicycloheptanyl, hydroxy, cyano, C₁₋₃ alkyloxy which is optionally partially or fully halogenated, NH₂C(O), mono- or di(C₁₋₃)alkylaminocarbonyl;
(j) C₅₋₇ cycloalkenyl selected from the group consisting of cyclopentenyl, cyclohexenyl, cyclohexadienyl, cycloheptenyl, cycloheptadienyl, bicyclohexenyl and bicycloheptenyl, wherein such cycloalkenyl group may optionally be substituted with one to three C₁₋₃ alkyl groups;
(k) cyano; and,
(I) methoxycarbonyl, ethoxycarbonyl and propoxycarbonyl;
R₂ is selected from the group consisting of:
a C₁₋₆ branched or unbranched alkyl which may optionally be partially or fully halogenated, acetyl, aroyl, C₁₋₄ branched or unbranched alkoxy, which may optionally be partially or fully halogenated, halogen, methoxycarbonyl and phenylsulfonyl;
R₃ is selected from the group consisting of:
g) a phenyl, naphthyl or heterocyclic group selected from the group consisting of pyridinyl, pyrimidinyl, pyrazinyl, pyridazinyl, pyrrolyl, imidazolyl, pyrazolyl, thienyl, furyl, tetrahydrofuryl, isoxazolyl, isothiazolyl, quinolinyl, isoquinolinyl, indolyl, benzimidazolyl, benzofuranyl, benzoxazolyl, benzisoxazolyl, benzpyrazolyl, benzothiofuranyl, cinnolinyl, pterindinyl, phthalazinyl, naphthypyridinyl, quinoxalinyl, quinazolinyl, purinyl and indazolyl; wherein such phenyl, naphthyl or heterocyclic group is optionally substituted with one to five groups selected from the group consisting of a C₁₋₆ branched or unbranched alkyl, phenyl, naphthyl, heterocycle selected from the group hereinabove described, C₁₋₆ branched or unbranched alkyl which is optionally partially or fully halogenated, cyclopropyl, cyclobutyl, cyclopentanyl, cyclohexanyl, cycloheptanyl, bicyclopentanyl, bicyclohexanyl, bicycloheptanyl, phenyl C₁₋₅ alkyl, naphthyl C₁₋₅ alkyl, halo, hydroxy, cyano, C₁₋₃ alkyloxy which may optionally be partially or fully halogenated, phenyloxy, naphthyloxy, heteraryloxy wherein the heterocyclic moiety is selected from the group hereinabove described, nitro, amino, mono- or di-(C₁₋₃)alkylamino, phenylamino, naphthylamino, heterocyclylamino wherein the heterocyclyl moiety is selected from the group hereinabove described, NH₂C(O), a mono- or di-(C₁₋₃)alkyl aminocarbonyl, C₁₋₅ alkyl-C(O)-C₁₋₄ alkyl, amino-C₁₋₅ alkyl, mono- or di-(C₁₋₃)alkylamino-C₁₋₅ alkyl, amino-S(O)₂, di-(C₁₋₃)alkylamino-S(O)₂, R₄ -C₁₋₅ alkyl, R₅-C₁₋₅ alkoxy, R₆―C(O)-C₁₋₅ alkyl and R₇-C₁₋₅ alkyl(R₈)N;
h) a fused aryl selected from the group consisting of benzocyclobutanyl, indanyl, indenyl, dihydronaphthyl, tetrahydronaphthyl, benzocycloheptanyl and benzocycloheptenyl, or a fused heterocyclyl selected from the group consisting of cyclopentenopyridine, cyclohexanopyridine, cyclopentanopyrimidine, cyclohexanopyrimidine, cyclopentanopyrazine, cyclohexanopyrazine, cyclopentanopyridazine, cyclohexanopyridazine, cyclopentanoquinoline, cyclohexanoquinoline, cyclopentanoisoquinoline, cyclohexanoisoquinoline, cyclopentanoindole, cyclohexanoindole, cyclopentanobenzimidazole, cyclohexanobenzimidazole, cyclopentanobenzoxazole, cyclohexanobenzoxazole, cyclopentanoimidazole, cyclohexanoimidazole, cyclopentanothiophene and cyclohexanothiophene; wherein the fused aryl or fused heterocyclyl ring is substituted with 0 to 3 groups independently selected from phenyl, naphthyl and heterocyclyl selected from the group consisting of pyridinyl, pyrimidinyl, pyrazinyl, pyridazinyl, pyrrolyl, imidazolyl, pyrazolyl, thienyl, furyl, isoxazolyl, and isothiazolyl, C₁₋₆ branched or unbranched alkyl which is optionally partially or fully halogenated, halo, cyano, C₁₋₃ alkyloxy which is optionally partially or fully halogenated, phenyloxy, naphthyloxy, heterocyclyloxy wherein the heterocyclyl moiety is selected from the group hereinabove described, nitro, amino, mono- or di-(C₁₋₃)alkylamino, phenylamino, naphthylamino, heterocyclylamino wherein the heterocyclyl moiety is selected from the group hereinabove described, NH₂C(O), a mono- or di-(C₁₋₃)alkyl aminocarbonyl, C₁₋₄ alkyl-OC(O), C₁₋₅ alkyl-C(O)-C₁₋₄ branched or unbranched alkyl, an amino-C₁₋₅ alkyl, mono- or di-(C₁₋₃)alkylamino-C₁₋₅ alkyl, R₉ -C₁₋₅ alkyl, R₁₀-C₁₋₅ alkoxy, R₁₁―C(O)-C₁₋₅ alkyl, and R₁₂-C₁₋₅alkyl(R₁₃)N;
i) cycloalkyl selected from the group consisting of cyclopentanyl, cyclohexanyl, cycloheptanyl, bicyclopentanyl, bicyclohexanyl and bicycloheptanyl, which the cycloalkyl may optionally be partially or fully halogenated and which may optionally be substituted with one to three C₁₋₃ alkyl groups;
j) C₅₋₇ cycloalkenyl, selected from the group consisting of cyclopentenyl, cyclohexenyl, cyclohexadienyl, cycloheptenyl, cycloheptadienyl, bicyclohexenyl and bicycloheptenyl, wherein such cycloalkenyl group may optionally be substituted with one to three C₁₋₃ alkyl groups; and
k) acetyl, aroyl, alkoxycarbonylalkyl or phenylsulfonyl;
I) C₁₋₆ branched or unbranched alkyl which may optionally be partially or fully halogenated;
or R₁ and R₂ taken together may optionally form a fused phenyl or pyridinyl ring,
and wherein each R₈, R₁₃ is independently selected from the group consisting of:
hydrogen and C₁₋₄ branched or unbranched alkyl which may optionally be partially or fully halogenated;
each R₄, R₅, R₆, R₇, R₉, R₁₀, R₁₁ and R₁₂ is independently selected from the group consisting of:
morpholine, piperidine, piperazine, imidazole and tetrazole;
m=0,1,2;
r = 0, 1, 2;
t = 0, 1, 2;
X = O or S and physiologically acceptable acids or salts thereof.

11. Pharmaceutical composition according to claim 6, **characterized in that** the p38 kinase inhibitor B is a compound of formula **5** 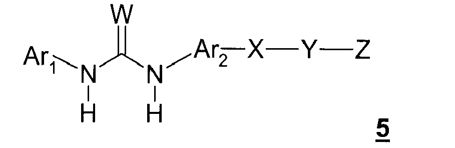 wherein:
Ar₁ is selected from the group consisting of:
pyrrole, pyrrolidine, pyrazole, imidazole, oxazole, thiazole, furan and thiophene;
wherein Ar₁ may be substituted by one or more R₁, R₂ or R₃;
Ar₂ is:
phenyl, naphthyl, quinoline, isoquinoline, tetrahydronaphthyl, tetrahydroquinoline, tetrahydroisoquinoline, benzimidazole, benzofuran, indanyl, indenyl or indole each being optionally substituted with zero to three R₂ groups;
X is:
a) a C₅₋₈ cycloalkyl or cycloalkenyl optionally substituted with 0-2 oxo groups or 0-3 C₁₋₄ branched or unbranched alkyl, C₁₋₄ alkoxy or C₁₋₄ alkylamino chains;
b) phenyl, furan, thiophene, pyrrole, imidazolyl, pyridine, pyrimidine, pyridinone, dihydropyridinone, maleimide, dihydromaleimide, piperdine, piperazine or pyrazine each being optionally independently substituted with 0-3 C₁₋₄ branched or unbranched alkyl, C₁₋₄alkoxy, hydroxy, nitrile, mono- or di-(C₁₋₃ alkyl)amino, C₁₋₆ alkyl-S(O)ₘ, or halogen;
Y is:
a bond or a C₁₋₄ saturated or unsaturated branched or unbranched carbon chain optionally partially or fully halogenated, wherein one or more methylene groups are optionally replaced by O, NH, S(O), S(O)₂ or S and wherein Y is optionally independently substituted with 0-2 oxo groups and one or more C₁₋₄ branched or unbranched alkyl which may be substituted by one or more halogen atoms;
Z is:
a) phenyl, pyridine, pyrimidine, pyridazine, imidazole, furan, thiophene, pyran, which are optionally substituted with one to three groups consisting of halogen, C₁₋₆ alkyl, C₁₋₆ alkoxy, hydroxy, mono- or di-(C₁₋₃ alkyl)amino, C₁₋₆ alkyl-S(O)ₘ , COOH and phenylamino wherein the phenyl ring is optionally substituted with one to two groups consisting of halogen, C₁₋₆ alkyl and C₁₋₆ alkoxy;
b) tetrahydropyran, tetrahydrofuran, 1,3-dioxolanone, 1,3-dioxanone, 1,4-dioxane, morpholine, thiomorpholine, thiomorpholine sulfoxide, piperidine, piperidinone, piperazine, tetrahydropyrimidone, cyclohexanone, cyclohexanol, pentamethylene sulfide, pentamethylene sulfoxide, pentamethylene sulfone, tetramethylene sulfide, tetramethylene sulfoxide or tetramethylene sulfone which are optionally substituted with one to three groups consisting of nitrile, C₁₋₆ alkyl, C₁₋₆ alkoxy, hydroxy, mono- or di-(C₁₋₃ alkyl)amino-C₁₋₃ alkyl, phenylamino-C₁₋₃ alkyl and C₁₋₃ alkoxy-C₁₋₃ alkyl;
c) C₁₋₆ alkoxy, secondary or tertiary amine wherein the amino nitrogen is covalently bonded to groups selected from the group consisting of C₁₋₃ alkyl, C₁₋₅ alkoxyalkyl, pyridinyl-C₁₋₃ alkyl, imidazolyl-C₁₋₃ alkyl, tetrahydrofuranyl-C₁₋₃ alkyl, phenylamino, wherein the phenyl ring is optionally substituted with one to two halogen, C₁₋₆ alkoxy, hydroxy or mono- or di-(C₁₋₃ alkyl)amino, C₁₋₆ alkyl-S(O)ₘ, and phenyl-S(O)ₘ, wherein the phenyl ring is optionally substituted with one to two halogen, C₁₋₆ alkoxy, hydroxy or mono- or di-(C₁₋₃ alkyl)amino;
R₁ is :
a) C₃₋₁₀ branched or unbranched alkyl optionally partially or fully halogenated and optionally substituted with one to three phenyl, naphthyl or heterocyclic groups selected from the group consisting of pyridinyl, pyrimidinyl, pyrazinyl, pyridazinyl, pyrrolyl, imidazolyl, pyrazolyl, thienyl, furyl, isoxazolyl and isothiazolyl; each such phenyl, naphthyl or heterocycle selected from the group hereinabove described in this paragraph, and being substituted with 0 to 5 groups selected from the group consisting of halogen, C₁₋₆ branched or unbranched alkyl which is optionally partially or fully halogenated, C₃₋₈ cycloalkyl, C₅₋₈ cycloalkenyl, hydroxy, nitrile, C₁₋₃ alkyloxy which is optionally partially or fully halogenated, NH₂C(O) and di(C₁₋₃)alkylaminocarbonyl;
b) C₃₋₇ cycloalkyl selected from the group consisting of cyclopropyl, cyclobutyl, cyclopentanyl, cyclohexanyl, cycloheptanyl, bicyclopentanyl, bicyclohexanyl and bicycloheptanyl each being optionally be partially or fully halogenated and optionally substituted with one to three C₁₋₃ alkyl groups, or an analog of such cycloalkyl group wherein one to three ring methylene groups are replaced by groups independently selected from the group consisting of O, S, CHOH, >C=O, >C=S and NH;
c) C₃₋₁₀ branched alkenyl optionally partially or fully halogenated and optionally substituted with one to three C₁₋₅ branched or unbranched alkyl, phenyl, naphthyl or heterocyclic groups, with each such heterocyclic group being independently selected from the group consisting of pyridinyl, pyrimidinyl, pyrazinyl, pyridazinyl, pyrrolyl, imidazolyl, pyrazolyl, thienyl, furyl, isoxazolyl and isothiazolyl, and each such phenyl, naphthyl or heterocyclic group being substituted with 0 to 5 groups selected from the group consisting of halogen, C₁₋₆ branched or unbranched alkyl which is optionally partially or fully halogenated, cyclopropyl, cyclobutyl, cyclopentanyl, cyclohexanyl, cycloheptanyl, bicyclopentanyl, bicyclohexanyl, bicycloheptanyl, hydroxy, nitrile, C₁₋₃ alkoxy which is optionally partially or fully halogenated, NH₂C(O) and mono- or di(C₁₋₃)alkylaminocarbonyl;
d) a C₅₋₇ cycloalkenyl selected from the group consisting of cyclopentenyl, cyclohexenyl, cyclohexadienyl, cycloheptenyl, cycloheptadienyl, bicyclohexenyl and bicycloheptenyl, wherein such cycloalkenyl group is optionally substituted with one to three C₁₋₃alkyl groups;
e) nitrile; or
f) C₁₋₆ branched or unbranched alkoxycarbonyl, C₁₋₆ branched or unbranched alkylaminocarbonyl, C₁₋₆ branched or unbranched alkylcarbonylamino-C₁₋₃-alkyl;
R₂ is:
a C₁₋₆ branched or unbranched alkyl optionally partially or fully halogenated, acetyl, aroyl, C₁₋₄ branched or unbranched alkoxy optionally partially or fully halogenated, halogen, methoxycarbonyl or phenylsulfonyl;
R₃ is:
a) phenyl, naphthyl or heterocyclic group selected from the group consisting of pyridinyl, pyrimidinyl, pyrazinyl, pyridazinyl, pyrrolyl, imidazolyl, pyrazolyl, thienyl, furyl, tetrahydrofuryl, isoxazolyl, isothiazolyl, quinolinyl, isoquinolinyl, indolyl, benzimidazolyl, benzofuranyl, benzoxazolyl, benzisoxazolyl, benzpyrazolyl, benzothiofuranyl, cinnolinyl, pterindinyl, phthalazinyl, naphthypyridinyl, quinoxalinyl, quinazolinyl, purinyl and indazolyl, wherein such phenyl, naphthyl or heterocyclic group is optionally substituted with one to five groups selected from the group consisting of phenyl, naphthyl, heterocycle selected from the group hereinabove described in this paragraph, C₁₋₆ branched or unbranched alkyl which is optionally partially or fully halogenated, cyclopropyl, cyclobutyl, cyclopentyl, cyclohexyl, cycloheptyl, bicyclopentyl, bicyclohexyl, bicycloheptyl, phenyl C₁₋₅alkyl, naphthyl C₁₋₅alkyl, halogen, hydroxy, nitrile, C₁₋₃ alkyloxy which may optionally be partially or fully halogenated, phenyloxy, naphthyloxy, heteraryloxy wherein the heterocyclic moiety is selected from the group hereinabove described in this paragraph, nitro, amino, mono- or di-(C₁₋₃)alkylamino, phenylamino, naphthylamino, heterocyclylamino wherein the heterocyclyl moiety is selected from the group hereinabove described in this paragraph, NH₂C(O), a mono- or di-(C₁₋₃)alkyl aminocarbonyl, C₁₋₅ alkyl-C(O)-C₁₋₄ alkyl, amino-C₁₋₅ alkyl, mono- or di-(C₁₋₃)alkylamino-C₁₋₅ alkyl, amino-S(O)₂, di-(C₁₋₃)alkylamino-S(O)₂, R₄ -C₁₋₅ alkyl, R₅ -C₁₋₅ alkoxy, R₆-C(O)-C₁₋₅ alkyl and R₇-C₁₋₅ alkyl(R₈)N, carboxy-mono- or di-(C₁₋₅)-alkyl-amino;
b) a fused aryl selected from the group consisting of benzocyclobutanyl, indanyl, indenyl, dihydronaphthyl, tetrahydronaphthyl, benzocycloheptanyl and benzocycloheptenyl, or a fused heterocyclyl selected from the group consisting of cyclopentenopyridine, cyclohexanopyridine, cyclopentanopyrimidine, cyclohexanopyrimidine, cyclopentanopyrazine, cyclohexanopyrazine, cyclopentanopyridazine, cyclohexanopyridazine, cyclopentanoquinoline, cyclohexanoquinoline, cyclopentanoisoquinoline, cyclohexanoisoquinoline, cyclopentanoindole, cyclohexanoindole, cyclopentanobenzimidazole, cyclohexanobenzimidazole, cyclopentanobenzoxazole, cyclohexanobenzoxazole, cyclopentanoimidazole, cyclohexanoimidazole, cyclopentanothiophene and cyclohexanothiophene; wherein the fused aryl or fused heterocyclyl ring is substituted with 0 to 3 groups independently selected from the group consisting of phenyl, naphthyl and heterocyclyl selected from the group consisting of pyridinyl, pyrimidinyl, pyrazinyl, pyridazinyl, pyrrolyl, imidazolyl, pyrazolyl, thienyl, furyl, isoxazolyl, and isothiazolyl, C₁₋₆ branched or unbranched alkyl which is optionally partially or fully halogenated, halogen, nitrile, C₁₋₃ alkoxy which is optionally partially or fully halogenated, phenyloxy, naphthyloxy, heterocyclyloxy wherein the heterocyclyl moiety is selected from the group hereinabove described in this paragraph, nitro, amino, mono- or di-(C₁₋₃)alkylamino, phenylamino, naphthylamino, heterocyclylamino wherein the heterocyclyl moiety is selected from the group hereinabove described in this paragraph, NH₂C(O), a mono- or di-(C₁₋₃)alkyl aminocarbonyl, C₁₋₄ alkyl-OC(O), C₁₋₅ alkyl-C(O)-C₁₋₄ branched or unbranched alkyl, an amino-C₁₋₅ alkyl, mono-or di-(C₁₋₃)alkylamino-C₁₋₅ alkyl, R₉-C₁₋₅ alkyl, R₁₀-C₁₋₅ alkoxy, R₁₁ -C(O)-C₁₋₅ alkyl, and R₁₂ -C₁₋₅ alkyl(R₁₃)N;
c) cycloalkyl selected from the group consisting of cyclopentyl, cyclohexyl, cycloheptyl, bicyclopentyl, bicyclohexyl and bicycloheptyl, wherein the cycloalkyl is optionally partially or fully halogenated and optionally substituted with one to three C₁₋₃ alkyl groups;
d) C₅₋₇ cycloalkenyl selected from the group consisting of cyclopentenyl, cyclohexenyl, cyclohexadienyl, cycloheptenyl, cycloheptadienyl, bicyclohexenyl and bicycloheptenyl, wherein such cycloalkenyl group is optionally substituted with one to three C₁₋₃ alkyl groups;
e) acetyl, aroyl, alkoxycarbonylalkyl or phenylsulfonyl; or
f) C₁₋₆ branched or unbranched alkyl optionally partially or fully halogenated;
or R₁ and R₂ taken together may optionally form a fused phenyl or pyridinyl ring;
each R₈ and R₁₃ is independently selected from the group consisting of:
hydrogen and C₁₋₄ branched or unbranched alkyl optionally be partially or fully halogenated;
each R₄, R₅, R₆, R₇, R₉, R₁₀, R₁₁ and R₁₂ is independently selected from the group consisting of morpholine, piperidine, piperazine, imidazole and tetrazole;
m is 0, 1 or 2;
W is O or S and pharmaceutically acceptable derivatives thereof.

12. Pharmaceutical composition according to claim 6, **characterized in that** the p38 kinase inhibitor **B** is a compound of formula **5a** 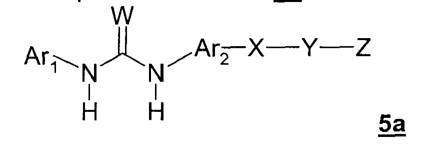 wherein:
Ar₁ is:
pyrrole, pyrrolidine, pyrazole, imidazole, oxazole, thiazole, furan and thiophene;
wherein Ar₁ is optionally substituted by one or more R₁, R₂ or R₃;
Ar₂ is:
phenyl, naphthyl, quinoline, isoquinoline, tetrahydronaphthyl, tetrahydroquinoline, tetrahydroisoquinoline, benzimidazole, benzofuran, indanyl, indenyl and indole each being optionally substituted with zero to three R₂ groups;
X is:
a C₅₋₈ cycloalkyl or cycloalkenyl optionally substituted with one to two oxo groups or one to three C₁₋₄ alkyl, C₁₋₄ alkoxy or C₁₋₄ alkylamino chains each being branched or unbranched;
phenyl, furanyl, thienyl, pyrrolyl, pyrazolyl, imidazolyl, pyridinyl, tetrahydropyridinyl, pyrimidinyl, pyridinonyl, dihydropyridinonyl, maleimidyl, dihydromaleimidyl, piperdinyl, benzimidazole, 3H-imidazo[4,5-b]pyridine, piperazinyl, pyridazinyl or pyrazinyl; each being optionally independently substituted with one to three C₁₋₄ alkyl, C₁₋₄alkoxy, hydroxy, nitrile, amino, mono- or di-(C₁₋₃ alkyl)amino, mono- or di-(C₁₋₃ alkylamino)carbonyl, NH₂C(O), C₁₋₆ alkyl-S(O)ₘ or halogen;
Y is:
a bond or a C₁₋₄ saturated or unsaturated branched or unbranched carbon chain optionally partially or fully halogenated, wherein one or more C atoms are optionally replaced by O, N, or S(O)ₘ and wherein Y is optionally independently substituted with one to two oxo groups, nitrile, phenyl, hydroxy or one or more C₁₋₄ alkyl optionally substituted by one or more halogen atoms;
Z is:
aryl, indanyl, heteroaryl selected from benzimidazolyl, pyridinyl, pyrimidinyl, pyridazinyl, pyrazinyl, imidazolyl, pyrazolyl, triazolyl, tetrazolyl, furanyl, thienyl and pyranyl, heterocycle selected from piperazinyl, tetrahydropyrimidonyl, cyclohexanonyl, cyclohexanolyl, 2-oxa- or 2-thia-5-aza-bicyclo[2.2.1]heptanyl, pentamethylene sulfidyl, pentamethylene sulfoxidyl, pentamethylene sulfonyl, tetramethylene sulfidyl, tetramethylene sulfoxidyl or tetramethylene sulfonyl, tetrahydropyranyl, tetrahydrofuranyl, 1,3-dioxolanonyl, 1,3-dioxanonyl, 1,4-dioxanyl, morpholino, thiomorpholino, thiomorpholino sulfoxidyl, thiomorpholino sulfonyl, piperidinyl, piperidinonyl, pyrrolidinyl and dioxolanyl, each of the aforementioned Z are optionally substituted with one to three halogen, C₁₋₆ alkyl, C₁₋₆ alkoxy, C₁₋₃ alkoxy-C₁₋₃ alkyl, C₁₋₆ alkoxycarbonyl, aroyl, heteroaroyl, heterocycleC₁₋₃acyl wherein the heteroaryl and heterocycle are as defined hereinabove in this paragraph, C₁₋₃acyl, oxo, hydroxy, pyridinyl-C₁₋₃ alkyl, imidazolyl-C₁₋₃ alkyl, tetrahydrofuranyl-C₁₋₃ alkyl, nitrile-C₁₋₃ alkyl, nitrile, carboxy, phenyl wherein the phenyl ring is optionally substituted with one to two halogen, C₁₋₆ alkoxy, hydroxy or mono- or di-(C₁₋₃ alkyl)amino, amino-S(O)ₘ, C₁₋₆ alkyl-S(O)ₘ or phenyl-S(O)ₘ wherein the phenyl ring is optionally substituted with one to two halogen, C₁₋₆ alkoxy, hydroxy, halogen or mono- or di-(C₁₋₃ alkyl)amino;
or Z is optionally substituted with one to three amino, aminocarbonyl or amino-C₁₋₃ alkyl wherein the N atom is optionally independently mono- or di-substituted by aminoC₁₋₆alkyl, C₁₋₃alkyl, arylC₀₋₃alkyl, C₁₋₅ alkoxyC₁₋₃ alkyl, C₁₋₅ alkoxy, aroyl, C₁₋₃acyl, C₁₋₃alkyl-S(O)ₘ- or arylC₀₋₃alkyl-S(O)ₘ- each of the aforementioned alkyl and aryl attached to the amino group is optionally substituted with one to two halogen, C₁₋₆ alkyl, C₁₋₆ alkoxy, hydroxy or mono-or di-(C₁₋₃ alkyl)amino;
or Z is optionally substituted with one to three aryl, heterocycle or heteroaryl as hereinabove described in this paragraph each in turn is optionally substituted by halogen, C₁₋₆ alkyl or C₁₋₆ alkoxy;
or Z is hydroxy, hydroxyC₁₋₃alkyl, halogen, nitrile, amino wherein the N atom is optionally independently mono- or di-substituted by C₁₋₆alkyl, aminoC₁₋₆alkyl, arylC₀₋₃alkyl, C₁₋₅ alkoxyC₁₋₃ alkyl, C₁₋₅ alkoxy, aroyl, C₁₋₃acyl, C₁₋₃alkyl-S(O)ₘ- , arylC₀₋₃alkyl-S(O)ₘ- , nitrileC₁₋₄alkyl or C₁₋₃alkoxyC₁₋₃alkyl, each of the aforementioned alkyl and aryl attached to the amino group is optionally substituted with one to two halogen, C₁₋₆ alkyl, C₁₋₆ alkoxy, hydroxy or mono-or di-(C₁₋₃ alkyl)amino, C₁₋₆ alkoxyheteroarylC₀₋₃alkyl, heteroarylC₀₋₃alkyl or heterocycyleC₀₋₃alkyl wherein the heteroaryl and heterocycle is hereinabove described in this paragraph,
or Z is C₁₋₆alkyl branched or unbranched, C₁₋₆alkoxy, C₁₋₃acylamino,
nitrileC₁₋₄alkyl, C₁₋₆ alkyl-S(O)ₘ, and phenyl-S(O)ₘ, wherein the phenyl ring is optionally substituted with one to two halogen, C₁₋₆ alkoxy, hydroxy or mono-or di-(C₁₋₃ alkyl)amino;
R₁ is:
a) C₁₋₁₀ branched or unbranched alkyl optionally partially or fully halogenated, and optionally substituted with one to three phenyl, naphthyl or heterocyclic groups selected from the group consisting of pyridinyl, pyrimidinyl, pyrazinyl, pyridazinyl, pyrrolyl, imidazolyl, pyrazolyl, thienyl, furyl, isoxazolyl and isothiazolyl; each such phenyl, naphthyl or heterocycle, selected from the group hereinabove described, being substituted with 0 to 5 groups selected from the group consisting of halogen, C₁₋₆ branched or unbranched alkyl which is optionally partially or fully halogenated, C₃₋₈ cycloalkyl, C₅₋₈ cycloalkenyl, hydroxy, nitrile, C₁₋₃ alkyloxy which is optionally partially or fully halogenated, NH₂C(O) and di(C₁₋₃)alkylaminocarbonyl;
b) C₃₋₇ cycloalkyl selected from the group consisting of cyclopropyl, cyclobutyl, cyclopentyl, cyclohexyl, cycloheptyl, bicyclopentyl, bicyclohexyl and bicycloheptyl, each optionally partially or fully halogenated and optionally substituted with one to three C₁₋₃ alkyl groups, or an analog of such cycloalkyl group wherein one to three ring methylene groups are replaced by groups independently selected from the group consisting of O, S, CHOH, >C=O, >C=S and NH;
c) C₃₋₁₀ branched alkenyl optionally partially or fully halogenated and optionally substituted with one to three C₁₋₅ branched or unbranched alkyl, phenyl, naphthyl or heterocyclic groups, with each such heterocyclic group being independently selected from the group consisting of pyridinyl, pyrimidinyl, pyrazinyl, pyridazinyl, pyrrolyl, imidazolyl, pyrazolyl, thienyl, furyl, isoxazolyl and isothiazolyl, and each such phenyl, naphthyl or heterocyclic group being substituted with 0 to 5 groups selected from the group consisting of halogen, C₁₋₆ branched or unbranched alkyl which is optionally partially or fully halogenated, cyclopropyl, cyclobutyl, cyclopentanyl, cyclohexanyl, cycloheptanyl, bicyclopentanyl, bicyclohexanyl, bicycloheptanyl, hydroxy, nitrile, C₁₋₃ alkoxy which is optionally partially or fully halogenated, NH₂C(O) and mono- or di(C₁₋₃)alkylaminocarbonyl;
d) a C₅₋₇ cycloalkenyl selected from the group consisting of cyclopentenyl, cyclohexenyl, cyclohexadienyl, cycloheptenyl, cycloheptadienyl, bicyclohexenyl and bicycloheptenyl, wherein such cycloalkenyl group is optionally substituted with one to three C₁₋₃ alkyl groups;
e) nitrile; or
f) C₁₋₆ branched or unbranched alkoxycarbonyl, C₁₋₆ branched or unbranched alkylaminocarbonyl, C₁₋₆ branched or unbranched alkylcarbonylamino-C₁₋₃-alkyl;
R₂ is:
a C₁₋₆ branched or unbranched alkyl optionally partially or fully halogenated and optionally substituted with nitrile,
or R₂ is acetyl, aroyl, C₁₋₄ branched or unbranched alkoxy optionally partially or fully halogenated, halogen, methoxycarbonyl or phenylsulfonyl;
R₃ is:
a) phenyl, naphthyl or heterocyclic group selected from the group consisting of pyridinyl, pyrimidinyl, pyrazinyl, pyridazinyl, pyrrolyl, imidazolyl, pyrazolyl, thienyl, furyl, tetrahydrofuryl, isoxazolyl, isothiazolyl, quinolinyl, isoquinolinyl, indolyl, benzimidazolyl, benzofuranyl, benzoxazolyl, benzisoxazolyl, benzpyrazolyl, benzothiofuranyl, cinnolinyl, pterindinyl, phthalazinyl, naphthypyridinyl, quinoxalinyl, quinazolinyl, purinyl and indazolyl, wherein such phenyl, naphthyl or heterocyclic group is optionally substituted with one to five groups selected from the group consisting of a phenyl, naphthyl, heterocycle selected from the group hereinabove described in this paragraph, C₁₋₆ branched or unbranched alkyl which is optionally partially or fully halogenated, cyclopropyl, cyclobutyl, cyclopentyl, cyclohexyl, cycloheptyl, bicyclopentyl, bicyclohexyl, bicycloheptyl, phenyl C₁₋₅ alkyl, naphthyl C₁₋₅ alkyl, halogen, hydroxy, oxo, nitrile, C₁₋₃ alkoxy optionally partially or fully halogenated, C₁₋₃ alkoxyC₁₋₅alkyl, C₁₋₃thioalkyl, C₁₋₃thioalkylC₁₋₅alkyl, phenyloxy, naphthyloxy, heteraryloxy wherein the heterocyclic moiety is selected from the group hereinabove described in this paragraph, nitro, amino, mono- or di-(C₁₋₃)alkylamino, phenylamino, naphthylamino, heterocyclylamino wherein the heterocyclyl moiety is selected from the group hereinabove described in this paragraph, NH₂C(O), a mono- or di-(C₁₋₃)alkyl aminocarbonyl, C₁₋₅ alkyl-C(O)-C₁₋₄ alkyl, amino-C₁₋₅ alkyl, mono- or di-(C₁₋₃)alkylamino-C₁₋₅ alkyl, amino-S(O)₂, di-(C₁₋₃)alkylamino-S(O)₂, R₄-C₁₋₅ alkyl, R₅-C₁₋₅alkoxy, R₆―C(O)-C₁₋₅ alkyl and R₇-C₁₋₅ alkyl(R₈)N, carboxy-mono- or di-(C₁₋₅)-alkyl-amino;
b) a fused aryl selected from the group consisting of benzocyclobutanyl, indanyl, indenyl, dihydronaphthyl, tetrahydronaphthyl, benzocycloheptanyl and benzocycloheptenyl, or a fused heterocyclyl selected from the group consisting of cyclopentenopyridine, cyclohexanopyridine, cyclopentanopyrimidine, cyclohexanopyrimidine, cyclopentanopyrazine, cyclohexanopyrazine, cyclopentanopyridazine, cyclohexanopyridazine, cyclopentanoquinoline, cyclohexanoquinoline, cyclopentanoisoquinoline, cyclohexanoisoquinoline, cyclopentanoindole, cyclohexanoindole, cyclopentanobenzimidazole, cyclohexanobenzimidazole, cyclopentanobenzoxazole, cyclohexanobenzoxazole, cyclopentanoimidazole, cyclohexanoimidazole, cyclopentanothiophene and cyclohexanothiophene; wherein the fused aryl or fused heterocyclyl ring is substituted with 0 to 3 groups independently selected from the group consisting of phenyl, naphthyl and heterocyclyl selected from the group consisting of pyridinyl, pyrimidinyl, pyrazinyl, pyridazinyl, pyrrolyl, imidazolyl, pyrazolyl, thienyl, furyl, isoxazolyl, and isothiazolyl, C₁₋₆ branched or unbranched alkyl which is optionally partially or fully halogenated, halogen, nitrile, C₁₋₃ alkoxy which is optionally partially or fully halogenated, phenyloxy, naphthyloxy, heterocyclyloxy wherein the heterocyclyl moiety is selected from the group hereinabove described, nitro, amino, mono- or di-(C₁₋₃)alkylamino, phenylamino, naphthylamino, heterocyclylamino wherein the heterocyclyl moiety is selected from the group hereinabove described, NH₂C(O), a mono- or di-(C₁₋₃)alkyl aminocarbonyl, C₁₋₄ alkyl-OC(O), C₁₋₅ alkyl-C(O)-C₁₋₄ branched or unbranched alkyl, an amino-C₁₋₅ alkyl, mono- or di-(C₁₋₃)alkylamino-C₁₋₅ alkyl, R₉-C₁₋₅ alkyl, R₁₀-C₁₋₅ alkoxy, R₁₁―C(O)-C₁₋₅ alkyl and R₁₂-C₁₋₅ alkyl(R₁₃)N;
c) cycloalkyl selected from the group consisting of cyclopropyl, cyclobutyl, cyclopentyl, cyclohexyl, cycloheptyl, bicyclopentyl, bicyclohexyl and bicycloheptyl, wherein the cycloalkyl is optionally partially or fully halogenated and optionally substituted with one to three C₁₋₃alkyl groups;
d) C₅₋₇ cycloalkenyl selected from the group consisting of cyclopentenyl, cyclohexenyl, cyclohexadienyl, cycloheptenyl, cycloheptadienyl, bicyclohexenyl and bicycloheptenyl, wherein such cycloalkenyl group is optionally substituted with one to three C₁₋₃ alkyl groups;
e) acetyl, aroyl, C₁₋₆alkoxycarbonylC₁₋₆alkyl or phenylsulfonyl; or
f) C₁₋₆ branched or unbranched alkyl optionally partially or fully halogenated;
or R₁ and R₂ taken together optionally form a fused phenyl or pyridinyl ring;
each R₈ and R₁₃ is independently selected from the group consisting of:
hydrogen and C₁₋₄ branched or unbranched alkyl optionally partially or fully halogenated;
each R₄, R₅, R₆, R₇, R₉, R₁₀, R₁₁ and R₁₂ is independently selected from the group consisting of morpholine, piperidine, piperazine, imidazole and tetrazole;
m is 0, 1 or 2;
W is O or S;
wherein X is directly attached to one or two -Y-Z, and
pharmaceutically acceptable derivatives thereof.

13. Pharmaceutical composition according to claim 6, **characterized in that** the p38 kinase inhibitor **B** is a compound of formula **6** 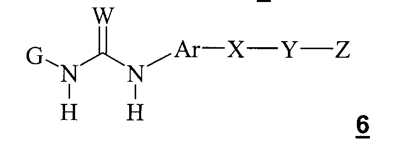 wherein:
G is:
an aromatic C₆₋₁₀ carbocycle or a nonaromatic C₃₋₁₀ carbocycle saturated or unsaturated;
a 6-10 membered heteroaryl containing 1 or more heteroatoms chosen from O, N and S;
a 5-8 membered monocyclic heterocycle containing one or more heteroatoms chosen from O, N and S;
or
an 8-11 membered bicyclic heterocycle, containing one or more heteroatoms chosen from O, N and S;
wherein G is substituted by one or more R₁, R₂ or R₃;
Ar is:
phenyl, naphthyl, quinolinyl, isoquinolinyl, tetrahydronaphthyl, tetrahydroquinolinyl, tetrahydroisoquinolinyl, benzimidazolyl, benzofuranyl, dihydrobenzofuranyl, indolinyl, benzothienyl, dihydrobenzothienyl, indanyl, indenyl or indolyl each being optionally substituted by one or more R₄ or R₅;
X is:
a C₅₋₈ cycloalkyl or cycloalkenyl optionally substituted with one to two oxo groups or one to three C₁₋₄ alkyl, C₁₋₄ alkoxy or C₁₋₄ alkylamino chains;
phenyl, furanyl, thienyl, pyrrolyl, pyrazolyl, imidazolyl, pyridinyl, pyrimidinyl, pyridinonyl, dihydropyridinonyl, maleimidyl, dihydromaleimidyl, piperdinyl, benzimidazole, 3H-imidazo[4,5-b]pyridine, piperazinyl, pyridazinyl or pyrazinyl;
Y is:
a bond or a C₁₋₄ saturated or unsaturated branched or unbranched carbon chain optionally partially or fully halogenated, wherein one or more methylene groups are optionally replaced by O, N, or S(O)ₘ and wherein Y is optionally independently substituted with one to two oxo groups, phenyl or one or more C₁₋₄ alkyl optionally substituted by one or more halogen atoms;
Z is:
phenyl, pyridinyl, pyrimidinyl, pyridazinyl, pyrazinyl, imidazolyl, pyrazolyl, triazolyl, tetrazolyl, furanyl, thienyl, pyranyl each being optionally substituted with one to three halogen, C₁₋₆ alkyl, C₁₋₆ alkoxy, hydroxy, amino, mono- or di-(C₁₋₃ alkyl)amino, C₁₋₆ alkyl-S(O)ₘ, CN, CONH₂, COOH or phenylamino wherein the phenyl ring is optionally substituted with one to two halogen, C₁₋₆ alkyl or C₁₋₆ alkoxy;
tetrahydropyranyl, tetrahydrofuranyl, 1,3-dioxolanonyl, 1,3-dioxanonyl, 1,4-dioxanyl, morpholinyl, thiomorpholinyl, thiomorpholino sulfoxidyl, thiomorpholino sulfonyl, piperidinyl, piperidinonyl, piperazinyl, tetrahydropyrimidonyl, cyclohexanonyl, cyclohexanolyl, pentamethylene sulfidyl, pentamethylene sulfoxidyl, pentamethylene sulfonyl, tetramethylene sulfide, tetramethylene sulfoxidyl or tetramethylene sulfonyl each being optionally substituted with one to three nitrile, C₁₋₆ alkyl, C₁₋₆ alkoxy, hydroxy, amino, mono- or di-(C₁₋₃ alkyl)amino-C₁₋₃ alkyl, CONH₂, phenylamino-C₁₋₃ alkyl or C₁₋₃ alkoxy-C₁₋₃ alkyl;
halogen, C₁₋₄ alkyl, nitrile, amino, hydroxy, C₁₋₆ alkoxy, NH₂C(O), mono- or di(C₁₋₃alkyl) aminocarbonyl, mono- or di(C₁₋₆alkyl)amino, secondary or tertiary amine wherein the amino nitrogen is covalently bonded to C₁₋₃ alkyl or C₁₋₅ alkoxyalkyl, pyridinyl-C₁₋₃ alkyl, imidazolyl-C₁₋₃ alkyl, tetrahydrofuranyl-C₁₋₃ alkyl, nitrile-C₁₋₃ alkyl, carboxamide-C₁₋₃ alkyl, phenyl, wherein the phenyl ring is optionally substituted with one to two halogen, C₁₋₆ alkoxy, hydroxy or mono- or di-(C₁₋₃ alkyl)amino, C₁₋₆ alkyl-S(O)ₘ, or phenyl-S(O)ₘ, wherein the phenyl ring is optionally substituted with one to two halogen, C₁₋₆ alkoxy, hydroxy, halogen or mono- or di-(C₁₋₃ alkyl)amino;
C₁₋₆ alkyl-S(O)ₘ, and phenyl-S(O)ₘ, wherein the phenyl ring is optionally substituted with one to two halogen, C₁₋₆ alkoxy, hydroxy or mono- or di-(C₁₋₃ alkyl)amino;
each R₁ is independently:
C₁₋₁₀ alkyl optionally be partially or fully halogenated, and optionally substituted with one to three C₃₋₁₀ cycloalkanyl, hydroxy, phenyl, naphthyl, pyridinyl, pyrimidinyl, pyrazinyl, pyridazinyl, pyrrolyl, imidazolyl, pyrazolyl, thienyl, furyl, isoxazolyl or isothiazolyl; each of the aforementioned being optionally substituted with one to five groups selected from halogen, C₁₋₆ alkyl which is optionally partially or fully halogenated, C₃₋₈ cycloalkanyl, C₅₋₈ cycloalkenyl, hydroxy, nitrile, C₁₋₃ alkoxy which is optionally partially or fully halogenated or NH₂C(O), mono- or di(C₁₋₃alkyl)amino, and mono- or di(C₁₋₃alkyl)aminocarbonyl;
cyclopropyloxy, cyclobutyloxy, cyclopentyloxy, cyclohexyloxy, or cycloheptyloxy each being optionally partially or fully halogenated and optionally substituted with one to three C₁₋₃ alkyl groups optionally partially or fully halogenated, CN, hydroxyC₁₋₃alkyl or aryl; or an analog of such cycloalkyl group wherein one to three ring methylene groups are independently replaced by O, S(O)ₘ, CHOH, >C=O, >C=S or NH;
phenyloxy or benzyloxy each being optionally partially or fully halogenated and optionally substituted with one to three C₁₋₃ alkyl groups optionally partially or fully halogenated, CN, hydroxyC₁₋₃alkyl or aryl; or an analog of such cycloaryl group wherein one to two ring methyne groups are independently replaced by N;
cyclopropanyl, cyclobutanyl, cyclopentanyl, cyclohexanyl, cycloheptanyl, bicyclopentanyl, bicyclohexanyl or bicycloheptanyl, each being optionally partially or fully halogenated and optionally substituted with one to three C₁₋₃ alkyl groups optionally partially or fully halogenated, CN, hydroxyC₁₋₃alkyl or aryl; or an analog of such cycloalkyl group wherein one to three ring methylene groups are independently replaced by O, S(O)ₘ, CHOH, >C=O, >C=S or NH;
C₃₋₁₀ branched or unbranced alkenyl each being optionally partially or fully halogenated, and optionally be substituted with one to three C₁₋₅ branched or unbranched alkyl, phenyl, naphthyl, pyridinyl, pyrimidinyl, pyrazinyl, pyridazinyl, pyrrolyl, imidazolyl, pyrazolyl, thienyl, furyl, isoxazolyl or isothiazolyl, each of the aforementioned being substituted with zero to five halogen, C₁₋₆ alkyl which is optionally partially or fully halogenated, cyclopropanyl, cyclobutanyl, cyclopentanyl, cyclohexanyl, cycloheptanyl, bicyclopentanyl, bicyclohexanyl and bicycloheptanyl, hydroxy, nitrile, C₁₋₃ alkyloxy which is optionally partially or fully halogenated, NH₂C(O), mono- or di(C₁₋₃alkyl)aminocarbonyl; the C₃₋₁₀ branched or unbranced alkenyl being optionally interrupted by one or more heteroatoms chosen from O, N and S(O)m;
cyclopentenyl, cyclohexenyl, cyclohexadienyl, cycloheptenyl, cycloheptadienyl, bicyclohexenyl or bicycloheptenyl, wherein such cycloalkenyl group is optionally substituted with one to three C₁₋₃ alkyl groups;
nitrile, halogen;
methoxycarbonyl, ethoxycarbonyl and propoxycarbonyl;
silyl containing three C₁₋₄ alkyl groups optionally partially or fully halogenated;
C₃₋₆ alkynyl branched or unbranched carbon chain optionally partially or fully halogenated, wherein one or more methylene groups are optionally replaced by O, NH or S(O)ₘ and wherein said alkynyl group is optionally independently substituted with one to two oxo groups, pyrrolidinyl, pyrrolyl, one or more C₁₋₄ alkyl optionally substituted by one or more halogen atoms, nitrile, morpholino, piperidinyl, piperazinyl, imidazolyl, phenyl, pyridinyl, tetrazolyl, or mono- or di(C₁₋₃alkyl)amino optionally substituted by one or more halogen atoms;
each R₂, R₄, and R₅ is
a C₁₋₆ branched or unbranched alkyl optionally partially or fully halogenated, acetyl, aroyl, C₁₋₄ branched or unbranched alkoxy, each being optionally partially or fully halogenated, halogen, nitrile, methoxycarbonyl, C₁₋₃ alkylS(O)ₘ optionally partially or fully halogenated, or phenylsulfonyl;
C₁₋₆ alkoxy, hydroxy, amino, or mono- or di-(C₁₋₄ alkyl)amino, nitrile, halogen;
OR₆;
nitro; or
mono- or di-(C₁₋₄ alkyl)amino-S(O)₂ optionally partially or fully halogenated, or H₂NSO₂;
each R₃ is independently:
phenyl, naphthyl, morpholinyl, pyridinyl, pyrimidinyl, pyrazinyl, pyridazinyl, pyrrolyl, pyrrolidinyl, imidazolyl, pyrazolyl, thiazolyl, oxazoyl, triazolyl, tetrazolyl, thienyl, furyl, tetrahydrofuryl, isoxazolyl, isothiazolyl, quinolinyl, isoquinolinyl, indolyl, benzimidazolyl, benzofuranyl, benzoxazolyl, benzisoxazolyl, benzpyrazolyl, benzothiofuranyl, cinnolinyl, pterindinyl, phthalazinyl, naphthypyridinyl, quinoxalinyl, quinazolinyl, purinyl or indazolyl, each of the aforementioned is optionally substituted with one to three phenyl, naphthyl, heterocycle or heteroaryl as hereinabove described in this paragraph, C₁₋₆ branched or unbranched alkyl which is optionally partially or fully halogenated, cyclopropanyl, cyclobutanyl, cyclopentanyl, cyclohexanyl, cycloheptanyl, bicyclopentanyl, bicyclohexanyl, bicycloheptanyl, phenyl C₁₋₅ alkyl, naphthyl C₁₋₅ alkyl, halogen, hydroxy, oxo, nitrile, C₁₋₃ alkyloxy optionally partially or fully halogenated, phenyloxy, naphthyloxy, heteroaryloxy or heterocyclicoxy wherein the heterocyclic or heteroaryl moiety is as hereinabove described in this paragraph, nitro, amino, mono- or di-(C₁₋₃alkyl)amino, phenylamino, naphthylamino, heteroaryl or heterocyclic amino wherein the heteroaryl heterocyclic moiety is as hereinabove described in this paragraph, NH₂C(O), a mono- or di-(C₁₋₃alkyl) aminocarbonyl, C₁₋₅ alkyl-C(O)-C₁₋₄ alkyl, amino-C₁₋₅ alkyl, mono- or di-(C₁₋₃alkyl)amino-C₁₋₅ alkyl, amino-S(O)₂, di-(C₁₋₃alkyl)amino-S(O)₂, R₇-C₁₋₅ alkyl, R₈-C₁₋₅ alkoxy, R₉-C(O)-C₁₋₅ alkyl, R₁₀-C₁₋₅ alkyl(R₁₁)N, carboxy-mono- or di-(C₁₋₅alkyl)-amino;
a fused aryl selected from benzocyclobutanyl, indanyl, indenyl, dihydronaphthyl, tetrahydronaphthyl, benzocycloheptanyl and benzocycloheptenyl, or a fused heteroaryl selected from cyclopentenopyridinyl, cyclohexanopyridinyl, cyclopentanopyrimidinyl, cyclohexanopyrimidinyl, cyclopentanopyrazinyl, cyclohexanopyrazinyl, cyclopentanopyridazinyl, cyclohexanopyridazinyl, cyclopentanoquinolinyl, cyclohexanoquinolinyl, cyclopentanoisoquinolinyl, cyclohexanoisoquinolinyl, cyclopentanoindolyl, cyclohexanoindolyl, cyclopentanobenzimidazolyl, cyclohexanobenzimidazolyl, cyclopentanobenzoxazolyl, cyclohexanobenzoxazolyl, cyclopentanoimidazolyl, cyclohexanoimidazolyl, cyclopentanothienyl and cyclohexanothienyl; wherein the fused aryl or fused heteroaryl ring is independently substituted with zero to three phenyl, naphthyl, pyridinyl, pyrimidinyl, pyrazinyl, pyridazinyl, pyrrolyl, imidazolyl, pyrazolyl, thienyl, furyl, isoxazolyl, isothiazolyl, C₁₋₆ alkyl which is optionally partially or fully halogenated, halogen, nitrile, C₁₋₃ alkyloxy which is optionally partially or fully halogenated, phenyloxy, naphthyloxy, heteroaryloxy or heterocyclicoxy wherein the heteroaryl or heterocyclic moiety is as hereinabove described in this paragraph, nitro, amino, mono- or di-(C₁₋₃alkyl)amino, phenylamino, naphthylamino, heteroaryl or heterocyclic amino wherein the heteroaryl or heterocyclic moiety is as hereinabove described in this paragraph, NH₂C(O), mono- or di-(C₁₋₃alkyl)aminocarbonyl, C₁₋₄ alkyl-OC(O), C₁₋₅ alkyl-C(O)-C₁₋₄ alkyl, amino-C₁₋₅ alkyl, mono- or di-(C₁₋₃)alkylamino-C₁₋₅ alkyl, R₁₂-C₁₋₅ alkyl, R₁₃-C₁₋₅ alkoxy, R₁₄-C(O)-C₁₋₅ alkyl or R₁₅-C₁₋₅ alkyl(R₁₆)N;
cyclopropanyl, cyclobutanyl, cyclopentanyl, cyclohexanyl, cycloheptanyl, bicyclopentanyl, bicyclohexanyl or bicycloheptanyl, each being optionally partially or fully halogenated and optionally substituted with one to three C₁₋₃ alkyl groups, or an analog of such cycloalkyl group wherein one to three ring methylene groups are independently replaced by O, S, CHOH, >C=O, >C=S or NH;
cyclopentenyl, cyclohexenyl, cyclohexadienyl, cycloheptenyl, cycloheptadienyl, bicyclohexenyl or bicycloheptenyl, each optionally substituted with one to three C₁₋₃ alkyl groups;
C₁₋₄ alkyl-phenyl-C(O)-C₁₋₄ alkyl-, C₁₋₄ alkyl-C(O)-C₁₋₄ alkyl- or C₁₋₄ alkylphenyl-S(O)ₘ-C₁₋₄ alkyl-;
C₁₋₆ alkyl or C₁₋₆ branched or unbranched alkoxy each of which is optionally partially or fully halogenated or optionally substituted with R₁₇;
OR₁₈ or C₁₋₆ alkyl optionally substituted with OR₁₈;
amino or mono- or di-(C₁₋₅alkyl)amino optionally substituted with R₁₉;
R₂₀C(O)N(R₂₁)-, R₂₂O- or R₂₃R₂₄NC(O)-; R₂₆(CH₂)ₘC(O)N(R₂₁)- or R₂₆C(O)(CH₂)ₘN(R₂₁)-;
C₂₋₆alkenyl substituted by R₂₃R₂₄NC(O)-;
C₂₋₆ alkynyl branched or unbranched carbon chain, optionally partially or fully halogenated, wherein one or more methylene groups are optionally replaced by O, NH, S(O)ₘ and wherein said alkynyl group is optionally independently substituted with one to two oxo groups, pyrroldinyl, pyrrolyl, morpholinyl, piperidinyl, piperazinyl, imidazolyl, phenyl, pyridinyl, tetrazolyl one or more C₁₋₄ alkyl optionally substituted by one or more halogen atoms, nitrile, morpholino, piperidinyl, piperazinyl, imidazolyl, phenyl, pyridinyl, tetrazolyl, or mono- or di(C₁₋₄ alkyl)amino optionally substituted by one or more halogen atoms; or
aroyl;
R₆ is a: C₁₋₄ alkyl optionally partially or fully halogenated and optionally substituted with R₂₆;
each R₇, R₈, R₉, R₁₀, R₁₂, R₁₃, R₁₄, R₁₅, R₁₇, R₁₉, R₂₅ and R₂₆ is independently: nitrile, phenyl, morpholino, piperidinyl, piperazinyl, imidazolyl, pyridinyl, tetrazolyl, amino or mono- or di-(C₁₋₄alkyl)amino optionally partially or fully halogenated;
each R₁₁ and R₁₆ is independently:
hydrogen or C₁₋₄ alkyl optionally partially or fully halogenated;
R₁₈ is independently:
hydrogen or a C₁₋₄ alkyl optionally independently substituted with oxo or R₂₅;
R₂₀ is independently:
C₁₋₁₀ alkyl optionally partially or fully halogenated, phenyl, or pyridinyl;
R₂₁ is independently:
hydrogen or C₁₋₃ alkyl optionally partially or fully halogenated;
each R₂₂, R₂₃ and R₂₄ is independently:
hydrogen, C₁₋₆ alkyl optionally partially or fully halogenated, said C₁₋₆ alkyl is optionally interrupted by one or more O, N or S, said C₁₋₆ alkyl also being independently optionally substituted by mono- or di-(C₁₋₃alkyl)aminocarbonyl, phenyl, pyridinyl, amino or mono- or di-(C₁₋₄alkyl)amino each of which is optionally partially or fully halogenated and optionally substituted with mono-or di-(C₁₋₃alkyl)amino;
or R₂₃ and R₂₄ taken together optionally form a heterocyclic or heteroaryl ring;
m = 0, 1 or 2;
W is O or S and
pharmaceutically acceptable derivatives thereof.

14. Pharmaceutical composition according to claim 6, **characterized in that** the p38 kinase inhibitor **B** is a compound of formula **7** 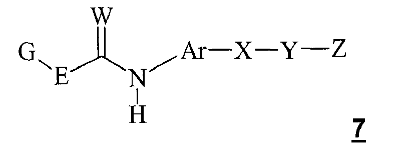 wherein:
E is carbon or a heteroatom group chosen from -O-, -NH- and -S-;
G is : an aromatic C₆₋₁₀ carbocycle or a nonaromatic C₃₋₁₀carbocycle saturated or u nsatu rated;
a 6-14 membered monocyclic, bicyclic or tricyclic heteroaryl containing 1 or more heteroatoms chosen from O, N and S;
a 6-8 membered monocyclic heterocycle containing one or more heteroatoms chosen from O, N and S;
or
an 8-11 membered bicyclic heterocycle, containing one or more heteroatoms chosen from O, N and S;
wherein G is optionally substituted by one or more R₁, R₂ or R₃;
Ar is: phenyl, naphthyl, quinolinyl, isoquinolinyl, tetrahydronaphthyl, tetrahydroquinolinyl, tetrahydroisoquinolinyl, benzimidazolyl, benzofuranyl, dihydrobenzofuranyl, indolinyl, benzothienyl, dihydrobenzothienyl, indanyl, indenyl or indolyl each being optionally substituted by one or more R₄ or R₅;
X is: a C₅₋₈ cycloalkyl or cycloalkenyl optionally substituted with one to two oxo groups or one to three C₁₋₄ alkyl, C₁₋₄ alkoxy or C₁₋₄ alkylamino chains each being branched or unbranched;
aryl, furanyl, thienyl, pyrrolyl, pyrazolyl, imidazolyl, pyridinyl, pyrimidinyl, pyridinonyl, dihydropyridinonyl, maleimidyl, dihydromaleimidyl, piperdinyl, benzimidazole, 3H-imidazo[4,5-b]pyridine, piperazinyl, pyridazinyl or pyrazinyl; each being optionally independently substituted with one to three C₁₋₄ alkyl, C₁₋₄alkoxy, hydroxy, nitrile, amino, mono- or di-(C₁₋₃ alkyl)amino, mono- or di-(C₁₋₃ alkylamino)carbonyl, NH₂C(O), C₁₋₆ alkyl-S(O)ₘ or halogen;
Y is: a bond or a C₁₋₄ saturated or unsaturated branched or unbranched carbon chain optionally partially or fully halogenated, wherein one or more C atoms are optionally replaced by O, N, or S(O)ₘ and wherein Y is optionally independently substituted with one to two oxo groups, nitrile, phenyl or one or more C₁₋₄ alkyl optionally substituted by one or more halogen atoms;
Z is: aryl, heteroaryl selected from pyridinyl, piperazinyl, pyrimidinyl, pyridazinyl, pyrazinyl, imidazolyl, pyrazolyl, triazolyl, tetrazolyl, furanyl, thienyl and pyranyl, heterocycle selected from tetrahydropyrimidonyl, cyclohexanonyl, cyclohexanolyl, 2-oxa- or 2-thia-5-aza-bicyclo[2.2.1]heptanyl, pentamethylene sulfidyl, pentamethylene sulfoxidyl, pentamethylene sulfonyl, tetramethylene sulfidyl, tetramethylene sulfoxidyl or tetramethylene sulfonyl, tetrahydropyranyl, tetrahydrofuranyl, 1,3-dioxolanonyl, 1,3-dioxanonyl, 1,4-dioxanyl, morpholino, thiomorpholino, thiomorpholino sulfoxidyl, thiomorpholino sulfonyl, piperidinyl, piperidinonyl, pyrrolidinyl and dioxolanyl, each of the aforementioned Z are optionally substituted with one to three halogen, C₁₋₆ alkyl, C₁₋₆ alkoxy, C₁₋₃ alkoxy-C₁₋₃ alkyl, C₁₋₆ alkoxycarbonyl, aroyl, C₁₋₃acyl, oxo, hydroxy, pyridinyl-C₁₋₃ alkyl, imidazolyl-C₁₋₃ alkyl, tetrahydrofuranyl-C₁₋₃ alkyl, nitrile-C₁₋₃ alkyl, nitrile, carboxy, phenyl wherein the phenyl ring is optionally substituted with one to two halogen, C₁₋₆ alkoxy, hydroxy or mono- or di-(C₁₋₃ alkyl)amino, C₁₋₆ alkyl-S(O)ₘ, or phenyl-S(O)ₘ wherein the phenyl ring is optionally substituted with one to two halogen, C₁₋₆ alkoxy, hydroxy, halogen or mono- or di-(C₁₋₃ alkyl)amino;
or Z is optionally substituted with one to three amino or amino-C₁₋₃ alkyl wherein the N atom is optionally independently mono- or di-substituted by aminoC₁₋₆alkyl, C₁₋₃alkyl, arylC₀₋₃alkyl, C₁₋₅ alkoxyC₁₋₃ alkyl, C₁₋₅ alkoxy, aroyl, C₁₋₃acyl, C₁₋₃alkyl-S(O)ₘ- or arylC₀₋₃alkyl-S(O)ₘ- each of the aforementioned alkyl and aryl attached to the amino group is optionally substituted with one to two halogen, C₁₋₆ alkyl or C₁₋₆ alkoxy;
or Z is optionally substituted with one to three aryl, heterocycle or heteroaryl as hereinabove described in this paragraph each in turn is optionally substituted by halogen, C₁₋₆ alkyl or C₁₋₆ alkoxy;
or Z is hydroxy, halogen, nitrile, amino wherein the N atom is optionally independently mono- or di-substituted by C₁₋₃acyl, C₁₋₆alkyl or C₁₋₃alkoxyC₁₋₃alkyl, C₁₋₆alkyl branched or unbranched, C₁₋₆alkoxy, C₁₋₃acylamino, nitrileC₁₋₄alkyl, C₁₋₆ alkyl-S(O)ₘ, and phenyl-S(O)ₘ, wherein the phenyl ring is optionally substituted with one to two halogen, C₁₋₆ alkoxy, hydroxy or mono- or di-(C₁₋₃ alkyl)amino;
each R₁ is independently:
C₁₋₁₀ alkyl branched or unbranched optionally partially or fully halogenated, wherein one or more C atoms are optionally independently replaced by O, N or S(O)ₘ, and wherein said C₁₋₁₀ alkyl is optionally substituted with one to three C₃₋₁₀ cycloalkyl, hydroxy, oxo, phenyl, naphthyl, pyridinyl, pyrimidinyl, pyrazinyl, pyridazinyl, pyrrolyl, pyrrolidinyl, imidazolyl, pyrazolyl, thienyl, furyl, dioxolanyl, isoxazolyl or isothiazolyl; each of the aforementioned being optionally substituted with one to five groups selected from halogen, C₁₋₆ alkyl which is optionally partially or fully halogenated, C₃₋₈ cycloalkanyl, C₅₋₈ cycloalkenyl, hydroxy, nitrile, C₁₋₃ alkoxy which is optionally partially or fully halogenated or NH₂C(O), mono- or di(C₁₋₃alkyl)amino, and mono- or di(C₁₋₃alkyl)aminocarbonyl;
or R₁ is
cyclopropyloxy, cyclobutyloxy, cyclopentyloxy, cyclohexyloxy, or cycloheptyloxy each being optionally partially or fully halogenated and optionally substituted with one to three C₁₋₃alkyl groups optionally partially or fully halogenated, nitrile, hydroxyC₁₋₃alkyl or aryl; or an analog of such cycloalkyl group wherein one to three ring methylene groups are independently replaced by O, S(O)ₘ, CHOH, >C=O, >C=S or NH;
phenyloxy or benzyloxy each being optionally partially or fully halogenated and optionally substituted with one to three C₁₋₃ alkyl groups optionally partially or fully halogenated, nitrile, hydroxyC₁₋₃alkyl or aryl; or an analog of such cycloaryl group wherein one to two ring methyne groups are independently replaced by N;
cyclopropyl, cyclobutyl, cyclopentyl, cyclohexyl, cycloheptyl, bicyclopentanyl, bicyclohexanyl or bicycloheptanyl, each being optionally partially or fully halogenated and optionally substituted with one to three C₁₋₃ alkyl optionally partially or fully halogenated, nitrile, hydroxyC₁₋₃alkyl or aryl; or an analog of such cycloalkyl group wherein one to three ring methylene groups are independently replaced by O, S(O)ₘ, CHOH, >C=O, >C=S or NH;
C₃₋₁₀ branched or unbranced alkenyl each being optionally partially or fully halogenated, and optionally substituted with one to three C₁₋₅ branched or unbranched alkyl, phenyl, naphthyl, pyridinyl, pyrimidinyl, pyrazinyl, pyridazinyl, pyrrolyl, imidazolyl, pyrazolyl, thienyl, furyl, isoxazolyl or isothiazolyl, each of the aforementioned being substituted with one to five halogen, C₁₋₆ alkyl which is optionally partially or fully halogenated, cyclopropanyl, cyclobutanyl, cyclopentanyl, cyclohexanyl, cycloheptanyl, bicyclopentanyl, bicyclohexanyl and bicycloheptanyl, hydroxy, nitrile, C₁₋₃ alkyloxy which is optionally partially or fully halogenated, NH₂C(O), mono- or di(C₁₋₃alkyl)aminocarbonyl; the C₃₋₁₀ branched or unbranced alkenyl being optionally interrupted by one or more heteroatoms chosen from O, N and S(O)ₘ;
cyclopentenyl, cyclohexenyl, cyclohexadienyl, cycloheptenyl, cycloheptadienyl, bicyclohexenyl or bicycloheptenyl, wherein such cycloalkenyl group is optionally substituted with one to three C₁₋₃ alkyl groups;
oxo, nitrile, halogen;
silyl containing three C₁₋₄ alkyl groups optionally partially or fully halogenated; or
C₃₋₆ alkynyl branched or unbranched carbon chain optionally partially or fully halogenated, wherein one or more methylene groups are optionally replaced by O, NH or S(O)ₘ and wherein said alkynyl group is optionally independently substituted with one to two oxo groups, hydroxy, pyrroldinyl, pyrrolyl, tetrahydropyranyl, one or more C₁₋₄ alkyl optionally substituted by one or more halogen atoms, nitrile, morpholino, piperidinyl, piperazinyl, imidazolyl, phenyl, pyridinyl, tetrazolyl, or mono- or di(C₁₋₃alkyl)amino optionally substituted by one or more halogen atoms;
each R₂, R₄, and R₅ is
a C₁₋₆ branched or unbranched alkyl optionally partially or fully halogenated, C₁₋₆acyl, aroyl, C₁₋₄ branched or unbranched alkoxy, each being optionally partially or fully halogenated, halogen, methoxycarbonyl, C₁₋₃ alkyl-S(O)ₘ optionally partially or fully halogenated, or phenyl-S(O)ₘ;
OR₆, C₁₋₆ alkoxy, hydroxy, nitrile, nitro, halogen;
or amino-S(O)ₘ- wherein the N atom is optionally independently mono- or di-substituted by C₁₋₆alkyl or arylC₀₋₃alkyl, or amino wherein the N atom is optionally independently mono- or di-substituted by C₁₋₃alkyl, arylC₀₋₃alkyl, C₁₋₆acyl, C₁₋₆alkyl-S(O)ₘ- or arylC₀₋₃alkyl-S(O)ₘ-, each of the aforementioned alkyl and aryl in this subparagraph are optionally partially or fully halogenated and optionally substituted with one to two C₁₋₆ alkyl or C₁₋₆ alkoxy;
each R₃ is independently:
phenyl, naphthyl, morpholino, pyridinyl, pyrimidinyl, pyrazinyl, pyridazinyl, pyrrolyl, pyrrolidinyl, imidazolyl, pyrazolyl, thiazolyl, oxazoyl, [1,3,4]oxadiazol, triazolyl, tetrazolyl, thienyl, furyl, tetrahydrofuryl, isoxazolyl, isothiazolyl, quinolinyl, isoquinolinyl, indolyl, benzimidazolyl, benzofuranyl, benzoxazolyl, benzisoxazolyl, benzpyrazolyl, benzothiofuranyl, cinnolinyl, pterindinyl, phthalazinyl, naphthypyridinyl, quinoxalinyl, quinazolinyl, purinyl or indazolyl, each of the aforementioned is optionally substituted with one to three phenyl, naphthyl, heterocycle or heteroaryl as hereinabove described in this paragraph, C₁₋₆ branched or unbranched alkyl which is optionally partially or fully halogenated, cyclopropanyl, cyclobutanyl, cyclopentanyl, cyclohexanyl, cycloheptanyl, bicyclopentanyl, bicyclohexanyl, bicycloheptanyl, phenyl C₁₋₅ alkyl, naphthyl C₁₋₅ alkyl, halogen, hydroxy, oxo, nitrile, C₁₋₃ alkoxy optionally partially or fully halogenated, phenyloxy, naphthyloxy, heteroaryloxy or heterocyclicoxy wherein the heterocyclic or heteroaryl moiety is as hereinabove described in this paragraph, nitro, amino, mono- or di-(C₁₋₃alky)lamino, phenylamino, naphthylamino, heteroaryl or heterocyclic amino wherein the heteroaryl heterocyclic moiety is as hereinabove described in this paragraph, NH₂C(O), a mono- or di-(C₁₋₃alkyl) aminocarbonyl, C₁₋₅ alkyl-C(O)-C₁₋₄ alkyl, amino-C₁₋₅ alkyl, mono- or di-(C₁₋₅alkyl)amino, mono- or di-(C₁₋₃alkyl)amino-C₁₋₅ alkyl, amino-S(O)₂, di-(C₁₋₃alkyl)amino-S(O)₂, R₇C₁₋₅ alkyl, R₈-C₁₋₅ alkoxy, R₉-C(O)-C₁₋₅ alkyl, R₁₀-C₁₋₅ alkyl(R₁₁)N, carboxy-mono- or di-(C₁₋₅alkyl)-amino;
a fused aryl selected from benzocyclobutanyl, indanyl, indenyl, dihydronaphthyl, tetrahydronaphthyl, benzocycloheptanyl and benzocycloheptenyl, or a fused heteroaryl selected from cyclopentenopyridinyl, cyclohexanopyridinyl, cyclopentanopyrimidinyl, cyclohexanopyrimidinyl, cyclopentanopyrazinyl, cyclohexanopyrazinyl, cyclopentanopyridazinyl, cyclohexanopyridazinyl, cyclopentanoquinolinyl, cyclohexanoquinolinyl, cyclopentanoisoquinolinyl, cyclohexanoisoquinolinyl, cyclopentanoindolyl, cyclohexanoindolyl, cyclopentanobenzimidazolyl, cyclohexanobenzimidazolyl, cyclopentanobenzoxazolyl, cyclohexanobenzoxazolyl, cyclopentanoimidazolyl, cyclohexanoimidazolyl, cyclopentanothienyl and cyclohexanothienyl; wherein the fused aryl or fused heteroaryl ring is independently substituted with zero to three phenyl, naphthyl, pyridinyl, pyrimidinyl, pyrazinyl, pyridazinyl, pyrrolyl, imidazolyl, pyrazolyl, thienyl, furyl, isoxazolyl, isothiazolyl, C₁₋₆ alkyl which is optionally partially or fully halogenated, halogen, nitrile, C₁₋₃ alkyloxy which is optionally partially or fully halogenated, phenyloxy, naphthyloxy, heteroaryloxy or heterocyclicoxy wherein the heteroaryl or heterocyclic moiety is as hereinabove described in this paragraph, nitro, amino, mono- or di-(C₁₋₃alkyl)amino, phenylamino, naphthylamino, heteroaryl or heterocyclic amino wherein the heteroaryl or heterocyclic moiety is as hereinabove described in this paragraph, NH₂C(O), mono- or di-(C₁₋₃alkyl)aminocarbonyl, C₁₋₄ alkyl-OC(O), C₁₋₅ alkyl-C(O)-C₁₋₄ alkyl, amino-C₁₋₅ alkyl, mono- or di-(C₁₋₃)alkylamino-C₁₋₅ alkyl, R₁₂-C₁₋₅ alkyl, R₁₃-C₁₋₅ alkoxy, R₁₄-C(O)-C₁₋₅ alkyl or R₁₅-C₁₋₅ alkyl(R₁₆)N;
cyclopropanyl, cyclobutanyl, cyclopentanyl, cyclohexanyl, cycloheptanyl, bicyclopentanyl, bicyclohexanyl or bicycloheptanyl, each being optionally be partially or fully halogenated and optionally substituted with one to three C₁₋₃ alkyl groups, or an analog of such cycloalkyl group wherein one to three ring methylene groups are independently replaced by O, S, CHOH, >C=O, >C=S or NH;
cyclopentenyl, cyclohexenyl, cyclohexadienyl, cycloheptenyl, cycloheptadienyl, bicyclohexenyl or bicycloheptenyl, each optionally substituted with one to three C₁₋₃ alkyl groups;
C₁₋₄ alkyl-phenyl-C(O)-C₁₋₄ alkyl-, C₁₋₄ alkyl-C(O)-C₁₋₄ alkyl- or C₁₋₄ alkylphenyl-S(O)ₘ-C₁₋₄ alkyl-;
C₁₋₆ alkyl or C₁₋₆ branched or unbranched alkoxy each of which is optionally partially or fully halogenated or optionally substituted with R₁₇;
OR₁₈ or C₁₋₆ alkyl optionally substituted with OR₁₈;
amino or mono- or di-(C₁₋₅alkyl)amino optionally substituted with R₁₉;
R₂₀C(O)N(R₂₁)-, R₂₂O- or R₂₃R₂₄NC(O)-; R₂₆(CH₂)ₘC(O)N(R₂₁)-, R₂₃R₂₄NC(O)-C₁₋₃alkoxy or R₂₆C(O)(CH₂)ₘN(R₂₁)-;
C₂₋₆alkenyl substituted by R₂₃R₂₄NC(O)-;
C₂₋₆ alkynyl branched or unbranched carbon chain, optionally partially or fully halogenated, wherein one or more methylene groups are optionally replaced by O, NH, S(O)ₘ and wherein said alkynyl group is optionally independently substituted with one to two oxo groups, pyrroldinyl, pyrrolyl, morpholino, piperidinyl, piperazinyl, imidazolyl, phenyl, pyridinyl, tetrazolyl one or more C₁₋₄ alkyl optionally substituted by one or more halogen atoms, nitrile, morpholino, piperidinyl, piperazinyl, imidazolyl, phenyl, pyridinyl, tetrazolyl, or mono- or di(C₁₋₄ alkyl)amino optionally substituted by one or more halogen atoms;
C₁₋₆acyl or aroyl;
R₆ is a:
C₁₋₄ alkyl optionally partially or fully halogenated and optionally substituted with R₂₆;
each R₇, R₈, R₉, R₁₀, R₁₂, R₁₃, R₁₄, R₁₅, R₁₇, R₁₉, R₂₅ and R₂₆ is independently:
nitrile, phenyl, morpholino, piperidinyl, piperazinyl, imidazolyl, pyridinyl, tetrazolyl, amino or mono- or di-(C₁₋₄alkyl)amino optionally partially or fully halogenated;
each R₁₁ and R₁₆ is independently:
hydrogen or C₁₋₄ alkyl optionally partially or fully halogenated;
R₁₈ is independently:
hydrogen or a C₁₋₄ alkyl optionally independently substituted with oxo or R₂₅;
R₂₀ is independently:
C₁₋₁₀ alkyl optionally partially or fully halogenated, phenyl, or pyridinyl;
R₂₁ is independently:
hydrogen or C₁₋₃ alkyl optionally partially or fully halogenated;
each R₂₂, R₂₃ and R₂₄ is independently:
hydrogen, C₁₋₆ alkyl optionally partially or fully halogenated, said C₁₋₆ alkyl is optionally interrupted by one or more O, N or S, said C₁₋₆ alkyl also being independently optionally substituted by mono- or di-(C₁₋₃alkyl)aminocarbonyl, phenyl, pyridinyl, amino or mono- or di-(C₁₋₄alkyl)amino each of which is optionally partially or fully halogenated and optionally substituted with mono-or di-(C₁₋₃alkyl)amino;
or R₂₃ and R₂₄ taken together optionally form a heterocyclic or heteroaryl ring;
m = 0, 1 or 2;
W is O or S and
pharmaceutically acceptable derivatives thereof.

15. Pharmaceutical composition according to one of claims 1 to 14, **characterised in that** the weight ratios of **A** to **B** are in the range from 1:300 to 20:1, preferably from 1:200 to 10:1.

16. Pharmaceutical composition according to one of claims 1 to 15, **characterised in that** a single application corresponds to a dosage of the active substance combination **A** and **B** of about 100 to 10000 µg, preferably 1000 to 9000 µg.

17. Pharmaceutical composition according to one of claims 1 to 16, **characterised in that** it is present in the form of a formulation suitable for inhalation.

18. Pharmaceutical composition according to claim 17, **characterised in that** it is a formulation selected from among inhalable powders, propellant-containing metering aerosols and propellant-free inhalable solutions or suspensions.

19. Pharmaceutical composition according to claim 18, **characterised in that** it is an inhalable powder which contains **A** and **B** in admixture with suitable physiologically acceptable excipients selected from among the monosaccharides, disaccharides, oligo- and polysaccharides, polyalcohols, salts, or mixtures of these excipients with one another.

20. Inhalable powder according to claim 19, **characterised in that** the excipient has a maximum average particle size of up to 250µm, preferably between 10 and 150µm.

21. Pharmaceutical composition according to claim 18, **characterised in that** it is an inhalable powder which contains only the active substances **A** and **B** as its ingredients.

22. Capsules, **characterised in that** they contain an inhalable powder according to claim 19, 20 or 21.

23. Pharmaceutical composition according to claim 18, **characterised in that** it is a propellant-containing inhalable aerosol which contains **A** and **B** in dissolved or dispersed form.

24. Pharmaceutical composition according to claim 18, **characterised in that** it is a propellant-free inhalable solution or suspension which contains water, ethanol or a mixture of water and ethanol as solvent.

25. Use of a capsule according to claim 22 in an inhaler, preferably in a Handyhaler.

26. Use of an inhalable solution according to claim 24 for nebulising in an inhaler according to WO 91/14468 or an inhaler as described in Figures 6a and 6b of WO 97/12687.

27. Use of an inhalable solution according to claim 26 for nebulising in an energy-operated free-standing or portable nebuliser which produces inhalable aerosols by means of ultrasound or compressed air in accordance with the Venturi principle or other principles.

28. Use of a composition according to one of claims 1 to 24 for preparing a medicament for treating inflammatory or obstructive diseases of the respiratory tract.
